(19) Europäisches Patentamt
European Patent Office
Office européen des brevets

(11) EP 3 737 233 B1

(12) **EUROPEAN PATENT SPECIFICATION**

(45) Date of publication and mention of the grant of the patent:
**19.03.2025 Bulletin 2025/12**

(21) Application number: **19738400.1**

(22) Date of filing: **09.01.2019**

(51) International Patent Classification (IPC):
***A61K 9/00*** *(2006.01)* ***A61K 9/06*** *(2006.01)*
***A61K 47/10*** *(2017.01)* ***A61K 45/06*** *(2006.01)*
***A61K 38/18*** *(2006.01)*

(52) Cooperative Patent Classification (CPC):
**A61K 45/06; A61K 9/0024; A61K 9/0046; A61K 9/06; A61K 38/185; A61K 47/10;**
A61K 47/14; A61K 47/32

(86) International application number:
**PCT/US2019/012941**

(87) International publication number:
**WO 2019/140012 (18.07.2019 Gazette 2019/29)**

(54) **GROWTH FACTOR OTIC FORMULATIONS**

WACHSTUMSFAKTORFORMULIERUNGEN FÜR DAS OHR

FORMULATIONS OTIQUES DE FACTEUR DE CROISSANCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.01.2018 US 201862615358 P**

(43) Date of publication of application:
**18.11.2020 Bulletin 2020/47**

(73) Proprietor: **Dompé farmaceutici S.p.A.**
**20122 Milan (IT)**

(72) Inventors:
- **PIU, Fabrice**
**San Diego, California 92121 (US)**
- **JACQUES, Bonnie**
**San Diego, California 92121 (US)**
- **TSIVKOVSKAIA, Natalia**
**San Diego, California 92121 (US)**
- **QI, Hong**
**San Diego, California 92121 (US)**
- **SAVEL, Robert**
**San Diego, California 92121 (US)**
- **COLEMAN, Scott**
**San Diego, California 92121 (US)**
- **ZHANG, Zhanpeng**
**San Diego, California 92121 (US)**
- **PASTUSZKA, Martha**
**San Diego, California 92121 (US)**

(74) Representative: **Dompé farmaceutici Spa**
**Via San Martino, 12-12/a**
**20122 Milano (IT)**

(56) References cited:
**WO-A1-2015/031393**
**WO-A2-2018/140792**
**JP-A- 2005 220 070**
**JP-A- 2005 220 070**
**US-A- 5 929 041**
**US-A1- 2016 243 028**
**US-A1- 2016 243 028**

- **ANONYMOUS ET AL: "Neurotrophin-3 regulates ribbon synapse density in the cochlea and induces synapse regeneration after acoustic trauma", ELIFE,2014,3,E03564, 20 October 2014 (2014-10-20), XP055836648, Retrieved from the Internet <URL:https://elifesciences.org/articles/03564> [retrieved on 20210901]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ZUCCOTTI A. ET AL: "Lack of Brain-Derived Neurotrophic Factor Hampers Inner Hair Cell Synapse Physiology, But Protects against Noise-Induced Hearing Loss", JOURNAL OF NEUROSCIENCE, 20 June 2012 (2012-06-20), pages 8545 - 8553, XP055836664, Retrieved from the Internet <URL:https://www.ncbi.nlm.nih.gov/pmc/articles/PMC6620992/pdf/zns8545.pdf> [retrieved on 20210901], DOI: 10.1523/JNEUROSCI.1247-12.2012
- BAFTI SEPAND: "Regeneration and protection of synapses after noise-induced Regeneration and protection of synapses after noise-induced cochlear synaptopathy cochlear synaptopathy", MS (MASTER OF SCIENCE) THESIS, UNIVERSITY OF IOWA, 2017., 1 January 2017 (2017-01-01), XP055836680, Retrieved from the Internet <URL:https://ir.uiowa.edu/cgi/viewcontent.cgi?article=7393&context=etd> [retrieved on 20210901], DOI: 10.17077/etd.arb68f5a
- ANDREW K WISE ET AL: "Drug delivery to the inner ear", JOURNAL OF NEURAL ENGINEERING, INSTITUTE OF PHYSICS PUBLISHING, BRISTOL, GB, vol. 9, no. 6, 27 November 2012 (2012-11-27), pages 65002, XP020234320, ISSN: 1741-2552, DOI: 10.1088/1741-2560/9/6/065002

## Description

### BACKGROUND OF THE DISCLOSURE

Vertebrates have a pair of ears, placed symmetrically on opposite sides of the head. The ear serves as both the sense organ that detects sound and the organ that maintains balance and body position. The ear is generally divided into three portions: the outer ear, auris media (or middle ear), and the auris interna (or inner ear).

Described herein is an otic formulation comprising a therapeutically effective amount of a growth factor and an auris-acceptable vehicle, and the use of said formulation in the treatment of hearing loss.

US 2016/ 243028 A1 discloses a method of treating or preventing hearing loss, the method comprising intratympanically administering an otic composition to a subject in need thereof, wherein the otic composition comprises an otic hair cell growth factor and an auris acceptable gel.

JP 2005 220070 A discloses a sustained otic formulation for inner-ear administration containing BDNF and a bioabsorbable polymer hydrogel.

WO 2015/031393 A1 discloses a method of treating a pediatric otic disease or condition associated with a microbial infection, the method comprising administering into the middle ear of a pediatric patient in need thereof an aqueous thermoreversible gel composition comprising about 6.0% by weight of micronized ciprofloxacin and 15-17% by weight of poloxamer 407.

Wan et al., "Neurotrophin-3 regulates ribbon synapse density in the cochlea and induces synapse regeneration after acoustic trauma", eLife. 2014 Oct 20;3:e03564, shows the highly localized effect of NT-3 on cochlear synaptogenesis, and that NT-3, but not BDNF, promoted recovery of cochlear function and ribbon synapse regeneration after acoustic trauma.

Zuccotti et al., "Lack of Brain-Derived Neurotrophic Factor Hampers Inner Hair Cell Synapse Physiology, But Protects against Noise-Induced Hearing Loss", The Journal of Neuroscience, 2012, 32(25):8545- 8553, discloses that BDNF expressed in the cochlea is useful for maintenance of adult inner hair cell (IHC) transmitter release sites, and that BDNF upholds opposing afferents in high-frequency turns and scales them down following noise exposure.

Bafti, S. "Regeneration And Protection Of Synapses After Noise-Induced Cochlear Synaptopathy", 2019, discloses a methodical investigation of the causes of noise-induced synaptopathy and excitotoxicity in the cochlea on the cellular level using combined in vitro and in vivo approaches.

Wise K., and Gillespie L., "Drug Delivery to the Inner Ear", J Neural Eng. 2012; 9(6): 065002, is a review on drug delivery to the inner ear.

WO 2018/140792 discloses a method of treating an otic condition in a subject in need thereof, comprising administering to the subject in need thereof an otic composition comprising a therapeutically effective amount of a non-natural neurotrophic agent, and a pharmaceutically acceptable carrier.

### SUMMARY OF THE DISCLOSURE

The invention is as defined in the appended claims.

In one aspect of the invention, there is provided an otic formulation comprising a therapeutically effective amount of a growth factor and an auris-acceptable vehicle, wherein the otic formulation is formulated to provide sustained release of the growth factor into the inner ear, wherein the auris-acceptable vehicle is a thermoreversible gel comprising poloxamer 407, wherein the otic formulation comprises from 0.0001% to 1% by weight of the growth factor, wherein the growth factor is brain-derived neurotrophic factor (BDNF), ciliary neurotrophic factor (CNTF), glial cell-line derived neurotrophic factor (GDNF), neurotrophin-3, neurotrophin-4, or any combination thereof, and wherein:

(i) the thermoreversible gel has a gelation viscosity from 15,000 cP to 3,000,000 cP;
(ii) the otic formulation has an osmolarity from 100 mOsm/L to 1000 mOsm/L; or
(iii) the otic formulation has a gelation temperature from 19°C to 42°C.

Preferably, said growth factor is neurotrophin-3.

Alternatively, said growth factor is preferably brain-derived neurotrophic factor (BDNF). Preferably, said otic formulation has a pH from 7.0 to 8.0.

Preferably, said otic formulation comprises from 14 wt% to 18 wt% poloxamer 407.

More preferably, said otic formulation comprises from 15 wt% to 17 wt% poloxamer 407. Preferably, said otic formulation further comprises at least one viscosity modulating agent. Preferably, said otic formulation comprises between 0.05% to 0.5% by weight of the growth factor.

Preferably, said otic formulation provides sustained release of the growth factor over a period of at least 7 days.

Preferably, said otic formulation is formulated to provide sustained release of the growth factor over a period of at least 5 days.

Preferably, said growth factor is essentially dissolved in the otic formulation.
According to another aspect of the present invention, said otic formulation is for use in the treatment of hearing loss.
Preferably, said formulation is for said use, wherein the ribbon synapses are repaired.

## BRIEF DESCRIPTION OF THE DRAWINGS

A better understanding of the features and advantages of the present disclosure will be obtained by reference to the following detailed description that sets forth illustrative embodiments, in which the principles of the disclosure are utilized, and the accompanying drawings of which:

**FIG. 1** illustrates the anatomy of the ear.
**FIG. 2** shows the visualization of synapses on hair cells.
**FIG. 3A** shows that treatment with sustained release BDNF formulations leads to improved hearing function (wave I amplitude) in noise exposed adult rats compared to vehicle treatment. **FIG. 3B** shows that treatment with sustained release BDNF formulations leads to an increased number of ribbon synapses per inner hair cell in noise exposed adult rats compared to vehicle treatment.
**FIG. 4** shows the perilymph concentrations of BDNF following a single IT injection in rats in poloxamer 407 formulation. BDNF was administered at the indicated doses. Perilymph BDNF is in arbitrary units.
**FIG.5A, 5B,** and **5C** show perilymph concentrations of BDNF following a single IT injection in rats in MCT formulations. BDNF was administered at a dose of 0.15% (1.5 mg/ml) in the MCT formulation containing different polymers: PVP (polyvinylpyrrolidone) at 2 or 10% **(FIG. 5A);** 0.15% carbomer **(FIG. 5B);** or P407 (poloxamer 407) at 10% **(FIG.5C).** Perilymph BDNF is in arbitrary units.
**FIG. 6A** schematically illustrates details of Example C. **FIG. 6B** shows DAPI-stained neurofilaments. **FIG. 6C** shows spiral ganglion neuron (SGN) survival normalized to 1nM NT-3 for treatment with various neurotrophins and antibodies. **FIG. 6D** shows SGN survival normalized to 1nM NT-3 for treatment at various concentrations of neurotrophins and antibodies.
**FIGs. 7A-7D** show changes in the complexities of SGNs upon treatment with various agents. **FIG. 7A** shows SGNs immunostained for neurofilament and DAPI and imaged at 20x. **FIG. 7B** shows roots (indicated with asterices), extremeties (indicated with white dots), nodes (indicated with arrrows), segments (indicated with discrete sections of neurite between nodes or extremities), and total neurite length measured for each neuron. **FIG. 7C** shows the percentage of SGNs having various numbers of roots upon treatment with 10 nM BDNF, 1 nM NT-3, or 1 nM M3 compared to the IgG-treated control (*$p<0.05$, **$p<0.005$, and $p<0.001$). **FIG. 7D** shows total neurite length (left panel) as well as total number of extremities, segments, and nodes per cell upon treatment with BDNF, NT-3, or M3.
**FIG. 8A** schematically depicts the experimental scheme of Example E. **FIG. 8B** shows stained neurofilaments for samples treated with no neurotrophin/antibody (upper left image), 10 nM BDNF (upper right image), 10 nM NT-3 (middle left image), 100 nM NT-3 (middle right image), 10 nM M3 (lower left image), and 100 nM M3 (lower right image). **FIGs. 8C** and **8D** show neurites per tissue at various concentrations of NT-3 **(FIG. 8C)** and BDNF **(FIG. 8D). FIG. 8E** shows neurites per tissue at various concentrations of hIgG4 (left) and M3 (right). **FIG. 8F** shows image analysis of explants. **FIG. 8G** shows the effect of BDNF, NT-3, and M3 on neurite complexity of SGN explants compared to IgG-treated or untreated controls.
**FIG. 9A** schematically depicts the experimental scheme of Example F. **FIG. 9B** shows estimation of type I SGNs based on counting inner hair cells (IHCs; triangles) and subtracting the number of fibers passing through to the outer hair cells (OHCs; circles). **FIG.** 9C shows SGN fibers/IHC normalized to CTL for various agents for no excitotoxin and 72 hour culture (left panel) and excitotoxin treatment and 18 hour culture (right panel). **FIG.** 9D shows images of cochlear explants taken under various conditions. **FIG. 9E** shows an image of cochlear explants with PSD-95, CtBP2, neurofilament (lines), and Myo7a. **FIG. 9F** shows puncta (PSD95)/IHC normalized to CTL for various Trk agonists for no excitotoxin and 72 hour culture (left panel) and excitotoxin and 72 hour culture (right panel) (*$p<0.05$ (CTL vs. NK)).
**FIG. 10** schematically illustrates non-invasive intratympanic delivery of an agent such as BDNF to the inner ear.
**FIG. 11** shows BDNF in perilymp (pg/ml) at various times post intratympanic injection for various concentrations of BDNF in P407.
**FIG. 12A** shows ABR threshold shift (dB SPL) at various frequencies for P407 vehicle alone and 0.05 mg/ml BDNF in P407. **FIG. 12B** shows ABR wave components (left panel) and noise-induced wave I deficits (right panel). **FIG. 12C** shows ABR wave I amplitudes measured in adult male rats after BDNF or vehicle treatment following noise exposure or in naive undamaged ears.
**FIG. 13A** shows inner hair cell innervation (upper panel) and hair cell ribbon synapses (lower panel). **FIG. 13B** shows high-magnification views of inner hair cells from the 40 kHz region from adult rats after BDNF or vehicle treatment following noise damage, or in a naive undamaged cochlea. Digital X-compressed cross-sections are shown at right.

**FIG. 13C** shows increased synaptic punca (defined by co-expression of CtBp2 and GluR2) per inner hair cell for each frequency region throughout the cochlea following BDNF treatment. **FIG. 13D** shows the cochlear frequency mapping.

**FIG. 14** schematically depicts the study design of Example H.

**FIG. 15** shows perilymph concentrations of BDNF following a single intratympanic injection in cats of BDNF poloxamer 407 formulation.

## DETAILED DESCRIPTION

Systemic administration of active agents is, in some instances, ineffectual in the treatment of diseases that affect inner ear structures. The cochlear canals and the cochlea, for example, are isolated from the circulatory system limiting systemic delivery of active agents to target sites in the inner ear. In some instances, systemic drug administration creates a potential inequality in drug concentration with higher circulating levels in the serum, and lower levels in the target auris interna organ structures. In certain instances, large amounts of drug are required to overcome this inequality in order to deliver sufficient, therapeutically effective quantities of a drug to auditory structures. In some instances, systemic drug administration also increases the likelihood of secondary systemic accumulation and consequent adverse side effects.

Currently available treatment for inner ear diseases also carries the risk of attendant side effects. For example, available methods require multiple daily doses (e.g., intratympanic injection or infusion) of drugs. In certain instances, multiple daily intratympanic injections cause patient discomfort and non-compliance. In certain instances, delivery of active agents to the inner ear via otic drops administered in the ear canal or via intratympanic injection is hindered by the biological barrier presented by the tympanic membrane the oval window membrane and/or the round window membrane. In some instances, delivery of active agents to the inner ear via otic drops or intratympanic injection causes osmotic imbalance in inner ear structures, introduces infections or other immune disorders as a result of microbial or endotoxin presence, or results in permanent structural damage (e.g. perforation of the tympanic membrane), resulting in hearing loss and the like.

Intratympanic injection of therapeutic agents is the technique of injecting a therapeutic agent behind the tympanic membrane into the auris media and/or auris interna. Some challenges remain with intratympanic injections. For example, access to the round window membrane, the site of drug absorption into the auris interna, is challenging in some instances. In addition, current regimens using intratympanic injections do not address changing the osmolarity and pH of the perilymph and endolymph, and introducing pathogens and endotoxins that directly or indirectly damage inner ear.

Provided herein in one aspect are otic formulations comprising a therapeutically effective amount of an active agent, such as a growth factor. In some embodiments, the growth factor is brain-derived neurotrophic factor (BDNF), ciliary neurotrophic factor (CNTF), glial cell-line derived neurotrophic factor (GDNF), neurotrophin-3, neurotrophin-4, or any combination thereof. In some embodiments, the growth factor is brain-derived neurotrophic factor (BDNF). In some embodiments, the otic formulations are auris-acceptable gels. In some embodiments, the otic formulations are triglyceride based auris-acceptable formulations.

These otic pharmaceutical formulations are suitable for drug delivery into the external, middle and/or inner ear. In some instances, these otic pharmaceutical formulations are suitable for administration to humans. In some instances, the otic formulations disclosed herein also meet stringent criteria for pH, osmolarity, ionic balance, sterility, endotoxin, and/or pyrogen levels. In some instances, the otic formulations are compatible with the microenvironment of the inner ear (e.g., the perilymph).

Accordingly, provided herein, in certain embodiments, are otic formulations that are controlled release auris-acceptable formulations that locally treat auris target structures and provide extended exposure of otic active agents to the target auris structures. In certain embodiments, the otic formulations described herein are designed for stringent osmolarity and pH ranges that are compatible with auditory structures and/or the endolymph and perilymph. In some embodiments, the otic formulations described herein are controlled release formulations that provide extended release for a period of at least 3 days and meet stringent sterility requirements. In some instances, otic formulations described herein contain lower endotoxin levels (e.g. < 0.5 EU/mL when compared to typically acceptable endotoxin levels of 0.5 EU/mL. In some instances, the otic formulations described herein contain low levels of colony forming units (e.g., <50 CFUs) per gram of the formulation . In some instances, the otic formulations described herein are substantially free of pyrogens and/or microbes. In some instances the otic formulations described herein are formulated to preserve the ionic balance of the endolymph and/or the perilymph.

In some instances, local administration of the otic formulations described herein avoids potential adverse side effects as a result of systemic administration of active agents. In some instances, the locally applied otic formulations described herein are compatible with auris structures. Such compatible auris structures include those associated with the outer, middle, and/or inner ear. In some embodiments, the otic formulations are administered either directly to the desired auris structure, e.g. the cochlear region, or administered to a structure in direct communication with areas of the auris structure; in the case of the cochlear region, for example, including the round window membrane, the crista fenestrae

cochleae or the oval window membrane.

In certain instances, the otic formulations disclosed herein controlled release formulations that provide a constant rate of release of a drug from the formulation and provide a constant prolonged source of exposure of an otic active agent to the inner ear of an individual or patient suffering from an otic disorder, reducing or eliminating any variabilities associated with other methods of treatment (such as, e.g., otic drops and/or multiple intratympanic injections).

In some embodiments, the otic formulations described herein provide extended release of the active ingredient(s) into the external ear. In some embodiments, the otic formulations described herein provide extended release of the active ingredient(s) into the middle and/or inner ear *(auris interna),* including the cochlea and vestibular labyrinth. In some embodiments, the otic formulations further comprise an immediate or rapid release component in combination with a controlled release component.

**Certain Definitions**

The term "auris-acceptable" with respect to a formulation, or ingredient, as used herein, includes having no persistent detrimental effect on the auris externa (or external ear or outer ear), auris media (or middle ear) and/or the auris interna (or inner ear) of the subject being treated. By "auris-pharmaceutically acceptable," as used herein, refers to a material, such as a carrier or diluent, which does not abrogate the biological activity or properties of the compound in reference to the auris externa (or external ear or outer ear), auris media (or middle ear) and/or the auris interna (or inner ear), and is relatively or is reduced in toxicity to the auris externa (or external ear or outer ear), auris media (or middle ear) and the auris interna (or inner ear), i.e., the material is administered to an individual without causing undesirable biological effects or interacting in a deleterious manner with any of the components of the formulation in which it is contained.

As used herein, amelioration or lessening of the symptoms of a particular otic disease, disorder or condition by administration of a particular compound or pharmaceutical formulation refers to any decrease of severity, delay in onset, slowing of progression, or shortening of duration, whether permanent or temporary, lasting or transient that is attributed to or associated with administration of the compound .

As used herein, the term "antimicrobial agent" refers to compounds that inhibit the growth, proliferation, or multiplication of microbes, or that kill microbes. Suitable "antimicrobial agents" are antibacterial agents (effective against bacteria), antiviral agents (effective against viruses), antifungal agents (effective against fungi), antiprotozoal (effective against protozoa), and/or antiparasitic to any class of microbial parasites. "Antimicrobial agents" work by any suitable mechanism against the microbes, including by being toxic or cytostatic.

"Antioxidants" are auris-pharmaceutically acceptable antioxidants, and include, for example, butylated hydroxytoluene (BHT), sodium ascorbate, ascorbic acid, sodium metabisulfite and tocopherol. In certain embodiments, antioxidants enhance chemical stability where required. Antioxidants are also used to counteract the ototoxic effects of certain therapeutic agents.

The term "auris-acceptable penetration enhancer" with respect to a formulation, composition or ingredient, as used herein, refers to the property of reducing barrier resistance.

"Auris externa" refers to the external (or outer) ear, and includes the pinna and the external auditory canal (EAC).

"Auris interna" refers to the inner ear, including the cochlea and the vestibular labyrinth, and the round window that connects the cochlea with the middle ear.

"Auris-interna bioavailability" or "Auris media bioavailability" refers to the percentage of the administered dose of compounds disclosed herein that becomes available in the inner or middle ear, respectively, of the animal or human being studied.

"Auris media" refers to the middle ear, including the tympanic cavity, auditory ossicles and oval window, which connects the middle ear with the inner ear.

"Auris-interna bioavailability" refers to the percentage of the administered dose of compounds disclosed herein that becomes available in the inner ear of the animal or human being studied.

"Balance disorder" refers to a disorder, illness, or condition which causes a subject to feel unsteady, or to have a sensation of movement. Included in this definition are dizziness, vertigo, disequilibrium, and pre-syncope. Diseases which are classified as balance disorders include Ramsay Hunt's Syndrome, Ménière's Disease, mal de debarquement, benign paroxysmal positional vertigo, labyrinthitis, and presbycusis.

"Blood plasma concentration" refers to the concentration of compounds provided herein in the plasma component of blood of a subject.

"Carrier materials" are excipients that are compatible with the otic agent, the auris media, the auris interna and the release profile properties of the auris-acceptable pharmaceutical formulations. Such carrier materials include, e.g., binders, suspending agents, disintegration agents, filling agents, surfactants, solubilizers, stabilizers, lubricants, wetting agents, diluents, and the like. "Auris-pharmaceutically compatible carrier materials" include acacia, gelatin, colloidal silicon dioxide, calcium glycerophosphate, calcium lactate, maltodextrin, glycerine, magnesium silicate, polyvinylpyrrolidone (PVP), cholesterol, cholesterol esters, sodium caseinate, soy lecithin, taurocholic acid, phosphatidylcholine,

sodium chloride, tricalcium phosphate, dipotassium phosphate, cellulose and cellulose conjugates, sugars sodium stearoyl lactylate, carrageenan, monoglyceride, diglyceride, pregelatinized starch, alginate, carbomer, hyaluronic acid (HA), poloxamer, dextran, and the like.

As used herein, the term "cytotoxic agent" refers to compounds that are cytotoxic (i.e., toxic to a cell) effective for the treatment of otic disorders, e.g., autoimmune diseases of the ear and cancer of the ear, and are suitable for use in the formulations disclosed herein.

The term "diluent" are chemical compounds that are used to dilute the otic agent prior to delivery and which are compatible with the auris media and/or auris interna.

"Dispersing agents," and/or "viscosity modulating agents" and/or "thickening agents" are materials that control the diffusion and homogeneity of the otic agent through liquid media. Examples of diffusion facilitators/dispersing agents include hydrophilic polymers, electrolytes, Tween ® 60 or 80, PEG, polyvinylpyrrolidone (PVP; commercially known as Plasdone®), and the carbohydrate-based dispersing agents such as, for example, hydroxypropyl celluloses (e.g., HPC, HPC-SL, and HPC-L), hydroxypropyl methylcelluloses (e.g., HPMC K100, HPMC K4M, HPMC K15M, HPMC E10M, and HPMC K100M), carboxymethylcellulose, carboxymethylcellulose sodium, methylcellulose, hydroxyethylcellulose, hydroxypropylcellulose, hydroxypropylmethylcellulose phthalate, hydroxypropylmethylcellulose acetate stearate (HPMCAS), noncrystalline cellulose, magnesium aluminum silicate, triethanolamine, polyvinyl alcohol (PVA), vinyl pyrrolidone/vinyl acetate copolymer (S630), 4-(1,1,3,3-tetramethylbutyl)-phenol polymer with ethylene oxide and formaldehyde (also known as tyloxapol), poloxamers (e.g., Pluronics F68®, F88®, F108®, and F127®, which are block copolymers of ethylene oxide and propylene oxide); and poloxamines (e.g., Tetronic 908®, also known as Poloxamine 908®, which is a tetrafunctional block copolymer derived from sequential addition of propylene oxide and ethylene oxide to ethylenediamine (BASF Corporation, Parsippany, N.J.)), polyvinylpyrrolidone K12, polyvinylpyrrolidone K17, polyvinylpyrrolidone K25, or polyvinylpyrrolidone K30, polyvinylpyrrolidone/vinyl acetate copolymer (S-630), polyethylene glycol, e.g., the polyethylene glycol has a molecular weight of about 300 to about 6000, or about 3350 to about 4000, or about 7000 to about 5400, sodium carboxymethylcellulose, methylcellulose, polysorbate-80, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, polysorbate-80, sodium alginate, polyethoxylated sorbitan monolaurate, polyethoxylated sorbitan monolaurate, povidone, carbomers, polyvinyl alcohol (PVA), alginates, chitosans, silicon dioxide, and combinations thereof. Plasticizers such as cellulose or triethyl cellulose are also be used as dispersing agents. Optional dispersing agents useful in liposomal dispersions and self-emulsifying dispersions of the otic agents disclosed herein are dimyristoyl phosphatidyl choline, natural phosphatidyl choline from eggs, natural phosphatidyl glycerol from eggs, cholesterol, and isopropyl myristate. In some embodiments, the "dispersing agent," and/or "viscosity modulating agent" and/or "thickening agent" is not a poloxamer.

"Drug absorption" or "absorption" refers to the process of movement of the otic agent from the localized site of administration, by way of example only, the round window membrane of the inner ear, and across a barrier (the round window membranes, as described below) into the auris interna or inner ear structures. The terms "co-administration" or the like, as used herein, are meant to encompass administration of the otic agent to a single patient, and are intended to include treatment regimens in which the otic agents are administered by the same or different route of administration or at the same or different time.

The terms "effective amount" or "therapeutically effective amount," as used herein, refer to a sufficient amount of the otic agent being administered that would be expected to relieve to some extent one or more of the symptoms of the disease or condition being treated. For example, the result of administration of the otic agents disclosed herein is reduction and/or alleviation of the signs, symptoms, or causes of any one of the diseases or conditions disclosed herein. For example, an "effective amount" for therapeutic uses is the amount of the otic agent, including a formulation as disclosed herein required to provide a decrease or amelioration in disease symptoms without undue adverse side effects. The term "therapeutically effective amount" includes, for example, a prophylactically effective amount. An "effective amount" of a otic agent composition disclosed herein is an amount effective to achieve a desired pharmacologic effect or therapeutic improvement without undue adverse side effects. It is understood that "an effective amount" or "a therapeutically effective amount" varies, in some embodiments, from subject to subject, due to variation in metabolism of the compound administered, age, weight, general condition of the subject, the condition being treated, the severity of the condition being treated, and the judgment of the prescribing physician. In some instances, it is also understood that "an effective amount" in an extended-release dosing format differs from "an effective amount" in an immediate-release dosing format based upon pharmacokinetic and pharmacodynamic considerations.

The terms "enhance" or "enhancing" refers to an increase or prolongation of either the potency or duration of a desired effect of the otic agent, or a diminution of any adverse symptomatology. For example, in reference to enhancing the effect of the otic agents disclosed herein, the term "enhancing" refers to the ability to increase or prolong, either in potency or duration, the effect of other therapeutic agents that are used in combination with the otic agents disclosed herein. An "enhancing-effective amount," as used herein, refers to an amount of an otic agent or other therapeutic agent that is adequate to enhance the effect of another therapeutic agent or otic agent in a desired system. When used in a patient,

amounts effective for this use will depend on the severity and course of the disease, disorder or condition, previous therapy, the patient's health status and response to the drugs, and the judgment of the treating physician.

The term "inhibiting" includes preventing, slowing, or reversing the development of a condition, including any of one of the conditions described herein, or advancement of a condition in a patient necessitating treatment.

The terms "kit" and "article of manufacture" are used as synonyms.

"Local anesthetic" means a substance which causes a reversible loss of sensation and/or a loss of nociception. Often, these substances function by decreasing the rate of the depolarization and repolarization of excitable membranes (for example, neurons). By way of non-limiting example, local anesthetics include lidocaine, benzocaine, prilocaine, and tetracaine.

As used herein, the term "otic agent" or "otic structure modulating agent" or "otic therapeutic agent" or "otic active agent" or "active agent" or "therapeutic agent" refers to compounds that are effective for the treatment of otic disorders, e.g., otitis media, otosclerosis, autoimmune diseases of the ear and cancer of the ear, and are suitable for use in the formulations disclosed herein. An "otic agent" or "otic structure modulating agent" or "otic therapeutic agent" or "otic active agent" or "active agent" includes compounds that act as an agonist, a partial agonist, an antagonist, a partial antagonist, an inverse agonist, a competitive antagonist, a neutral antagonist, an orthosteric antagonist, an allosteric antagonist, a positive allosteric modulator of an otic structure modulating target, a negative allosteric modulator of an otic structure modulating target, or combinations thereof.

The term "otic intervention" means an external insult or trauma to one or more auris structures and includes implants, otic surgery, injections, cannulations, or the like. Implants include auris-interna or auris-media medical devices, examples of which include cochlear implants, hearing sparing devices, hearing-improvement devices, short electrodes, micro-prostheses or piston-like prostheses; needles; stem cell transplants; drug delivery devices; any cell-based therapeutic; or the like. Otic surgery includes middle ear surgery, inner ear surgery, tympanostomy, cochleostomy, labyrinthotomy, mastoidectomy, stapedectomy, stapedotomy, endolymphatic sacculotomy, or the like. Injections include intratympanic injections, intracochlear injections, injections across the round window membrane or the like. Cannulations include intratympanic, intracochlear, endolymphatic, perilymphatic or vestibular cannulations, or the like.

The term "penetration enhancer" refers to an agent that reduces barrier resistance (e.g., barrier resistance of the round window membrane, BLB or the like).

"Pharmacodynamics" refers to the factors which determine the biologic response observed relative to the concentration of drug at the desired site within the auris media and/or auris interna.

"Pharmacokinetics" refers to the factors which determine the attainment and maintenance of the appropriate concentration of drug at the desired site within the auris media and/or auris interna.

In prophylactic applications, formulations containing the otic agents described herein are administered to a patient susceptible to or otherwise at risk of a particular disease, disorder or condition. Such an amount is defined to be a "prophylactically effective amount or dose." In this use, the precise amounts also depend on the patient's state of health, weight, and the like.

A "prodrug" refers to the otic agent that is converted into the parent drug *in vivo.* In certain embodiments, a prodrug is enzymatically metabolized by one or more steps or processes to the biologically, pharmaceutically or therapeutic form of the compound. To produce a prodrug, a pharmaceutically active compound is modified such that the active compound will be regenerated upon *in vivo* administration. In one embodiment, the prodrug is designed to alter the metabolic stability or the transport characteristics of a drug, to mask side effects or toxicity, or to alter other characteristics or properties of a drug. Compounds provided herein, in some embodiments, are derivatized into suitable prodrugs.

"Solubilizers" refers to auris-acceptable compounds such as triacetin, triethylcitrate, ethyl oleate, ethyl caprylate, sodium lauryl sulfate, sodium doccusate, vitamin E TPGS, dimethylacetamide, N-methylpyrrolidone, N-hydroxyethylpyrrolidone, polyvinylpyrrolidone, hydroxypropylmethyl cellulose, hydroxypropyl cyclodextrins, ethanol, n-butanol, isopropyl alcohol, cholesterol, bile salts, polyethylene glycol 200-600, glycofurol, transcutol®, propylene glycol, and dimethyl isosorbide and the like.

"Stabilizers" refers to compounds such as any antioxidation agents, buffers, acids, preservatives and the like that are compatible with the environment of the auris media and/or auris interna. Stabilizers include agents that will do any of (1) improve the compatibility of excipients with a container, or a delivery system, including a syringe or a glass bottle, (2) improve the stability of a component of the formulation, or (3) improve formulation stability.

"Steady state," as used herein, is when the amount of drug administered to the auris media and/or auris interna is equal to the amount of drug eliminated within one dosing interval resulting in a plateau or constant levels of drug exposure within the targeted structure.

As used herein, the term "subject" is used to mean an animal, preferably a mammal, including a human or non-human. The terms patient and subject are used interchangeably.

"Surfactants" refers to compounds that are auris-acceptable, such as sodium lauryl sulfate, sodium docusate, Tween 60 or 80, triacetin, vitamin E TPGS, phospholipids, lecithins, phosphatidyl cholines (c8-c18), phosphatidylethanolamines (c8-c18), phosphatidylglycerols (c8-c18), sorbitan monooleate, polyoxyethylene sorbitan monooleate, polysorbates, polaxomers, bile salts, glyceryl monostearate, copolymers of ethylene oxide and propylene oxide, e.g., Pluronic® (BASF), and the like. Some other surfactants include polyoxyethylene fatty acid glycerides and vegetable oils, e.g., polyoxyethylene (60) hydrogenated castor oil; and polyoxyethylene alkylethers and alkylphenyl ethers, *e.g.,* octoxynol 10, octoxynol 40. In some embodiments, surfactants are included to enhance physical stability or for other purposes.

The terms "treat," "treating" or "treatment," as used herein, include alleviating, abating or ameliorating a disease or condition or the associated symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or controlling or stopping the symptoms of the disease or condition either prophylactically and/or therapeutically.

The term "substantially low degradation products" means about 10% by weight of the active agent are degradation products of the active agent. In further embodiments, the term means less than 10% by weight of the active agent are degradation products of the active agent. In further embodiments, the term means less than 9% by weight of the active agent are degradation products of the active agent. In further embodiments, the term means less than 8% by weight of the active agent are degradation products of the active agent. In further embodiments, the term means less than 7% by weight of the active agent are degradation products of the active agent. In further embodiments, the term means less than 6% by weight of the active agent are degradation products of the active agent. In further embodiments, the term means less than 5% by weight of the active agent are degradation products of the active agent. In further embodiments, the term means less than 4% by weight of the active agent are degradation products of the active agent. In further embodiments, the term means less than 3% by weight of the active agent are degradation products of the active agent. In yet further embodiments, the term means less than 2% by weight of the active agent are degradation products of the active agent. In further embodiments, the term means less than 1% by weight of the active agent are degradation products of the active agent. In some embodiments, any individual impurity (e.g., metal impurity, degradation products of active agent and/or excipients, or the like) present in a formulation described herein is less than 5%, less than 2%, or less than 1% by weight of the active agent. In some embodiments the formulation does not contain precipitate during storage or change in color after manufacturing and storage.

Pharmaceutically acceptable derivatives of a compound include salts, esters, enol ethers, enol esters, acetals, ketals, orthoesters, hemiacetals, hemiketals, acids, bases, solvates, hydrates, or prodrugs thereof. In some embodiments, such derivatives are be readily prepared by those of skill in this art using known methods for such derivatization. Pharmaceutically acceptable salts include amine salts, such as N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2- pyrrolidin-1'-ylmethylbenzimidazole, diethylamine, and other alkylamines, piperazine and tris(hydroxymethyl)aminomethane; alkali metal salts, such as lithium, potassium and sodium; alkali earth metal salts, such as barium, calcium and magnesium; transition metal salts, such as zinc; and inorganic salts, such as sodium hydrogen phosphate and disodium phosphate; and also including, but salts of mineral acids, such as hydrochlorides and sulfates; and salts of organic acids, such as acetates, lactates, malates, tartrates, citrates, ascorbates, succinates, butyrates, valerates, mesylates, and fumarates. Pharmaceutically acceptable esters include alkyl, alkenyl, alkynyl, aryl, aralkyl, and cycloalkyl esters of acidic groups, including carboxylic acids, phosphoric acids, phosphinic acids, sulfonic acids, sulfinic acids, and boronic acids. Pharmaceutically acceptable enol ethers include derivatives of formula C=C(OR), where R is hydrogen, alkyl, alkenyl, alkynyl, aryl, aralkyl, and cycloalkyl. Pharmaceutically acceptable enol esters include derivatives of formula C=C(OC(O)R), where R is hydrogen, alkyl, alkenyl, alkynyl, aryl, aralkyl, and cycloalkyl. Pharmaceutically acceptable solvates and hydrates are complexes of a compound with one or more solvent or water molecules, or 1 to about 100, or 1 to about 10, or one to about 2, 3 or 4, solvent or water molecules.

It is to be understood that the compounds provided herein may contain chiral centers. Such chiral centers may be of either the (R) or (S) configuration, or may be a mixture thereof. Thus, the compounds provided herein may be enantiomerically pure, or be stereoisomeric or diastereomeric mixtures. As such, one of skill in the art will recognize that administration of a compound in its (R) form is equivalent, for compounds that undergo epimerization in vivo, to administration of the compound in its (S) form.

The instant disclosure is meant to include all such possible isomers, as well as, their racemic and optically pure forms. Optically active (+) and (-), (R)- and (S)-, or (D)- and (L)-isomers are prepared in some instances using chiral synthons or chiral reagents, or resolved using conventional techniques, such as reverse phase HPLC. When the compounds described herein contain olefinic double bonds or other centers of geometric asymmetry, and unless specified otherwise, it is intended that the compounds include both E and Z geometric isomers. Likewise, all tautomeric forms are also intended to be included.

As used herein, alkyl, alkenyl, and alkynyl carbon chains, if not specified, contain from 1 to 20 carbons, 1 to 16 carbons or 1 to 6 carbons and are straight or branched. In certain embodiments, alkyl, alkenyl, and alkynyl carbon chains

contain from 1 to 6 carbons. Alkenyl carbon chains of from 2 to 20 carbons, in certain embodiments, contain 1 to 8 double bonds, and the alkenyl carbon chains of 2 to 16 carbons, in certain embodiments, contain 1 to 5 double bonds. The alkenyl carbon chains of 2 to 6 carbons, in certain embodiments, contain 1 to 2 double bonds. Alkynyl carbon chains of from 2 to 20 carbons, in certain embodiments, contain 1 to 8 triple bonds, and the alkynyl carbon chains of 2 to 16 carbons, in certain embodiments, contain 1 to 5 triple bonds. Alkynyl carbon chains of from 2 to 6 carbons, in certain embodiments, contain 1 to 2 triple bonds. Exemplary alkyl, alkenyl and alkynyl groups herein include methyl, ethyl, propyl, isopropyl, isobutyl, n-butyl, sec-butyl, tert-butyl, isopentyl, neopentyl, tert- pentyl, isohexyl, vinyl, 1-propenyl, 2-propenyl, isopropenyl, 1-butenyl, 2-butenyl, 3- butenyl, 1,3-butadienyl, ethynyl, 1-propynyl, and 2-propynyl. As used herein, lower alkyl, lower alkenyl, and lower alkynyl refer to carbon chains having from about 1 or about 2 carbons up to about 6 carbons.

The term "cycloalkyl" refers to a saturated mono- or multicyclic ring system, in certain embodiments of 3 to 10 carbon atoms, in other embodiments of 3 to 6 carbon atoms; cycloalkenyl and cycloalkynyl refer to mono- or multicyclic ring systems that respectively include at least one double bond and at least one triple bond. Cycloalkenyl and cycloalkynyl groups may, in certain embodiments, contain 3 to 10 carbon atoms, with cycloalkenyl groups, in further embodiments, containing 4 to 7 carbon atoms and cycloalkynyl groups, in further embodiments, containing 8 to 10 carbon atoms. The ring systems of the cycloalkyl, cycloalkenyl, and cycloalkynyl groups may be composed of one ring or two or more rings which may be joined together in a fused, bridged, or spiro-connected fashion.

The term "aryl" refers to aromatic monocyclic or multicyclic groups containing from 6 to 19 carbon atoms. Aryl groups include groups such as fluorenyl, substituted fluorenyl, phenyl, substituted phenyl, naphthyl, and substituted naphthyl.

The term "aralkyl" refers to an alkyl group in which one of the hydrogen atoms of the alkyl is replaced by an aryl.

**Anatomy of the Ear**

The ear serves as both the sense organ that detects sound and the organ that maintains balance and body position. The ear is generally divided into three portions: the outer ear, middle ear and the inner ear (or auris interna). As shown in **FIG. 1,** the outer ear is the external portion of the organ and is composed of the pinna (auricle), the auditory canal (external auditory meatus) and the outward facing portion of the tympanic membrane, also known as the ear drum. The pinna, which is the fleshy part of the externa ear that is visible on the side of the head, collects sound waves and directs them toward the auditory canal. Thus, the function of the outer ear, in part, is to collect and direct sound waves towards the tympanic membrane and the middle ear.

The middle ear is an air-filled cavity, called the tympanic cavity, behind the tympanic membrane. The tympanic membrane, also known as the ear drum, is a thin membrane that separates the external ear from the middle ear. The middle ear lies within the temporal bone, and includes within this space the three ear bones (auditory ossicles): the malleus, the incus and the stapes. The auditory ossicles are linked together via tiny ligaments, which form a bridge across the space of the tympanic cavity. The malleus, which is attached to the tympanic membrane at one end, is linked to the incus at its anterior end, which in turn is linked to the stapes. The stapes is attached to the oval window, one of two windows located within the tympanic cavity. A fibrous tissue layer, known as the annular ligament connects the stapes to the oval window. Sound waves from the outer ear first cause the tympanic membrane to vibrate. The vibration is transmitted across to the cochlea through the auditory ossicles and oval window, which transfers the motion to the fluids in the auris interna. Thus, the auditory ossicles are arranged to provide a mechanical linkage between the tympanic membrane and the oval window of the fluid-filled auris interna, where sound is transformed and transduced to the auris interna for further processing. Stiffness, rigidity or loss of movement of the auditory ossicles, tympanic membrane or oval window leads to hearing loss, e.g. otosclerosis, or rigidity of the stapes bone.

The tympanic cavity also connects to the throat via the eustachian tube. The eustachian tube provides the ability to equalize the pressure between the outside air and the middle ear cavity. The round window, a component of the auris interna but which is also accessible within the tympanic cavity, opens into the cochlea of the auris interna. The round window is covered by a membrane, which consists of three layers: an external or mucous layer, an intermediate or fibrous layer, and an internal membrane, which communicates directly with the cochlear fluid. The round window, therefore, has direct communication with the auris interna via the internal membrane.

Movements in the oval and round window are interconnected, i.e. as the stapes bone transmits movement from the tympanic membrane to the oval window to move inward against the auris interna fluid, the round window is correspondingly pushed out and away from the cochlear fluid. This movement of the round window allows movement of fluid within the cochlea, which eventually leads in turn to movement of the cochlear inner hair cells, allowing hearing signals to be transduced. Stiffness and rigidity in the round window leads to hearing loss because of the lack of ability of movement in the cochlear fluid. Recent studies have focused on implanting mechanical transducers onto the round window, which bypasses the normal conductive pathway through the oval window and provides amplified input into the cochlear chamber.

Auditory signal transduction takes place in the auris interna. The fluid-filled inner ear, or auris interna, consists of

two major components: the cochlear and the vestibular apparatus.

The cochlea is the portion of the auris interna related to hearing. The cochlea is a tapered tube-like structure which is coiled into a shape resembling a snail. The inside of the cochlea is divided into three regions, which is further defined by the position of the vestibular membrane and the basilar membrane. The portion above the vestibular membrane is the scala vestibuli, which extends from the oval window to the apex of the cochlea and contains perilymph fluid, an aqueous liquid low in potassium and high in sodium content. The basilar membrane defines the scala tympani region, which extends from the apex of the cochlea to the round window and also contains perilymph. The basilar membrane contains thousands of stiff fibers, which gradually increase in length from the round window to the apex of the cochlea. The fibers of the basement membrane vibrate when activated by sound. In between the scala vestibuli and the scala tympani is the cochlear duct, which ends as a closed sac at the apex of the cochlea. The cochlear duct contains endolymph fluid, which is similar to cerebrospinal fluid and is high in potassium.

The Organ of Corti, the sensory organ for hearing, is located on the basilar membrane and extends upward into the cochlear duct. The Organ of Corti contains hair cells, which have hairlike projections that extend from their free surface, and contacts a gelatinous surface called the tectorial membrane. Although hair cells have no axons, they are surrounded by sensory nerve fibers that form the cochlear branch of the vestibulocochlear nerve (cranial nerve VIII).

As discussed, the oval window, also known as the elliptical window communicates with the stapes to relay sound waves that vibrate from the tympanic membrane. Vibrations transferred to the oval window increases pressure inside the fluid-filled cochlea via the perilymph and scala vestibuli/scala tympani, which in turn causes the membrane on the round window to expand in response. The concerted inward pressing of the oval window/outward expansion of the round window allows for the movement of fluid within the cochlea without a change of intra-cochlear pressure. However, as vibrations travel through the perilymph in the scala vestibuli, they create corresponding oscillations in the vestibular membrane. These corresponding oscillations travel through the endolymph of the cochlear duct, and transfer to the basilar membrane. When the basilar membrane oscillates, or moves up and down, the Organ of Corti moves along with it. The hair cell receptors in the Organ of Corti then move against the tectorial membrane, causing a mechanical deformation in the tectorial membrane. This mechanical deformation initiates the nerve impulse which travels via the vestibulocochlear nerve to the central nervous system, mechanically transmitting the sound wave received into signals that are subsequently processed by the central nervous system.

The auris interna is located in part within the osseous or bony labyrinth, an intricate series of passages in the temporal bone of the skull. The vestibular apparatus is the organ of balance and consists of the three semi-circular canals and the vestibule. The three semi-circular canals are arranged relative to each other such that movement of the head along the three orthogonal planes in space is detected by the movement of the fluid and subsequent signal processing by the sensory organs of the semi-circular canals, called the crista ampullaris. The crista ampullaris contains hair cells and supporting cells, and is covered by a dome-shaped gelatinous mass called the cupula. The hairs of the hair cells are embedded in the cupula. The semi-circular canals detect dynamic equilibrium, the equilibrium of rotational or angular movements.

When the head turns rapidly, the semicircular canals move with the head, but endolymph fluid located in the membranous semi-circular canals tends to remain stationary. The endolymph fluid pushes against the cupula, which tilts to one side. As the cupula tilts, it bends some of the hairs on the hair cells of the crista ampullaris, which triggers a sensory impulse. Because each semicircular canal is located in a different plane, the corresponding crista ampullaris of each semi-circular canal responds differently to the same movement of the head. This creates a mosaic of impulses that are transmitted to the central nervous system on the vestibular branch of the vestibulocochlear nerve. The central nervous system interprets this information and initiates the appropriate responses to maintain balance. Of importance in the central nervous system is the cerebellum, which mediates the sense of balance and equilibrium.

The vestibule is the central portion of the auris interna and contains mechanoreceptors bearing hair cells that ascertain static equilibrium, or the position of the head relative to gravity. Static equilibrium plays a role when the head is motionless or moving in a straight line. The membranous labyrinth in the vestibule is divided into two sac-like structures, the utricle and the saccule. Each structure in turn contains a small structure called a macula, which is responsible for maintenance of static equilibrium. The macula consists of sensory hair cells, which are embedded in a gelatinous mass (similar to the cupula) that covers the macula. Grains of calcium carbonate, called otoliths, are embedded on the surface of the gelatinous layer.

When the head is in an upright position, the hairs are straight along the macula. When the head tilts, the gelatinous mass and otoliths tilts correspondingly, bending some of the hairs on the hair cells of the macula. This bending action initiates a signal impulse to the central nervous system, which travels via the vestibular branch of the vestibulocochlear nerve, which in turn relays motor impulses to the appropriate muscles to maintain balance.

In some instances, the otic formulations described herein are placed in the outer ear. In some instances, the otic formulations described herein are placed in the middle or inner ear, including the cochlea and vestibular labyrinth: one option is to use a syringe/needle or pump and inject the formulation across the tympanic membrane (the eardrum). In some instances, for cochlear and vestibular labyrinth delivery, one option is to deliver the active ingredient across the round

window membrane or even by microinjection directly into the auris interna also known as cochlear microperfusion.

**Diseases or Conditions of the Ear (any reference to methods of treatment do not form part of the invention)**

In some embodiments, the otic formulations described herein are suitable for the treatment and/or prevention of diseases or conditions associated with the outer, middle, and/or inner ear. In some embodiments, the otic formulations described herein are suitable for the treatment and/or prevention of diseases or conditions associated with the outer ear. In some embodiments, the otic formulations described herein are suitable for the treatment and/or prevention of diseases or conditions associated with the middle ear. In some embodiments, the otic formulations described herein are suitable for the treatment and/or prevention of diseases or conditions associated with the inner ear. In some embodiments, the otic formulations described herein reduce, reverse and/or ameliorate symptoms of otic diseases or conditions, such as any one of these disclosed herein. These disorders or conditions have many causes, which include infection, injury, inflammation, tumors, and adverse response to drugs or other chemical agents.

The otic formulations described herein are useful for treating ear pruritus, otitis externa, otalgia, tinnitus, vertigo, ear fullness, hearing loss, or a combination thereof. In some embodiments, the otic formulations described herein are used to for the treatment and/or prevention of Ménière's disease, sensorineural hearing loss, noise induced hearing loss, presbycusis (age related hearing loss), auto immune ear disease, tinnitus, ototoxicity, excitotoxicity, endolymphatic hydrops, labyrinthitis, Ramsay Hunt's Syndrome, vestibular neuronitis, microvascular compression syndrome, hyperacusis, presbystasis, central auditory processing disorder, or auditory neuropathy. In some embodiments, the otic formulations described herein are used for the improvement of cochlea implant performance. In some embodiments, the otic formulations are used for the treatment and/or prevention of Ménière's disease. In some embodiments, the otic formulations are used for the treatment and/or prevention of sensorineural hearing loss. In some embodiments, the otic formulations are used for the treatment and/or prevention of noise induced hearing loss. In some embodiments, the otic formulations are used for the treatment and/or prevention of presbycusis (age related hearing loss). In some embodiments, the otic formulations are used for the treatment and/or prevention of auto immune ear disease. In some embodiments, the otic formulations are used for the treatment and/or prevention of tinnitus. In some embodiments, the otic formulations are used for the treatment and/or prevention of ototoxicity. In some embodiments, the otic formulations are used for the treatment and/or prevention of excitotoxicity. In some embodiments, the otic formulations are used for the treatment and/or prevention of endolymphatic hydrops. In some embodiments, the otic formulations are used for the treatment and/or prevention of labyrinthitis. In some embodiments, the otic formulations are used for the treatment and/or prevention of Ramsay Hunt's Syndrome. In some embodiments, the otic formulations are used for the treatment and/or prevention of vestibular neuronitis. In some embodiments, the otic formulations are used for the treatment and/or prevention of microvascular compression syndrome. In some embodiments, the otic formulations are used for the treatment and/or prevention of hyperacusis. In some embodiments, the otic formulations are used for the treatment and/or prevention of presbystasis. In some embodiments, the otic formulations are used for the treatment and/or prevention of central auditory processing disorder. In some embodiments, the otic formulations are used for the treatment and/or prevention of auditory neuropathy. In some embodiments, the otic formulations are used for the improvement of cochlea implant performance.

*Ear Pruritus*

Ear pruritus, or itchy ear canal, is a tickling or irritating sensation that causes a desire or reflex to scratch the affected area. In some cases, redness, swelling, soreness, and flaking may develop in the affected area. Ear pruritus is caused by a variety of agents. In some embodiments, ear pruritus occurs due to either primary microbial infection within the ear or as a secondary infection from the body where it is then spread into the ear canal. In some embodiments, skin conditions, such as eczema or psoriasis, lead to skin irritations within the ear canal. Further, external irritants such as hairspray, shampoo, shower gel, or allergen, such as dust, pets, and pollen, lead to ear pruritus in some instances. In some embodiments, ear pruritus serves as an early sign for more serious complications such as otitis externa.

*Otalgia*

Otalgia, also known as earache or ear pain, is classified into two types, primary otalgia and referred otalgia. Primary otalgia is ear pain which originates from inside of the ear. Referred otalgia is ear pain which originates from the outside of the ear. Although the etiology of referred otalgia can be complex, several well-known culprits include dental disorders, sinusitis, neck problems, tonsillitis, pharyngitis, and sensory branches from the vagus and glossopharyngeal nerves. In some cases, referred otalgia has been associated with head and neck malignancies.

*Ear Fullness*

Ear fullness or aural fullness is described as a feeling that the ears are clogged, stuffed, or congested. Similar to otalgia, the etiology of ear fullness is diverse with numerous underlying causes. Generally, ear fullness may also be accompanied by tinnitus, otalgia, and impaired hearing.

*Hearing Loss*

Hearing loss is a partial or total impairment to hearing. Hearing loss is classified into three types, conductive hearing loss, sensorineural hearing loss, and mixed hearing loss. Conductive hearing loss occurs when sound is not conducted efficiently through the external auditory canal to the tympanic membrane or eardrum. In some embodiments, conductive hearing loss involves a reduction in sound level or the ability to hear faint sounds. Treatment involves corrective medical or surgical procedures. Sensorineural hearing loss occurs when there is damage to the cochlea (inner ear), or to the nerve pathways from the cochlea to the brain. This type of hearing loss generally leads to permanent hearing loss. Mixed hearing loss is a combination of conductive hearing loss and sensorineural hearing loss in which damage occurs along both the outer and inner ear regions.

The degree or severity of hearing loss is categorized into seven groups ranging from normal, slight, mild, moderate, moderately severe, severe to profound. In addition, hearing loss is stratified based on frequency in some instances. For example, a hearing loss that only affects the high tones is referred to as a high frequency hearing loss, whereas that which affects the low tones is referred to as a low frequency hearing loss. In some cases, hearing loss affects both high and low frequencies.

Hearing loss is often accompanied by additional causes and symptoms such as ceruminosis, otitis externa, otalgia, tinnitus and vertigo. In some embodiments, it has been shown that ceruminosis can decrease hearing acuity by 40-45 dB. Such impairment, especially in the geriatric population can cause difficulties in communication and even physical immobility. The otic formulations disclosed herein are useful for the treatment of hearing loss.

*Hair Cell Regeneration*

Hair cells in the mammalian cochlea are important for hearing. The inner and outer hair cells in the Organ of Corti sense vibrations in cochlea fluid produced by sound and transduce these into auditory nerve responses that travel to the brain for sound to be perceived. Loss of hair cells has been implicated hearing loss caused by age, exposure to loud noise, ototoxic drugs, and genetic factors. In birds and amphibians, damage to hair cells triggers mechanisms that cause epithelial cells (supporting cells) in the cochlea to transdifferentiate into new hair cells and to divide and regenerate new supporting cells and hair cells to restore hearing. This ability to regenerate hair cells has been lost in mammals.

The otic formulations described herein are useful for the regeneration of otic hair cells.

*Vertigo*

Vertigo is described as a feeling of spinning or swaying while the body is stationary. There are two types of vertigo. Subjective vertigo is the false sensation of movement of the body. Objective vertigo is the perception that one's surrounding are in motion. It is often accompanied by nausea, vomiting, and difficulty maintaining balance. Otitis externa can induce vertigo.

Ménière's *Disease*

Ménière's Disease is an idiopathic condition characterized by sudden attacks of vertigo, nausea and vomiting that lasts for 3 to 24 hours, and subside gradually. Progressive hearing loss, tinnitus and a sensation of pressure in the ears accompanies the disease through time. The cause of Ménière's disease is likely related to an imbalance of auris interna fluid homeostasis, including an increase in production or a decrease in resorption of auris interna fluid.

The cause of symptoms associated with Ménière's disease is likely an imbalance of inner ear fluid homeostasis, including an increase in production or a decrease in reabsorption of inner ear fluid.

Recent studies of the vasopressin (VP)-mediated aquaporin 2 (AQP2) system in the auris interna suggest a role for VP in inducing endolymph production, thereby increasing pressure in the vestibular and cochlear structures). VP levels were found to be upregulated in endolymphatic hydrops (Ménière's Disease) cases, and chronic administration of VP in guinea pigs was found to induce endolymphatic hydrops. Treatment with VP antagonists, including infusion of OPC-31260 (a competitive antagonist of $V_2$-R) into the scala tympani resulted in a marked reduction of Ménière's disease symptoms. Other VP antagonists include WAY-140288, CL-385004, tolvaptan, conivaptan, SR 121463A and VPA 985. (Sanghi et al. Eur. Heart J. (2005) 26:538-543; Palm et al. Nephrol. Dial Transplant (1999) 14:2559-2562).

Other studies suggest a role for estrogen-related receptor β/NR3B2 (ERR/Nr3b2) in regulating endolymph production, and therefore pressure in the vestibular/cochlear apparatus. Knock-out studies in mice demonstrate the role of the protein product of the Nr3b2 gene in regulating endolymph fluid production. Nr3b2 expression has been localized in the endolymph-secreting strial marginal cells and vestibular dark cells of the cochlea and vestibular apparatus, respectively. Moreover, conditional knockout of the Nr3b2 gene results in deafness and diminished endolymphatic fluid volume. In some instances, treatment with antagonists to ERR/Nr3b2 assist in reducing endolymphatic volume, and thus alter pressure in the auris interna structures.

Other treatments are aimed at dealing with the immediate symptoms and prevention of recurrence. Low-sodium diets, avoidance of caffeine, alcohol, and tobacco have been advocated. Medications that temporarily relieve vertigo attacks include antihistamines (including meclizine (Antivert, Bonine, Dramamine, Driminate) and other antihistamines), and central nervous system agents, including barbiturates and/or benzodiazepines, including lorazepam or diazepam. Other examples of drugs that are useful in relieving symptoms include muscarinic antagonists, including scopolamine. Nausea and vomiting are relieved by suppositories containing antipsychotic agents, including the phenothiazine agent prochlorperazine (Compazine, Buccastem, Stemetil and Phenotil).

Surgical procedures have also been used to relieve symptoms of Ménière's disease, including destruction of vestibular function to relieve vertigo symptoms. These procedures aim to either reduce fluid pressure in the inner ear and/or to destroy inner ear balance function. An endolymphatic shunt procedure, which relieves fluid pressure, are placed in the inner ear to relieve symptoms of vestibular dysfunction. Severing of the vestibular nerve is also employed, which controls vertigo while preserving hearing.

Another approach to destruction of vestibular function for the treatment of severe Ménière's disease is intratympanic application of an agent that destroys sensory hair cell function in the vestibular system, thereby eradicating inner ear balance function. Various antimicrobial agents are used in the procedure, including aminoglycosides such as gentamicin and streptomycin. The agents are injected through the tympanic membrane using a small needle, a tympanostomy tube with or without a wick, or surgical catheters. Various dosing regimens are used to administer the antimicrobial agents, including a low dose method in which less of the agents are administered over longer periods of time (e.g., one month between injections), and high dose methods in which more of the agents are administered over a shorter time frame (e.g., every week). Although the high dose method is typically more effective, it is more risky, as it results in hearing loss in some cases.

Ménière's *Syndrome*

Ménière's Syndrome, which displays similar symptoms as Ménière's disease, is attributed as a secondary affliction to another disease process, e.g. thyroid disease or auris interna inflammation due to syphilis infection. Ménière's syndrome, thus, are secondary effects to various process that interfere with normal production or resorption of endolymph, including endocrine abnormalities, electrolyte imbalance, autoimmune dysfunction, medications, infections (e.g. parasitic infections), or hyperlipidemia. Treatment of patients afflicted with Ménière's Syndrome is similar to Ménière's Disease.

*Sensorineural Hearing Loss*

Sensorineural hearing loss is a type of hearing loss which results from defects (congenital and acquired) in the vestibulocochlear nerve (also known as cranial nerve VIII), or sensory cells of the inner ear. The majority of defects of the inner ear are defects of otic hair cells.

Aplasia of the cochlea, chromosomal defects, and congenital cholesteatoma are examples of congenital defects which result in sensorineural hearing loss. By way of non-limiting example, inflammatory diseases (e.g. suppurative labyrinthitis, meningitis, mumps, measles, viral syphilis, and autoimmune disorders), Ménière's Disease, exposure to ototoxic drugs (e.g. aminoglycosides, loop diuretics, antimetabolites, salicylates, and cisplatin), physical trauma, presbycusis, and acoustic trauma (prolonged exposure to sound in excess of 90 dB) all result in acquired sensorineural hearing loss.

If the defect resulting in sensorineural hearing loss is a defect in the auditory pathways, the sensorineural hearing loss is called central hearing loss. If the defect resulting in sensorineural hearing loss is a defect in the auditory pathways, the sensorineural hearing loss is called cortical deafness.

In some instances, sensorineural hearing loss occurs when the components of the auris interna or accompanying neural components are affected, and contain a neural, i.e. when the auditory nerve or auditory nerve pathways in the brain are affected, or sensory component. Sensory hearing loss are hereditary, or it are caused by acoustic trauma (i.e. very loud noises), a viral infection, drug-induced or Ménière's disease. In some instances, neural hearing loss occurs as a result of brain tumors, infections, or various brain and nerve disorders, such as stroke. Some hereditary diseases, such as Refsum disease (defective accumulation of branched fatty acids), also cause neural disorders affecting hearing loss. Auditory nerve pathways are damaged by demyelinating diseases, e.g. idiopathic inflammatory demyelinating disease (including

multiple sclerosis), transverse myelitis, Devic's disease, progressive multifocal leukoencephalopathy, Guillain-Barre syndrome, chronic inflammatory demyelinating polyneuropathy and anti-MAG peripheral neuropathy.

The incidence of sudden deafness, or sensorineural hearing loss, occurs in about 1 in 5000 individuals, and are caused by viral or bacterial infections, e.g. mumps, measles, influenza, chickenpox, cytomegalovirus, syphilis or infectious mononucleosis, or physical injury to the inner ear organ. In some cases, no cause is identified. In some cases, tinnitus and vertigo accompany sudden deafness, which subsides gradually. Oral corticosteroids are frequently prescribed to treat sensorineural hearing loss. In some cases, surgical intervention is necessary. Other treatments include AM-101 and AM-111, compounds under development for the treatment of auris interna tinnitus and acute sensorineural hearing loss. (Auris Medical AG, Basel, Switzerland).

*Noise induced hearing loss*

Noise induced hearing loss (NIHL) is caused upon exposure to sounds that are too loud or loud sounds that last a long time. In some instances, hearing loss occurs from prolonged exposure to loud noises, such as loud music, heavy equipment or machinery, airplanes, or gunfire. Long or repeated or impulse exposure to sounds at or above 85 decibels cause hearing loss in some cases. NIHL causes damage to the hair cells and/or the auditory nerve. The hair cells are small sensory cells that convert sound energy into electrical signals that travel to the brain. In some cases, impulse sound results in immediate hearing loss that is permanent. This kind of hearing loss are accompanied by tinnitus-a ringing, buzzing, or roaring in the ears or head-which subsides over time in some cases. Hearing loss and tinnitus are experienced in one or both ears, and tinnitus continue constantly or occasionally throughout a lifetime in some instances. Permanent damage to hearing loss is often diagnosed. Continuous exposure to loud noise also damages the structure of hair cells, resulting in hearing loss and tinnitus, although the process occurs more gradually than for impulse noise.

An otoprotectant reverses, reduces, or ameliorates NIHL. Examples of otoprotectants that treat or prevent NIHL include D-methionine, L-methionine, ethionine, hydroxyl methionine, methioninol, amifostine, mesna (sodium 2-sulfanylethanesulfonate), a mixture of D and L methionine, normethionine, homomethionine, S-adenosyl-L-methionine), diethyldithiocarbamate, ebselen (2-phenyl-1, 2-benzisoselenazol-3(2H)-one), sodium thiosulfate, AM-111 (a cell permeable JNK inhibitor, (Laboratoires Auris SAS)), leucovorin, leucovorin calcium, dexrazoxane, or combinations thereof.

*Presbycusis (Age Related Hearing Loss)*

Presbycusis (age related hearing loss (ARHL)) is the progressive bilateral loss of hearing that results from aging. Most hearing loss occurs at higher frequencies (i.e. frequencies above 15 or 16 Hz) making it difficult to hear a female voice (as opposed to male voice), and an inability to differentiate between high-pitched sounds (such as "s" and "th"). It is difficult to filter out background noise. The disorder is most often treated by the implantation of a hearing aid and/or the administration of pharmaceutical agents which prevent the buildup of ROS.

The disorder is caused by changes in the physiology of the inner ear, the middle ear, and/or the VIII nerve. Changes in the inner ear resulting in presbycusis include epithelial atrophy with loss of otic hair cells and/or stereocilia, atrophy of nerve cells, atrophy of the stria vascularis, and the thickening/stiffening of the basilar membrane. Additional changes which contribute to presbycusis include the accumulation of defects in the tympanic membrane and the ossicles.

In some instances, changes leading to presbycusis occur due to the accumulation of mutations in DNA, and mutations in mitochondrial DNA; however, the changes are exacerbated by exposure to loud noise, exposure to ototoxic agents, infections, and/or the lessening of blood flow to the ear. The latter is attributable to atherosclerosis, diabetes, hypertension, and smoking.

Presbycusis, or age-related hearing loss, occurs as a part of normal aging, and occurs as a result of degeneration of the receptor cells in the spiral Organ of Corti in the auris interna. Other causes are also attributed to a decrease in a number of nerve fibers in the vestibulocochlear nerve, as well as a loss of flexibility of the basilar membrane in the cochlea. Most commonly, it arises from changes in the inner ear as one ages, but it also results from changes in the middle ear, or from complex changes along the nerve pathways from the ear to the brain. Certain medical conditions and medications also play a role. In some instances, presbycusis results from a gradual loss of spiral ganglion neuron afferent fibers and their synapses with hair cells (ribbon synapses), causing a disconnection between the sensory cells that detect sound and the auditory nerve that transmits this information to the auditory brain. Loss of spiral ganglion neurons and hair cells also occurs. In some cases, prior exposure to loud noise or other otic insults exacerbates this ageing process, leading to an accelerated loss of hearing. Presbycusis also involves "hidden hearing loss", an inability to detect sound against a background noise ("speech-in-noise") despite a lack of marked changes in hearing thresholds. These more subtle decrements in hearing have been associated with a loss of spiral ganglion neuron afferent fibers and their synaptic connections with hair cells (ribbon synapses).

In some embodiments, the agents described herein repair ribbon synapses and restores hearing function. In some embodiments, the ribbon synapeses are damaged due to noise trauma or exposure.

*Autoimmune Inner Ear Disease*

Autoimmune inner ear disease (AIED) is one of the few reversible causes of sensorineural hearing loss. It is a rare disorder appearing in both adults and children that often involves a bilateral disturbance of the audio and vestibular functions of the auris interna. The origin of AIED is likely autoantibodies and/or immune cells attacking inner ear structures, but are associated with other autoimmune conditions. In many cases, AIED occurs without systemic autoimmune symptoms, but up to one-third of patients also suffer from a systemic autoimmune illness, such as inflammatory bowel disease, rheumatoid arthritis, Ankylosing spondylitis, Systemic Lupus Erythematosus (SLE), Sjõgren's Syndrome, Cogan's disease, ulcerative colitis, Wegener's granulomatosis and scleroderma. Behçet's disease, a multisystem disease, also commonly has audiovestibular problems. There is some evidence for food-related allergies as a cause for cochlear and vestibular autoimmunity, but there is presently no agreement as to its importance in the aetiology of the disease. A classification scheme for AIED has been developed (Harris and Keithley, (2002) Autoimmune inner ear disease, in Otorhinolaryngology Head and Neck Surgery. 91, 18-32).

The immune system normally performs a crucial role in protecting the auris interna from invasive pathogens such as bacteria and viruses. However, in AIED the immune system itself begins to damage the delicate auris interna tissues. It is well established that the auris interna is fully capable of mounting a localized immune response to foreign antigens. When a foreign antigen enters the auris interna, it is first processed by immunocompetent cells which reside in and around the endolymphatic sac. Once the foreign antigen has been processed by these immunocompetent cells, these cells secrete various cytokines which modulate the immune response of the auris interna. One result of this cytokine release is to facilitate the influx of inflammatory cells which are recruited from the systemic circulation. These systemic inflammatory cells enter the cochlea via diapedesis through the spiral modiolar vein and its tributaries and begin to participate in antigen uptake and deregulation just as it occurs in other parts of the body. Interleukin 1 (IL-1) plays an important role in modulating the innate (nonspecific) immune response and is a known activator of resting T helper cells and B-cells. T helper cells, once activated by IL-1, produce IL-2. IL-2 secretion results in differentiation of pluripotent T-cells into helper, cytotoxic and suppressor T-cell subtypes. IL-2 also assists T helper cells in the activation of B lymphocytes and probably plays a pivotal role in the immunoregulation of the immune response of the auris interna. IL-2 has been identified within the perilymph of the auris interna as early as 6 h after antigen challenge with peak levels at 18 h after antigen challenge. The perilymphatic levels of IL-2 then dissipate, and it is no longer present within the perilymph at 120 hours post antigen challenge (Gloddek, Acta Otolaryngol. (1989) 108, 68-75).

Both IL-1$\beta$ and tumor necrosis factor-$\alpha$ (TNF-$\alpha$) play a key role in the initiation and amplification of the immune response. IL-1$\beta$ is expressed by the fibrocytes of the spiral ligament in the presence of trauma such as surgical trauma or acoustic trauma in a nonspecific response. THF-$\alpha$ is expressed either by infiltrating systemic cells or by resident cells contained within the endolymphatic sac in the presence of antigen. THF-$\alpha$ is released as part of the adaptive (specific) immune response in animal models. When antigen is injected into the auris internas of mice, IL-1$\beta$ and TNF-$\alpha$ are both expressed and a vigorous immune response occurs. However, when antigen is introduced to the auris interna via the cerebral spinal fluid without trauma to the auris interna, only TNF-$\alpha$ is expressed and the immune response in minimal (Satoh, J. Assoc. Res. Otolaryngol. (2003), 4, 139-147). Importantly, cochlear trauma in isolation also results in a minimal immune response. These results suggest that both the nonspecific and specific components of the immune response act in concert in the auris interna to achieve a maximal response.

Accordingly, if the cochlea is traumatized and an antigen is injected (or in the case of autoimmune disease, the patient has immune cells directed against auris interna antigens), both the nonspecific and the specific immune responses are activated simultaneously. This results in the concurrent production of IL-1$\beta$ as well as THF-$\alpha$ which causes a greatly amplified level of inflammation leading to substantial damage to the auris interna. Subsequent experiments in animal models confirm that an important step in immune-mediated damage requires that the auris interna be conditioned by the non-specific innate immune response before the specific adaptive immune response leads to enough inflammation to result in damage. As a result, in some instances, agents which downregulate or block the specific immune responses prevent the excessive immune response seen when both the specific and nonspecific immune responses are simultaneously activated.

*Tinnitus*

Tinnitus is defined as the perception of sound in the absence of any external stimuli. In some cases, it occurs in one or both ears, continuously or sporadically, and is most often described as a ringing sound. It is most often used as a diagnostic symptom for other diseases. There are two types of tinnitus: objective and subjective. The former is a sound created in the body which is audible to anyone. The latter is audible only to the affected individual. Studies estimate that over 50 million Americans experience some form of tinnitus. Of those 50 million, about 12 million experience severe tinnitus.

In certain instances, tinnitus results from damage to otic structures (e.g. stereocilia), the dysfunction of one or

more molecular receptors, and/or the dysfunction of one or more neural pathways. In certain instances, tinnitus results from excitotoxicity caused by abnormal activity of an NMDA receptor. In certain instances, tinnitus results from by dysfunction of an $\alpha$9 and/or $\alpha$10 acetylcholine receptor. In certain instances, tinnitus results from damage to the vestibulocochlear nerve. In certain embodiments, a reduction in neurotransmitter reuptake (e.g. the increase in extracellular neurotransmitters) treats, and/or ameliorates the symptoms of tinnitus. In certain embodiments, antagonism of an NK1 receptor treats, and/or ameliorates the symptoms of tinnitus. In certain embodiments, a reduction in neurotransmitter reuptake and antagonism of an NK1 receptor treats, and/or ameliorates the symptoms of tinnitus.

There are several treatments for tinnitus. Lidocaine, administered by IV, reduces or eliminates the noise associated with tinnitus in about 60-80% of sufferers. Selective neurotransmitter reuptake inhibitors, such as nortriptyline, sertraline, and paroxetine, have also demonstrated efficacy against tinnitus. Benzodiazepines are also prescribed to treat tinnitus.

*Ototoxicity*

Ototoxicity refers to hearing loss caused by a toxin. The hearing loss are due to trauma to otic hair cells, the cochlea, and/or the cranial nerve VIII. Multiple drugs are known to be ototoxic. Often ototoxicity is dose-dependent. It is permanent or reversible upon withdrawal of the drug.

Known ototoxic drugs include the aminoglycoside class of antibiotics (e.g., gentamicin, and amikacin), some members of the macrolide class of antibiotics (e.g., erythromycin), some members of the glycopeptide class of antibiotics (e.g., vancomycin), salicylic acid, nicotine, some chemotherapeutic agents (e.g., actinomycin, bleomycin, cisplatin, carboplatin and vincristine), and some members of the loop diuretic family of drugs (e.g., furosemide).

Cisplatin and the aminoglycoside class of antibiotics induce the production of reactive oxygen species ("ROS"). ROS damages cells directly by damaging DNA, polypeptides, and/or lipids. Antioxidants prevent damage of ROS by preventing their formation or scavenging free radicals before they damage the cell. Both cisplatin and the aminoglycoside class of antibiotics are also thought to damage the ear by binding melanin in the stria vascularis of the inner ear.

Salicylic acid is classified as ototoxic as it inhibits the function of the polypeptide prestin. Prestin mediates outer otic hair cell motility by controlling the exchange of chloride and carbonate across the plasma membrane of outer otic hair cells. It is only found in the outer otic hair cells, not the inner otic hair cells. Accordingly, in some embodiments, the use of the controlled release auris-formulations described herein, ameliorates or lessens ototoxic effects of chemotherapy, including cisplatin treatment, aminoglycoside or salicylic acid administration, or other ototoxic agents.

*Excitotoxicity*

Excitotoxicity refers to the death or damaging of neurons and/or otic hair cells by glutamate and/or similar substances.

Glutamate is the most abundant excitatory neurotransmitter in the central nervous system. Pre-synaptic neurons release glutamate upon stimulation. It flows across the synapse, binds to receptors located on post-synaptic neurons, and activates these neurons. The glutamate receptors include the NMDA, AMPA, and kainate receptors. Glutamate transporters are tasked with removing extracellular glutamate from the synapse. Certain events (e.g. ischemia or stroke) damage the transporters. This results in excess glutamate accumulating in the synapse. Excess glutamate in synapses results in the over-activation of the glutamate receptors.

The AMPA receptor is activated by the binding of both glutamate and AMPA. Activation of certain isoforms of the AMPA receptor results in the opening of ion channels located in the plasma membrane of the neuron. When the channels open, $Na^+$ and $Ca^{2+}$ ions flow into the neuron and $K^+$ ions flow out of the neuron.

The NMDA receptor is activated by the binding of both glutamate or NMDA together with a co-agonist glycine or D-serine. Activation of the NMDA receptor, results in the opening of ion channels located in the plasma membrane of the neuron. However, these channels are blocked by $Mg^{2+}$ ions. Activation of the AMPA receptor results in the expulsion of $Mg^{2+}$ ions from the ion channels into the synapse. When the ion channels open, and the $Mg^{2+}$ ions evacuate the ion channels, $Na^+$ and $Ca^{2+}$ ions flow into the neuron, and $K^+$ ions flow out of the neuron.

Excitotoxicity occurs when the NMDA receptor and AMPA receptors are over-activated by the binding of excessive amounts of ligands, for example, abnormal amounts of glutamate. The over-activation of these receptors causes excessive opening of the ion channels under their control. This allows abnormally high levels of $Ca^{2+}$ and $Na^+$ to enter the neuron. The influx of these levels of $Ca^{2+}$ and $Na^+$ into the neuron causes the neuron to fire more often, resulting in a rapid buildup of free radicals and inflammatory compounds within the cell. The free radicals eventually damage the mitochondria, depleting the cell's energy stores. Furthermore, excess levels of $Ca^{2+}$ and $Na^+$ ions activate excess levels of enzymes including phospholipases, endonucleases, and proteases. The over-activation of these enzymes results in damage to the cytoskeleton, plasma membrane, mitochondria, and DNA of the sensory neuron.

*Endolymphatic Hydrops*

Endolymphatic hydrops refers to an increase in the hydraulic pressure within the endolymphatic system of the inner ear. The endolymph and perilymph are separated by thin membranes which contain multiple nerves. Fluctuation in the pressure stresses the membranes and the nerves they house. If the pressure is great enough, disruptions form in the membranes. This results in a mixing of the fluids which leads to a depolarization blockade and transient loss of function. Changes in the rate of vestibular nerve firing often lead to vertigo. Further, the Organ of Corti are affected in some cases. Distortions of the basilar membrane and the inner and outer hair cells leads to hearing loss and/or tinnitus.

Causes include metabolic disturbances, hormonal imbalances, autoimmune disease, and viral, bacterial, or fungal infections. Symptoms include hearing loss, vertigo, tinnitus, and aural fullness. Nystagmus is also present in some instances. Treatment includes systemic administration of benzodiazepine, diuretics (to decrease the fluid pressure), corticosteroids, and/or anti-bacterial, anti-viral, or anti-fungal agents.

*Labyrinthitis*

Labyrinthitis is an inflammation of the labyrinths of the ear which contain the vestibular system of the inner ear. Causes include bacterial, viral, and fungal infections. It is also caused by a head injury or allergies in some instances. Symptoms of labyrinthitis include difficulty maintaining balance, dizziness, vertigo, tinnitus, and hearing loss. In some cases, recovery takes one to six weeks; however, chronic symptoms are present for years.

There are several treatments for labyrinthitis. Prochlorperazine is often prescribed as an antiemetic. Serotonin-reuptake inhibitors have been shown to stimulate new neural growth within the inner ear. Additionally, treatment with antibiotics is prescribed if the cause is a bacterial infection, and treatment with corticosteroids and antivirals is recommended if the condition is caused by a viral infection.

*Ramsay Hunt's Syndrome (Herpes Zoster Infection)*

Ramsay Hunt's syndrome is caused by a herpes zoster infection of the auditory nerve. The infection causes severe ear pain, hearing loss, vertigo, as well as blisters on the outer ear, in the ear canal, as well as on the skin of the face or neck supplied by the nerves. In some cases, facial muscles also become paralyzed if the facial nerves are compressed by the swelling. Hearing loss are temporary or permanent, with vertigo symptoms usually lasting from several days to weeks.

Treatment of Ramsay Hunt's syndrome includes administration of antiviral agents, including acyclovir. Other antiviral agents include famciclovir and valacyclovir. Combination of antiviral and corticosteroid therapy are also employed to ameliorate herpes zoster infection. Analgesics or narcotics are also administered to relieve the pain, and diazempam or other central nervous system agents to suppress vertigo. Capsaicin, lidocaine patches and nerve blocks are also used. Surgery is also performed on compressed facial nerves to relieve facial paralysis.

*Vestibular Neuronitis*

Vestibular neuronitis, or vestibular neuropathy, is an acute, sustained dysfunction of the peripheral vestibular system. It is theorized that vestibular neuronitis is caused by a disruption of afferent neuronal input from one or both of the vestibular apparatuses. Sources of this disruption include viral infection, and acute localized ischemia of the vestibular nerve and/or labyrinth. Vestibular neuronitis is characterized by sudden vertigo attacks, which manifests as a single attack of vertigo, a series of attacks, or a persistent condition which diminishes over a matter of weeks. Symptoms typically include nausea, vomiting, and previous upper respiratory tract infections, although there are generally no auditory symptoms. The first attack of vertigo is usually severe, leading to nystagmus, a condition characterized by flickering of the eyes involuntarily toward the affected side. Hearing loss does not usually occur.

In some instances, vestibular neuronitis is caused by inflammation of the vestibular nerve, the nerve that connects the inner ear to the brain, and is likely caused by viral infection. Diagnosis of vestibular neuronitis usually involves tests for nystagmus using electronystagmography, a method of electronically recording eye movements. Magnetic resonance imaging is also performed to determine if other causes play a role in the vertigo symptoms.

Treatment of vestibular neuronitis typically involves alleviating the symptoms of the condition, primarily vertigo, until the condition clears on its own. Treatment of vertigo is often identical to Ménière's disease, and optionally includes meclizine, lorazepam, prochlorperazine, or scopolamine. Fluids and electrolytes are intravenously administered if the vomiting is severe. Corticosteroids, such as prednisolone, are also given if the condition is detected early enough.

In some instances, the formulations disclosed herein are administered for the treatment of vestibular neuronitis. Further, the formulations are administered with other agents that are typically used to treat symptoms of the condition, including anticholinergics, antihistamines, benzodiazepines, or steroids. Treatment of vertigo is identical to Ménière's disease, and include meclizine, lorazepam, prochlorperazine or scopolamine. Fluids and electrolytes are also intravenously administered if the vomiting is severe.

The most significant finding when diagnosing vestibular neuronitis is spontaneous, unidirectional, horizontal nystagmus. It is often accompanied by nausea, vomiting, and vertigo. It is, generally, not accompanied by hearing loss or other auditory symptoms.

There are several treatments for vestibular neuronitis. H1-receptor antagonists, such as dimenhydrinate, diphenhydramine, meclizine, and promethazine, diminish vestibular stimulation and depress labyrinthine function through anticholinergic effects. Benzodiazepines, such as diazepam and lorazepam, are also used to inhibit vestibular responses due to their effects on the $GABA_A$ receptor. Anticholinergics, for example scopolamine, are also prescribed. They function by suppressing conduction in the vestibular cerebellar pathways. Finally, corticosteroids (i.e. prednisone) are prescribed to ameliorate the inflammation of the vestibular nerve and associated apparatus.

*Microvascular compression syndrome*

Microvascular compression syndrome (MCS), also called "vascular compression" or "neurovascular compression", is a disorder characterized by vertigo and tinnitus. It is caused by the irritation of Cranial Nerve VIII by a blood vessel. Other symptoms found in subjects with MCS include severe motion intolerance, and neuralgic like "quick spins". MCS is treated with carbamazepine, TRILEPTAL®, and baclofen. It is also surgically treated for some cases.

*Auditory Nerve Tumors*

Auditory nerve tumors, including acoustic neuroma, acoustic neurinoma, vestibular schwannoma and eighth nerve tumor) are tumors that originate in Schwann cells, cells that wrap around a nerve. Auditory nerve tumors account for approximately 7-8% of all tumors originating in the skull, and are often associated with the diagnosis of neurofibromatosis in a patient. Depending upon the location of the tumor, some symptoms include hearing loss, tinnitus, dizziness and loss of balance. In some cases, other more serious symptoms develop as the tumor becomes larger, which compresses against the facial or trigeminal nerve, which affect connections between the brain and the mouth, eye or jaw. Smaller tumors are removed by microsurgery, or stereotactic radiosurgical techniques, including fractionated stereotactic radiotherapy. Malignant Schwannomas are treated with chemotherapeutic agents, including vincristine, adriamycin, cyclophosphamide and imidazole carboxamide.

*Auditory Neuropathy*

Auditory neuropathy (AN) is also known as auditory neuropathy/auditory dys-synchrony (AN/AD) or auditory neuropathy spectrum disorder (ANSD). Auditory neuropathy describes hearing loss in which the outer hair cells within the cochlea are present and functional, but auditory information is not properly transmitted to the auditory nerve and brain.

*Benign Paroxysmal Positional Vertigo*

Benign paroxysmal positional vertigo is caused by the movement of free floating calcium carbonate crystals (otoliths) from the utricle to one of the semicircular canals, most often the posterior semicircular canal. Movement of the head results in the movement of the otoliths causing abnormal endolymph displacement and a resultant sensation of vertigo. The episodes of vertigo usually last for about a minute and are rarely accompanied by other auditory symptoms.

*Cancer of the Ear*

Although the cause is unknown, cancer of the ear is often associated with long-term and untreated otitis, suggesting a link between chronic inflammation and development of the cancer, at least in some cases. In some instances, tumors in the ear are benign or malignant, and they exist in the external, middle, or inner ear. Symptoms of ear cancer include otorrhea, otalgia, hearing loss, facial palsy, tinnitus, and vertigo. Treatment options are limited, and include surgery, radiotherapy, chemotherapy, and combinations thereof. Also, additional pharmaceutical agents are used to treat symptoms or conditions associated with the cancer, including corticosteroids in the case of facial palsy, and antimicrobial agents when otitis is present.

Systemic administration of conventional cytotoxic agents have been used to treat cancer of the ear, including systemic administration of cyclophosphamide (in CHOP chemotherapy) in combination with radiotherapy and methotrexate, and perfusion of methotrexate through the external carotid artery. However, such treatments requiring systemic administration of the active agents suffer from the same drawbacks discussed herein. Namely, relatively high doses of the agents are required to achieve the necessary therapeutic doses in the ear, which result in an increase of undesired, adverse side effects. Accordingly, in some embodiments, the local administration of the active agents in the formulations

disclosed herein results in treatment of cancer of the ear with lower effective doses, and with a decrease in the incidence and/or severity of side effects. Typical side effects of systemic administration of cytotoxic agents, e.g., methotrexate, cyclophosphamide, and thalidomide, for the treatment of cancer of the ear include anemia, neutropenia, bruising, nausea, dermatitis, hepatitis, pulmonary fibrosis, teratogenicity, peripheral neuropathy, fatigue, constipation, deep vein thrombosis, pulmonary edema, atelectasis, aspiration pneumonia, hypotension, bone marrow suppression, diarrhea, darkening of skin and nails, alopecia, changes in hair color and texture, lethargy, hemorrhagic cystitis, carcinoma, mouth sores, and decreased immunity.

In some instances, the compounds have anti-inflammatory properties and are used in the formulations disclosed herein for the treatment of inflammatory disorders of the ear, including AIED.

Although systemic administration of methotrexate, cyclophosphamide, and thalidomide is currently used to treat or is being investigated for the treatment of otic disorders, such as inflammatory otic disorders, including AIED, Ménière's disease, and Behçet's disease, as well as cancer of the ear, the cytotoxic agents are not without the potential for serious adverse side effects. Moreover, cytotoxic agents, which demonstrate efficacy but are otherwise not approvable because of safety considerations, are also contemplated within the embodiments disclosed herein. The localized application of the active agents to the target otic structures for treatment of autoimmune and/or inflammatory disorders, as well as cancer of the ear, results in the reduction or elimination of adverse side effects experienced with systemic treatment. The localized treatment with the active agents contemplated herein reduce the amount of agent needed for effective treatment of the targeted disorder due, for example, to increased retention of the active agents in the auris interna and/or media, to the existence of the biological blood barrier in the auris interna, or to the lack of sufficient systemic access to the auris media.

In some instances, active agents used in the formulations disclosed herein are metabolites, salts, polymorphs, prodrugs, analogues, and derivatives of active agents. In some instances, the active agents are metabolites, salts, polymorphs, prodrugs, analogues, and derivatives of active agents that retain at least partially the cytotoxicity and anti-inflammatory properties of the parent compounds.

*Central Auditory Processing Disorder*

Central auditory processing disorder (CAPD), also referred as auditory processing disorder (APD), is a general term for describing a variety of disorders that affect the way the brain processes auditory information. Individuals with CAPD usually have normal structure and function of the outer, middle and inner ear (peripheral hearing). However, these individuals are unable to process the auditory information, which leads to difficulties in recognizing and interpreting sounds, especially the sounds from speech. Central auditory processing disorder is either developmental or acquired and is believed to arise from dysfunction in the central nervous system.

*Cholesteatoma*

A cholesteatoma is a hyperproliferative cyst often found in the middle ear. Cholesteatoma are classified as congenital or acquired. Acquired cholesteatomas result from retraction of the ear drum (primary) and/or a tear in the ear drum (secondary).

The most common primary cholesteatoma results from the pars flaccida retracting into the epitympanum. As the pars flaccida continues to retract, the lateral wall of the epitympanum slowly erodes. This produces a defect in the lateral wall of the epitympanum that slowly expands. A less common type of primary acquired cholesteatoma results from the retraction of the posterior quadrant of the tympanic membrane retracts into the posterior middle ear. As the tympanic membrane retracts, squamous epithelium envelops the stapes and retracts into the sinus tympani. Secondary cholesteatomas result from injury to the tympanic membrane (e.g. a perforation resulting from otitis media; trauma; or a surgically-induced injury).

Complications associated with a growing cholesteatoma include injury to the osteoclasts and, in some cases, deterioration of the thin bone layer separating the top of the ear from the brain. Damage to the osteoclasts results from the persistent application of pressure to the bones resulting from the expansion of the cholesteatoma. Additionally, the presence of multiple cytokines (e.g. TNF-$\alpha$, TGF-$\beta$1, TGF-$\beta$2, Il-1, and IL-6) in the epithelium of the cholesteatoma results in further degradation of the surrounding bones.

Patients with a cholesteatoma often present with earache, hearing loss, mucopurulent discharge, and/or dizziness. Physical examination confirms the presence of a cholesteatoma. Symptoms that are identified upon physical examination include damage to the ossicles, and a canal filled with mucopus and granulation tissue.

There is currently no effective medical therapy for cholesteatomas. As a cholesteatoma has no blood supply, it cannot be treated with systemic antibiotics. Topical administration of antibiotics often fails to treat a cholesteatoma.

*Drug-Induced Inner Ear Damage*

Damage to all or a portion of the ear (e.g., inner ear) may be caused by the administration of drugs, including certain antibiotics, diuretics (e.g. ethacrynic acid and furosemide), aspirin, aspirin-like substances (e.g. salicylates) and quinine. Deterioration of the auris interna organ are hastened by impaired kidney function, which results in decreased clearance of the affecting drugs and their metabolites. In some instances, these drugs affect both hearing and equilibrium; however, these drugs likely affect hearing to a greater extent.

For example, neomycin, kanamycin, amikacin have a greater effect on hearing than on balance. The antibiotics viomycin, gentamicin and tobramycin affect both hearing and equilibrium. Streptomycin, another commonly administered antibiotic, induces vertigo more than loss of hearing, and leads to Dandy's syndrome, where walking in the dark becomes difficult and induces a sensation of the environment moving with each step. Aspirin, when taken in very high doses, also leads to temporary hearing loss and tinnitus, a condition where sound is perceived in the absence of external sound. Similarly, quinine, ethacryinic acid and furosemide result in temporary or permanent hearing loss in some instances.

*Hereditary Disorders*

Hereditary disorders, including Scheibe, Mondini-Michelle, Waardenburg, Michel, Alexander's ear deformity, hypertelorism, Jervell-Lange Nielson, Refsum and Usher syndromes, are found in approximately 20% of patients with sensorineural hearing loss. In some instances, congenital ear malformations result from defects in the development of the membranous labyrinthine, the osseous labyrinthine, or both. Along with profound hearing loss and vestibular function abnormalities, hereditary deformities are associated with other dysfunctions, including development of recurring meningitis, cerebral spinal fluid (CSF) leaks, as well as perilymphatic fistulas. Treatment of chronic infections is necessitated in hereditary disorder patients.

*Inflammatory Disorders of the Auris Media*

Otitis media (OM), which includes acute otitis media (AOM), otitis media with effusion (OME) and chronic otitis media as examples, is a condition affecting both adults and children. OM susceptibility is multifactorial and complex, including environmental, microbial and host factors. Bacterial infection accounts for a large percentage of OM cases, with more than 40% of cases attributed to *Streptococcus pneumoniae* infection. However, viral causes, as well as other microbial agents, also account for OM conditions in some cases.

Regardless of the causative agent, increases in cytokine production, including interleukins and TNF, have been observed in the effluent media of individuals afflicted with OM. IL-1$\beta$, IL-6 and TNF-$\alpha$ are acute-phase cytokines that promote acute inflammatory response after infection with viruses and bacteria. Genetic studies supports this link between cytokines and OM by demonstrating a correlation in the occurrence of TNF-$\alpha$ SNP (single-nucleotide polymorphisms) and an increased susceptibility for OM in pediatric patients suffering from AOM and with a subsequent need for placement of tympanostomy tubes. In animal models of OM induced with pneumococci inoculations, TNF-$\alpha$ and interleukins levels were found to increase in early developmental phase of OM, with TNF-$\alpha$ levels steadily increasing 72 hours after inoculation. Moreover, higher TNF-$\alpha$ levels have been associated with a history of multiple tympanostomy tube placements, indicating a role for TNF-$\alpha$ in chronic OM cases. Finally, direct injection of TNF-$\alpha$ and interleukins has been shown to induce middle ear inflammation in a guinea pig model. These studies support the role that cytokines play in the origin and maintenance of OM in the auris media.

In some instances, because OM is caused by a virus, bacteria or both, it is often difficult to identify the exact cause and thus the most appropriate treatment. Treatment options of OM in the auris media include treatment with antibiotics, such as amoxicillin, clavulanate acid, trimethoprim-sulfamethoxazole, cefuroxime, clarithromycin and azithromycin and other cephalosporins, macrolides, penicillins or sulfonamides. Surgical intervention is also available, including a myringotomy, an operation to insert a tympanostomy tube through the tympanic membrane and into the patient's middle ear to drain the fluid and balance the pressure between the outer and middle ear. In some instances, antipyretics and analgesics, including benzocaine, ibuprofen and acetaminophen, are also prescribed to treat accompanying fever or pain effects. Pre-treatment with TNF-$\alpha$ inhibitors in experimental lipopolysaccharide (LPS)-induced OM animal models has been shown to suppress development of OM, suggesting a role in the treatment of OM or OME. In addition, treatment of such conditions include use of TNF-$\alpha$ inhibitors in combination with other inflammatory response mediators, including platelet activating factor antagonists, nitric oxide synthase inhibitors, and histamine antagonists.

In addition, other otic disorders have inflammatory response aspects or are tangentially related to autoimmune conditions, including Ménière's disease and non-sudden hearing loss or noise induced hearing loss. These disorders are also explicitly contemplated as benefiting from the otic formulations disclosed herein, and therefore are within the scope of the embodiments disclosed.

*Hyperacusis*

Hyperacusis is a condition characterized by an increased sensitivity to certain frequency and volume ranges of sound (a collapsed tolerance to usual environmental sound). In some cases, hyperacusis occur gradually and in other cases, hyperacusis appears suddenly. A person with severe hyperacusis has difficulty tolerating everyday sounds, wherein some of these sounds seem unpleasantly or painfully loud to the afflicted person but not to others.

*Inflammatory Disorders of the Auris externa*

Otitis externa (OE), also referred to as swimmer's ear, is an inflammation and/or infection of the external ear. OE is often caused by bacteria in the outer ear, which establish infection following damage to the skin of the ear canal. Primary bacterial pathogens that cause OE are *Pseudomonas* aeruginosa and *Staphylococcus aureus,* but the condition is associated with the presence of many other strains of gram positive and negative bacteria. OE is also sometimes caused by fungal infection in the outer ear, including *Candida albicans* and *Aspergillus.* Symptoms of OE include otalgia, swelling, and otorrhea. If the condition progresses significantly, OE causes temporary conductive hearing loss as a result of the swelling and discharge.

Treatment of OE involves eliminating the aggravating pathogen from the ear canal and reducing inflammation, which is usually accomplished by administering combinations of antimicrobial agents, e.g., antibacterial and antifungal agents, with anti-inflammatory agents, e.g., steroids. Typical antibacterial agents for the treatment of OE include aminoglycosides (e.g., neomycin, gentamycin, and tobramycin), polymyxins (e.g., polymyxin B), fluoroquinolone (e.g., ofloxacin and ciprofloxacin), cephalosporins (e.g., cefuroxime, cefaclor, cefprozil, loracarbef, cefindir, cefixime, cefpodoxime proxetil, ceftibuten, and ceftriaxone), penicillins (e.g., amoxicillin, amoxicillin-clavulanate, and penicillinase-resistant penicillins), and combinations thereof. Typical antifungal agents for the treatment of OE include clotrimazole, thimerosal, M-cresyl acetate, tolnaftate, itraconazole, and combinations thereof. Acetic acid is also administered to the ear, alone and in combination with other agents, to treat bacterial and fungal infections. Ear drops are often used as the vehicle for administration of the active agents. In the case that ear swelling has progressed substantially and ear drops do not penetrate significantly into the ear canal, a wick is inserted into the ear canal to facilitate penetration of the treatment solutions. Oral antibiotics are also administered in the case of extensive soft tissue swelling that extends to the face and neck. When the pain of OE is extremely severe such that it interferes with normal activity, e.g., sleeping, pain relievers such as topical analgesics or oral narcotics are given until the underlying inflammation and infection are alleviated.

Notably, some types of topical ear drops, such as ear drops containing neomycin, are safe and effective for use in the ear canal, but are irritating and even ototoxic to the auris media, prompting concern that such topical preparations should not be used unless the tympanic membrane is known to be intact. In some instances, the utilization of the formulations disclosed herein for the treatment of OE allows for use of active agents that are potentially damaging to the auris media, even when the tympanic membrane is not intact. Specifically, the controlled release formulations disclosed herein, in some instances, is applied locally in the external ear with improved retention time, thus eliminating concern that the active agents will leak out of the ear canal into the auris media. Furthermore, otoprotectants are optionally added when ototoxic agents, such as neomycin, are used.

In some instances, treatment of severe OE with the formulations disclosed herein, such as highly viscous and/or mucoadhesive formulations, also obviates the need for extended use of an ear wick. Specifically, in some instances, the formulations disclosed herein have increased retention time in the ear canal as a result of the formulation technology, thus eliminating the need for a device to maintain their presence in the outer ear. In some embodiments, the formulations are applied in the outer ear with a needle or an ear dropper, and the active agents are maintained at the site of inflammation without the aid of an ear wick.

In some instances, the treatment of OE with the otic formulations disclosed herein encompasses the treatment of granular myringitis, a specific form of OE characterized by chronic inflammation of the pars tensa of the tympanic membrane. The outer epithelial and underlying fibrous layers of the tympanic membrane are replaced by a proliferating granulation tissue. The predominant symptom is foul-smelling otorrhea. A variety of bacteria and fungi cause the condition, including *Proteus* and *Pseudomonas* species. Accordingly, the otic formulations disclosed herein are useful for the treatment of granular myringitis.

In some instances, the treatment of OE with otic formulations disclosed herein encompasses the treatment of chronic stenosing otitis externa. Chronic stenosing otitis externa is characterized by repeated infections, typically caused by bacteria or fungi. The primary symptoms are pruritus in the ear canal, otorrhea, and chronic swelling. Otic agent formulations disclosed herein are useful for the treatment of chronic stenosing otitis externa.

In some instances, the treatment of OE with the otic formulations disclosed herein encompasses the treatment of malignant or necrotizing external otitis, an infection involving the temporal and adjacent bones. Malignant external otitis is typically a complication of external otitis. It occurs primarily in persons with compromised immunity, especially in older persons with diabetes mellitus. Malignant external otitis is often caused by the bacteria *Pseudomonas aeruginosa.*

Treatment typically involves correction of immunosuppression when possible, in conjunction with antibacterial therapy and pain relievers. Accordingly, , the otic formulations disclosed herein are useful for the treatment of malignant or necrotizing external otitis.

Otitis media (OM), which includes acute otitis media (AOM), chronic otitis media, otitis media with effusion (OME), secretory otitis media, and chronic secretory otitis media as examples, is a condition affecting both adults and children. OM susceptibility is multifactorial and complex, including environmental, microbial and host factors. Bacterial infection accounts for a large percentage of OM cases, with more than 40% of cases attributed to *Streptococcus pneumoniae* infection. However, viruses, as well as other microbes, also account for OM conditions in some cases.

In some instances, because OM is caused by a virus, bacteria or both, it is often difficult to identify the exact cause and thus the most appropriate treatment. Treatment options for OM include antibiotics, such as penicillins (e.g., amoxicillin and amoxicillin-clavulanate), clavulanate acid, trimethoprim-sulfamethoxazole, cephalosporins (e.g., cefuroxime, cefaclor, cefprozil, loracarbef, cefindir, cefixime, cefpodoxime proxetil, ceftibuten, and ceftriaxone), macrolides and azalides (e.g., erythromycin, clarithromycin, and azithromycin), sulfonamides, and combinations thereof. Surgical intervention is also available, including myringotomy, an operation to insert a tympanostomy tube through the tympanic membrane and into the patient's middle ear to drain the fluid and balance the pressure between the outer and middle ear. Antipyretics and analgesics, including benzocaine, ibuprofen and acetaminophen, are also prescribed to treat accompanying fever or pain effects.

Regardless of the causative agent, increases in cytokine production, including interleukins and TNF, have been observed in the effluent media of individuals afflicted with OM. IL-1$\beta$, IL-6 and TNF-$\alpha$ are acute-phase cytokines that promote acute inflammatory response after infection with viruses and bacteria. Moreover, higher TNF-$\alpha$ levels have been associated with a history of multiple tympanostomy tube placements, indicating a role for TNF-$\alpha$ in chronic OM cases. Finally, direct injection of TNF-$\alpha$ and interleukins has been shown to induce middle ear inflammation in a guinea pig model. These studies support the role that cytokines play in the origin and maintenance of OM in the auris media. Thus, treatment of OM includes the use of antimicrobial agents in conjunction with anti-inflammatory agents to eliminate the pathogen and treat the symptoms of inflammation. Such treatments include use of steroids, TNF-$\alpha$ inhibitors, platelet activating factor antagonists, nitric oxide synthase inhibitors, histamine antagonists, and combinations thereof.

Mastoiditis is an infection of the mastoid process, which is the portion of the temporal bone behind the ear. It is typically caused by untreated acute otitis media. Mastoiditis is acute or chronic. Symptoms include pain, swelling, and tenderness in the mastoid region, as well as otalgia, erythematous, and otorrhea. Mastoiditis typically occurs as bacteria spread from the middle ear to the mastoid air cells, where the inflammation causes damage to the bony structures. The most common bacterial pathogens are *Streptococcus pneumoniae, Streptococcus pyogenes, Staphylococcus aureus,* and gram-negative bacilli. Accordingly, the otic agent formulations disclosed herein comprising are effective against the bacteria are useful for the treatment of mastoiditis, including acute mastoiditis and chronic mastoiditis.

Bullous myringitis is an infection of the tympanic membrane, caused by a variety of bacteria and viruses, including *Mycoplasma* bacteria. The infection leads to inflammation of the tympanic membrane and nearby canal, and causes the formation of blisters on the ear drum. The primary symptom of Bullous myringitis is pain, which are relieved through the administration of analgesics. In some instances, the antimicrobial formulations disclosed herein comprising antibacterial and antiviral agents are useful for the treatment of Bullous myringitis.

Eustachian tubal catarrh, or Eustachian salpingitis, is caused from inflammation and swelling of the Eustachian tubes, resulting in a build-up of catarrh. In some instances, the antimicrobial formulations disclosed herein are useful for the treatment of Eustachian salpingitis.

Labyrinthitis, e.g., serous labyrinthitis, is an inflammation of the inner ear that involves one or more labyrinths housing the vestibular system. The primary symptom is vertigo, but the condition is also characterized by hearing loss, tinnitus, and nystagmus. Labyrinthitis maybe acute, lasting for one to six weeks and being accompanied by severe vertigo and vomiting, or chronic, with symptoms lasting for months or even years. Labyrinthitis is typically caused by viral or bacterial infection. In some instances, the antimicrobial formulations disclosed herein comprising antibacterial and antiviral agents are useful for the treatment of labyrinthitis.

Facial nerve neuritis is a form of neuritis, an inflammation of the peripheral nervous system, afflicting the facial nerve. The primary symptoms of the condition are a tingling and burning sensation, and stabbing pains in the affected nerves. In severe cases, there are numbness, loss of sensation, and paralysis of the nearby muscles. The condition is typically caused by herpes zoster or herpes simplex viral infection, but has also been associated with bacterial infection, e.g., leprosy. In some instances, the antimicrobial formulations disclosed herein comprising antibacterial and antiviral agents are useful for the treatment of facial nerve neuritis.

In some instances, antimicrobial formulations disclosed herein are also useful for the treatment of temporal bone osteoradionecrosis.

*Kinetosis*

Kinetosis, also known as motion sickness, is a condition in which there is a disconnection between visually perceived movement and the vestibular system's sense of movement. Dizziness, fatigue, and nausea are the most common symptoms of kinetosis. Dimenhydrinate, cinnarizine, and meclizine are all systemic treatments for kinetosis. Additionally, benzodiazepines and antihistamines have demonstrated efficacy in treating kinetosis.

*Mal de Debarquement*

Mal de debarquement is a condition which usually occurs subsequent to a sustained motion event, for example, a cruise, car trip, or airplane ride. It is characterized by a persistent sense of motion, difficulty maintaining balance, fatigue, and cognitive impairment. Symptoms also include dizziness, headaches, hyperacusis, and/or tinnitus. Symptoms often last in excess of a month. Treatment includes benzodiazepines, diuretics, sodium channel blockers, and tricyclic antidepressants.

*Other Microbial Infections Causing Cochleovestibular Disorders*

Other microbial infections are known to cause cochleovestibular disorders, including hearing loss. Such infections include rubella, cytomegalovirus, mononucleosis, varicella zoster (chicken pox), pneumonia, *Borrelia* species of bacteria (Lyme disease), and certain fungal infections. Accordingly, the controlled release antimicrobial agent formulations disclosed herein are also used for localized treatment of these infections in the ear.

*Otic Disorders caused by Free Radicals*

Free radicals are highly reactive atoms, molecules, or ions the reactivity of which results from the presence of unpaired electrons. Reactive oxygen species ("ROS") form as a result of sequential reduction of molecular oxygen. Examples of reactive oxygen species of interest ("ROS") include superoxide, hydrogen peroxide, and hydroxyl radicals. ROS are naturally produced as a by-product of the production of ATP. In some cases, ROS also results from the use of cisplatin, and aminoglycosides. Further, stress to stereocilia caused by acoustic trauma results in otic hair cells producing ROS.

In some instances, ROS damages cells directly by damaging nuclear DNA and mitochondrial DNA. Damage to the former leads to mutations which impair the functioning of auditory cells and/or apoptosis. Damage to the latter often results in decreased energy production and increased ROS production both of which leads to impaired cellular functioning or apoptosis. Further, ROS also damages or kills cells by oxidizing the polydesaturated fatty acids which comprise lipids, oxidizing the amino acids which comprise proteins, and oxidizing co-factors necessary for the activity of enzymes. Antioxidants ameliorate damage by caused by ROS by preventing their formation, or scavenging the ROS before they damage the cell.

Damage to mitochondria by ROS is often seen in hearing loss, especially hearing loss due to aging. The loss of ATP correlates to a loss in neural functioning in the inner ear. It also leads to physiological changes in the inner ear. Further, damage to mitochondria often results in an increased rate of cellular degradation and apoptosis of inner ear cells. The cells of the stria vascularis are the most metabolically active due to the vast energy requirements needed to maintain the ionic balance of fluids in the inner ear. Thus, the cells of the stria vascularis are most often damaged or killed due to damage of the mitochondria.

*Otosclerosis*

Otosclerosis is an abnormal growth of bone in the middle ear, which prevents structures within the ear from transducing vibration, which causes hearing loss. Otosclerosis usually affects the ossicles, in particular the stapes, which rests in the entrance to the cochlea in the oval window. The abnormal bone growth fixates the stapes onto the oval window, preventing sound passing waves from traveling to the cochlea. Otosclerosis causes a sensorineural hearing loss, i.e. damaged nerve fibers or hearing hair cells, or conductive hearing loss.

In some cases, treatment of otosclerosis includes surgery to remove the fixated stapes bone, called a stapedectomy. In some cases, fluoride treatment is also be used, which will not reverse the hearing loss but slows the development of otosclerosis.

*Presbystasis*

Presbystasis, also known as disequilibrium of aging, is a disorder wherein affected individuals have generalized

imbalance, but without spinning vertigo. The generalized imbalance is typically noticed during walking.

*Postural Vertigo*

Postural vertigo, otherwise known as positional vertigo, is characterized by sudden violent vertigo that is triggered by certain head positions. This condition is caused by damaged semicircular canals caused by physical injury to the auris interna, otitis media, ear surgery, or blockage of the artery to the auris interna.

Vertigo onset in patients with postural vertigo usually develops when a person lies on one ear or tilts the head back to look up. Vertigo is accompanied by nystagmus. In severe cases of postural vertigo, the vestibular nerve is severed to the affected semicircular canal. Treatment of vertigo is often identical to Ménière's disease, and includes meclizine, lorazepam, prochlorperazine or scopolamine. Fluids and electrolytes are intravenously administered if the vomiting is severe.

*Recurrent Vestibulopathy*

Recurrent vestibulopathy is a condition wherein the subject experiences multiple episodes of severe vertigo. The episodes of vertigo last for minutes or hours. Unlike Ménière's Disease, it is not accompanied by hearing loss. In some cases it develops into Ménière's Disease or Benign Paroxysmal Positional Vertigo. Treatment is similar to that of Ménière's Disease.

*Syphilis Infection*

Syphilis infection also leads to congenital prenatal hearing loss, affecting approximately 11.2 per 100,000 live births in the United States, as well as sudden hearing loss in adults. Syphilis is a venereal disease, caused by the spirochete Treponema pallidum, which in its secondary and tertiary stages results in auditory and vestibular disorders due to membranous labyrinthitis, and secondarily include meningitis.

Both acquired and congenital syphilis cause otic disorders. Symptoms of cochleovestibular disorders resulting from syphilis are often similar to those of other otic disorders, such as AIED and Ménière's disease, and include tinnitus, deafness, vertigo, malaise, sore throat, headaches, and skin rashes. In some instances, syphilis infection leads to congenital prenatal hearing loss, affecting approximately 11.2 per 100,000 live births in the United States, as well as sudden hearing loss in adults.

Treatment with steroids and antibiotics, including penicillins (e.g. benzathine penicillin G (BICILLIN LA®), is effective in eradicating the spirochete organism. However, Treponema remains in the cochlear and vestibular endolymph even after eradication from other sites in the body. Accordingly, long term treatment with penicillins is warranted to achieve eradication of the spirochete organism from the endolymph fluid.

Treatment of otosyphilis (syphilis presenting otic symptoms) typically includes a combination of steroids (e.g., prednisolone) and antibacterial agents (e.g., benzathine penicillin G (BICILLIN LA®), penicillin G procaine, doxycycline, tetracycline, ceftriaxone, azithromycin). Such treatments are effective in eradicating the spirochete organism. However, *Treponema* remains in the cochlear and vestibular endolymph even after eradication from other sites in the body. Accordingly, long term treatment with a penicillins is required to achieve complete eradication of the spirochete organism from the endolymph fluid. Also, in the case of a severe or advanced case of syphilis, a uricosuric drug, such as probenecid, are administered in conjunction with the antibacterial agent to increase its efficacy.

*Temporal Bone Fractures*

The temporal bone, which contains part of the ear canal, the middle ear and the auris interna, is subject to fractures from blows to the skull or other injuries. Bleeding from the ear or patchy bruising is symptomatic of a fracture to the temporal bone, and requires a computed tomography (CT) scan for accurate diagnosis. Temporal bone fractures rupture the eardrum, causing facial paralysis and sensorineural hearing loss.

Treatment of detected temporal bone fractures includes an antibiotic regimen to prevent meningitis, or an infection of brain tissue. In addition, surgery is performed to relieve any subsequent pressure on the facial nerve due to swelling or infection.

*Temporomandibular Joint Disease*

Some evidence exists for a relationship between temporomandibular joint disease (TMD) and auris interna disorders. Anatomical studies demonstrate the possible involvement of the trigeminal nerve, where trigeminal innervation of the vascular system has been shown to control cochlear and vestibular labyrinth function. Additionally, in some cases,

projections of ophthalmic fibers of the trigeminal Gasser ganglion to the cochlea through the basilar and anterior inferior cerebellar arteries play an important role in the vascular tone in quick vasodilatory response to metabolic stresses, e.g. intense noise. Auris interna diseases and symptoms, such as sudden hearing loss, vertigo and tinnitus, originate from reduction of the cochlear blood flow due to the presence of abnormal activity in the trigeminal ganglion, for example from migraine or by the central excitatory effect originated in chronic or deep pain produced by TMD.

Similarly, other researchers have found that the trigeminal ganglion also innervates the ventral cochlear nucleus and the superior olivary complex, which interfere with auditory pathways leading to the auditory cortex where constant peripheral somatic signals from the ophthalmic and mandibular trigeminal peripheral innervation occurs in TMD cases. These somatosensory and auditory system interactions via the central nervous system explain otic symptoms in the absence of existing disease in the ear, nose, or throat.

Accordingly, forceful muscle contractions in TMD elicit modulations in the neurological and auditory and equilibrium functions. For example, the auditory and vestibular modulations occur as a result of hypertonicity and muscular spasm, which in turn irritates nerves and blood vessels that affect auris interna function by muscular trapping. Relief of the affected nerve or muscular contractions act to relieve auditory or vestibular symptoms. Medications, including barbiturates or diazepam, thus relieve auditory or vestibular dysfunction in TMD patients.

*Utricular Dysfunction*

The utricle is one of the two otolith organs found in the vestibular labyrinth. It is responsive to both gravity and linear acceleration along the horizontal plane. Utricular dysfunction is a disorder caused by damage to the utricle. It is often characterized by a subject's perception of tilting or imbalance.

*Vertigo*

Vertigo is described as a feeling of spinning or swaying while the body is stationary. There are two types of vertigo. Subjective vertigo is the false sensation of movement of the body. Objective vertigo is the perception that one's surrounding are in motion. It is often accompanied by nausea, vomiting, and difficulty maintaining balance.

While not wishing to be bound by any one theory, it is hypothesized that vertigo is caused by an over-accumulation of fluid in the endolymph. This fluid imbalance results in increased pressure on the cells of the inner ear which leads to the sensation of movement. The most common cause of vertigo is benign paroxysmal positional vertigo, or BPPV. In some cases, it is brought on by a head injury, or a sudden change of blood pressure. It is a diagnostic symptom of several diseases including superior canal dehiscence syndrome.

**Local otic administration (any reference to methods of treatment do not form part of the invention)**

Also provided herein are formulations for local delivery of therapeutic agents (otic agents) to auris externa, auris media, and/or auris interna structures. In some embodiments, local delivery of the therapeutic agent (otic agent) overcomes the toxic and attendant side effects of systemic delivery. In some embodiments, access to the vestibular and cochlear apparatus is through the auris media and includes the round window membrane, the oval window/stapes footplate, the annular ligament and through the otic capsule/temporal bone.

Provided herein, in certain embodiments, are otic formulations that remain in contact with the target auditory surfaces (e.g., the round window) for extended periods of time. In some embodiments, the otic formulations further comprise mucoadhesives that allow the otic formulations to adhere to otic mucosal surfaces. In some instances, the formulations described herein avoid attenuation of therapeutic benefit due to drainage or leakage of active agents via the eustachian tube.

In some embodiments, the localized treatment of the auris externa, auris media and/or auris interna affords the use of previously undesired therapeutic agents, including agents with poor PK profiles, poor uptake, low systemic release and/or toxicity issues. In some embodiments, localized targeting of the otic agent formulations reduces the risk of adverse effects with previously characterized toxic or ineffective therapeutic agents (otic active agents). Accordingly, also contemplated within the scope of the embodiments described herein is the use of active agents and/or agents that have been previously rejected by practitioners because of adverse effects or ineffectiveness of the therapeutic agent (otic agent).

In some embodiments, specifically targeting the auris externa, auris media and/or auris interna structures avoids the adverse side effects usually associated with systemic treatment. In some embodiments, the otic formulations described herein are controlled release therapeutic agent formulations that treat otic disorders by providing a constant, variable and/or extended source of a therapeutic agent (otic agent) to the individual or patient suffering from an otic disorder, thereby reducing or eliminating the variability of treatment. Accordingly, one embodiment disclosed herein is to provide a formulation that enables at least one therapeutic agent (otic agent) to be released in therapeutically effective

doses either at variable or constant rates such as to ensure a continuous release of the at least one therapeutic agent (otic agent). In some embodiments, the therapeutic agents (otic agents) disclosed herein are administered as an immediate release formulation . In other embodiments, the therapeutic agents (otic agents) are administered as a controlled release formulation, released either continuously or in a pulsatile manner, or variants of both. In still other embodiments, the therapeutic agent (otic agent) formulation is administered as both an immediate release and controlled release formulation , released either continuously or in a pulsatile manner, or variants of both. The release is optionally dependent on environmental or physiological conditions, for example, the external ionic environment (see, e.g. Oros® release system, Johnson & Johnson).

In addition, the otic formulations included herein also optionally include carriers, adjuvants, such as preserving, stabilizing, wetting or emulsifying agents, solution promoters, salts for regulating the osmotic pressure, and/or buffers. Such carriers, adjuvants, and other excipients are compatible with the environment in the auris externa, auris media and/or auris interna. Accordingly, specifically contemplated are carriers, adjuvants and excipients that lack ototoxicity or are minimally ototoxic in order to allow effective treatment of the otic disorders contemplated herein with minimal side effects in the targeted regions or areas. To prevent ototoxicity, otic formulations disclosed herein are optionally targeted to distinct regions of the auris externa, auris media and/or auris interna, including the tympanic cavity, vestibular bony and membranous labyrinths, cochlear bony and membranous labyrinths and other anatomical or physiological structures located within the auris interna.

**Treatment (any reference to methods of treatment do not form part of the invention)**

Provided herein are otic formulations suitable for the treatment of any otic condition, disease or disorder (e.g., outer, middle and/or inner ear disorders) described herein. The formulations described herein are suitable for the treatment of any disease described herein. In some instances, the treatment is long-term treatment for chronic recurring disease. In some instances, the treatment is prophylactic administration of an otic formulation described herein for the treatment of any otic disease or disorder described herein. In some instances, prophylactic administration avoids occurrence of disease in individuals suspected of having a disease or in individuals genetically predisposed to an otic disease or disorder. In some instances the treatment is preventive maintenance therapy. In some instances, preventive maintenance therapy avoids recurrence of a disease.

In some instances, because of their otic compatibility and improved sterility, the otic formulations described herein are safe for long-term administration. In some embodiments, the otic formulations described herein have very low ototoxicity.

In some embodiments, the otic formulations described herein provide a steady sustained release of a therapeutic agent (otic agent) for a period of at least one day, three days, five days, one week, two weeks, three weeks, a month, two months, three months, four months, five months, six months, or a year. In some embodiments, the otic formulations described herein provide a steady sustained release of a therapeutic agent (otic agent) for a period of at least three days. In some embodiments, the otic formulations described herein provide a steady sustained release of a therapeutic agent (otic agent) for a period of at least five days. In some embodiments, the otic formulations described herein provide a steady sustained release of a therapeutic agent (otic agent) for a period of at least one week. In some embodiments, the otic formulations described herein provide a steady sustained release of a therapeutic agent (otic agent) for a period of at least two weeks. In some embodiments, the otic formulations described herein provide a steady sustained release of a therapeutic agent (otic agent) for a period of at least three weeks. In some embodiments, the otic formulations described herein provide a steady sustained release of a therapeutic agent (otic agent) for a period of at least a month. In some embodiments, the otic formulations described herein provide a steady sustained release of a therapeutic agent (otic agent) for a period of at least two months. In some embodiments, the otic formulations described herein provide a steady sustained release of a therapeutic agent (otic agent) for a period of at least three months. In some embodiments, the otic formulations described herein provide a steady sustained release of a therapeutic agent (otic agent) for a period of at least four months. In some embodiments, the otic formulations described herein provide a steady sustained release of a therapeutic agent (otic agent) for a period of at least five months. In some embodiments, the otic formulations described herein provide a steady sustained release of a therapeutic agent (otic agent) for a period of at least six months. In some embodiments, the otic formulations described herein provide a steady sustained release of a therapeutic agent (otic agent) for a period of at least a year.

In some embodiments, the otic formulations described herein provide a steady sustained release of a therapeutic agent (otic agent) for a period of about a day, three days, five days, one week, two weeks, three weeks, a month, two months, three months, four months, five months, six months, or a year. In some embodiments, the otic formulations described herein provide a steady sustained release of a therapeutic agent (otic agent) for a period of about three days. In some embodiments, the otic formulations described herein provide a steady sustained release of a therapeutic agent (otic agent) for a period of about five days. In some embodiments, the otic formulations described herein provide a steady sustained release of a therapeutic agent (otic agent) for a period of about one week. In some embodiments, the otic

formulations described herein provide a steady sustained release of a therapeutic agent for a period of about two weeks. In some embodiments, the otic formulations described herein provide a steady sustained release of a therapeutic agent (otic agent) for a period of about three weeks. In some embodiments, the otic formulations described herein provide a steady sustained release of a therapeutic agent (otic agent) for a period of about a month. In some embodiments, the otic formulations described herein provide a steady sustained release of a therapeutic agent (otic agent) for a period of about two months. In some embodiments, the otic formulations described herein provide a steady sustained release of a therapeutic agent (otic agent) for a period of about three months. In some embodiments, the otic formulations described herein provide a steady sustained release of a therapeutic agent (otic agent) for a period of about four months. In some embodiments, the otic formulations described herein provide a steady sustained release of a therapeutic agent (otic agent) for a period of about five months. In some embodiments, the otic formulations described herein provide a steady sustained release of a therapeutic agent (otic agent) for a period of about six months. In some embodiments, the otic formulations described herein provide a steady sustained release of a therapeutic agent (otic agent) for a period of about a year.

In one aspect, provided herein are controlled release formulations to treat and/or prevent diseases or conditions associated with the ear. In some instances, these diseases or conditions associated with the ear include the outer, the middle ear and/or inner ear.

In some embodiments, disease or condition is ear pruritus, otitis externa, otalgia, tinnitus, vertigo, ear fullness, hearing loss, or a combination thereof.

Other otic diseases or conditions include autoimmune inner ear disorder (AIED), Ménière's disease, endolymphatic hydrops, noise induced hearing loss (NIHL), sensorineural hearing loss (SNL), tinnitus, otosclerosis, balance disorders, vertigo and the like. In some embodiments, the disease or condition associated with the ear is Ménière's disease, sensorineural hearing loss, noise induced hearing loss, presbycusis (age related hearing loss), auto immune ear disease, tinnitus, ototoxicity, excitotoxicity, endolymphatic hydrops, labyrinthitis, Ramsay Hunt's Syndrome, vestibular neuronitis, microvascular compression syndrome, hyperacusis, presbystasis, central auditory processing disorder, auditory neuropathy, or improvement of cochlea implant performance.

The etiology of several ear diseases or disorders consists of a syndrome of progressive hearing loss, including noise induced hearing loss and age-related hearing loss, dizziness, nausea, nystagmus, vertigo, tinnitus, inflammation, swelling, infection and/or congestion. These disorders have many causes, such as infection, exposure to noise, injury, inflammation, tumors, and/or adverse response to drugs or other chemical agents. Several causes of hearing and/or equilibrium impairment are attributed to inflammation and/or an autoimmune disorder and/or a cytokine-mediated inflammatory response.

**Therapeutic Agents (any reference to methods of treatment do not form part of the invention)**

In some embodiments, the otic formulations described herein have pH and osmolarity that are auris-acceptable. In some embodiments, the otic formulations described herein meet the stringent sterility requirements described herein and are compatible with the endolymph and/or the perilymph. Pharmaceutical agents that are used in conjunction with the formulations disclosed herein include agents that ameliorate or lessen otic disorders, including auris interna disorders, and their attendant symptoms, which include hearing loss, nystagmus, vertigo, tinnitus, inflammation, swelling, infection and congestion. Otic disorders have many causes and include infection, injury, inflammation, tumors and adverse response to drugs or other chemical agents that are responsive to the pharmaceutical agents disclosed herein. In some embodiments, pharmaceutically active metabolites, salts, polymorphs, prodrugs, analogues, and derivatives of the otic agents disclosed herein are used in the formulations.

For some embodiments, wherein the formulation is designed such that the active ingredient has limited or no systemic release, therapeutic agents that produce systemic toxicities (e.g., liver toxicity) or have poor PK characteristics (e.g. short half-life) are also optionally used. Thus, in some embodiments, therapeutic agents that have been previously shown to be toxic, harmful or non-effective during systemic application, for example through toxic metabolites formed after hepatic processing, toxicity of the drug in particular organs, tissues or systems, through high levels needed to achieve efficacy, through the inability to be released through systemic pathways or through poor PK characteristics, are useful. The formulations disclosed herein are contemplated to be targeted directly to otic structures where treatment is needed; for example, one embodiment contemplated is the direct application of the otic formulations disclosed herein onto the round window membrane or the crista fenestrae cochlea of the auris interna, allowing direct access and treatment of the auris interna, or inner ear components. In other embodiments, the otic formulations may be applied directly to the oval window. In yet other embodiments, direct access may be obtained by microinjection directly into the auris interna, for example, through cochlear microperfusion. Such embodiments also optionally comprise a drug delivery device, wherein the drug delivery device delivers the otic formulations through use of a needle and syringe, a pump, a microinjection device, a spongy material or any combination thereof.

In still other embodiments, application of any otic formulation described herein is targeted to the auris media through piercing of the intratympanic membrane and applying the otic agent formulation directly to the auris media

structures affected, including the walls of the tympanic cavity or auditory ossicles. In some embodiments, the auris active agent formulations disclosed herein are confined to the targeted auris media structure, and will not be lost, for example, through diffusion or leakage through the eustachian tube or pierced tympanic membrane. In some embodiments, the otic formulations disclosed herein are delivered to the auris externa in any suitable manner, including by cotton swab, injection or ear drops. Also, in other embodiments, the otic formulations described herein are targeted to specific regions of the auris externa by application with a needle and syringe, a pump, a microinjection device, a spongy material, or any combination thereof.

**Growth Factors (of which neurotrophic growth factors are specifically claimed)**

Contemplated for use with the formulations disclosed herein are agents that modulate the degeneration of neurons and/or hair cells of the auris, promote the survival and/or growth of neurons and/or hair cells of the auris, and agents for treating or ameliorating hearing loss or reduction resulting from destroyed, stunted, malfunctioning, damaged, fragile or missing hairs in the inner ear. Accordingly, some embodiments incorporate the use of agents which promote the survival of neurons and otic hair cells, and/or the growth of neurons and otic hair cells. In some embodiments, the agent which promotes the survival of otic hair cells is a growth factor. In some embodiments, the growth factor is a neurotroph. In certain instances, neurotrophs are growth factors which prevent cells from initiating apoptosis, repair damaged neurons and otic hair cells, and/or induce differentiation in progenitor cells. In some embodiments, the growth factor is brain-derived neurotrophic factor (BDNF), ciliary neurotrophic factor (CNTF), glial cell-line derived neurotrophic factor (GDNF), neurotrophin-3, neurotrophin-4, and/or combinations thereof. In some embodiments, the growth factor is brain-derived neurotrophic factor (BDNF). In some embodiments, the growth factor is ciliary neurotrophic factor (CNTF). In some embodiments, the growth factor is glial cell-line derived neurotrophic factor (GDNF). In some embodiments, the growh factor is neurotrophin-3. In some embodiments, the growth factor is neurotrophin-4. In some embodiments, the growth factor is a fibroblast growth factor (FGF), an insulin-like growth factor (IGF), an epidermal growth factor (EGF), a platlet-derived growth factor (PGF) and/or agonists thereof. In some embodiments, the growth factor is an agonist of the fibroblast growth factor (FGF) receptor, the insulin-like growth factor (IGF) receptor, the epidermal growth factor (EGF) receptor, and/or the platlet-derived growth factor. In some embodiments, the growth factor is hepatocyte growth factor.

In some embodiments, the growth factor is an epidermal growth factor (EGF). In some embodiments, the EGF is heregulin (HRG). In certain instances, HRG stimulates the proliferation of utricular sensory epithelium. In certain instances, HRG-binding receptors are found in the vestibular and auditory sensory epithelium.

In some embodiments, the growth factor is an insulin-like growth factor (IGF). In some embodiments, the IGF is IGF-1. In some embodiments, the IGF-1 is mecasermin. In certain instances, IGF-1 attenuates the damage induced by exposure to an aminoglycoside. In certain instances, IGF-1 stimulates the differentiation and/or maturation of cochlear ganglion cells.

In some embodiments, the FGF receptor agonist is FGF-2. In some embodiments, the IGF receptor agonist is IGF-1. Both the FGF and IGF receptors are found in the cells comprising the utricle epithelium.

In some embodiments, the growth factor is hepatocyte growth factor (HGF). In some instances, HGF protects cochlear hair cells from noise-induced damage and reduces noise-exposure-caused ABR threshold shifts.

Also contemplated for use in the otic formulations described herein are growth factors including Erythropoietin (EPO), Granulocyte-colony stimulating factor (G-CSF), Granulocyte-macrophage colony stimulating factor (GM-CSF), Growth differentiation factor-9 (GDF9), Insulin-like growth factor (IGF), Myostatin (GDF-8), Platelet-derived growth factor (PDGF), Thrombopoietin (TPO), Transforming growth factor alpha (TGF-$\alpha$), Transforming growth factor beta (TGF-$\beta$), Vascular endothelial growth factor (VEGF) or combinations thereof.

*Neurotrophs (which are specifically claimed)*

In some embodiments, the growth factor is a neurotroph. In certain instances, neurotrophs are growth factors which prevent cells from initiating apoptosis, repair damaged neurons and otic hair cells, and/or induce differentiation in progenitor cells. In some embodiments, the neurotroph is brain-derived neurotrophic factor (BDNF), ciliary neurotrophic factor (CNTF), glial cell-line derived neurotrophic factor (GDNF), neurotrophin-3, neurotrophin-4, and/or combinations thereof.

In some embodiments, the neurotroph is BDNF. In certain instances, BDNF is a neurotroph which promotes the survival of existing neurons (e.g. spiral ganglion neurons), and otic hair cells by repairing damaged cells, inhibiting the production of ROS, and inhibiting the induction of apoptosis. In certain embodiments, it also promotes the differentiation of neural and otic hair cell progenitors. Further, in certain embodiments, it protects the Cranial Nerve VII from degeneration. In some embodiments, BDNF is administered in conjunction with fibroblast growth factor.

In some embodiments, the neurotroph is neurotrophin-3. In certain embodiments, neurotrophin-3 promotes the survival of existing neurons and otic hair cells, and promotes the differentiation of neural and otic hair cell progenitors.

Further, in certain embodiments, it protects the VII nerve from degeneration.

In some embodiments, the neurotroph is CNTF. In certain embodiments, CNTF promotes the synthesis of neurotransmitters and the growth of neuritis. In some embodiments, CNTF is administered in conjunction with BDNF.

In some embodiments, the neurotroph is GDNF. In certain embodiments, GDNF expression is increased by treatment with ototoxic agents. Further, in certain embodiments, cells treated with exogenous GDNF have higher survival rates after trauma then untreated cells.

In some embodiments, the neurotroph is a Trk agonist, such as a TrkB or TrkC agonist. For example, BDNF and NT-3 activate TrkB and TrkC on spiral ganglion neurons to prompt survival, neurite growth, and synapse formation. Other Trk agonists including TrkC monoclonal antibody agonists M1, M2, and M7 humanized and TrkB monoclonal antibody agonists M3 and M6 humanized and M4 and M5 mouse are also contemplated for use provided herein.

**Amount of Therapeutic Agent**

In some embodiments, the otic formulation comprises between about 0.0001% to about 99.9999% by weight of the weight of the therapeutic agent (e.g., growth factor), or pharmaceutically acceptable prodrug or salt thereof. In some embodiments, the otic formulation comprises between about 0.0001% to about 20% by weight of the weight of the therapeutic agent, or pharmaceutically acceptable prodrug or salt thereof. In some embodiments, the otic formulation comprises between about 0.0001% to about 15% by weight of the weight of the therapeutic agent, or pharmaceutically acceptable prodrug or salt thereof. In some embodiments, the otic formulation comprises between about about 0.0001% to about 10% by weight of the weight of the therapeutic agent, or pharmaceutically acceptable prodrug or salt thereof. In some embodiments, the otic formulation comprises between about 0.0001% to about 5% by weight of the weight of the therapeutic agent, or pharmaceutically acceptable prodrug or salt thereof. In some embodiments, the otic formulation comprises between about 0.0001% to about 1% by weight of the weight of the therapeutic agent, or pharmaceutically acceptable prodrug or salt thereof. In some embodiments, the otic formulation comprises between about 0.05% to about 0.5% by weight of the therapeutic agent, or pharmaceutically acceptable prodrug or salt thereof.

In some embodiments, the otic formulation comprises between about 0.001% to about 99.99% by weight of the therapeutic agent, or pharmaceutically acceptable prodrug or salt thereof. In some embodiments, the otic formulation comprises between about 0.001% to about 99.9% by weight of the therapeutic agent, or pharmaceutically acceptable prodrug or salt thereof. In some embodiments, the otic formulation comprises between about 0.001% to about 99% by weight of the therapeutic agent, or pharmaceutically acceptable prodrug or salt thereof. In some embodiments, the otic formulation comprises between about 0.001% to about 90% by weight of the therapeutic agent, or pharmaceutically acceptable prodrug or salt thereof. In some embodiments, the otic formulation comprises between about 0.001% to about 80% by weight of the therapeutic agent, or pharmaceutically acceptable prodrug or salt thereof. In some embodiments, the otic formulation comprises between about 0.001% to about 70% by weight of the therapeutic agent, or pharmaceutically acceptable prodrug or salt thereof. In some embodiments, the otic formulation comprises between about 0.001% to about 60% by weight of the therapeutic agent, or pharmaceutically acceptable prodrug or salt thereof. In some embodiments, the otic formulation comprises between about 0.001% to about 50% by weight of the therapeutic agent, or pharmaceutically acceptable prodrug or salt thereof. In some embodiments, the otic formulation comprises between about 0.001% to about 40% by weight of the therapeutic agent, or pharmaceutically acceptable prodrug or salt thereof. In some embodiments, the otic formulation comprises between about 0.001% to about 30% by weight of the therapeutic agent, or pharmaceutically acceptable prodrug or salt thereof. In some embodiments, the otic formulation comprises between about 0.001% to about 20% by weight of the therapeutic agent, or pharmaceutically acceptable prodrug or salt thereof. In some embodiments, the otic formulation comprises between about 0.001% to about 15% by weight of the therapeutic agent, or pharmaceutically acceptable prodrug or salt thereof. In some embodiments, the otic formulation comprises between about 0.001% to about 10% by weight of the therapeutic agent, or pharmaceutically acceptable prodrug or salt thereof. In some embodiments, the otic formulation comprises between about 0.001% to about 7% by weight of the therapeutic agent, or pharmaceutically acceptable prodrug or salt thereof. In some embodiments, the otic formulation comprises between about 0.001% to about 5% by weight of the therapeutic agent, or pharmaceutically acceptable prodrug or salt thereof. In some embodiments, the otic formulation comprises between about 0.001% to about 3% by weight of the therapeutic agent, or pharmaceutically acceptable prodrug or salt thereof. In some embodiments, the otic formulation comprises between about 0.001% to about 2% by weight of the therapeutic agent, or pharmaceutically acceptable prodrug or salt thereof. In some embodiments, the otic formulation comprises between about 0.001% to about 1% by weight of the therapeutic agent, or pharmaceutically acceptable prodrug or salt thereof. In some embodiments, the otic formulation comprises between about 0.05% to about 0.5% by weight of the therapeutic agent, or pharmaceutically acceptable prodrug or salt thereof.

In some embodiments, the otic formulation comprises between about 0.01% to about 99.99% by weight of the therapeutic agent (e.g., growth factor), or pharmaceutically acceptable prodrug or salt thereof. In some embodiments, the otic formulation comprises between about 0.01% to about 99.9% by weight of the therapeutic agent, or pharmaceutically acceptable prodrug or salt thereof. In some embodiments, the otic formulation comprises between about 0.01% to about

99% by weight of the therapeutic agent, or pharmaceutically acceptable prodrug or salt thereof. In some embodiments, the otic formulation comprises between about 0.01% to about 90% by weight of the therapeutic agent, or pharmaceutically acceptable prodrug or salt thereof. In some embodiments, the otic formulation comprises between about 0.01% to about 80% by weight of the therapeutic agent, or pharmaceutically acceptable prodrug or salt thereof. In some embodiments, the otic formulation comprises between about 0.01% to about 70% by weight of the therapeutic agent, or pharmaceutically acceptable prodrug or salt thereof. In some embodiments, the otic formulation comprises between about 0.01% to about 60% by weight of the therapeutic agent, or pharmaceutically acceptable prodrug or salt thereof. In some embodiments, the otic formulation comprises between about 0.01% to about 50% by weight of the therapeutic agent, or pharmaceutically acceptable prodrug or salt thereof. In some embodiments, the otic formulation comprises between about 0.01% to about 40% by weight of the therapeutic agent, or pharmaceutically acceptable prodrug or salt thereof. In some embodiments, the otic formulation comprises between about 0.01% to about 30% by weight of the therapeutic agent, or pharmaceutically acceptable prodrug or salt thereof. In some embodiments, the otic formulation comprises between about 0.01% to about 20% by weight of the therapeutic agent, or pharmaceutically acceptable prodrug or salt thereof. In some embodiments, the otic formulation comprises between about 0.01% to about 15% by weight of the therapeutic agent, or pharmaceutically acceptable prodrug or salt thereof. In some embodiments, the otic formulation comprises between about 0.01% to about 10% by weight of the therapeutic agent, or pharmaceutically acceptable prodrug or salt thereof. In some embodiments, the otic formulation comprises between about 0.01% to about 7% by weight of the therapeutic agent, or pharmaceutically acceptable prodrug or salt thereof. In some embodiments, the otic formulation comprises between about 0.01% to about 5% by weight of the therapeutic agent, or pharmaceutically acceptable prodrug or salt thereof. In some embodiments, the otic formulation comprises between about 0.01% to about 3% by weight of the therapeutic agent, or pharmaceutically acceptable prodrug or salt thereof. In some embodiments, the otic formulation comprises between about 0.01% to about 2% by weight of the therapeutic agent, or pharmaceutically acceptable prodrug or salt thereof. In some embodiments, the otic formulation comprises between about 0.01% to about 1% by weight of the therapeutic agent, or pharmaceutically acceptable prodrug or salt thereof.

In some embodiments, the otic formulation comprises between about 0.1% to about 40% by weight of the therapeutic agent (e.g., growth factor), or pharmaceutically acceptable prodrug or salt thereof. In some embodiments, the otic formulation comprises between about 0.1% to about 30% by weight of the therapeutic agent, or pharmaceutically acceptable prodrug or salt thereof. In some embodiments, the otic formulation comprises between about 0.10% to about 20% by weight of the therapeutic agent, or pharmaceutically acceptable prodrug or salt thereof. In some embodiments, the otic formulation comprises between about 0.10% to about 15% by weight of the therapeutic agent, or pharmaceutically acceptable prodrug or salt thereof. In some embodiments, the otic formulation comprises between about 0.10% to about 10% by weight of the therapeutic agent, or pharmaceutically acceptable prodrug or salt thereof. In some embodiments, the otic formulation comprises between about 0.10% to about 7% by weight of the therapeutic agent, or pharmaceutically acceptable prodrug or salt thereof. In some embodiments, the otic formulation comprises between about 0.10% to about 5% by weight of the therapeutic agent, or pharmaceutically acceptable prodrug or salt thereof. In some embodiments, the otic formulation comprises between about 0.10% to about 3% by weight of the therapeutic agent, or pharmaceutically acceptable prodrug or salt thereof. In some embodiments, the otic formulation comprises between about 0.10% to about 2% by weight of the therapeutic agent, or pharmaceutically acceptable prodrug or salt thereof. In some embodiments, the otic formulation comprises between about 0.10% to about 1% by weight of the therapeutic agent, or pharmaceutically acceptable prodrug or salt thereof.

In some embodiments, the otic formulation comprises between about 1% to about 40% by weight of the therapeutic agent (e.g., growth factor), or pharmaceutically acceptable prodrug or salt thereof. In some embodiments, the otic formulation comprises between about 1% to about 30% by weight of the therapeutic agent, or pharmaceutically acceptable prodrug or salt thereof. In some embodiments, the otic formulation comprises between about 1% to about 20% by weight of the therapeutic agent, or pharmaceutically acceptable prodrug or salt thereof. In some embodiments, the otic formulation comprises between about 1% to about 15% by weight of the therapeutic agent, or pharmaceutically acceptable prodrug or salt thereof. In some embodiments, the otic formulation comprises between about 1% to about 10% by weight of the therapeutic agent, or pharmaceutically acceptable prodrug or salt thereof. In some embodiments, the otic formulation comprises between about 1% to about 7% by weight of the therapeutic agent, or pharmaceutically acceptable prodrug or salt thereof. In some embodiments, the otic formulation comprises between about 1% to about 5% by weight of the therapeutic agent, or pharmaceutically acceptable prodrug or salt thereof. In some embodiments, the otic formulation comprises between about 1% to about 3% by weight of the therapeutic agent, or pharmaceutically acceptable prodrug or salt thereof. In some embodiments, the otic formulation comprises between about 1% to about 2% by weight of the therapeutic agent, or pharmaceutically acceptable prodrug or salt thereof.

In some embodiments, the otic formulation comprises about 0.0001%, about 0.0002%, about 0.0003%, about 0.0004%, about 0.0005%, about 0.0006%, about 0.0007%, about 0.0008%, about 0.0009%, about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%,

about 0.1%, about 0.2%, about 0.3%, about 0.4%, about 0.5%, about 0.6%, about 0.7%, about 0.8%, about 0.9%, about 1%, about 2%, about 3%, about 4%, about 5%, about 6%, about 7%m about 8%, about 9%, about 10%, about about 11%, about 12%, about 13%, about 14%, about 15%, about 16%, about 17%, about 18%, about 19%, about 20%, about 25%, about 30%, about 35%, or about 40% by weight of the therapeutic agent (e.g., growth factor), or pharmaceutically acceptable prodrug or salt thereof.

In some embodiments, the otic formulation comprises about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2% about 0.3%, about 0.4%, about 0.5%, about 0.6%, about 0.7%, about 0.8%, about 0.9%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 25%, about 30%, about 35%, or about 40% by weight of the therapeutic agent (e.g., growth factor), or pharmaceutically acceptable prodrug or salt thereof. In some embodiments, the otic formulation comprises about 0.01% by weight of the therapeutic agent, or pharmaceutically acceptable prodrug or salt thereof. In some embodiments, the otic formulation comprises about 0.02% by weight of the therapeutic agent, or pharmaceutically acceptable prodrug or salt thereof. In some embodiments, the otic formulation comprises about 0.03% by weight of the therapeutic agent, or pharmaceutically acceptable prodrug or salt thereof. In some embodiments, the otic formulation comprises about 0.04% by weight of the therapeutic agent, or pharmaceutically acceptable prodrug or salt thereof. In some embodiments, the otic formulation comprises about 0.05% by weight of the therapeutic agent, or pharmaceutically acceptable prodrug or salt thereof. In some embodiments, the otic formulation comprises about 0.06% by weight of the therapeutic agent, or pharmaceutically acceptable prodrug or salt thereof. In some embodiments, the otic formulation comprises about 0.07% by weight of the therapeutic agent, or pharmaceutically acceptable prodrug or salt thereof. In some embodiments, the otic formulation comprises about 0.08% by weight of the therapeutic agent, or pharmaceutically acceptable prodrug or salt thereof. In some embodiments, the otic formulation comprises about 0.09% by weight of the therapeutic agent, or pharmaceutically acceptable prodrug or salt thereof. In some embodiments, the otic formulation comprises about 0.1% by weight of the therapeutic agent, or pharmaceutically acceptable prodrug or salt thereof. In some embodiments, the otic formulation comprises about 0.2% by weight of the therapeutic agent, or pharmaceutically acceptable prodrug or salt thereof. In some embodiments, the otic formulation comprises about 0.3% by weight of the therapeutic agent, or pharmaceutically acceptable prodrug or salt thereof. In some embodiments, the otic formulation comprises about 0.4% by weight of the therapeutic agent, or pharmaceutically acceptable prodrug or salt thereof. In some embodiments, the otic formulation comprises about 0.5% by weight of the therapeutic agent, or pharmaceutically acceptable prodrug or salt thereof. In some embodiments, the otic formulation comprises about 0.6% by weight of the therapeutic agent, or pharmaceutically acceptable prodrug or salt thereof. In some embodiments, the otic formulation comprises about 0.7% by weight of the therapeutic agent, or pharmaceutically acceptable prodrug or salt thereof. In some embodiments, the otic formulation comprises about 0.8% by weight of the therapeutic agent, or pharmaceutically acceptable prodrug or salt thereof. In some embodiments, the otic formulation comprises about 0.9% by weight of the therapeutic agent, or pharmaceutically acceptable prodrug or salt thereof. In some embodiments, the otic formulation comprises about 1% by weight of the therapeutic agent, or pharmaceutically acceptable prodrug or salt thereof. In some embodiments, the otic formulation comprises about 2% by weight of the therapeutic agent, or pharmaceutically acceptable prodrug or salt thereof. In some embodiments, the otic formulation comprises about 3% by weight of the therapeutic agent, or pharmaceutically acceptable prodrug or salt thereof. In some embodiments, the otic formulation comprises about 4% by weight of the therapeutic agent, or pharmaceutically acceptable prodrug or salt thereof. In some embodiments, the otic formulation comprises about 5% by weight of the therapeutic agent, or pharmaceutically acceptable prodrug or salt thereof. In some embodiments, the otic formulation comprises about 6% by weight of the therapeutic agent, or pharmaceutically acceptable prodrug or salt thereof. In some embodiments, the otic formulation comprises about 7% by weight of the therapeutic agent, or pharmaceutically acceptable prodrug or salt thereof. In some embodiments, the otic formulation comprises about 8% by weight of the therapeutic agent, or pharmaceutically acceptable prodrug or salt thereof. In some embodiments, the otic formulation comprises about 9% by weight of the therapeutic agent, or pharmaceutically acceptable prodrug or salt thereof. In some embodiments, the otic formulation comprises about 10% by weight of the therapeutic agent, or pharmaceutically acceptable prodrug or salt thereof. In some embodiments, the otic formulation comprises about 11% by weight of the therapeutic agent, or pharmaceutically acceptable prodrug or salt thereof. In some embodiments, the otic formulation comprises about 12% by weight of the therapeutic agent, or pharmaceutically acceptable prodrug or salt thereof. In some embodiments, the otic formulation comprises about 13% by weight of the therapeutic agent, or pharmaceutically acceptable prodrug or salt thereof. In some embodiments, the otic formulation comprises about 14% by weight of the therapeutic agent, or pharmaceutically acceptable prodrug or salt thereof. In some embodiments, the otic formulation comprises about 15% by weight of the therapeutic agent, or pharmaceutically acceptable prodrug or salt thereof. In some embodiments, the otic formulation comprises about 16% by weight of the therapeutic agent, or pharmaceutically acceptable prodrug or salt thereof. In some embodiments, the otic formulation comprises about 17% by weight of the therapeutic agent, or

pharmaceutically acceptable prodrug or salt thereof. In some embodiments, the otic formulation comprises about 18% by weight of the therapeutic agent, or pharmaceutically acceptable prodrug or salt thereof. In some embodiments, the otic formulation comprises about 19% by weight of the therapeutic agent, or pharmaceutically acceptable prodrug or salt thereof. In some embodiments, the otic formulation comprises about 20% by weight of the therapeutic agent, or pharmaceutically acceptable prodrug or salt thereof.

**Devices (which are not part of the claimed invention but may be used in conjunction with the claimed formulations)**

Disclosed but not claimed is the use of devices for the delivery of the pharmaceutical formulations disclosed herein, or alternatively for the measurement or surveillance of the function of the auris formulations disclosed herein. For example pumps, osmotic devices or other means of mechanically delivering pharmaceutical formulations are used for the delivery of the pharmaceutical formulations disclosed herein. Reservoir devices are optionally used with the pharmaceutical drug delivery units, and reside either internally along with the drug delivery unit, or externally of the auris structures.

Disclosed but not claimed is the use of mechanical or imaging devices to monitor or survey the hearing, balance or other auris disorder. For example, magnetic resonance imaging (MRI) devices are specifically contemplated within the scope of the embodiments, wherein the MRI devices (for example, 3 Tesla MRI devices) are capable of evaluating Ménière Disease progression and subsequent treatment with the pharmaceutical formulations disclosed herein. *See,* Carfrae et al. Laryngoscope 118:501-505 (March 2008). Whole body scanners, or alternatively cranial scanners, are contemplated, as well as higher resolution (7 Tesla, 8 Tesla, 9.5 Tesla or 11 Tesla for humans) are optionally used in MRI scanning.

**Visualization of otic formulations**

Also provided herein in some embodiments are otic formulations that comprise a dye (e.g., a Trypan blue dye, Evans blue dye) or other tracer compound. In some instances, addition of an auris-compatible dye to an otic formulation described herein aids visualization of any administered formulation in an ear (e.g., a rodent ear and/or a human ear). In certain embodiments, an otic formulation comprising a dye or other tracer compound eliminates the need for invasive procedures that are currently used in animal models to monitor the concentrations of drugs in the endolymph and/or perilymph.

In some instances, intratympanic injections require the need of a specialist and the formulation needs to be delivered to a specific site of the ear to maximize efficiency of the medication delivered. In certain instances, a visualization technique for any formulation described herein allows for visualization of a dosing site (e.g., the round window) so that the medication is applied in the proper place. In some instances, a formulation comprising a dye allows visualization of the formulation during administration of the formulation to an ear (e.g., a human ear), ensures that the medication will be delivered at the intended site, and avoids any complications due to incorrect placement of a formulation . The inclusion of a dye to help enhance the visualization of the formulation when applied, and the ability to visually inspect the location of the formulation after administration without further intervention, represents an advance over currently available methods for testing intratympanic therapeutics in animal models and/or human trials. In some embodiments, dyes that are compatible with the otic formulations described herein include Evans blue (e.g., 0.5% of the total weight of an otic formulation), Methylene blue (e.g., 1% of the total weight of an otic formulation), Isosulfan blue (e.g., 1% of the total weight of an otic formulation), Trypan blue (e.g., 0.15% of the total weight of an otic formulation), and/or indocyanine green (e.g., 25mg/vial). Other common dyes, e.g., FD&C red 40, FD&C red 3, FD&C yellow 5, FD&C yellow 6, FD&C blue 1, FD&C blue2, FD&C green 3, fluorescence dyes (e.g., Fluorescein isothiocyanate, rhodamine, Alexa Fluors, DyLight Fluors) and/or dyes that are visualizable in conjunction with non-invasive imaging techniques such as MRI, CAT scans, PET scans or the like (e.g., Gadolinium-based MRI dyes, iodine-base dyes, barium-based dyes or the like) are also contemplated for use with any otic formulation described herein. Other dyes that are compatible with any formulation described herein are listed in the Sigma-Aldrich catalog under dyes . In some embodiments, concentration of a dye in any otic formulation described herein is less than 2%, less than 1.5%, less than 1%, less than 0.5%, less than 0.25%, less than 0.1%, or less than 100 ppm of the total weight and/or volume of any formulation described herein.

In certain embodiments of such auris-compatible formulations that comprise a dye, the ability to visualize a controlled release otic formulation comprising a dye in an ear meets a long standing need for suitable testing methods that are applicable to the development of intratympanic otic formulations suitable for human use. In certain embodiments of such auris-compatible formulations that comprise a dye, the ability to visualize a controlled release otic formulation comprising a dye allows for testing of any otic formulation described herein in human clinical trials.

**General Methods of Sterilization (reference to methods of treatment do not form part of the invention)**

The environment of the inner ear is an isolated environment. The endolymph and the perilymph are static fluids

and are not in contiguous contact with the circulatory system. The blood - labyrinth - barrier (BLB), which includes a blood-endolymph barrier and a blood-perilymph barrier, consists of tight junctions between specialized epithelial cells in the labyrinth spaces (i.e., the vestibular and cochlear spaces). The presence of the BLB limits delivery of active agents to the isolated microenvironment of the inner ear. Auris hair cells are bathed in endolymphatic or perilymphatic fluids and cochlear recycling of potassium ions is important for hair cell function. When the inner ear is infected, there is an influx of leukocytes and/or immunoglobins (e.g. in response to a microbial infection) into the endolymph and/or the perilymph and the delicate ionic composition of inner ear fluids is upset by the influx of leukocytes and/or immunoglobins. In certain instances, a change in the ionic composition of inner ear fluids results in hearing loss, loss of balance and/or ossification of auditory structures. In certain instances, even trace amounts of pyrogens and/or microbes trigger infections and related physiological changes in the isolated microenvironment of the inner ear.

In one aspect, provided herein are otic formulations that are sterilized with stringent sterility requirements and are suitable for administration to the middle and/or inner ear. In some embodiments, the otic formulations described herein are auris compatible formulations. In some embodiment, the otic formulations are substantially free of pyrogens and/or microbes.

Provided herein are otic formulations that ameliorate or lessen otic disorders described herein. In some embodiments, the formulations are sterilized. Included within the embodiments disclosed herein are means and processes for sterilization of a pharmaceutical formulation disclosed herein for use in humans. The goal is to provide a safe pharmaceutical product, relatively free of infection causing micro-organisms. The U. S. Food and Drug Administration has provided regulatory guidance in the publication "Guidance for Industry: Sterile Drug Products Produced by Aseptic Processing" available at: http://www.fda.gov/cder/guidance/5882fnl.htm. No specific guidelines are available for safe pharmaceutical products for treatment of the inner ear.

As used herein, sterilization means a process used to destroy or remove microorganisms that are present in a product or packaging. Any suitable method available for sterilization of objects and formulations is used. Available methods for the inactivation of microorganisms include the application of extreme heat, lethal chemicals, or gamma radiation. In some embodiments is a process for the preparation of an otic therapeutic formulation comprising subjecting the formulation to a sterilization method selected from heat sterilization, chemical sterilization, radiation sterilization or filtration sterilization. The method used depends largely upon the nature of the device or formulation to be sterilized. Detailed descriptions of many methods of sterilization are given in Chapter 40 of Remington: The Science and Practice of Pharmacy published by Lippincott, Williams & Wilkins.

*Sterilization by Heat*

Many methods are available for sterilization by the application of extreme heat. One method is through the use of a saturated steam autoclave. In this method, saturated steam at a temperature of at least 121 °C is allowed to contact the object to be sterilized. The transfer of heat is either directly to the microorganism, in the case of an object to be sterilized, or indirectly to the microorganism by heating the bulk of an aqueous solution to be sterilized. This method is widely practiced as it allows flexibility, safety and economy in the sterilization process.

Dry heat sterilization is a method which is used to kill microorganisms and perform depyrogenation at elevated temperatures. This process takes place in an apparatus suitable for heating HEPA-filtered microorganism-free air to temperatures of at least 130-180 °C for the sterilization process and to temperatures of at least 230-250 °C for the depyrogenation process. Water to reconstitute concentrated or powdered formulations is also sterilized by autoclave.

*Chemical Sterilization*

Chemical sterilization methods are an alternative for products that do not withstand the extremes of heat sterilization. In this method, a variety of gases and vapors with germicidal properties, such as ethylene oxide, chlorine dioxide, formaldehyde or ozone are used as the anti-apoptotic agents. The germicidal activity of ethylene oxide, for example, arises from its ability to serve as a reactive alkylating agent. Thus, the sterilization process requires the ethylene oxide vapors to make direct contact with the product to be sterilized.

*Radiation Sterilization*

One advantage of radiation sterilization is the ability to sterilize many types of products without heat degradation or other damage. The radiation commonly employed is beta radiation or alternatively, gamma radiation from a $^{60}Co$ source. The penetrating ability of gamma radiation allows its use in the sterilization of many product types, including solutions, compositions and heterogeneous mixtures. The germicidal effects of irradiation arise from the interaction of gamma radiation with biological macromolecules. This interaction generates charged species and free radicals. Subsequent chemical reactions, such as rearrangements and cross-linking processes, result in the loss of normal function for

these biological macromolecules. The formulations described herein are also optionally sterilized using beta irradiation.

*Filtration*

Filtration sterilization is a method used to remove but not destroy microorganisms from solutions. Membrane filters are used to filter heat-sensitive solutions. Such filters are thin, strong, homogenous polymers of mixed cellulosic esters (MCE), polyvinylidene fluoride (PVF; also known as PVDF), or polytetrafluoroethylene (PTFE) and have pore sizes ranging from 0.1 to 0.22 μm. Solutions of various characteristics are optionally filtered using different filter membranes. For example, PVF and PTFE membranes are well suited to filtering organic solvents while aqueous solutions are filtered through PVF or MCE membranes. Filter apparatus are available for use on many scales ranging from the single point-of-use disposable filter attached to a syringe up to commercial scale filters for use in manufacturing plants. The membrane filters are sterilized by autoclave or chemical sterilization. Validation of membrane filtration systems is performed following standardized protocols (Microbiological Evaluation of Filters for Sterilizing Liquids, Vol 4, No. 3. Washington, D.C: Health Industry Manufacturers Association, 1981) and involve challenging the membrane filter with a known quantity (ca. $10^{7}/cm^{2}$) of unusually small microorganisms, such as Brevundimonas diminuta (ATCC 19146).

Pharmaceutical formulations are optionally sterilized by passing through membrane filters. In some embodiments, formulations comprising nanoparticles (U.S. Pat No. 6,139,870) or multilamellar vesicles (Richard et al., International Journal of Pharmaceutics (2006), 312(1-2):144-50) are amenable to sterilization by filtration through 0.22 μm filters without destroying their organized structure.

Methods disclosed but not claimed include sterilizing the formulation (or components thereof) by means of filtration sterilization. In another embodiment the otic formulation comprises a particle wherein the particle formulation is suitable for filtration sterilization. In a further embodiment said particle formulation comprises particles of less than 300 nm in size, of less than 200 nm in size, of less than 100 nm in size. In another embodiment the otic formulation comprises a particle formulation wherein the sterility of the particle is ensured by sterile filtration of the precursor component solutions. In another embodiment the otic formulation comprises a particle formulation wherein the sterility of the particle formulation is ensured by low temperature sterile filtration. In a further embodiment, said low temperature sterile filtration occurs at a temperature between 0 and 30 °C, or between 0 and 20 °C, or between 0 and 10 °C, or between 10 and 20 °C, or between 20 and 30 °C. In another embodiment is a process for the preparation of an auris-acceptable particle formulation comprising: filtering the aqueous solution containing the particle formulation at low temperature through a sterilization filter; lyophilizing the sterile solution; and reconstituting the particle formulation with sterile water prior to administration. In some embodiments, a formulation described herein is manufactured as a suspension in a single vial formulation containing the micronized active pharmaceutical ingredient. A single vial formulation is prepared by aseptically mixing a sterile poloxamer solution with sterile micronized active ingredient (e.g., ketamine) and transferring the formulation to sterile pharmaceutical containers. In some embodiments, a single vial containing a formulation described herein as a suspension is resuspended before dispensing and/or administration.

In specific embodiments, filtration and/or filling procedures are carried out at about 5°C below the gel temperature (Tgel) of a formulation described herein and with viscosity below a theoretical value of 100cP to allow for filtration in a reasonable time using a peristaltic pump.

In another embodiment the otic formulation comprises a nanoparticle formulation wherein the nanoparticle formulation is suitable for filtration sterilization. In a further embodiment the nanoparticle formulation comprises nanoparticles of less than 300 nm in size, of less than 200 nm in size, or of less than 100 nm in size. In another embodiment the otic formulation comprises a microsphere formulation wherein the sterility of the microsphere is ensured by sterile filtration of the precursor organic solution and aqueous solutions. In another embodiment, the sterility of the otic formulation is ensured by low temperature sterile filtration. In a further embodiment, the low temperature sterile filtration occurs at a temperature between 0 and 30 °C, or between 0 and 20 °C, or between 0 and 10 °C, or between 10 and 20 °C, or between 20 and 30 °C. In another embodiment is a process for the preparation of an auris-acceptable thermoreversible gel formulation comprising: filtering the aqueous solution containing the thermoreversible gel components at low temperature through a sterilization filter; lyophilizing the sterile solution; and reconstituting the thermoreversible gel formulation with sterile water prior to administration.

In certain embodiments, the active ingredients are dissolved in a suitable vehicle (e.g. a buffer) and sterilized separately (e.g. by heat treatment, filtration, gamma radiation); the remaining excipients are sterilized in a separate step by a suitable method (e.g. filtration and/or irradiation of a cooled mixture of excipients); the two solutions that were separately sterilized are then mixed aseptically to provide a final otic formulation .

In some instances, conventionally used methods of sterilization (e.g., heat treatment (e.g., in an autoclave), gamma irradiation, filtration) lead to irreversible degradation of the therapeutic agent in the formulation .

In some instances, conventionally used methods of sterilization (e.g., heat treatment (e.g., in an autoclave), gamma irradiation, filtration) lead to irreversible degradation of polymeric components (e.g., thermosetting, gelling or mucoadhesive polymer components) and/or the active agent in the formulation. In some instances, sterilization of an auris

formulation by filtration through membranes (e.g., 0.2 □M membranes) is not possible if the formulation comprises thixotropic polymers that gel during the process of filtration.

Accordingly, provided herein are methods for sterilization of auris formulations that prevent degradation of polymeric components (e.g., thermosetting and/or gelling and/or mucoadhesive polymer components) and/or the therapeutic agent during the process of sterilization. In some embodiments, degradation of the therapeutic agent is reduced or eliminated through the use of specific pH ranges for buffer components and specific proportions of gelling agents in the formulations. In some embodiments, the choice of an appropriate gelling agent and/or thermosetting polymer allows for sterilization of formulations described herein by filtration. In some embodiments, the use of an appropriate thermosetting polymer and an appropriate copolymer (e.g., a gelling agent) in combination with a specific pH range for the formulation allows for high temperature sterilization of formulations described with substantially no degradation of the therapeutic agent or the polymeric excipients. An advantage of the methods of sterilization provided herein is that, in certain instances, the formulations are subjected to terminal sterilization via autoclaving without any loss of the active agent and/or excipients and/or polymeric components during the sterilization step and are rendered substantially free of microbes and/or pyrogens.

*Microorganisms*

Provided herein are otic formulations that ameliorate or lessen otic disorders described herein. In some embodiments, the formulations are substantially free of microorganisms. Acceptable sterility levels are based on applicable standards that define therapeutically acceptable otic formulations , including United States Pharmacopeia Chapters <1111> et seq. For example, acceptable sterility levels include 10 colony forming units (cfu) per gram of formulation , 50 cfu per gram of formulation , 100 cfu per gram of formulation , 500 cfu per gram of formulation or 1000 cfu per gram of formulation . In addition, acceptable sterility levels include the exclusion of specified objectionable microbiological agents. By way of example, specified objectionable microbiological agents include Escherichia coli (E. coli), Salmonella sp., Pseudomonas aeruginosa (P. aeruginosa) and/or other specific microbial agents.

Sterility of the otic formulation is confirmed through a sterility assurance program in accordance with United States Pharmacopeia Chapters <61>, <62> and <71>. A key component of the sterility assurance quality control, quality assurance and validation process is the method of sterility testing. Sterility testing, by way of example only, is performed by two methods. The first is direct inoculation wherein a sample of the formulation to be tested is added to growth medium and incubated for a period of time up to 21 days. Turbidity of the growth medium indicates contamination. Drawbacks to this method include the small sampling size of bulk materials which reduces sensitivity, and detection of microorganism growth based on a visual observation. An alternative method is membrane filtration sterility testing. In this method, a volume of product is passed through a small membrane filter paper. The filter paper is then placed into media to promote the growth of microorganisms. This method has the advantage of greater sensitivity as the bulk product is sampled. The commercially available Millipore Steritest sterility testing system is optionally used for determinations by membrane filtration sterility testing. For the filtration testing of creams or ointments Steritest filter system No. TLHVSL210 are used. For the filtration testing of emulsions or viscous products Steritest filter system No. TLAREM210 or TDAREM210 are used. For the filtration testing of pre-filled syringes Steritest filter system No. TTHASY210 are used. For the filtration testing of material dispensed as an aerosol or foam Steritest filter system No. TTHVA210 are used. For the filtration testing of soluble powders in ampoules or vials Steritest filter system No. TTHADA210 or TTHADV210 are used.

Testing for E. coli and Salmonella includes the use of lactose broths incubated at 30 - 35 °C for 24-72 hours, incubation in MacConkey and/or EMB agars for 18-24 hours, and/or the use of Rappaport medium. Testing for the detection of P. aeruginosa includes the use of NAC agar. United States Pharmacopeia Chapter <62> further enumerates testing procedures for specified objectionable microorganisms.

In certain embodiments, any otic formulation described herein has less than about 60 colony forming units (CFU), less than about 50 colony forming units, less than about 40 colony forming units, or less than about 30 colony forming units of microbial agents per gram of formulation. In certain embodiments, the otic formulations described herein are formulated to be isotonic with the endolymph and/or the perilymph.

*Endotoxins*

Provided herein are otic formulations that ameliorate or lessen otic disorders described herein.. In some embodiments, the otic formulations are substantially free of endotoxins. An additional aspect of the sterilization process is the removal of by-products from the killing of microorganisms (hereinafter, "Product"). The process of depyrogenation removes pyrogens from the sample. Pyrogens are endotoxins or exotoxins which induce an immune response. An example of an endotoxin is the lipopolysaccharide (LPS) molecule found in the cell wall of gram-negative bacteria. While sterilization procedures such as autoclaving or treatment with ethylene oxide kill the bacteria, the LPS residue induces a proinflammatory immune response, such as septic shock. Because the molecular size of endotoxins varies widely, the

presence of endotoxins is expressed in "endotoxin units" (EU). One EU is equivalent to 100 picograms of E. coli LPS. In some cases, humans develop a response to as little as 5 EU/kg of body weight. The sterility is expressed in any units as recognized in the art. In certain embodiments, otic formulations described herein contain lower endotoxin levels (e.g. < 4 EU/kg of body weight of a subject) when compared to conventionally acceptable endotoxin levels (e.g., 5 EU/kg of body weight of a subject). In some embodiments, the otic formulation has less than about 5 EU/kg of body weight of a subject. In other embodiments, the otic formulation has less than about 4 EU/kg of body weight of a subject. In additional embodiments, the otic formulation has less than about 3 EU/kg of body weight of a subject. In additional embodiments, the otic formulation has less than about 2 EU/kg of body weight of a subject.

In some embodiments, the otic formulation has less than about 5 EU/kg of formulation. In other embodiments, the otic therapeutic formulation has less than about 4 EU/kg of formulation. In additional embodiments, the otic formulation has less than about 3 EU/kg of formulation. In some embodiments, the otic formulation has less than about 5 EU/kg Product. In other embodiments, the otic formulation has less than about 1 EU/kg Product. In additional embodiments, the otic formulation has less than about 0.2 EU/kg Product. In some embodiments, the otic formulation has less than about 5 EU/g of unit or Product. In other embodiments, the otic formulation has less than about 4 EU/ g of unit or Product. In additional embodiments, the otic formulation has less than about 3 EU/g of unit or Product. In some embodiments, the otic formulation has less than about 5 EU/mg of unit or Product. In other embodiments, the otic formulation has less than about 4 EU/ mg of unit or Product. In additional embodiments, the otic formulation has less than about 3 EU/mg of unit or Product. In certain embodiments, otic formulations described herein contain from about 1 to about 5 EU/mL of formulation . In certain embodiments, otic formulations described herein contain from about 2 to about 5 EU/mL of formulation , from about 3 to about 5 EU/mL of formulation , or from about 4 to about 5 EU/mL of formulation .

In certain embodiments, otic formulations described herein contain lower endotoxin levels (e.g. < 0.5 EU/mL of formulation ) when compared to conventionally acceptable endotoxin levels (e.g., 0.5 EU/mL of formulation ). In some embodiments, the otic formulation has less than about 0.5 EU/mL of formulation . In other embodiments, the otic formulation has less than about 0.4 EU/mL of formulation . In additional embodiments, the otic formulation has less than about 0.2 EU/mL of formulation .

Pyrogen detection, by way of example only, is performed by several methods. Suitable tests for sterility include tests described in United States Pharmacopoeia (USP) <71> Sterility Tests (23rd edition, 1995). The rabbit pyrogen test and the Limulus amebocyte lysate test are both specified in the United States Pharmacopeia Chapters <85> and <151> (USP23/NF 18, Biological Tests, The United States Pharmacopeial Convention, Rockville, MD, 1995). Alternative pyrogen assays have been developed based upon the monocyte activation-cytokine assay. Uniform cell lines suitable for quality control applications have been developed and have demonstrated the ability to detect pyrogenicity in samples that have passed the rabbit pyrogen test and the Limulus amebocyte lysate test (Taktak et al, J. Pharm. Pharmacol. (1990), 43:578-82). In an additional embodiment, the otic formulation is subject to depyrogenation. In a further embodiment, the process for the manufacture of the otic formulation comprises testing the formulation for pyrogenicity. In certain embodiments, the formulations described herein are substantially free of pyrogens.

### pH and Osmolarity

Described herein are otic formulations with an ionic balance that is compatible with the perilymph and/or the endolymph and does not cause any change in cochlear potential. In specific embodiments, osmolarity/osmolality of the present formulations is adjusted, for example, by the use of appropriate salt concentrations (e.g., concentration of sodium salts) or the use of tonicity agents which renders the formulations endolymph-compatible and/or perilymph compatible (i.e. isotonic with the endolymph and/or perilymph). In some instances, the endolymph-compatible and/or perilymph-compatible formulations described herein cause minimal disturbance to the environment of the inner ear and cause minimum discomfort (e.g., vertigo) to a mammal (e.g., a human) upon administration. In some embodiments, the formulations described herein are free of preservatives and cause minimal disturbance (e.g., change in pH or osmolarity, irritation) in auditory structures. In some embodiments, the formulations described herein comprise antioxidants that are non-irritating and/or non-toxic to otic structures.

As used herein, "practical osmolarity" means the osmolarity of a formulation that is measured by including the active agent and all excipients except the gelling and/or the thickening agent (e.g., polyoxyethylene-polyoxypropylene copolymers, carboxymethylcellulose or the like). The practical osmolarity of a formulation described herein is measured by any suitable method, e.g., a freezing point depression method as described in Viegas et. al., Int. J. Pharm., 1998, 160, 157-162. In some instances, the practical osmolarity of a formulation described herein is measured by vapor pressure osmometry (e.g., vapor pressure depression method) that allows for determination of the osmolarity of a formulation at higher temperatures. In some instances, vapor pressure depression method allows for determination of the osmolarity of a formulation comprising a gelling agent (e.g., a thermoreversible polymer) at a higher temperature wherein the gelling agent is in the form of a gel. In some embodiments, the practical osmolality of an otic formulation described herein is from about 100 mOsm/kg to about 1000 mOsm/kg, from about 200 mOsm/kg to about 800 mOsm/kg, from about 250 mOsm/kg

to about 500 mOsm/kg, or from about 250 mOsm/kg to about 320 mOsm/kg, or from about 250 mOsm/kg to about 350 mOsm/kg or from about 280 mOsm/kg to about 320 mOsm/kg. In some embodiments, the formulations described herein have a practical osmolarity of about 100 mOsm/L to about 1000 mOsm/L, about 200 mOsm/L to about 800 mOsm/L, about 250 mOsm/L to about 500 mOsm/L, about 250 mOsm/L to about 350 mOsm/L, about 250 mOsm/L to about 320 mOsm/L, or about 280 mOsm/L to about 320 mOsm/L.

In some embodiments, the osmolarity at a target site of action (e.g., the perilymph) is about the same as the delivered osmolarity (i.e., osmolarity of materials that cross or penetrate the round window membrane) of any formulation described herein. In some embodiments, the formulations described herein have a deliverable osmolarity of about 150 mOsm/L to about 500 mOsm/L, about 250 mOsm/L to about 500 mOsm/L, about 250 mOsm/L to about 350 mOsm/L, about 280 mOsm/L to about 370 mOsm/L or about 250 mOsm/L to about 320 mOsm/L.

The main cation present in the endolymph is potassium. In addition the endolymph has a high concentration of positively charged amino acids. The main cation present in the perilymph is sodium. In certain instances, the ionic composition of the endolymph and perilymph regulate the electrochemical impulses of hair cells. In certain instances, any change in the ionic balance of the endolymph or perilymph results in a loss of hearing due to changes in the conduction of electrochemical impulses along otic hair cells. In some embodiments, a formulation disclosed herein does not disrupt the ionic balance of the perilymph. In some embodiments, a formulation disclosed herein has an ionic balance that is the same as or substantially the same as the perilymph. In some embodiments, a formulation disclosed herein does not disrupt the ionic balance of the endolymph. In some embodiments, a formulation disclosed herein has an ionic balance that is the same as or substantially the same as the endolymph. In some embodiments, a formulation described herein is formulated to provide an ionic balance that is compatible with inner ear fluids (i.e., endolymph and/or perilymph).

The endolymph and the perilymph have a pH that is close to the physiological pH of blood. The endolymph has a pH range of about 7.2-7.9; the perilymph has a pH range of about 7.2 - 7.4. The *in situ* pH of the proximal endolymph is about 7.4 while the pH of distal endolymph is about 7.9.

In some embodiments, the pH of a formulation described herein is adjusted (e.g., by use of a buffer) to an endolymph-compatible pH range of about 7.0 to 8.0, and a preferred pH range of about 7.2 - 7.9. In some embodiments, the pH of the formulations described herein is adjusted (e.g., by use of a buffer) to a perilymph -compatible pH of about 7.0 - 7.6, and a preferred pH range of about 7.2-7.4.

In some embodiments, useful formulations also include one or more pH adjusting agents or buffering agents. Suitable pH adjusting agents or buffers include acetate, bicarbonate, ammonium chloride, citrate, phosphate, pharmaceutically acceptable salts thereof and combinations or mixtures thereof.

In one embodiment, when one or more buffers are utilized in the formulations of the present disclosure, they are combined, e.g., with a pharmaceutically acceptable vehicle and are present in the final formulation , e.g., in an amount ranging from about 0.1% to about 20%, from about 0.5% to about 10%. In certain embodiments of the present disclosure, the amount of buffer included in the formulations are an amount such that the pH of the formulation does not interfere with the body's natural buffering system. In some embodiments, from about 5 mM to about 200 mM concentration of a buffer is present in the formulation . In certain embodiments, from about a 20 mM to about a 100 mM concentration of a buffer is present. In other embodiments, the concentration of buffer is such that a pH of the formulation is between 3 and 9, between 5 and 8, or alternatively between 6 and 7. In other embodiments, the pH of the formulation is about 7. In one embodiment is a buffer such as acetate or citrate at slightly acidic pH. In one embodiment the buffer is a sodium acetate buffer having a pH of about 4.5 to about 6.5. In another embodiment the buffer is a sodium acetate buffer having a pH of about 5.5 to about 6.0. In a further embodiment the buffer is a sodium acetate buffer having a pH of about 6.0 to about 6.5. In one embodiment the buffer is a sodium citrate buffer having a pH of about 5.0 to about 8.0. In another embodiment the buffer is a sodium citrate buffer having a pH of about 5.5 to about 7.0. In one embodiment the buffer is a sodium citrate buffer having a pH of about 6.0 to about 6.5.

In some embodiments, the concentration of buffer is such that a pH of the formulation is between 6 and 9, between 6 and 8, between 6 and 7.6, between 7 and 8. In other embodiments, the pH of the formulation is about 6.0, about 6.5, about 7 or about 7.5. In one embodiment is a buffer such as tris(hydroxymethyl)aminomethane, bicarbonate, carbonate or phosphate at slightly basic pH. In one embodiment, the buffer is a sodium bicarbonate buffer having a pH of about 7.5 to about 8.5. In another embodiment the buffer is a sodium bicarbonate buffer having a pH of about 7.0 to about 8.0. In a further embodiment the buffer is a sodium bicarbonate buffer having a pH of about 6.5 to about 7.0. In one embodiment the buffer is a sodium phosphate dibasic buffer having a pH of about 6.0 to about 9.0. In another embodiment the buffer is a sodium phosphate dibasic buffer having a pH of about 7.0 to about 8.5. In one embodiment the buffer is a sodium phosphate dibasic buffer having a pH of about 7.5 to about 8.0.

In one embodiment, diluents are also used to stabilize compounds because they provide a more stable environment. Salts dissolved in buffered solutions (which also provide pH control or maintenance) are utilized as diluents in the art, including a phosphate buffered saline solution.

In a specific embodiment the pH of a formulation described herein is between about 6.0 and about 7.6, between 7 and about 7.8, between about 7.0 and about 7.6, between about 7.2 and about 7.6, or between about 7.2 and about 7.4. In

certain embodiments the pH of a formulation described herein is about 6.0, about 6.5, about 7.0, about 7.1, about 7.2, about 7.3, about 7.4, about 7.5, or about 7.6. In some embodiments, the pH of any formulation described herein is designed to be compatible with the targeted otic structure (e.g., endolymph, perilymph or the like).

In some embodiments, any formulation described herein has a pH that allows for sterilization (e.g., by filtration or aseptic mixing or heat treatment and/or autoclaving (e.g., terminal sterilization)) of a formulation without degradation of the therapeutic agent. In order to reduce hydrolysis and/or degradation of the therapeutic agent during sterilization, the buffer pH is designed to maintain pH of the formulation in the 7-8 range during the process of sterilization.

In specific embodiments, any formulation described herein has a pH that allows for terminal sterilization (e.g., by heat treatment and/or autoclaving) of a formulation without degradation of the therapeutic agent. For example, in order to reduce hydrolysis and/or degradation of the therapeutic agent during autoclaving, the buffer pH is designed to maintain pH of the formulation in the 7-8 range at elevated temperatures. Any appropriate buffer is used depending on the therapeutic agent used in the formulation . In some instances, since $pK_a$ of TRIS decreases as temperature increases at approximately -0.03/°C and $pK_a$ of PBS increases as temperature increases at approximately 0.003/°C, autoclaving at 250°F (121°C) results in a significant downward pH shift (i.e. more acidic) in the TRIS buffer whereas a relatively much less upward pH shift in the PBS buffer and therefore much increased hydrolysis and/or degradation of an otic agent in TRIS than in PBS. In some embodiments, degradation of a therapeutic agent is reduced by the use of an appropriate of a buffer as described herein.

In some embodiments, a pH of between about 6.0 and about 7.6, between about 7 and about 7.8, between about 7.0 and about 7.6, between about 7.2 and 7.6, between about 7.2 and about 7.4 is suitable for sterilization (e.g., by filtration or aseptic mixing or heat treatment and/or autoclaving (e.g., terminal sterilization)) of formulations described herein. In specific embodiments a formulation pH of about 6.0, about 6.5, about 7.0, about 7.1, about 7.2, about 7.3, about 7.4, about 7.5, or about 7.6 is suitable for sterilization (e.g., by filtration or aseptic mixing or heat treatment and/or autoclaving (e.g., terminal sterilization)) of any formulation described herein.

In some embodiments, the formulations described herein have a pH between about 3 and about 9, or between about 4 and 8, or between about 5 and 8, or between about 6 and about 7, or between about 6.5 and about 7, or between about 5.5 and about 7.5, or between about 7.1 and about 7.7, and have a concentration of active pharmaceutical ingredient between about 0.1 mM and about 100 mM. In some embodiments, the formulations described herein have a pH between about 5 and about 8, or between about 6 and about 7, or between about 6.5 and about 7, or between about 5.5 and about 7.5, or between about 7.1 and about 7.7, and have a concentration of active pharmaceutical ingredient between about 1 and about 100 mM. In some embodiments, the formulations described herein have a pH between about 5 and about 8, or between about 6 and about 7, or between about 6.5 and about 7, or between about 5.5 and about 7.5, or between about 7.1 and about 7.7, and have a concentration of active pharmaceutical ingredient between about 50 and about 80 mM. In some embodiments, the concentration of active pharmaceutical ingredient between about 10 and about 100 mM. In other embodiments, the concentration of active pharmaceutical ingredient between about 20 and about 80 mM. In additional embodiments, the concentration of active pharmaceutical ingredient between about 10 and about 50 mM.

In some embodiments, the formulations have a pH as described herein, and include a thickening agent (i.e., a viscosity enhancing agent or viscosity modulating agent) such as, by way of non-limiting example, a cellulose based thickening agent described herein. In some instances, the addition of a thickening agent and a pH of formulation as described herein, allows for sterilization of a formulation described herein without any substantial degradation of the therapeutic agent in the otic formulation . In some embodiments, the amount of thickening agent in any formulation described herein is about 1%, 5%, about 10%, or about 15% of the total weight of the formulation . In some embodiments, the amount of thickening agent in any formulation described herein is about 0.5%, about 1%, about 1.5%, about 2%, about 2.5%, about 3%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10%, or about 15%. In some instances, the addition of a secondary polymer (e.g., a thickening agent) and a pH of formulation as described herein, allows for sterilization of a formulation described herein without any substantial degradation of the otic agent and/or the polymer components in the otic formulation. In some embodiments, the ratio of a thermoreversible poloxamer to a thickening agent in a formulation that has a pH as described herein is about 40:1, about 35:1, about 30:1, about 25:1, about 20:1, about 15:1, about 10:1, or about 5:1. For example, in certain embodiments, a sustained and/or extended release formulation described herein comprises a combination of poloxamer 407 (pluronic F127) and carboxymethylcellulose (CMC) in a ratio of about 40:1, about 35:1, about 30:1, about 25:1, about 20:1, about 15:1, about 10:1, or about 5:1.4%, about 4.5%, or about 5% of the total weight of the formulation.

In some embodiments, the amount of thermoreversible polymer in any formulation described herein is about 0.01%, about 0.05%, about 0.1%, about 0.5%, about 1%, about 5%, about 10%, about 15%, about 20%, about 25%, about 30%, about 35%, or about 40% of the total weight of the formulation. In some embodiments, the amount of thermoreversible polymer in any formulation described herein is about 0.01%, about 0.05%, about 0.1%, about 0.5%, about 1%, about 5%, about 10%, about 11%, about 12%, about 13%, about 14%, about 15%, about 16%, about 17%, about 18%, about 19%, about 20%, about 21%, about 22%, about 23%, about 24%, or about 25% of the total weight of the formulation.

In some embodiments, the amount of thermoreversible polymer (e.g., pluronic F127) in any formulation described herein is about 7.5% of the total weight of the formulation. In some embodiments, the amount of thermoreversible polymer (e.g., pluronic F127) in any formulation described herein is about 10% of the total weight of the formulation. In some embodiments, the amount of thermoreversible polymer (e.g., pluronic F127) in any formulation described herein is about 11% of the total weight of the formulation. In some embodiments, the amount of thermoreversible polymer (e.g., pluronic F127) in any formulation described herein is about 12% of the total weight of the formulation. In some embodiments, the amount of thermoreversible polymer (e.g., pluronic F127) in any formulation described herein is about 13% of the total weight of the formulation. In some embodiments, the amount of thermoreversible polymer (e.g., pluronic F127) in any formulation described herein is about 14% of the total weight of the formulation. In some embodiments, the amount of thermoreversible polymer (e.g., pluronic F127) in any formulation described herein is about 15% of the total weight of the formulation. In some embodiments, the amount of thermoreversible polymer (e.g., pluronic F127) in any formulation described herein is about 16% of the total weight of the formulation. In some embodiments, the amount of thermoreversible polymer (e.g., pluronic F127) in any formulation described herein is about 17% of the total weight of the formulation. In some embodiments, the amount of thermoreversible polymer (e.g., pluronic F127) in any formulation described herein is about 18% of the total weight of the formulation. In some embodiments, the amount of thermoreversible polymer (e.g., pluronic F127) in any formulation described herein is about 19% of the total weight of the formulation. In some embodiments, the amount of thermoreversible polymer (e.g., pluronic F127) in any formulation described herein is about 20% of the total weight of the formulation. In some embodiments, the amount of thermoreversible polymer (e.g., pluronic F127) in any formulation described herein is about 21% of the total weight of the formulation. In some embodiments, the amount of thermoreversible polymer (e.g., pluronic F127) in any formulation described herein is about 23% of the total weight of the formulation. In some embodiments, the amount of thermoreversible polymer (e.g., pluronic F127) in any formulation described herein is about 25% of the total weight of the formulation.

In some embodiments, the amount of thickening agent (e.g., a gelling agent) in any formulation described herein is about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1%, about 5%, about 10%, or about 15% of the total weight of the formulation. In some embodiments, the amount of thickening agent (e.g., a gelling agent) in any formulation described herein is about 0.1%, 0.5%, about 1%, about 1.5%, about 2%, about 2.5%, about 3%, about 3.5%, about 4%, about 4.5%, or about 5% of the total weight of the formulation.

In some embodiments, the pharmaceutical formulations described herein are stable with respect to pH over a period of any of at least about 1 day, at least about 2 days, at least about 3 days, at least about 4 days, at least about 5 days, at least about 6 days, at least about 1 week, at least about 2 weeks, at least about 3 weeks, at least about 4 weeks, at least about 5 weeks, at least about 6 weeks, at least about 7 weeks, at least about 8 weeks, at least about 1 month, at least about 2 months, at least about 3 months, at least about 4 months, at least about 5 months, or at least about 6 months. In other embodiments, the formulations described herein are stable with respect to pH over a period of at least about 1 week. Also described herein are formulations that are stable with respect to pH over a period of at least about 1 month.

*Tonicity Agents*

In general, the endolymph has a higher osmolality than the perilymph. For example, the endolymph has an osmolality of about 304 mOsm/kg $H_2O$ while the perilymph has an osmolality of about 294 mOsm/kg $H_2O$. In some embodiments, formulations described herein are formulated to provide an osmolarity of about 250 to about 320 mM (osmolality of about 250 to about 320 mOsm/kg $H_2O$); and preferably about 270 to about 320 mM (osmolality of about 270 to about 320 mOsm/kg $H_2O$). In certain embodiments, tonicity agents are added to the formulations described herein in an amount as to provide a practical osmolality of an otic formulation of about 100 mOsm/kg to about 1000 mOsm/kg, from about 200 mOsm/kg to about 800 mOsm/kg, from about 250 mOsm/kg to about 500 mOsm/kg, from about 250 mOsm/kg to about 350 mOsm/kg, or from about 280 mOsm/kg to about 320 mOsm/kg. In some embodiments, the formulations described herein have a practical osmolarity of about 100 mOsm/L to about 1000 mOsm/L, about 200 mOsm/L to about 800 mOsm/L, about 250 mOsm/L to about 500 mOsm/L, about 250 mOsm/L to about 350 mOsm/L, about 280 mOsm/L to about 320 mOsm/L, or about 250 mOsm/L to about 320 mOsm/L.

In specific embodiments, osmolarity/osmolality of the present formulations is adjusted, for example, by the use of appropriate salt concentrations (e.g., concentration of potassium salts) or the use of tonicity agents which renders the formulations endolymph-compatible and/or perilymph-compatible (i.e. isotonic with the endolymph and/or perilymph. In some instances, the endolymph-compatible and/or perilymph-compatible formulations described herein cause minimal disturbance to the environment of the inner ear and cause minimum discomfort (e.g., vertigo and/or nausea) to a mammal upon administration.

In some embodiments, the deliverable osmolarity of any formulation described herein is designed to be isotonic with the targeted otic structure (e.g., endolymph, perilymph, or the like). In specific embodiments, auris formulations described herein are formulated to provide a delivered perilymph-suitable osmolarity at the target site of action of about

250 to about 320 mOsm/L and preferably about 270 to about 320 mOsm/L. In specific embodiments, auris formulations described herein are formulated to provide a delivered perilymph-suitable osmolality at the target site of action of about 250 to about 320 mOsm/kg $H_2O$ or an osmolality of about 270 to about 320 mOsm/kg $H_2O$. In specific embodiments, the deliverable osmolarity/osmolality of the formulations (i.e., the osmolarity/osmolality of the formulation in the absence of gelling or thickening agents (e.g., thermoreversible gel polymers) is adjusted, for example, by the use of appropriate salt concentrations (e.g., concentration of potassium or sodium salts) or the use of tonicity agents which renders the formulations endolymph-compatible and/or perilymph-compatible (i.e. isotonic with the endolymph and/or perilymph) upon delivery at the target site. The osmolarity of a formulation comprising a thermoreversible gel polymer is an unreliable measure due to the association of varying amounts of water with the monomeric units of the polymer. The practical osmolarity of a formulation (i.e., osmolarity in the absence of a gelling or thickening agent (e.g. a thermoreversible gel polymer) is a reliable measure and is measured by any suitable method (e.g., freezing point depression method, vapor depression method). In some instances, the formulations described herein provide a deliverable osmolarity (e.g., at a target site (e.g., perilymph) that causes minimal disturbance to the environment of the inner ear and causes minimum discomfort (e.g., vertigo and/or nausea) to a mammal upon administration.

In some embodiments, any formulation described herein is isotonic with the perilymph. Isotonic formulations are provided by the addition of a tonicity agent. Suitable tonicity agents include any pharmaceutically acceptable sugar, salt or any combinations or mixtures thereof, such as dextrose, glycerin, mannitol, sorbitol, sodium chloride, and other electrolytes.

Useful otic formulations include one or more salts in an amount required to bring osmolality of the formulation into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite, and ammonium sulfate.

In further embodiments, the tonicity agents are present in an amount as to provide a final osmolality of an otic formulation of about 100 mOsm/kg to about 500 mOsm/kg, from about 200 mOsm/kg to about 400 mOsm/kg, from about 250 mOsm/kg to about 350 mOsm/kg or from about 280 mOsm/kg to about 320 mOsm/kg. In some embodiments, the formulations described herein have a osmolarity of about 100 mOsm/L to about 500 mOsm/L, about 200 mOsm/L to about 400 mOsm/L, about 250 mOsm/L to about 350 mOsm/L, or about 280 mOsm/L to about 320 mOsm/L. In some embodiments, the osmolarity of any formulation described herein is designed to be isotonic with the targeted otic structure (e.g., endolymph, perilymph or the like). In some embodiments, the formulations described herein have a pH and/or practical osmolarity as described herein, and have a concentration of active pharmaceutical ingredient from about 0.0001% to about 60%, from about 0.001% to about 40%, from about 0.01% to about 20%, from about 0.01% to about 10%, from about 0.01% to about 7.5%, from about 0.01% to about 6%, from about 0.01 to about 5%, from about 0.1 to about 10%, or from about 0.1 to about 6% of the active ingredient by weight of the formulation.

In some embodiments, the formulations described herein have a pH and osmolarity as described herein, and have a concentration of active pharmaceutical ingredient between about 1 μM and about 10 μM, between about 1 mM and about 100 mM, between about 0.1 mM and about 100 mM, between about 0.1 mM and about 100 nM. In some embodiments, the formulations described herein have a pH and osmolarity as described herein, and have a concentration of active pharmaceutical ingredient between about 0.01 - about 20%, between about 0.01 - about 10%, between about 0.01 - about 7%, between about 0.01 - 5%, between about 0.01 - about 3%, between about 0.01 - about 2% of the active ingredient by weight of the formulation . In some embodiments, the formulations described herein have a pH and osmolarity as described herein, and have a concentration of active pharmaceutical ingredient between about 0.1 - about 70 mg/mL, between about 1 mg - about 70 mg/mL, between about 1 mg - about 50 mg/mL, between about 1 mg/mL and about 20 mg/mL, between about 1 mg/mL to about 10 mg/mL, between about 1 mg/mL to about 5 mg/mL, or between about 0.5 mg/mL to about 5 mg/mL of the active agent by volume of the formulation .

**Tunable release characteristics**

The release of the therapeutic agent described herein from any formulation, or device described herein is optionally tunable to the desired release characteristics. In some embodiments, a formulation described herein is a solution that is substantially free of gelling components. In such instances, the formulation provides essentially immediate release of the therapeutic agent. In some of such embodiments, the formulation is useful in perfusion of otic structures, e.g., during surgery.

In some of such embodiments, the formulation provides release of the therapeutic agent from about 2 days to about 4 days.

In some embodiments, a formulation described herein, further comprises a gelling agent (e.g., poloxamer 407) and provides release of the therapeutic agent over a period of from about 1 day to about 3 days. In some embodiments, a formulation described herein, further comprises a gelling agent (e.g., poloxamer 407) and provides release of the therapeutic agent over a period of from about 1 day to about 5 days. In some embodiments, a formulation described

herein, further comprises a gelling agent (e.g., poloxamer 407) and provides release of the therapeutic agent over a period of from about 2 days to about 7 days.

In some embodiments, a formulation described herein further comprises from about 14 to about 17% of a gelling agent (e.g., poloxamer 407), and provides extended sustained release over a period of from about 1 week to about 3 weeks. In some embodiments, a formulation described herein, further comprises from about 18 to about 21% of a gelling agent (e.g., poloxamer 407) and, provides extended sustained release over a period of from about 3 weeks to about 6 weeks.

In some embodiments, the viscosity of any formulation described herein, is designed to provide a suitable rate of release from an auris compatible gel. In some embodiments, the concentration of a thickening agent (e.g., gelling components such as polyoxyethylene-polyoxypropylene copolymers) allows for a tunable mean dissolution time (MDT). The MDT is inversely proportional to the release rate of an active agent from a formulation or device described herein. Experimentally, the released active agent is optionally fitted to the Korsmeyer-Peppas equation

$$\frac{Q}{Q_\alpha} = kt^n + b$$

where Q is the amount of active agent released at time $t$, $Q_\alpha$ is the overall released amount of active agent, $k$ is a release constant of the nth order, $n$ is a dimensionless number related to the dissolution mechanism, and b is the axis intercept, characterizing the initial burst release mechanism wherein $n$=1 characterizes an erosion controlled mechanism. The mean dissolution time (MDT) is the sum of different periods of time the drug molecules stay in the matrix before release, divided by the total number of molecules, and is optionally calculated by:

$$MDT = \frac{nk^{-1/n}}{n+1}$$

For example, a linear relationship between the mean dissolution time (MDT) of a formulation or device and the concentration of the gelling agent (e.g., poloxamer) indicates that the active agent is released due to the erosion of the polymer gel (e.g., poloxamer) and not via diffusion. In another example, a non-linear relationship indicates release of otic agent via a combination of diffusion and/or polymer gel degradation. In another example, a faster gel elimination time course of a formulation or device (a faster release of active agent) indicates lower mean dissolution time (MDT). The concentration of gelling components and/or active agent in a formulation are tested to determine suitable parameters for MDT. In some embodiments, injection volumes are also tested to determine suitable parameters for preclinical and clinical studies. The gel strength and concentration of the active agent affects release kinetics of the active agent from the formulation. At low poloxamer concentration, elimination rate is accelerated (MDT is lower). An increase in the active agent concentration in the formulation or device prolongs residence time and/or MDT of the active agent in the ear.

In some embodiments, the MDT for poloxamer from a formulation or device described herein is at least 6 hours. In some embodiments, the MDT for poloxamer from a formulation or device described herein is at least 10 hours.

In some embodiments, the MDT for an active agent from a formulation or device described herein is from about 30 hours to about 48 hours. In some embodiments, the MDT for an active agent from a formulation or device described herein is from about 30 hours to about 96 hours. In some embodiments, the MDT for an active agent from a formulation or device described herein is from about 30 hours to about 1 week. In some embodiments, the MDT for an active agent from a formulation or device described herein is from about 1 week to about 6 weeks.

In certain embodiments, any controlled-release otic formulation described herein increases the exposure of an active agent and increases the Area Under the Curve (AUC) in otic fluids (e.g., endolymph and/or perilymph) by about 30%, about 40%, about 50%, about 60%, about 70%, about 80%, about 90%, about 100%, or higher than 100%, compared to an otic formulation that is not a controlled-release otic formulation. In certain embodiments, any controlled-release otic formulation described herein increases the exposure time of an active agent and decreases the $C_{max}$ in otic fluids (e.g., endolymph and/or perilymph) by about 40%, about 30%, about 20%, or about 10%, compared to a formulation that is not a controlled-release otic formulation. In certain embodiments, any controlled-release otic formulation described herein alters (e.g. reduces) the ratio of $C_{max}$ to $C_{min}$ compared to a formulation that is not a controlled-release otic formulation. In certain embodiments, any controlled-release otic formulation described herein increases the exposure of an active agent and increases the length of time that the concentration of the active agent is above $C_{min}$ by about 30%, about 40%, about 50%, about 60%, about 70%, about 80%, or about 90% compared to a formulation that is not a controlled-release otic formulation. In certain embodiments, the increase in exposure of an active agent and the increase in the length of time that the concentration of the active agent is above $C_{min}$ by a controlled-release otic formulation described herein is greater than 100% compared to a formulation that is not a controlled-release otic formulation. In certain instances, controlled-release

otic formulations described herein delay the time to $C_{max}$. In certain instances, the controlled steady release of a drug prolongs the time the concentration of the active agent will stay above the $C_{min}$. In some embodiments, otic formulations described herein prolong the residence time of an active agent in the inner ear and provide a stable drug exposure profile. In some instances, an increase in concentration of an active agent in the otic formulation saturates the clearance process and allows for a more rapid and stable steady state to be reached.

In certain instances, once exposure to an active agent (e.g., concentration in the endolymph or perilymph) reaches steady state, the concentration of an active agent in the endolymph or perilymph stays at or about the therapeutic dose for an extended period of time (e.g., one day, 2 days, 3 days, 4 days, 5 days, 6 days, 1 week, 3 weeks, 6 weeks, or 2 months). In some embodiments, the steady state concentration of an active agent released from a controlled-release otic formulation described herein is from about 20 to about 50 times the steady state concentration of an active agent released from a formulation that is not a controlled-release otic formulation.

**Particle size**

Size reduction is used to increase surface area and/or modulate formulation dissolution properties. It is also used to maintain a consistent average particle size distribution (PSD) (e.g., micrometer-sized particles, nanometer-sized particles or the like) for any formulation described herein. In some embodiments, the formulation comprises micrometer-sized particles. In some embodiments, the formulation comprises nanometer-sized particles. In some instances, any formulation described herein comprises multiparticulates, i.e., a plurality of particle sizes (e.g., micronized particles, nano-sized particles, non-sized particles); i.e., the formulation is a multiparticulate formulation . In some embodiments, any formulation described herein comprises one or more multiparticulate (e.g., micronized) therapeutic agents. Micronization is a process of reducing the average diameter of particles of a solid material. Micronized particles are from about micrometer-sized in diameter to about picometer -sized in diameter. In some embodiments, the use of multiparticulates (e.g., micronized particles) of a therapeutic agent, or an otic agent, allows for extended and/or sustained release of the therapeutic agent from any formulation described herein compared to a formulation comprising non-multiparticulate (e.g., non-micronized) therapeutic agent. In some instances, formulations containing multiparticulate (e.g., micronized) therapeutic agents are ejected from a 1mL syringe adapted with a 27G needle without any plugging or clogging. In some embodiments, the therapeutic agent is essentially in the form of micronized particles. In some embodiments, the therapeutic agent is essentially in the form of microsized particles. In some embodiments, the therapeutic agent is essentially in the form of nanosized particles.

In some embodiments, the particle size of the formulation described herein increases the retention time of the formulation described herein. In some embodiments, the particle size of the formulation described herein provides slow release of the therapeutic agent. In some embodiments, the particle size of the formulation described herein provides sustained release of the therapeutic agent. In some embodiments, the particle size is less than 450 nm, less than 400 nm, less than 350 nm, less than 300 nm, less than 275 nm, less than 250 nm, less than 225 nm, less than 200 nm in size, less than 175 nm, less than 150 nm, or less than 125 nm, or less than 100 nm. In some embodiments, the particle size is less than 300 nm. In some embodiments, the particle size is less than 250 nm. In some embodiments, the particle size is less than 200 nm.

In some instances, any particle in any formulation described herein is a coated particle (e.g., a coated micronized particle) and/or a microsphere and/or a liposomal particle. Particle size reduction techniques include, by way of example, grinding, milling (e.g., air-attrition milling (jet milling), ball milling), coacervation, high pressure homogenization, spray drying and/or supercritical fluid crystallization. In some instances, particles are sized by mechanical impact (e.g., by hammer mills, ball mill and/or pin mills). In some instances, particles are sized via fluid energy (e.g., by spiral jet mills, loop jet mills, and/or fluidized bed jet mills). In some embodiments formulations described herein comprise crystalline particles. In some embodiments, formulations described herein comprise amorphous particles. In some embodiments, formulations described herein comprise therapeutic agent particles wherein the therapeutic agent is a free base, or a salt, or a prodrug of a therapeutic agent, or any combination thereof.

In some instances, a combination of a therapeutic agent and a salt of the therapeutic agent is used to prepare pulsed release otic formulations using the procedures described herein. In some formulations, a combination of a micronized therapeutic agent (and/or salt or prodrug thereof) and coated particles (e.g., nanoparticles, liposomes, microspheres) is used to prepare pulsed release otic formulations using any procedure described herein.

In some embodiments, a pulsed release profile is achieved by solubilizing up to 40% of the delivered dose of the therapeutic agent (e.g., micronized therapeutic agent, or free base or salt or prodrug thereof; multiparticulate therapeutic agent, or free base or salt or prodrug thereof) with the aid of cyclodextrins, surfactants (e.g., poloxamers (407, 338, 188), tween (80, 60, 20,81), PEG-hydrogenated castor oil, cosolvents like N-methyl-2-Pyrrolidone or the like and preparing pulsed release formulations using any procedure described herein.

In some specific embodiments, any otic formulation described herein comprises one or more micronized therapeutic agents. In some of such embodiments, a micronized therapeutic agent comprises micronized particles,

coated (e.g., with an extended release coat) micronized particles, or a combination thereof. In some of such embodiments, a micronized therapeutic agent comprising micronized particles, coated micronized particles, or a combination thereof, comprises a therapeutic agent as a free base, a salt, a prodrug or any combination thereof.

**Controlled Release Otic Formulations (of which auris-acceptable thermoreversible gels are specifically claimed)**

In certain embodiments, any controlled release otic formulation described herein increases the exposure of a therapeutic agent and increases the Area Under the Curve (AUC) in otic fluids (e.g., endolymph and/or perilymph) by about 30%, about 40%, about 50%, about 60%, about 70%, about 80%, or about 90% compared to a formulation that is not a controlled release otic formulation . In certain embodiments, any controlled release otic formulation described herein increases the exposure of a therapeutic agent and decreases the $C_{max}$ in otic fluids (e.g., endolymph and/or perilymph) by about 40%, about 30%, about 20%, or about 10%, compared to a formulation that is not a controlled release otic formulation . In certain embodiments, any controlled release otic formulation described herein alters (e.g. reduces) the ratio of $C_{max}$ to $C_{min}$ compared to a formulation that is not a controlled release otic formulation. In certain embodiments, the ratio of $C_{max}$ to $C_{min}$ is 10:1, 9:1, 8:1, 7:1, 6:1, 5:1, 4:1, 3:1, 2:1 or 1:1. In certain embodiments, any controlled release otic formulation described herein increases the exposure of a therapeutic agent and increases the length of time that the concentration of a therapeutic agent is above $C_{min}$ by about 30%, about 40%, about 50%, about 60%, about 70%, about 80% or about 90% compared to a formulation that is not a controlled release otic formulation . In certain instances, controlled release formulations described herein delay the time to $C_{max}$. In certain instances, the controlled steady release of a drug prolongs the time the concentration of the drug will stay above the $C_{min}$. In some embodiments, auris formulations described herein prolong the residence time of a drug in the inner ear. In certain instances, once drug exposure (e.g., concentration in the endolymph or perilymph) of a drug reaches steady state, the concentration of the drug in the endolymph or perilymph stays at or about the therapeutic dose for an extended period of time (e.g., one day, 2 days, 3 days, 4 days, 5 days, 6 days, 1 week, 2 weeks, 3 weeks, a month, two months, three months, six months, or one year).

In some embodiments, the otic formulations described herein deliver an active agent to the external, middle and/or inner ear, including the cochlea and vestibular labyrinth. In some embodiments, local otic delivery of the auris formulations described herein allows for controlled release of active agents to auris structures and overcomes the drawbacks associated with systemic administration (e.g., low bioavailability of the drug in the endolymph or perilymph, variability in concentration of the drug in the external, middle and/or internal ear).

Controlled-release options include liposomes, cyclodextrins, biodegradable polymers, dispersible polymers, emulsions, microspheres or microparticles, other viscous media, paints, foams, in spongy materials, liposomes, nanocapsules or nanospheres, and combinations thereof; other options or components include mucoadhesives, penetration enhancers, bioadhesives, antioxidants, surfactants, buffering agents, diluents, salts and preservatives. To the extent viscosity considerations potentially limit the use of a syringe/needle delivery system, thermoreversible gels or post-administration viscosity-enhancing options are also envisioned, as well as alternative delivery systems, including pumps, microinjection devices and the like.

In one embodiment of the otic formulations described herein, the otic formulation is provided as a thickened liquid formulation , also referred to herein as "auris-acceptable thickened liquid formulation ," "auris thickened liquid formulations " or variations thereof. All of the components of the thickened liquid formulation must be compatible with the auris interna. Further, the thickened liquid formulation provides controlled release of the therapeutic agent to the desired site within the auris interna for some embodiments. In some embodiments, the thickened liquid formulation also has an immediate or rapid release component for delivery of the therapeutic agent to the desired target site.

In one embodiment of the otic formulations described herein, the otic formulation is provided as a suspension formulation , also referred to herein as "auris-acceptable suspension formulation ," "auris suspension formulations " or variations thereof. All of the components of the suspension formulation must be compatible with the auris interna. Further, the suspension formulation provides controlled release of the therapeutic agent to the desired site within the auris interna for some embodiments. In some embodiments, the suspension formulation also has an immediate or rapid release component for delivery of the therapeutic agent to the desired target site.

In one embodiment of the otic formulations described herein, the otic formulation is provided as a solution formulation , also referred to herein as "auris-acceptable solution formulation," "auris solution formulations " or variations thereof. All of the components of the solution formulation must be compatible with the auris interna. Further, the solution formulation provides controlled release of the therapeutic agent to the desired site within the auris interna for some embodiments. In some embodiments, the solution formulation also has an immediate or rapid release component for delivery of the therapeutic agent to the desired target site.

In one embodiment of the otic formulations described herein, the otic formulation is provided as a gel formulation , also referred to herein as "auris-acceptable gel formulation ," "auris gel formulations " or variations thereof. All of the components of the gel formulation must be compatible with the auris interna. Further, the gel formulation provides

controlled release of the therapeutic agent to the desired site within the auris interna for some embodiments. In some embodiments, the gel formulation also has an immediate or rapid release component for delivery of the therapeutic agent to the desired target site.

In some embodiments, the formulations described herein are bimodal formulations and comprise an immediate release component and an extended release component. In some instances, bimodal formulations allow for a constant rate of release of an immediate release component (multiparticulate agent (e.g., micronized active agent)) and a constant rate of release of an extended release component (e.g., an encapsulated active agent that serves as a depot for extending the release of an active agent). In other embodiments, the otic formulations described herein are administered as a controlled release formulation , released either continuously or in a pulsatile manner, or variants of both. In still other embodiments, the active agent formulation is administered as both an immediate release and controlled release formulation , released either continuously or in a pulsatile manner, or variants of both. In certain embodiments, the formulations comprise an excipient that increases the release rate of the therapeutic agent. In certain embodiments, the formulations comprise an excipient that decreases the release rate of the therapeutic agent. In certain embodiments, the formulations comprise penetration enhancers that allow for delivery of the active agents across the oval window or the round window of the ear.

In some embodiments, the otic formulations are biodegradable. In other embodiments, the otic formulations include a mucoadhesive excipient to allow adhesion to the external mucous membrane of the round window. In yet other embodiments, the otic formulations include a penetration enhancer excipient; in further embodiments, the otic formulation contains a viscosity enhancing agent. In other embodiments, the otic pharmaceutical formulations provide an auris-acceptable microsphere or microparticle; in still other embodiments, the otic pharmaceutical formulations provide an auris-acceptable liposome, in yet other embodiments, the otic pharmaceutical formulations provide an auris-acceptable paint or foam. In other embodiments, the otic pharmaceutical formulations provide an auris-acceptable spongy material.

The formulations disclosed herein alternatively encompass an otoprotectant agent in addition to the at least one active agent and/or excipients, including such as antioxidants, alpha lipoic acid, calcium, fosfomycin or iron chelators, to counteract potential ototoxic effects that arise from the use of specific therapeutic agents or excipients, diluents, or carriers.

One aspect of the embodiments disclosed herein is to provide a controlled release formulation for the treatment of fluid homeostasis disorders. The controlled release aspect of the formulations disclosed herein is imparted through a variety of agents, including excipients, agents or materials that are acceptable for use in the auris interna or other otic structure. By way of example only, such excipients, agents or materials include an auris-acceptable polymer, an auris-acceptable viscosity enhancing agent, an auris-acceptable microsphere, an auris-acceptable liposome, an auris-acceptable nanocapsule or nanosphere, or combinations thereof.

Thus, provided herein are pharmaceutical formulations that include at least one auris therapeutic agent and auris-acceptable diluent(s), excipient(s), and/or carrier(s). In some embodiments, the pharmaceutical formulations include other medicinal or pharmaceutical agents, carriers, adjuvants, such as preserving, stabilizing, wetting or emulsifying agents, solution promoters, salts for regulating the osmotic pressure, and/or buffers. In other embodiments, the pharmaceutical formulations also contain other therapeutic substances.

### Auris-Acceptable Gel Formulations

In some embodiments, the auris-acceptable formulations described herein are gel formulations .

In some embodiments, the otic gel formulations that include at least therapeutic agent and a pharmaceutically acceptable diluent(s), excipient(s), or carrier(s). In some embodiments, the otic gel formulations include other medicinal or pharmaceutical agents; carriers; adjuvants; preserving, stabilizing, wetting or emulsifying agents; solution promoters; salts for regulating the osmotic pressure; and/or buffers. In some embodiments, the otic gel formulations comprise (i) a therapeutic agent, (ii) a gelling and viscosity enhancing agent, (iii) a pH adjusting agent, and (iv) sterile water.

Gels, sometimes referred to as jellies, have been defined in various ways. For example, the United States Pharmacopoeia defines gels as semisolid systems consisting of either suspensions made up of small inorganic particles or large organic molecules interpenetrated by a liquid. Gels include a single-phase or a two-phase system. A single-phase gel consists of organic macromolecules distributed uniformly throughout a liquid in such a manner that no apparent boundaries exist between the dispersed macromolecules and the liquid. Some single-phase gels are prepared from synthetic macromolecules (e.g., carbomer) or from natural gums (e.g., tragacanth). In some embodiments, single-phase gels are generally aqueous but will also be made using alcohols and oils. Two-phase gels consist of a network of small discrete particles.

Gels can also be classified as being hydrophobic or hydrophilic. In certain embodiments, the base of a hydrophobic gel consists of a liquid paraffin with polyethylene or fatty oils gelled with colloidal silica or aluminum or zinc soaps. In contrast, the base of hydrophilic gels usually consists of water, glycerol, or propylene glycol gelled with a suitable gelling agent (e.g., tragacanth, starch, cellulose derivatives, carboxyvinylpolymers, and magnesium-aluminum silicates).

In certain embodiments, the rheology of the formulations or devices disclosed herein is pseudo plastic, plastic, thixotropic, or dilatant.

In one embodiment the enhanced viscosity auris-acceptable formulation described herein is not a liquid at room temperature. In certain embodiments, the enhanced viscosity formulation is characterized by a phase transition between room temperature and body temperature (including an individual with a serious fever, e.g., up to about 42 °C). In some embodiments, the phase transition occurs at about 1 °C below body temperature, at about 2 °C below body temperature, at about 3 °C below body temperature, at about 4 °C below body temperature, at about 6 °C below body temperature, at about 8 °C below body temperature, or at about 10 °C below body temperature. In some embodiments, the phase transition occurs at about 15 °C below body temperature, at about 20 °C below body temperature, or at about 25 °C below body temperature. In specific embodiments, the gelation temperature (Tgel) of a formulation described herein is about 20 °C, about 25 °C, or about 30 °C. In certain embodiments, the gelation temperature (Tgel) of a formulation described herein is about 35 °C or about 40 °C. In one embodiment, administration of any formulation described herein at about body temperature reduces or inhibits vertigo associated with intratympanic administration of otic formulations. Included within the definition of body temperature is the body temperature of a healthy individual or an unhealthy individual, including an individual with a fever (up to ~42 °C). In some embodiments, the pharmaceutical formulations or devices described herein are liquids at about room temperature and are administered at or about room temperature, reducing or ameliorating side effects such as, for example, vertigo.

Polymers composed of polyoxypropylene and polyoxyethylene form thermoreversible gels when incorporated into aqueous solutions. These polymers have the ability to change from the liquid state to the gel state at temperatures close to body temperature, therefore allowing useful formulations that are applied to the targeted auris structure(s). The liquid state-to-gel state phase transition is dependent on the polymer concentration and the ingredients in the solution.

Poloxamer 407 (PF-127) is a nonionic surfactant composed of polyoxyethylene-polyoxypropylene copolymers. Other poloxamers include 188 (F-68 grade), 237 (F-87 grade), and 338 (F-108 grade). Aqueous solutions of poloxamers are stable in the presence of acids, alkalis, and metal ions. PF-127 is a commercially available polyoxyethylene-polyoxypropylene triblock copolymer of general formula E106 P70 E106, with an average molar mass of 13,000. The polymer can be further purified by suitable methods that will enhance gelation properties of the polymer. It contains approximately 70% ethylene oxide, which accounts for its hydrophilicity. It is one of the series of poloxamer ABA block copolymers, whose members share the chemical formula shown below.

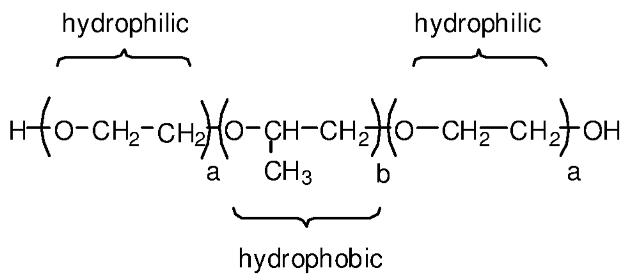

PF-127 is of particular interest since concentrated solutions (>20% w/w) of the copolymer are transformed from low viscosity transparent solutions to solid gels on heating to body temperature. This phenomenon, therefore, suggests that when placed in contact with the body, the gel preparation will form a semi-solid structure and a sustained release depot. Furthermore, PF-127 has good solubilizing capacity, low toxicity and is, therefore, considered a good medium for drug delivery systems.

In an alternative embodiment, the thermogel is a PEG-PLGA-PEG triblock copolymer (Jeong et al, Nature (1997), 388:860-2; Jeong et al, J. Control. Release (2000), 63:155-63; Jeong et al, Adv. Drug Delivery Rev. (2002), 54:37-51). The polymer exhibits sol-gel behavior over a concentration of about 5% w/w to about 40% w/w. Depending on the properties desired, the lactide/glycolide molar ratio in the PLGA copolymer ranges from about 1:1 to about 20:1. The resulting coploymers are soluble in water and form a free-flowing liquid at room temperature but form a hydrogel at body temperature. A commercially available PEG-PLGA-PEG triblock copolymer is RESOMER RGP t50106 manufactured by Boehringer Ingelheim. This material is composed of a PGLA copolymer of 50:50 poly(DL-lactide-co-glycolide), is 10% w/w of PEG, and has a molecular weight of about 6000.

ReGel® is a tradename of MacroMed Incorporated for a class of low molecular weight, biodegradable block copolymers having reverse thermal gelation properties as described in U.S. Pat. Nos. 6,004,573, 6,117949, 6,201,072, and 6,287,588. It also includes biodegradable polymeric drug carriers disclosed in pending U.S. patent application Ser. Nos. 09/906,041, 09/559,799 and 10/919,603. The biodegradable drug carrier comprises ABA-type or BAB-type triblock copolymers, or mixtures thereof, wherein the A-blocks are relatively hydrophobic and comprise biodegradable polyesters

or poly(orthoester)s, and the B-blocks are relatively hydrophilic and comprise polyethylene glycol (PEG), said copolymers having a hydrophobic content of between 50.1 to 83% by weight and a hydrophilic content of between 17 to 49.9% by weight, and an overall block copolymer molecular weight of between 2000 and 8000 Daltons. The drug carriers exhibit water solubility at temperatures below normal mammalian body temperatures and undergo reversible thermal gelation to then exist as a gel at temperatures equal to physiological mammalian body temperatures. The biodegradable, hydrophobic A polymer block comprises a polyester or poly(ortho ester), in which the polyester is synthesized from monomers selected from the group consisting of D,L-lactide, D-lactide, L-lactide, D,L-lactic acid, D-lactic acid, L-lactic acid, glycolide, glycolic acid, ε-caprolactone, ε-hydroxyhexanoic acid, γ-butyrolactone, γ-hydroxybutyric acid, δ-valerolactone, δ-hydroxyvaleric acid, hydroxybutyric acids, malic acid, and copolymers thereof and having an average molecular weight of between about 600 and 3000 Daltons. The hydrophilic B-block segment is preferably polyethylene glycol (PEG) having an average molecular weight of between about 500 and 2200 Daltons.

Additional biodegradable thermoplastic polyesters include AtriGel® (provided by Atrix Laboratories, Inc.) and/or those disclosed, e.g., in U.S. Patent Nos. 5,324,519; 4,938,763; 5,702,716; 5,744,153; and 5,990,194; wherein the suitable biodegradable thermoplastic polyester is disclosed as a thermoplastic polymer. Examples of suitable biodegradable thermoplastic polyesters include polylactides, polyglycolides, polycaprolactones, copolymers thereof, terpolymers thereof, and any combinations thereof. In some such embodiments, the suitable biodegradable thermoplastic polyester is a polylactide, a polyglycolide, a copolymer thereof, a terpolymer thereof, or any combination thereof. In one embodiment, the biodegradable thermoplastic polyester is 50/50 poly(DL-lactide-co-glycolide) having a carboxy terminal group; is present in about 30 wt. % to about 40 wt. % of the formulation; and has an average molecular weight of about 23,000 to about 45,000. Alternatively, in another embodiment, the biodegradable thermoplastic polyester is 75/25 poly (DL-lactide-co-glycolide) without a carboxy terminal group; is present in about 40 wt. % to about 50 wt. % of the formulation; and has an average molecular weight of about 15,000 to about 24,000. In further or alternative embodiments, the terminal groups of the poly(DL-lactide-co-glycolide) are either hydroxyl, carboxyl, or ester depending upon the method of polymerization. Polycondensation of lactic or glycolic acid provides a polymer with terminal hydroxyl and carboxyl groups. Ring-opening polymerization of the cyclic lactide or glycolide monomers with water, lactic acid, or glycolic acid provides polymers with the same terminal groups. However, ring-opening of the cyclic monomers with a monofunctional alcohol such as methanol, ethanol, or 1-dodecanol provides a polymer with one hydroxyl group and one ester terminal groups. Ring-opening polymerization of the cyclic monomers with a diol such as 1,6-hexanediol or polyethylene glycol provides a polymer with only hydroxyl terminal groups.

Since the polymer systems of thermoreversible gels dissolve more completely at reduced temperatures, methods of solubilization include adding the required amount of polymer to the amount of water to be used at reduced temperatures. Generally after wetting the polymer by shaking, the mixture is capped and placed in a cold chamber or in a thermostatic container at about 0-10 °C in order to dissolve the polymer. The mixture is stirred or shaken to bring about a more rapid dissolution of the thermoreversible gel polymer. The active agent and various additives such as buffers, salts, and preservatives are subsequently added and dissolved. In some instances the active agent and/or other pharmaceutically active agent is suspended if it is insoluble in water. The pH is modulated by the addition of appropriate buffering agents. Round window membrane mucoadhesive characteristics are optionally imparted to a thermoreversible gel by incorporation of round window membrane mucoadhesive carbomers, such as Carbopol® 934P, to the formulation (Majithiya et al., AAPS PharmSciTech (2006), 7(3), p. E1; EP0551626).

In one embodiment are auris-acceptable pharmaceutical gel formulations which do not require the use of an added viscosity enhancing agent or viscosity modulating agent. Such gel formulations incorporate at least one pharmaceutically acceptable buffer. In one aspect is a gel formulation and a pharmaceutically acceptable buffer. In another embodiment, the pharmaceutically acceptable excipient or carrier is a gelling agent.

In other embodiments, useful auris-acceptable pharmaceutical formulations also include one or more pH adjusting agents or buffering agents to provide an endolymph or perilymph suitable pH. Suitable pH adjusting agents or buffers include to acetate, bicarbonate, ammonium chloride, citrate, phosphate, pharmaceutically acceptable salts thereof, and combinations or mixtures thereof. Such pH adjusting agents and buffers are included in an amount required to maintain pH of the formulation from a pH of about 5 to about 9, in one embodiment a pH from about 6.5 to about 7.5, and in yet another embodiment at a pH of about 6.5, 6.6, 6.7, 6.8, 6.9, 7.0, 7.1, 7.2, 7.3, 7.4, 7.5, 7.6, 7.7, 7.8, 7.9, or 8.0. In one embodiment, when one or more buffers are utilized in the formulations of the present disclosure, they are combined, e.g., with a pharmaceutically acceptable vehicle and are present in the final formulation, e.g., in an amount ranging from about 0.1% to about 20%, from about 0.5% to about 10%. In certain embodiments of the present disclosure, the amount of buffer included in the gel formulations are an amount such that the pH of the gel formulation does not interfere with the auris media or auris interna's natural buffering system, or does not interfere with the natural pH of the endolymph or perilymph, depending on where in the cochlea the otic formulation is targeted. In some embodiments, from about 10 mM to about 200 mM concentration of a buffer is present in the gel formulation. In certain embodiments, from about a 5 mM to about a 200 mM concentration of a buffer is present. In certain embodiments, from about a 20 mM to about a 100 mM concentration of a buffer is present. In one embodiment is a buffer such as acetate or citrate at slightly acidic pH. In one embodiment the buffer

is a sodium acetate buffer having a pH of about 4.5 to about 6.5. In one embodiment the buffer is a sodium citrate buffer having a pH of about 5.0 to about 8.0, or about 5.5 to about 7.0.

In an alternative embodiment, the buffer used is tris(hydroxymethyl)aminomethane, bicarbonate, carbonate, or phosphate at slightly basic pH. In one embodiment, the buffer is a sodium bicarbonate buffer having a pH of about 6.5 to about 8.5, or about 7.0 to about 8.0. In another embodiment the buffer is a sodium phosphate dibasic buffer having a pH of about 6.0 to about 9.0.

Also described herein are controlled-release formulations or devices a viscosity enhancing agent or viscosity modulating agent. Suitable viscosity-enhancing agents or viscosity modulating agents include by way of example only, gelling agents and suspending agents. In one embodiment, the enhanced viscosity formulation does not include a buffer. In other embodiments, the enhanced viscosity formulation includes a pharmaceutically acceptable buffer. Sodium chloride or other tonicity agents are optionally used to adjust tonicity, if necessary.

By way of example only, the auris-acceptable viscosity agent includes hydroxypropyl methylcellulose, hydroxyethyl cellulose, polyvinylpyrrolidone, carboxymethyl cellulose, polyvinyl alcohol, sodium chondroitin sulfate, sodium hyaluronate. Other viscosity enhancing agents compatible with the targeted auris structure include acacia (gum arabic), agar, aluminum magnesium silicate, sodium alginate, sodium stearate, bladderwrack, bentonite, carbomer, carrageenan, Carbopol, xanthan, cellulose, microcrystalline cellulose (MCC), ceratonia, chitin, carboxymethylated chitosan, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethylcellulose (CMC), silicon dioxide, polyvinylpyrrolidone (PVP: povidone), Splenda® (dextrose, maltodextrin and sucralose), or combinations thereof. In specific embodiments, the viscosity-enhancing excipient is a combination of MCC and CMC. In another embodiment, the viscosity-enhancing agent is a combination of carboxymethylated chitosan, or chitin, and alginate. The combination of chitin and alginate with the active agent disclosed herein acts as a controlled-release formulation, restricting the diffusion of the active agent from the formulation. Moreover, the combination of carboxymethylated chitosan and alginate is optionally used to assist in increasing the permeability of the active agent through the round window membrane.

In some embodiments is an enhanced viscosity formulation, comprising from about 0.1 mM and about 100 mM of an active agent, a pharmaceutically acceptable viscosity enhancer or viscosity modulating agent, and water for injection, the concentration of the viscosity enhancer or viscosity modulating agent in the water being sufficient to provide an enhanced viscosity formulation with a final viscosity from about 100 to about 100,000 cP. In certain embodiments, the viscosity of the gel is in the range from about 100 to about 50,000 cP, about 100 cP to about 1,000 cP, about 500 cP to about 1500 cP, about 1000 cP to about 3000 cP, about 2000 cP to about 8,000 cP, about 4,000 cP to about 50,000 cP, about 10,000 cP to about 500,000 cP, about 15,000 cP to about 1,000,000 cP. In certain embodiments, the viscosity of the gel is in the range from about 100 to about 50,000 cP, about 100 cP to about 1,000 cP, about 500 cP to about 1500 cP, about 1000 cP to about 3000 cP, about 2000 cP to about 8,000 cP, about 4,000 cP to about 50,000 cP, about 10,000 cP to about 500,000 cP, about 15,000 cP to about 3,000,000 cP. In other embodiments, when an even more viscous medium is desired, the biocompatible gel comprises at least about 35%, at least about 45%, at least about 55%, at least about 65%, at least about 70%, at least about 75%, or even at least about 80% or so by weight of the active agent. In highly concentrated samples, the biocompatible enhanced viscosity formulation comprises at least about 25%, at least about 35%, at least about 45%, at least about 55%, at least about 65%, at least about 75%, at least about 85%, at least about 90%, at least about 95%, or more by weight of the active agent.

In some embodiments, the viscosity of the gel formulations presented herein are measured by any means described. For example, in some embodiments, an LVDV-II+CP Cone Plate Viscometer and a Cone Spindle CPE-40 is used to calculate the viscosity of the gel formulation described herein. In other embodiments, a Brookfield (spindle and cup) viscometer is used to calculate the viscosity of the gel formulation described herein. In some embodiments, the viscosity ranges referred to herein are measured at room temperature. In other embodiments, the viscosity ranges referred to herein are measured at body temperature (e.g., at the average body temperature of a healthy human).

In one embodiment, the pharmaceutically acceptable enhanced viscosity auris-acceptable formulation comprises at least one active agent and at least one gelling agent. Suitable gelling agents for use in preparation of the gel formulation include celluloses, cellulose derivatives, cellulose ethers (e.g., carboxymethylcellulose, ethylcellulose, hydroxyethylcellulose, hydroxymethylcellulose, hydroxypropylmethylcellulose, hydroxypropylcellulose, methylcellulose), guar gum, xanthan gum, locust bean gum, alginates (e.g., alginic acid), silicates, starch, tragacanth, carboxyvinyl polymers, carrageenan, paraffin, petrolatum, and any combinations or mixtures thereof. In some other embodiments, hydroxypropylmethylcellulose (Methocel®) is utilized as the gelling agent. In certain embodiments, the viscosity enhancing agents or viscosity modulating agents described herein are also utilized as the gelling agent for the gel formulations

presented herein.

In one specific embodiment of the auris-acceptable controlled-release formulations described herein, the active agent is provided in a gel matrix, also referred to herein as "auris-acceptable gel formulations", "auris interna-acceptable gel formulations", "auris media-acceptable gel formulations", "auris externa-acceptable gel formulations", "auris gel formulations", or variations thereof. All of the components of the gel formulation must be compatible with the targeted auris structure. Further, the gel formulations provide controlled-release of the active agent to the desired site within the targeted auris structure; in some embodiments, the gel formulation also has an immediate or rapid release component for delivery of the active agent to the desired target site. In other embodiments, the gel formulation has a sustained release component for delivery of the active agent. In some embodiments, the auris gel formulations are biodegradable. In other embodiments, the auris gel formulations include a mucoadhesive excipient to allow adhesion to the external mucous layer of the round window membrane. In yet other embodiments, the auris gel formulations include a penetration enhancer excipient; in further embodiments, the auris gel formulation contains a viscosity enhancing agent sufficient to provide a viscosity of from about 10 to about 1,000,000 centipoise, from about 500 and 1,000,000 centipoise; from about 750 to about 1,000,000 centipoise; from about 1000 to about 1,000,000 centipoise; from about 1000 to about 400,000 centipoise; from about 2000 to about 100,000 centipoise; from about 3000 to about 50,000 centipoise; from about 4000 to about 25,000 centipoise; from about 5000 to about 20,000 centipoise; or from about 6000 to about 15,000 centipoise. In some embodiments, the auris gel formulation contains a viscosity enhancing agent sufficient to provide a viscosity of from about 50,0000 to about 1,000,000 centipoise. In some embodiments, the auris gel formulation contains a viscosity enhancing agent sufficient to provide a viscosity of from about 50,0000 to about 3,000,000 centipoise.

In some embodiments, the otic pharmaceutical formulations or devices described herein are low viscosity formulations or devices at body temperature. In some embodiments, low viscosity formulations or devices contain from about 1% to about 10% of a viscosity enhancing agent or viscosity modulating agent (e.g., gelling components such as polyoxyethylene-polyoxypropylene copolymers). In some embodiments, low viscosity formulations or devices contain from about 2% to about 10% of a viscosity enhancing agent or viscosity modulating agent (e.g., gelling components such as polyoxyethylene-polyoxypropylene copolymers). In some embodiments, low viscosity formulations or devices contain from about 5% to about 10% of a viscosity enhancing agent or viscosity modulating agent (e.g., gelling components such as polyoxyethylene-polyoxypropylene copolymers). In some embodiments, low viscosity formulations or devices are substantially free of a viscosity enhancing agent or viscosity modulating agent (e.g., gelling components such as polyoxyethylene-polyoxypropylene copolymers). In some embodiments, a low viscosity otic formulation or device described herein provides an apparent viscosity of from about 100 cP to about 10,000 cP. In some embodiments, a low viscosity otic formulation or device described herein provides an apparent viscosity of from about 500 cP to about 10,000 cP. In some embodiments, a low viscosity otic formulation or device described herein provides an apparent viscosity of from about 1000 cP to about 10,000 cP. In some of such embodiments, a low viscosity otic formulation or device is administered in combination with an external otic intervention, e.g., a surgical procedure including middle ear surgery, inner ear surgery, typanostomy, cochleostomy, labyrinthotomy, mastoidectomy, stapedectomy, stapedotomy, endolymphatic sacculotomy, or the like. In some of such embodiments, a low viscosity otic formulation or device is administered during an otic intervention. In other such embodiments, a low viscosity otic formulation or device is administered before the otic intervention.

In some embodiments, the otic pharmaceutical formulations or devices described herein are high viscosity formulations or devices at body temperature. In some embodiments, high viscosity formulations or devices contain from about 10% to about 25% of a viscosity enhancing agent or viscosity modulating agent (e.g., gelling components such as polyoxyethylene-polyoxypropylene copolymers). In some embodiments, high viscosity formulations or devices contain from about 14% to about 22% of a viscosity enhancing agent or viscosity modulating agent (e.g., gelling components such as polyoxyethylene-polyoxypropylene copolymers). In some embodiments, high viscosity formulations or devices contain from about 15% to about 21% of a viscosity enhancing agent or viscosity modulating agent (e.g., gelling components such as polyoxyethylene-polyoxypropylene copolymers). In some embodiments, a high viscosity otic formulation or device described herein provides an apparent viscosity of from about 100,000 cP to about 3,000,000 cP. In some embodiments, a high viscosity otic formulation or device described herein provides an apparent viscosity of from about 100,000 cP to about 1,000,000 cP. In some embodiments, a high viscosity otic formulation or device described herein provides an apparent viscosity of from about 150,000 cP to about 500,000 cP. In some embodiments, a high viscosity otic formulation or device described herein provides an apparent viscosity of from about 250,000 cP to about 500,000 cP. In some of such embodiments, a high viscosity formulation or device is a liquid at room temperature and gels at about between room temperature and body temperature (including an individual with a serious fever, e.g., up to about 42 °C). In some embodiments, an otic high viscosity formulation or device is administered as monotherapy for treatment of an otic disease or condition described herein. In some embodiments, an otic high viscosity formulation or device is administered in combination with an external otic intervention, e.g., a surgical procedure including middle ear surgery, inner ear surgery, typanostomy, cochleostomy, labyrinthotomy, mastoidectomy, stapedectomy, stapedotomy, endolymphatic sacculotomy, or the like. In some of such embodiments, a high viscosity otic formulation or device is administered after the otic

intervention. In other such embodiments, a high viscosity otic formulation or device is administered before the otic intervention.

In other embodiments, the otic pharmaceutical formulations described herein further provide an auris-acceptable hydrogel; in yet other embodiments, the otic pharmaceutical formulations provide an auris-acceptable microsphere or microparticle; in still other embodiments, the otic pharmaceutical formulations provide an auris-acceptable liposome. In some embodiments, the otic pharmaceutical formulations provide an auris-acceptable foam; in yet other embodiments, the otic pharmaceutical formulations provide an auris-acceptable paint; in still further embodiments, otic pharmaceutical formulations provide an auris-acceptable in situ forming spongy material. In some embodiments, the otic pharmaceutical formulations provide an auris-acceptable solvent release gel. In some embodiments, the otic pharmaceutical formulations provide an actinic radiation curable gel. Further embodiments include a thermoreversible gel in the otic pharmaceutical formulation, such that upon preparation of the gel at room temperature or below, the formulation is a fluid, but upon application of the gel into or near the auris interna and/or auris media target site, including the tympanic cavity, round window membrane, or the crista fenestrae cochleae, the otic-pharmaceutical formulation stiffens or hardens into a gel-like substance.

In further or alternative embodiments, the otic gel formulations are capable of being administered on or near the round window membrane via intratympanic injection. In other embodiments, the otic gel formulations are administered on or near the round window or the crista fenestrae cochleae through entry via a post-auricular incision and surgical manipulation into or near the round window or the crista fenestrae cochleae area. Alternatively, the otic gel formulation is applied via syringe and needle, wherein the needle is inserted through the tympanic membrane and guided to the area of the round window or crista fenestrae cochleae. The otic gel formulations are then deposited on or near the round window or crista fenestrae cochleae for localized treatment of autoimmune otic disorders. In other embodiments, the otic gel formulations are applied via microcathethers implanted into the patient, and in yet further embodiments the formulations are administered via a pump device onto or near the round window membrane. In still further embodiments, the otic gel formulations are applied at or near the round window membrane via a microinjection device. In yet other embodiments, the otic gel formulations are applied in the tympanic cavity. In some embodiments, the otic gel formulations are applied on the tympanic membrane. In still other embodiments, the otic gel formulations are applied onto or in the auditory canal.

**Triglyceride Based Otic Formulations**

Provided herein in one embodiment are otic formulations comprising triglycerides. Triglycerides are esters derived from glycerol and three fatty acids. In some instances, these fatty acids are saturated fatty acids, unsaturated fatty acids, or a combination thereof. Provided herein in one aspect, is an otic formulation comprising a therapeutic agent, or pharmaceutically acceptable prodrug or salt thereof; and triglycerides comprising medium chain fatty acids; wherein the triglycerides are present in an amount that is sufficient to stabilize the therapeutic agent for injection into the ear, and wherein the otic pharmaceutical formulation comprises at least about 50% by weight of the triglycerides.

In some instances, these triglycerides are medium chain triglycerides (MCTs). In some embodiments, these triglycerides comprise medium chain fatty acids. In some embodiments, these triglycerides are derived from glycerol and medium-chain fatty acids. In some embodiments, these triglycerides are derived from glycerol and at least two medium-chain fatty acids. In some embodiments, these triglycerides are derived from glycerol, two medium-chain fatty acids, and one long-chain fatty acid. In some embodiments, these triglycerides are derived from glycerol, and three medium-chain fatty acids.

In some embodiments, the triglycerides are derived from glycerol and medium chain fatty acids. In some embodiments, the triglycerides are derived from glycerol and at least two medium-chain fatty acids. In some embodiments, each medium chain fatty acid independently comprises 6 to 12 carbon atoms in the carbon chain. In some embodiments, each medium chain fatty acid independently comprises 8 to 12 carbon atoms in the carbon chain. In some embodiments, each medium chain fatty acid independently comprises 6, 7, 8, 9, 10, 11, or 12 carbon atoms in the carbon chain. In some embodiments, each medium chain fatty acid independently comprises 8 or 10 carbon atoms in the carbon chain. In some embodiments, the medium chain fatty acids are caproic acid (hexanoic acid), enanthic acid (heptanoic acid), caprylic acid (octanoic acid), pelargonic acid (nonanoic acid), capric acid (decanoic acid), undecylenic acid (undec-10-enoic acid), lauric acid (dodecanoic acid), or a combination thereof. In some embodiments, the medium chain fatty acids are caprylic acid (octanoic acid), capric acid (decanoic acid), or a combination thereof.

In some embodiments, the triglycerides comprising medium chain fatty acids are balassee oil, coconut oil, cohune oil, palm kernel oil, tucum oil, or combinations thereof. In some embodiments, triglycerides comprising medium chain fatty acids are coconut oil, cohune oil, palm kernel oil, tucum oil, or any combinations thereof. In some embodiments, the triglycerides comprising medium chain fatty acids are balassee oil. In some embodiments, the triglycerides comprising medium chain fatty acids are coconut oil. In some embodiments, the triglycerides comprising medium chain fatty acids are cohune oil. In some embodiments, the triglycerides comprising medium chain fatty acids are palm kernel oil. In some embodiments, the triglycerides comprising medium chain fatty acids are tucum oil.

In some embodiments, the otic pharmaceutical formulation has triglycerides in an amount that is sufficient to stabilize the therapeutic agent for injection into the ear. In some embodiments, the otic pharmaceutical formulation has triglycerides in an amount that is sufficient to provide sufficient retention time in the ear. In some embodiments, the ear is the outer ear, middle ear, or inner ear. In some embodiments, the otic pharmaceutical formulation has triglycerides in an amount that is sufficient to provide sustained release of the therapeutic agent. In some embodiments, the otic formulation has triglycerides in an amount that is sufficient to allow delivery of the formulation via a narrow gauge needle.

In some embodiments, the otic pharmaceutical formulation comprises between about 50% to about 99.9% by weight of the triglycerides. In some embodiments, the otic pharmaceutical formulation comprises between about 55% to about 99.9% by weight of the triglycerides. In some embodiments, the otic pharmaceutical formulation comprises between about 60% to about 99.9% by weight of the triglycerides. In some embodiments, the otic pharmaceutical formulation comprises between about 65% to about 99.9% by weight of the triglycerides. In some embodiments, the otic pharmaceutical formulation comprises between about 70% to about 99.9% by weight of the triglycerides. In some embodiments, the otic pharmaceutical formulation comprises between about 75% to about 99.9% by weight of the triglycerides. In some embodiments, the otic pharmaceutical formulation comprises between about 80% to about 99.9% by the weight of the triglycerides. In some embodiments, the otic pharmaceutical formulation comprises between about 85% to about 99.9% by the weight of the triglycerides. In some embodiments, the otic pharmaceutical formulation comprises between about 90% to about 99.9% by the weight of the triglycerides. In some embodiments, the otic pharmaceutical formulation comprises between about 95% to about 99.9% by the weight of the triglycerides.

In some embodiments, the otic pharmaceutical formulation comprises between about 50% to about 99.99% by weight of the triglycerides. In some embodiments, the otic pharmaceutical formulation comprises between about 55% to about 99.99% by weight of the triglycerides. In some embodiments, the otic pharmaceutical formulation comprises between about 60% to about 99.99% by weight of the triglycerides. In some embodiments, the otic pharmaceutical formulation comprises between about 65% to about 99.99% by weight of the triglycerides. In some embodiments, the otic pharmaceutical formulation comprises between about 70% to about 99.99% by weight of the triglycerides. In some embodiments, the otic pharmaceutical formulation comprises between about 75% to about 99.99% by weight of the triglycerides. In some embodiments, the otic pharmaceutical formulation comprises between about 80% to about 99.99% by weight of the triglycerides. In some embodiments, the otic pharmaceutical formulation comprises between about 85% to about 99.99% by weight of the triglycerides. In some embodiments, the otic pharmaceutical formulation comprises between about 90% to about 99.99% by weight of the triglycerides. In some embodiments, the otic pharmaceutical formulation comprises between about 95% to about 99.99% by weight of the triglycerides.

In some embodiments, the otic pharmaceutical formulation comprises between about 50% to about 95% by weight of the triglycerides. In some embodiments, the otic pharmaceutical formulation comprises between about 55% to about 95% by weight of the triglycerides. In some embodiments, the otic pharmaceutical formulation comprises between about 60% to about 95% by weight of the triglycerides. In some embodiments, the otic pharmaceutical formulation comprises between about 65% to about 95% by weight of the triglycerides. In some embodiments, the otic pharmaceutical formulation comprises between about 70% to about 95% by weight of the triglycerides. In some embodiments, the otic pharmaceutical formulation comprises between about 75% to about 95% by weight of the triglycerides. In some embodiments, the otic pharmaceutical formulation comprises between about 80% to about 95% by weight of the triglycerides. In some embodiments, the otic pharmaceutical formulation comprises between about 85% to about 95% by weight of the triglycerides. In some embodiments, the otic pharmaceutical formulation comprises between about 90% to about 95% by weight of the triglycerides.

In some embodiments, the otic pharmaceutical formulation comprises between about 50% to about 55% by weight of the triglycerides. In some embodiments, the otic pharmaceutical formulation comprises between about 55% to about 60% by weight of the triglycerides. In some embodiments, the otic pharmaceutical formulation comprises between about 60% to about 65% by weight of the triglycerides. In some embodiments, the otic pharmaceutical formulation comprises between about 65% to about 70% by weight of the triglycerides. In some embodiments, the otic pharmaceutical formulation comprises between about 70% to about 75% by weight of the triglycerides. In some embodiments, the otic pharmaceutical formulation comprises between about 75% to about 80% by weight of the triglycerides. In some embodiments, the otic pharmaceutical formulation comprises between about 80% to about 85% by weight of the triglycerides. In some embodiments, the otic pharmaceutical formulation comprises between about 85% to about 90% by weight of the triglycerides. In some embodiments, the otic pharmaceutical formulation comprises between about 90% to about 95% by weight of the triglycerides. In some embodiments, the otic pharmaceutical formulation comprises between about 95% to about 99% by weight of the triglycerides. In some embodiments, the otic pharmaceutical formulation comprises between about 95% to about 99.9% by weight of the triglycerides. In some embodiments, the otic pharmaceutical formulation comprises between about 95% to about 99.99% by weight of the triglycerides.

In some embodiments, the otic pharmaceutical formulation comprises between about 50% to about 60% by weight of the triglycerides. In some embodiments, the otic pharmaceutical formulation comprises between about 60% to about 70% by weight of the triglycerides. In some embodiments, the otic pharmaceutical formulation comprises between

about 70% to about 80% by weight of the triglycerides. In some embodiments, the otic pharmaceutical formulation comprises between about 80% to about 90% by weight of the triglycerides. In some embodiments, the otic pharmaceutical formulation comprises between about 90% to about 99% by weight of the triglycerides. In some embodiments, the otic pharmaceutical formulation comprises between about 90% to about 99.9% by weight of the triglycerides. In some embodiments, the otic pharmaceutical formulation comprises between about 90% to about 99.99% by weight of the triglycerides.

In some embodiments, the otic pharmaceutical formulation comprises about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% , or about 99% by weight of the triglycerides.

In some embodiments, the otic pharmaceutical formulation comprises about 50% by weight of the triglycerides. In some embodiments, the otic pharmaceutical formulation comprises about 51% by weight of the triglycerides. In some embodiments, the otic pharmaceutical formulation comprises about 52% by weight of the triglycerides. In some embodiments, the otic pharmaceutical formulation comprises about 53% by weight of the triglycerides. In some embodiments, the otic pharmaceutical formulation comprises about 54% by weight of the triglycerides. In some embodiments, the otic pharmaceutical formulation comprises about 55% by weight of the triglycerides. In some embodiments, the otic pharmaceutical formulation comprises about 56% by weight of the triglycerides. In some embodiments, the otic pharmaceutical formulation comprises about 57% by weight of the triglycerides. In some embodiments, the otic pharmaceutical formulation comprises about 58% by weight of the triglycerides. In some embodiments, the otic pharmaceutical formulation comprises about 59% by weight of the triglycerides. In some embodiments, the otic pharmaceutical formulation comprises about 60% by weight of the triglycerides. In some embodiments, the otic pharmaceutical formulation comprises about 61% by weight of the triglycerides. In some embodiments, the otic pharmaceutical formulation comprises about 62% by weight of the triglycerides. In some embodiments, the otic pharmaceutical formulation comprises about 63% by weight of the triglycerides. In some embodiments, the otic pharmaceutical formulation comprises about 64% by weight of the triglycerides. In some embodiments, the otic pharmaceutical formulation comprises about 65% by weight of the triglycerides. In some embodiments, the otic pharmaceutical formulation comprises about 66% by weight of the triglycerides. In some embodiments, the otic pharmaceutical formulation comprises about 67% by weight of the triglycerides. In some embodiments, the otic pharmaceutical formulation comprises about 68% by weight of the triglycerides. In some embodiments, the otic pharmaceutical formulation comprises about 69% by weight of the triglycerides. In some embodiments, the otic pharmaceutical formulation comprises about 70% by weight of the triglycerides. In some embodiments, the otic pharmaceutical formulation comprises about 71% by weight of the triglycerides. In some embodiments, the otic pharmaceutical formulation comprises about 72% by weight of the triglycerides. In some embodiments, the otic pharmaceutical formulation comprises about 73% by weight of the triglycerides. In some embodiments, the otic pharmaceutical formulation comprises about 74% by weight of the triglycerides. In some embodiments, the otic pharmaceutical formulation comprises about 75% by weight of the triglycerides. In some embodiments, the otic pharmaceutical formulation comprises about 76% by weight of the triglycerides. In some embodiments, the otic pharmaceutical formulation comprises about 77% by weight of the triglycerides. In some embodiments, the otic pharmaceutical formulation comprises about 78% by weight of the triglycerides. In some embodiments, the otic pharmaceutical formulation comprises about 79% by weight of the triglycerides. In some embodiments, the otic pharmaceutical formulation comprises about 80% by weight of the triglycerides. In some embodiments, the otic pharmaceutical formulation comprises about 81% by weight of the triglycerides. In some embodiments, the otic pharmaceutical formulation comprises about 82% by weight of the triglycerides. In some embodiments, the otic pharmaceutical formulation comprises about 83% by weight of the triglycerides. In some embodiments, the otic pharmaceutical formulation comprises about 84% by weight of the triglycerides. In some embodiments, the otic pharmaceutical formulation comprises about 85% by weight of the triglycerides. In some embodiments, the otic pharmaceutical formulation comprises about 86% by weight of the triglycerides. In some embodiments, the otic pharmaceutical formulation comprises about 87% by weight of the triglycerides. In some embodiments, the otic pharmaceutical formulation comprises about 88% by weight of the triglycerides. In some embodiments, the otic pharmaceutical formulation comprises about 89% by weight of the triglycerides. In some embodiments, the otic pharmaceutical formulation comprises about 90% by weight of the triglycerides. In some embodiments, the otic pharmaceutical formulation comprises about 91% by weight of the triglycerides. In some embodiments, the otic pharmaceutical formulation comprises about 92% by weight of the triglycerides. In some embodiments, the otic pharmaceutical formulation comprises about 93% by weight of the triglycerides. In some embodiments, the otic pharmaceutical formulation comprises about 94% by weight of the triglycerides. In some embodiments, the otic pharmaceutical formulation comprises about 95% by weight of the triglycerides. In some

embodiments, the otic pharmaceutical formulation comprises about 96% by weight of the triglycerides. In some embodiments, the otic pharmaceutical formulation comprises about 97% by weight of the triglycerides. In some embodiments, the otic pharmaceutical formulation comprises about 98% by weight of the triglycerides. In some embodiments, the otic pharmaceutical formulation comprises about 99% by weight of the triglycerides.

In some embodiments, the triglycerides in any one of the otic formulations described herein are replaced with at least one of the following components in the corresponding amounts of triglyceride in the formulation disclosed herein: mineral oil or any corresponding higher alkanes; Vaseline (petroleum jelly); silicone oil (polydimethylsiloxane) in different molecular weights; beeswax dissolved in any of the oils disclosed herein.

In some embodiments, the otic formulation further comprises at least one viscosity modulating agent. In some embodiments, the at least one viscosity modulating agent is silicon dioxide, povidone, carbomer, poloxamer, or a combination thereof. In some embodiments, the viscosity modulating agent is silicon dioxide. In some embodiments, the viscosity modulating agent is povidone. In some embodiments, the viscosity modulating agent is carbomer. In some embodiments, the viscosity modulating agent is poloxamer. In some embodiments, the viscosity modulating agents are silicon dioxide and povidone. In some embodiments, the viscosity modulating agents are silicon dioxide and carbomer. In some embodiments, the viscosity modulating agents are silicon dioxide and poloxamer. In some embodiments, the poloxamer is P407.

In some embodiments, the otic formulation comprises between about 0.01% to about 40% by weight of the povidone. In some embodiments, the otic formulation comprises between about 0.01% to about 35% by weight of the povidone. In some embodiments, the otic formulation comprises between about 0.01% to about 30% by weight of the povidone. In some embodiments, the otic formulation comprises between about 0.01% to about 25% by weight of the povidone. In some embodiments, the otic formulation comprises between about 0.01% to about 20% by weight of the povidone. In some embodiments, the otic formulation comprises between about 0.01% to about 15% by weight of the povidone. In some embodiments, the otic formulation comprises between about 0.01% to about 10% by weight of the povidone. In some embodiments, the otic formulation comprises about 0.01% to about 7% by weight of the povidone. In some embodiments, the otic formulation comprises between about 0.01% to about 5% by weight of the povidone. In some embodiments, the otic formulation comprises between about 0.01% to about 3% by weight of the povidone. In some embodiments, the otic formulation comprises between about 0.01% to about 2% by weight of the povidone. In some embodiments, the otic formulation comprises about 0.01% to about 1% by weight of the povidone.

In some embodiments, the otic formulation comprises about 0.01% by weight of the povidone. In some embodiments, the otic formulation comprises about 0.02% by weight of the povidone. In some embodiments, the otic formulation comprises about 0.03% by weight of the povidone. In some embodiments, the otic formulation comprises about 0.04% by weight of the povidone. In some embodiments, the otic formulation comprises about 0.05% by weight of the povidone. In some embodiments, the otic formulation comprises about 0.06% by weight of the povidone. In some embodiments, the otic formulation comprises about 0.07% by weight of the povidone. In some embodiments, the otic formulation comprises about 0.08% by weight of the povidone. In some embodiments, the otic formulation comprises about 0.09% by weight of the povidone. In some embodiments, the otic formulation comprises about 0.1% by weight of the povidone. In some embodiments, the otic formulation comprises about 0.2% by weight of the povidone. In some embodiments, the otic formulation comprises about 0.3% by weight of the povidone. In some embodiments, the otic formulation comprises about 0.4% by weight of the povidone. In some embodiments, the otic formulation comprises about 0.5% by weight of the povidone. In some embodiments, the otic formulation comprises about 0.6% by weight of the povidone. In some embodiments, the otic formulation comprises about 0.7% by weight of the povidone. In some embodiments, the otic formulation comprises about 0.8% by weight of the povidone. In some embodiments, the otic formulation comprises about 0.9% by weight of the povidone. In some embodiments, the otic formulation comprises about 1% by weight of the povidone. In some embodiments, the otic formulation comprises about 2% by weight of the povidone. In some embodiments, the otic formulation comprises about 3% by weight of the povidone. In some embodiments, the otic formulation comprises about 4% by weight of the povidone. In some embodiments, the otic formulation comprises about 5% by weight of the povidone. In some embodiments, the otic formulation comprises about 6% by weight of the povidone. In some embodiments, the otic formulation comprises about 7% by weight of the povidone. In some embodiments, the otic formulation comprises about 8% by weight of the povidone. In some embodiments, the otic formulation comprises about 9% by weight of the povidone. In some embodiments, the otic formulation comprises about 10% by weight of the povidone. In some embodiments, the otic formulation comprises about 11% by weight of the povidone. In some embodiments, the otic formulation comprises about 12% by weight of the povidone. In some embodiments, the otic formulation comprises about 13% by weight of the povidone. In some embodiments, the otic formulation comprises about 14% by weight of the povidone. In some embodiments, the otic formulation comprises about 15% by weight of the povidone. In some embodiments, the otic formulation comprises about 16% by weight of the povidone. In some embodiments, the otic formulation comprises about 17% by weight of the povidone. In some embodiments, the otic formulation comprises about 18% by weight of the povidone. In some embodiments, the otic formulation comprises about 19% by weight of the povidone. In some embodiments, the otic formulation comprises about 20% by weight of the povidone. In some

embodiments, the otic formulation comprises about 25% by weight of the povidone. In some embodiments, the otic formulation comprises about 30% by weight of the povidone. In some embodiments, the otic formulation comprises about 35% by weight of the povidone. In some embodiments, the otic formulation comprises about 40% by weight of the povidone.

In some embodiments, the otic formulation comprises between about 0.01% to about 40% by weight of the carbomer. In some embodiments, the otic formulation comprises between about 0.01% to about 35% by weight of the carbomer. In some embodiments, the otic formulation comprises between about 0.01% to about 30% by weight of the carbomer. In some embodiments, the otic formulation comprises between about 0.01% to about 25% by weight of the carbomer. In some embodiments, the otic formulation comprises between about 0.01% to about 20% by weight of the carbomer. In some embodiments, the otic formulation comprises between about 0.01% to about 15% by weight of the carbomer. In some embodiments, the otic formulation comprises between about 0.01% to about 10% by weight of the carbomer. In some embodiments, the otic formulation comprises about 0.01% to about 7% by weight of the carbomer. In some embodiments, the otic formulation comprises between about 0.01% to about 5% by weight of the carbomer. In some embodiments, the otic formulation comprises between about 0.01% to about 3% by weight of the carbomer. In some embodiments, the otic formulation comprises between about 0.01% to about 2% by weight of the carbomer. In some embodiments, the otic formulation comprises about 0.01% to about 1% by weight of the carbomer.

In some embodiments, the otic formulation comprises about 0.01% by weight of the carbomer. In some embodiments, the otic formulation comprises about 0.02% by weight of the carbomer. In some embodiments, the otic formulation comprises about 0.03% by weight of the carbomer. In some embodiments, the otic formulation comprises about 0.04% by weight of the carbomer. In some embodiments, the otic formulation comprises about 0.05% by weight of the carbomer. In some embodiments, the otic formulation comprises about 0.06% by weight of the carbomer. In some embodiments, the otic formulation comprises about 0.07% by weight of the carbomer. In some embodiments, the otic formulation comprises about 0.08% by weight of the carbomer. In some embodiments, the otic formulation comprises about 0.09% by weight of the carbomer. In some embodiments, the otic formulation comprises about 0.1% by weight of the carbomer. In some embodiments, the otic formulation comprises about 0.2% by weight of the carbomer. In some embodiments, the otic formulation comprises about 0.3% by weight of the carbomer. In some embodiments, the otic formulation comprises about 0.4% by weight of the carbomer. In some embodiments, the otic formulation comprises about 0.5% by weight of the carbomer. In some embodiments, the otic formulation comprises about 0.6% by weight of the carbomer. In some embodiments, the otic formulation comprises about 0.7% by weight of the carbomer. In some embodiments, the otic formulation comprises about 0.8% by weight of the carbomer. In some embodiments, the otic formulation comprises about 0.9% by weight of the carbomer. In some embodiments, the otic formulation comprises about 1% by weight of the carbomer. In some embodiments, the otic formulation comprises about 2% by weight of the carbomer. In some embodiments, the otic formulation comprises about 3% by weight of the carbomer. In some embodiments, the otic formulation comprises about 4% by weight of the carbomer. In some embodiments, the otic formulation comprises about 5% by weight of the carbomer. In some embodiments, the otic formulation comprises about 6% by weight of the carbomer. In some embodiments, the otic formulation comprises about 7% by weight of the carbomer. In some embodiments, the otic formulation comprises about 8% by weight of the carbomer. In some embodiments, the otic formulation comprises about 9% by weight of the carbomer. In some embodiments, the otic formulation comprises about 10% by weight of the carbomer. In some embodiments, the otic formulation comprises about 11% by weight of the carbomer. In some embodiments, the otic formulation comprises about 12% by weight of the carbomer. In some embodiments, the otic formulation comprises about 13% by weight of the carbomer. In some embodiments, the otic formulation comprises about 14% by weight of the carbomer. In some embodiments, the otic formulation comprises about 15% by weight of the carbomer. In some embodiments, the otic formulation comprises about 16% by weight of the carbomer. In some embodiments, the otic formulation comprises about 17% by weight of the carbomer. In some embodiments, the otic formulation comprises about 18% by weight of the carbomer. In some embodiments, the otic formulation comprises about 19% by weight of the carbomer. In some embodiments, the otic formulation comprises about 20% by weight of the carbomer. In some embodiments, the otic formulation comprises about 25% by weight of the carbomer. In some embodiments, the otic formulation comprises about 30% by weight of the carbomer. In some embodiments, the otic formulation comprises about 35% by weight of the carbomer. In some embodiments, the otic formulation comprises about 40% by weight of the carbomer.

In some embodiments, the otic formulation comprises between about 0.01% to about 40% by weight of the poloxamer. In some embodiments, the otic formulation comprises between about 0.01% to about 35% by weight of the poloxamer. In some embodiments, the otic formulation comprises between about 0.01% to about 30% by weight of the poloxamer. In some embodiments, the otic formulation comprises between about 0.01% to about 25% by weight of the poloxamer. In some embodiments, the otic formulation comprises between about 0.01% to about 20% by weight of the poloxamer. In some embodiments, the otic formulation comprises between about 0.01% to about 15% by weight of the poloxamer. In some embodiments, the otic formulation comprises between about 0.01% to about 10% by weight of the poloxamer. In some embodiments, the otic formulation comprises about 0.01% to about 7% by weight of the poloxamer. In

some embodiments, the otic formulation comprises between about 0.01% to about 5% by weight of the poloxamer. In some embodiments, the otic formulation comprises between about 0.01% to about 3% by weight of the poloxamer. In some embodiments, the otic formulation comprises between about 0.01% to about 2% by weight of the poloxamer. In some embodiments, the otic formulation comprises about 0.01% to about 1% by weight of the poloxamer.

In some embodiments, the otic formulation comprises about 0.01% by weight of the poloxamer. In some embodiments, the otic formulation comprises about 0.02% by weight of the poloxamer. In some embodiments, the otic formulation comprises about 0.03% by weight of the poloxamer. In some embodiments, the otic formulation comprises about 0.04% by weight of the poloxamer. In some embodiments, the otic formulation comprises about 0.05% by weight of the poloxamer. In some embodiments, the otic formulation comprises about 0.06% by weight of the poloxamer. In some embodiments, the otic formulation comprises about 0.07% by weight of the poloxamer. In some embodiments, the otic formulation comprises about 0.08% by weight of the poloxamer. In some embodiments, the otic formulation comprises about 0.09% by weight of the poloxamer. In some embodiments, the otic formulation comprises about 0.1% by weight of the poloxamer. In some embodiments, the otic formulation comprises about 0.2% by weight of the poloxamer. In some embodiments, the otic formulation comprises about 0.3% by weight of the poloxamer. In some embodiments, the otic formulation comprises about 0.4% by weight of the poloxamer. In some embodiments, the otic formulation comprises about 0.5% by weight of the poloxamer. In some embodiments, the otic formulation comprises about 0.6% by weight of the poloxamer. In some embodiments, the otic formulation comprises about 0.7% by weight of the poloxamer. In some embodiments, the otic formulation comprises about 0.8% by weight of the poloxamer. In some embodiments, the otic formulation comprises about 0.9% by weight of the poloxamer. In some embodiments, the otic formulation comprises about 1% by weight of the poloxamer. In some embodiments, the otic formulation comprises about 2% by weight of the poloxamer. In some embodiments, the otic formulation comprises about 3% by weight of the poloxamer. In some embodiments, the otic formulation comprises about 4% by weight of the poloxamer. In some embodiments, the otic formulation comprises about 5% by weight of the poloxamer. In some embodiments, the otic formulation comprises about 6% by weight of the poloxamer. In some embodiments, the otic formulation comprises about 7% by weight of the poloxamer. In some embodiments, the otic formulation comprises about 8% by weight of the poloxamer. In some embodiments, the otic formulation comprises about 9% by weight of the poloxamer. In some embodiments, the otic formulation comprises about 10% by weight of the poloxamer. In some embodiments, the otic formulation comprises about 11% by weight of the poloxamer. In some embodiments, the otic formulation comprises about 12% by weight of the poloxamer. In some embodiments, the otic formulation comprises about 13% by weight of the poloxamer. In some embodiments, the otic formulation comprises about 14% by weight of the poloxamer. In some embodiments, the otic formulation comprises about 15% by weight of the poloxamer. In some embodiments, the otic formulation comprises about 16% by weight of the poloxamer. In some embodiments, the otic formulation comprises about 17% by weight of the poloxamer. In some embodiments, the otic formulation comprises about 18% by weight of the poloxamer. In some embodiments, the otic formulation comprises about 19% by weight of the poloxamer. In some embodiments, the otic formulation comprises about 20% by weight of the poloxamer. In some embodiments, the otic formulation comprises about 25% by weight of the poloxamer. In some embodiments, the otic formulation comprises about 30% by weight of the poloxamer. In some embodiments, the otic formulation comprises about 35% by weight of the poloxamer. In some embodiments, the otic formulation comprises about 40% by weight of the poloxamer.

In some embodiments, the otic formulation comprises between about 0.01% to about 20% by weight of the silicon dioxide. In some embodiments, the otic formulation comprises between about 0.01% to about 15% by weight of the silicon dioxide. In some embodiments, the otic formulation comprises between about 0.01% to about 10% by weight of the silicon dioxide. In some embodiments, the otic formulation comprises about 0.01% to about 7% by weight of the silicon dioxide. In some embodiments, the otic formulation comprises between about 0.01% to about 5% by weight of the silicon dioxide. In some embodiments, the otic formulation comprises between about 0.01% to about 3% by weight of the silicon dioxide. In some embodiments, the otic formulation comprises between about 0.01% to about 2% by weight of the silicon dioxide. In some embodiments, the otic formulation comprises about 0.01% to about 1% by weight of the silicon dioxide.

In some embodiments, the otic formulation comprises about 0.01% by weight of the silicon dioxide. In some embodiments, the otic formulation comprises about 0.02% by weight of the silicon dioxide. In some embodiments, the otic formulation comprises about 0.03% by weight of the silicon dioxide. In some embodiments, the otic formulation comprises about 0.04% by weight of the silicon dioxide. In some embodiments, the otic formulation comprises about 0.05% by weight of the silicon dioxide. In some embodiments, the otic formulation comprises about 0.06% by weight of the silicon dioxide. In some embodiments, the otic formulation comprises about 0.07% by weight of the silicon dioxide. In some embodiments, the otic formulation comprises about 0.08% by weight of the silicon dioxide. In some embodiments, the otic formulation comprises about 0.09% by weight of the silicon dioxide. In some embodiments, the otic formulation comprises about 0.1% by weight of the silicon dioxide. In some embodiments, the otic formulation comprises about 0.2% by weight of the silicon dioxide. In some embodiments, the otic formulation comprises about 0.3% by weight of the silicon dioxide. In some embodiments, the otic formulation comprises about 0.4% by weight of the silicon dioxide. In some embodiments, the otic formulation comprises about 0.5% by weight of the silicon dioxide. In some embodiments, the otic formulation comprises

about 0.6% by weight of the silicon dioxide. In some embodiments, the otic formulation comprises about 0.7% by weight of the silicon dioxide. In some embodiments, the otic formulation comprises about 0.8% by weight of the silicon dioxide. In some embodiments, the otic formulation comprises about 0.9% by weight of the silicon dioxide. In some embodiments, the otic formulation comprises about 1% by weight of the silicon dioxide. In some embodiments, the otic formulation comprises about 2% by weight of the silicon dioxide. In some embodiments, the otic formulation comprises about 3% by weight of the silicon dioxide. In some embodiments, the otic formulation comprises about 4% by weight of the silicon dioxide. In some embodiments, the otic formulation comprises about 5% by weight of the silicon dioxide. In some embodiments, the otic formulation comprises about 6% by weight of the silicon dioxide. In some embodiments, the otic formulation comprises about 7% by weight of the silicon dioxide. In some embodiments, the otic formulation comprises about 8% by weight of the silicon dioxide. In some embodiments, the otic formulation comprises about 9% by weight of the silicon dioxide. In some embodiments, the otic formulation comprises about 10% by weight of the silicon dioxide. In some embodiments, the otic formulation comprises about 11% by weight of the silicon dioxide. In some embodiments, the otic formulation comprises about 12% by weight of the silicon dioxide. In some embodiments, the otic formulation comprises about 13% by weight of the silicon dioxide. In some embodiments, the otic formulation comprises about 14% by weight of the silicon dioxide. In some embodiments, the otic formulation comprises about 15% by weight of the silicon dioxide. In some embodiments, the otic formulation comprises about 16% by weight of the silicon dioxide. In some embodiments, the otic formulation comprises about 17% by weight of the silicon dioxide. In some embodiments, the otic formulation comprises about 18% by weight of the silicon dioxide. In some embodiments, the otic formulation comprises about 19% by weight of the silicon dioxide. In some embodiments, the otic formulation comprises about 20% by weight of the silicon dioxide.

In some embodiments, the viscosity modulating agent is silicon dioxide. In some embodiments, the viscosity modulating agent is a polymer, such as povidone, carbomer, or poloxamer. In some embodiments, the viscosity modulating agent is a polysaccharide, such as dextran, alginate, or hyaluronic acid. In some embodiments, the viscosity modulating agent is cellulose-based, such as hydroxypropyl cellulose, hydroxypropyl methylcellulose, carboxymethylcellulose, carboxymethylcellulose sodium, methylcellulose, hydroxyethylcellulose, hydroxypropylcellulose, hydroxypropylmethylcellulose phthalate, hydroxypropylmethylcellulose acetate stearate (HPMCAS), and noncrystalline cellulose. In some embodiments, the viscosity modulating agent is polyvinyl alcohol (PVA). In some embodiments, the viscosity modulating agent is polyethylene glycol (PEG) based.

In some embodiments, the otic formulation comprises between about 0.01% to about 40% by weight of the viscosity modulating agent(s). In some embodiments, the otic formulation comprises between about 0.01% to about 35% by weight of the viscosity modulating agent(s). In some embodiments, the otic formulation comprises between about 0.01% to about 30% by weight of the viscosity modulating agent(s). In some embodiments, the otic formulation comprises between about 0.01% to about 25% by weight of the viscosity modulating agent(s). In some embodiments, the otic formulation comprises between about 0.01% to about 20% by weight of the viscosity modulating agent(s). In some embodiments, the otic formulation comprises between about 0.01% to about 15% by weight of the viscosity modulating agent(s). In some embodiments, the otic formulation comprises between about 0.01% to about 10% by weight of the viscosity modulating agent(s). In some embodiments, the otic formulation comprises about 0.01% to about 7% by weight of the viscosity modulating agent(s). In some embodiments, the otic formulation comprises between about 0.01% to about 5% by weight of the viscosity modulating agent(s). In some embodiments, the otic formulation comprises between about 0.01% to about 3% by weight of the viscosity modulating agent(s). In some embodiments, the otic formulation comprises between about 0.01% to about 2% by weight of the viscosity modulating agent(s). In some embodiments, the otic formulation comprises about 0.01% to about 1% by weight of the viscosity modulating agent(s).

In some embodiments, the otic formulation comprises about 0.01% by weight of the viscosity modulating agent(s). In some embodiments, the otic formulation comprises about 0.02% by weight of the viscosity modulating agent(s). In some embodiments, the otic formulation comprises about 0.03% by weight of the viscosity modulating agent(s). In some embodiments, the otic formulation comprises about 0.04% by weight of the viscosity modulating agent(s). In some embodiments, the otic formulation comprises about 0.05% by weight of the viscosity modulating agent(s). In some embodiments, the otic formulation comprises about 0.06% by weight of the viscosity modulating agent(s). In some embodiments, the otic formulation comprises about 0.07% by weight of the viscosity modulating agent(s). In some embodiments, the otic formulation comprises about 0.08% by weight of the viscosity modulating agent(s). In some embodiments, the otic formulation comprises about 0.09% by weight of the viscosity modulating agent(s). In some embodiments, the otic formulation comprises about 0.1% by weight of the viscosity modulating agent(s). In some embodiments, the otic formulation comprises about 0.2% by weight of the viscosity modulating agent(s). In some embodiments, the otic formulation comprises about 0.3% by weight of the viscosity modulating agent(s). In some embodiments, the otic formulation comprises about 0.4% by weight of the viscosity modulating agent(s). In some embodiments, the otic formulation comprises about 0.5% by weight of the viscosity modulating agent(s). In some embodiments, the otic formulation comprises about 0.6% by weight of the viscosity modulating agent(s). In some embodiments, the otic formulation comprises about 0.7% by weight of the viscosity modulating agent(s). In some embodiments, the otic formulation comprises about 0.8% by weight of the viscosity modulating agent(s). In some

embodiments, the otic formulation comprises about 0.9% by weight of the viscosity modulating agent(s). In some embodiments, the otic formulation comprises about 1% by weight of the viscosity modulating agent(s). In some embodiments, the otic formulation comprises about 2% by weight of the viscosity modulating agent(s). In some embodiments, the otic formulation comprises about 3% by weight of the viscosity modulating agent(s). In some embodiments, the otic formulation comprises about 4% by weight of the viscosity modulating agent(s). In some embodiments, the otic formulation comprises about 5% by weight of the viscosity modulating agent(s). In some embodiments, the otic formulation comprises about 6% by weight of the viscosity modulating agent(s). In some embodiments, the otic formulation comprises about 7% by weight of the viscosity modulating agent(s). In some embodiments, the otic formulation comprises about 8% by weight of the viscosity modulating agent(s). In some embodiments, the otic formulation comprises about 9% by weight of the viscosity modulating agent(s). In some embodiments, the otic formulation comprises about 10% by weight of the viscosity modulating agent(s). In some embodiments, the otic formulation comprises about 11% by weight of the viscosity modulating agent(s). In some embodiments, the otic formulation comprises about 12% by weight of the viscosity modulating agent(s). In some embodiments, the otic formulation comprises about 13% by weight of the viscosity modulating agent(s). In some embodiments, the otic formulation comprises about 14% by weight of the viscosity modulating agent(s). In some embodiments, the otic formulation comprises about 15% by weight of the viscosity modulating agent(s). In some embodiments, the otic formulation comprises about 16% by weight of the viscosity modulating agent(s). In some embodiments, the otic formulation comprises about 17% by weight of the viscosity modulating agent(s). In some embodiments, the otic formulation comprises about 18% by weight of the viscosity modulating agent(s). In some embodiments, the otic formulation comprises about 19% by weight of the viscosity modulating agent(s). In some embodiments, the otic formulation comprises about 20% by weight of the viscosity modulating agent(s). In some embodiments, the otic formulation comprises about 25% by weight of the viscosity modulating agent(s). In some embodiments, the otic formulation comprises about 30% by weight of the viscosity modulating agent(s). In some embodiments, the otic formulation comprises about 35% by weight of the viscosity modulating agent(s). In some embodiments, the otic formulation comprises about 40% by weight of the viscosity modulating agent(s).

In some embodiments, the otic formulations described herein further comprise cholesterol. In some embodiments, the otic formulation comprises between about 0.01% to about 40% by weight of the cholesterol. In some embodiments, the otic formulation comprises between about 0.01% to about 35% by weight of the cholesterol. In some embodiments, the otic formulation comprises between about 0.01% to about 30% by weight of the cholesterol. In some embodiments, the otic formulation comprises between about 0.01% to about 25% by weight of the cholesterol. In some embodiments, the otic formulation comprises between about 0.01% to about 20% by weight of the cholesterol. In some embodiments, the otic formulation comprises between about 0.01% to about 15% by weight of the cholesterol. In some embodiments, the otic formulation comprises between about 0.01% to about 10% by weight of the cholesterol. In some embodiments, the otic formulation comprises about 0.01% to about 7% by weight of the cholesterol. In some embodiments, the otic formulation comprises between about 0.01% to about 5% by weight of the cholesterol. In some embodiments, the otic formulation comprises between about 0.01% to about 3% by weight of the cholesterol. In some embodiments, the otic formulation comprises between about 0.01% to about 2% by weight of the cholesterol. In some embodiments, the otic formulation comprises about 0.01% to about 1% by weight of the cholesterol.

In some embodiments, the otic formulation comprises about 0.01% by weight of the cholesterol. In some embodiments, the otic formulation comprises about 0.02% by weight of the cholesterol. In some embodiments, the otic formulation comprises about 0.03% by weight of the cholesterol. In some embodiments, the otic formulation comprises about 0.04% by weight of the cholesterol. In some embodiments, the otic formulation comprises about 0.05% by weight of the cholesterol. In some embodiments, the otic formulation comprises about 0.06% by weight of the cholesterol. In some embodiments, the otic formulation comprises about 0.07% by weight of the cholesterol. In some embodiments, the otic formulation comprises about 0.08% by weight of the cholesterol. In some embodiments, the otic formulation comprises about 0.09% by weight of the cholesterol. In some embodiments, the otic formulation comprises about 0.1% by weight of the cholesterol. In some embodiments, the otic formulation comprises about 0.2% by weight of the cholesterol. In some embodiments, the otic formulation comprises about 0.3% by weight of the cholesterol. In some embodiments, the otic formulation comprises about 0.4% by weight of the cholesterol. In some embodiments, the otic formulation comprises about 0.5% by weight of the cholesterol. In some embodiments, the otic formulation comprises about 0.6% by weight of the cholesterol. In some embodiments, the otic formulation comprises about 0.7% by weight of the cholesterol. In some embodiments, the otic formulation comprises about 0.8% by weight of the cholesterol. In some embodiments, the otic formulation comprises about 0.9% by weight of the cholesterol. In some embodiments, the otic formulation comprises about 1% by weight of the cholesterol. In some embodiments, the otic formulation comprises about 2% by weight of the cholesterol. In some embodiments, the otic formulation comprises about 3% by weight of the cholesterol. In some embodiments, the otic formulation comprises about 4% by weight of the cholesterol. In some embodiments, the otic formulation comprises about 5% by weight of the cholesterol. In some embodiments, the otic formulation comprises about 6% by weight of the cholesterol. In some embodiments, the otic formulation comprises about 7% by weight of the

cholesterol. In some embodiments, the otic formulation comprises about 8% by weight of the cholesterol. In some embodiments, the otic formulation comprises about 9% by weight of the cholesterol. In some embodiments, the otic formulation comprises about 10% by weight of the cholesterol. In some embodiments, the otic formulation comprises about 11% by weight of the cholesterol. In some embodiments, the otic formulation comprises about 12% by weight of the cholesterol. In some embodiments, the otic formulation comprises about 13% by weight of the cholesterol. In some embodiments, the otic formulation comprises about 14% by weight of the cholesterol. In some embodiments, the otic formulation comprises about 15% by weight of the cholesterol. In some embodiments, the otic formulation comprises about 16% by weight of the cholesterol. In some embodiments, the otic formulation comprises about 17% by weight of the cholesterol. In some embodiments, the otic formulation comprises about 18% by weight of the cholesterol. In some embodiments, the otic formulation comprises about 19% by weight of the cholesterol. In some embodiments, the otic formulation comprises about 20% by weight of the cholesterol. In some embodiments, the otic formulation comprises about 25% by weight of the cholesterol. In some embodiments, the otic formulation comprises about 30% by weight of the cholesterol. In some embodiments, the otic formulation comprises about 35% by weight of the cholesterol. In some embodiments, the otic formulation comprises about 40% by weight of the cholesterol.

In some embodiments, the triglycerides are a mixture of long-chain triglycerides and medium-chain triglycerides. In some embodiments, the ratio of long-chain triglycerides to medium-chain triglycerides is from about 0.01:99.99 to about 99.99:0.01. In some embodiments, the ratio of long-chain triglycerides to medium-chain triglycerides is from about 0.1:99.9 to about 99.9:0.1. In some embodiments, the ratio of long-chain triglycerides to medium-chain triglycerides is about 0.1:99.9. In some embodiments, the ratio of long-chain triglycerides to medium-chain triglycerides is about 0.5:99.5. In some embodiments, the ratio of long-chain triglycerides to medium-chain triglycerides is about 1.0:99.0. In some embodiments, the ratio of long-chain triglycerides to medium-chain triglycerides is about 5.0:95.0. In some embodiments, the ratio of long-chain triglycerides to medium-chain triglycerides is about 10.0:80.0. In some embodiments, the ratio of long-chain triglycerides to medium-chain triglycerides is about 15.0:85.0. In some embodiments, the ratio of long-chain triglycerides to medium-chain triglycerides is about 20.0:80.0. In some embodiments, the ratio of long-chain triglycerides to medium-chain triglycerides is about 25.0:75.0. In some embodiments, the ratio of long-chain triglycerides to medium-chain triglycerides is about 30.0:70.0. In some embodiments, the ratio of long-chain triglycerides to medium-chain triglycerides is about 35.0:65.0. In some embodiments, the ratio of long-chain triglycerides to medium-chain triglycerides is about 40.0:60.0. In some embodiments, the ratio of long-chain triglycerides to medium-chain triglycerides is about 45.0:55.0. In some embodiments, the ratio of long-chain triglycerides to medium-chain triglycerides is about 50.0:50.0. In some embodiments, the ratio of long-chain triglycerides to medium-chain triglycerides is about 55.0:45.0. In some embodiments, the ratio of long-chain triglycerides to medium-chain triglycerides is about 60.0:40.0. In some embodiments, the ratio of long-chain triglycerides to medium-chain triglycerides is about 65.0:35.0. In some embodiments, the ratio of long-chain triglycerides to medium-chain triglycerides is about 70.0:30.0. In some embodiments, the ratio of long-chain triglycerides to medium-chain triglycerides is about 75.0:25.0. In some embodiments, the ratio of long-chain triglycerides to medium-chain triglycerides is about 80.0:20.0. In some embodiments, the ratio of long-chain triglycerides to medium-chain triglycerides is about 85.0:15.0. In some embodiments, the ratio of long-chain triglycerides to medium-chain triglycerides is about 90.0:10.0. In some embodiments, the ratio of long-chain triglycerides to medium-chain triglycerides is about 95.0:5.0. In some embodiments, the ratio of long-chain triglycerides to medium-chain triglycerides is about 99.0:1.0. In some embodiments, the ratio of long-chain triglycerides to medium-chain triglycerides is about 99.9:0.1.

Long-chain triglycerides (LCTs) are triglycerides are derived from glycerol and at least two long-chain fatty acids. In some embodiments, the triglycerides are derived from glycerol and three long-chain fatty acids. In some embodiments, the triglycerides are derived from glycerol, two long-chain fatty acids, and one medium-chain fatty acid.

In some embodiments, the long-chain triglycerides are derived from glycerol and at least two long-chain fatty acids. In some embodiments, each long-chain fatty acid independently comprises greater than 12 carbon atoms in the carbon chain. In some embodiments, each long-chain fatty acid independently comprises 13 to 38 carbon atoms in the carbon chain. In some embodiments, the long-chain fatty acid is a saturated long-chain fatty acids, an unsaturated long-chain fatty acid, or a combination thereof. In some embodiments, each long-chain fatty acid independently comprises 13, 14, 15, 16, 17, 18, 19, 20, 21, 22, 23, 24, 25, 26, 27, 28, 29, 30, 31, 32, 33, 34, 35, 36, 37, or 38 carbon atoms in the carbon chain. In some embodiments, each long-chain fatty acid independently comprises 13 to 24 carbon atoms in the carbon chain. In some embodiments, the long-chain fatty acid is a saturated long-chain fatty acid, an unsaturated long-chain fatty acid, or a combination thereof. In some embodiments, each long-chain fatty acid independently comprises 13, 14, 15, 16, 17, 18, 19, 20, 21, 22, 23, or 24 carbon atoms in the carbon chain. In some embodiments, each long-chain fatty acid independently comprises 13 to 22 carbon atoms in the carbon chain. In some embodiments, the long-chain fatty acids are a saturated long-chain fatty acid, an unsaturated long-chain fatty acid, or a combination thereof. In some embodiments, each long-chain fatty acid independently comprises 13, 14, 15, 16, 17, 18, 19, 20, 21, or 22 carbon atoms in the carbon chain.

In some embodiments, each long-chain fatty acid is independently tridecylic acid (tridecanoic acid), myristic acid (tetradecanoic acid), pentadecylic acid (pentadecanoic acid), palmitic acid (hexadecanoic acid) , margaric acid (heptadecanoic acid), stearic acid (octadecanoic acid), nonadecylic acid (nonadecanoic acid), arachidic acid (eicosanoic acid), heneicosylic acid (heneicosanoic acid), behenic acid (docosanoic acid), tricosylic acid (tricosanoic acid), lignoceric acid (tetracosanoic acid), pentacosylic acid (pentacosanoic acid), cerotic acid (hexacosanoic acid), heptacosylic acid (heptacosanoic acid), montanic acid (octacosanoic acid), nonacosylic acid (nonacosanoic acid), melissic acid (triacontanoic acid), henatriacontylic acid (henatriacontanoic acid), lacceroic acid (dotriacontanoic acid), psyllic acid (tritriacontanoic acid), geddic acid (tetratriacontanoic acid), ceroplastic acid (pentatriacontanoic acid), hexatriacontylic acid (hexatriacontanoic acid), heptatriacontanoic acid (heptatriacontanoic acid), or octatriacontanoic acid.

In some embodiments, each long-chain fatty acid is independently $\alpha$-linolenic acid, stearidonic acid, eicosapentaenoic acid, docosahexaenoic acid, linoleic acid, $\gamma$-linolenic acid, dihomo-$\gamma$-linolenic acid, arachidonic acid, docosatetraenoic acid, palmitoleic acid, vaccenic acid, paullinic acid, oleic acid, elaidic acid, gondoic acid, erucic acid, nervonic acid, or mead acid.

In some embodiments, the otic triglyceride based pharmaceutical formulations have triglycerides in an amount that is sufficient to stabilize the therapeutic agent for injection into the ear. In some embodiments, the injection is into the outer ear. In some embodiments, the injection is into the middle ear. In some embodiments, the injection is intratympanic. In some embodiments, the injection is into the inner ear. In some embodiments, the otic triglyceride based pharmaceutical formulations have triglycerides in an amount that is sufficient to provide sufficient retention time in the ear. In some embodiments, the sufficient retention time in the ear is for the middle ear. In some embodiments, the sufficient retention time in the ear is for the inner ear. In some embodiments, the sufficient retention time in the ear is for the outer ear. In some embodiments, the outer ear is the external auditory canal, the outer surface of the tympanic membrane, or a combination thereof. In some embodiments, the outer ear is the external auditory canal. In some embodiments, the otic triglyceride based pharmaceutical formulations have triglycerides in an amount that is sufficient to provide sustained release of the therapeutic agent. In some embodiments, the sustained release of the therapeutic agent is in the outer ear. In some embodiments, the sustained release of the therapeutic agent is in the middle ear. In some embodiments, the sustained release of the therapeutic agent is in the inner ear.

## Auris-acceptable Formulations

In some embodiments, the otic formulations described herein are thickened liquid formulations . The otic formulations described herein are suspension formulations . The otic formulations described herein are solution formulations . In some embodiments, the otic formulations have greater viscosity than an aqueous liquid formulation. In some embodiments, the formulation has a viscosity of greater than 1 cP (centipoise). In some embodiments, the formulation has a viscosity of at least about 10 cP, about 20 cP, about 30 cP, about 40 cP, about 50 cP, about 60 cP, about 70 cP, about 80 cP, about 90 cP, about 100 cP, about 200 cP, about 300 cP, about 400 cP, about 500 cP, about 600 cP, about 700 cP, about 800 cP, about 900 cP, about 1,000 cP, about 2,000 cP, about 3,000 cP, about 4,000 cP, about 5,000 cP, about 6,000 cP, about 7,000 cP, about 8,000 cP, about 9,000 cP, about 10,000 cP, about 15,000 cP, or about 20,000 cP. In some embodiments, the formulation has a viscosity of less than about 1,000 cP. In some embodiments, the formulation has a viscosity of less than about 10,000 cP. In some embodiments, the formulation has a viscosity of about 2 cP to about 250,000 cP, about 2 cP to about 100,000 cP, about 2 cP to about 50,000 cP, about 2 cP to about 25,000 cP, about 2 cP to about 10,000 cP, about 2 cP to about 5,000 cP, about 2 cP to about 1,000 cP, about 2 cP to about 500 cP, about 2 cP to about 250 cP, about 2 cP to about 100 cP, about 2 cP to about 90 cP, about 2 cP to about 80 cP, about 2 cP to about 70 cP, about 2 cP to about 60 cP, about 2 cP to about 50 cP, about 2 cP to about 40 cP, about 2 cP to about 30 cP, about 2 cP to about 20 cP, or about 2 cP to about 10 cP. In some embodiments, the liquid formulation has a viscosity of about 2 cP, about 5 cP, about 10 cP, about 20 cP, about 30 cP, about 40 cP, about 50 cP, about 60 cP, about 70 cP, about 80 cP, about 90 cP, about 100 cP, about 200 cP, about 300 cP, about 400 cP, about 500 cP, about 600 cP, about 700 cP, about 800 cP, about 900 cP, about 1,000 cP, about 5,000 cP, about 10,000 cP, about 20,000 cP, about 50,000 cP, about 100,000 cP, or about 250,000 cP.

In some embodiments, the formulation has a viscosity between about 10 cP to about 20,000 cP. In some embodiments, the formulation has a viscosity between about 10 cP to about 10,000 cP. In some embodiments, the formulation has a viscosity between about 10 cP to about 5,000 cP. In some embodiments, the formulation has a viscosity between about 10 cP to about 1,000 cP. In some embodiments, the formulation has a viscosity between about 10 cP to about 500 cP. In some embodiments, the formulation has a viscosity between about 10 cP to about 250 cP. In some embodiments, the formulation has a viscosity between about 10 cP to about 100 cP. In some embodiments, the formulation has a viscosity between about 10 cP to about 50 cP.

In some embodiments, the formulation has a viscosity of about 10 cP. In some embodiments, the formulation has a viscosity of about 20 cP. In some embodiments, the formulation has a viscosity of about 30 cP. In some embodiments, the formulation has a viscosity of about 40 cP. In some embodiments, the formulation has a viscosity of about 50 cP. In some embodiments, the formulation has a viscosity of about 60 cP. In some embodiments, the formulation has a viscosity of about 70 cP. In some embodiments, the formulation has a viscosity of about 80 cP. In some embodiments, the formulation has a viscosity of about 90 cP. In some embodiments, the formulation has a viscosity of about 100 cP. In some

embodiments, the formulation has a viscosity of about 150 cP. In some embodiments, the formulation has a viscosity of about 200 cP. In some embodiments, the formulation has a viscosity of about 250 cP. In some embodiments, the formulation has a viscosity of about 300 cP. In some embodiments, the formulation has a viscosity of about 350 cP. In some embodiments, the formulation has a viscosity of about 400 cP. In some embodiments, the formulation has a viscosity of about 450 cP. In some embodiments, the formulation has a viscosity of about 500 cP. In some embodiments, the formulation has a viscosity of about 550 cP. In some embodiments, the formulation has a viscosity of about 600 cP. In some embodiments, the formulation has a viscosity of about 650 cP. In some embodiments, the formulation has a viscosity of about 700 cP. In some embodiments, the formulation has a viscosity of about 750 cP. In some embodiments, the formulation has a viscosity of about 800 cP. In some embodiments, the formulation has a viscosity of about 850 cP. In some embodiments, the formulation has a viscosity of about 900 cP. In some embodiments, the formulation has a viscosity of about 950 cP. In some embodiments, the formulation has a viscosity of about 1,000 cP. In some embodiments, the formulation has a viscosity of about 1,500 cP. In some embodiments, the formulation has a viscosity of about 2,000 cP. In some embodiments, the formulation has a viscosity of about 2,500 cP. In some embodiments, the formulation has a viscosity of about 3,000 cP. In some embodiments, the formulation has a viscosity of about 3,500 cP. In some embodiments, the formulation has a viscosity of about 4,000 cP. In some embodiments, the formulation has a viscosity of about 4,500 cP. In some embodiments, the formulation has a viscosity of about 5,000 cP. In some embodiments, the formulation has a viscosity of about 5,500 cP. In some embodiments, the formulation has a viscosity of about 6,000 cP. In some embodiments, the formulation has a viscosity of about 6,500 cP. In some embodiments, the formulation has a viscosity of about 7,000 cP. In some embodiments, the formulation has a viscosity of about 7,500 cP. In some embodiments, the formulation has a viscosity of about 8,000 cP. In some embodiments, the formulation has a viscosity of about 8,500 cP. In some embodiments, the formulation has a viscosity of about 9,000 cP. In some embodiments, the formulation has a viscosity of about 9,500 cP. In some embodiments, the formulation has a viscosity of about 10,000 cP. In some embodiments, the formulation has a viscosity of about 20,000 cP.

In some embodiments, the otic formulation is free or substantially free of viscosity modulating agent. In some embodiments, the otic formulation contains at least one viscosity modulating agent that provides the otic formulation with a viscosity of at least about 10 cP, about 20 cP, about 30 cP, about 40 cP, about 50 cP, about 60 cP, about 70 cP, about 80 cP, about 90 cP, about 100 cP, about 200 cP, about 300 cP, about 400 cP, about 500 cP, about 600 cP, about 700 cP, about 800 cP, about 900 cP, about 1000 cP, about 2,000 cP, about 3,000 cP, about 4,000 cP, about 5,000 cP, about 6,000 cP, about 7,000 cP, about 8,000 cP, about 9,000 cP, about 10,000 cP, about 15,000 cP, or about 20,000 cP. In some embodiments, the formulation contains at least one viscosity modulating agent that provides the otic formulation with a viscosity of less than about 1,000 cP. In some embodiments, the formulation contains at least one viscosity modulating agent that provides the otic formulation with a viscosity of less than about 10,000 cP. In some embodiments, the otic formulation contains at least one viscosity modulating agent that provides the formulation with a viscosity of about 2 cP to about 250,000 cP, about 2 cP to about 100,000 cP, about 2 cP to about 50,000 cP, about 2 cP to about 25,000 cP, about 2 cP to about 10,000 cP, about 2 cP to about 5,000 cP, about 2 cP to about 1,000 cP, about 2 cP to about 500 cP, about 2 cP to about 250 cP, about 2 cP to about 100 cP, about 2 cP to about 90 cP, about 2 cP to about 80 cP, about 2 cP to about 70 cP, about 2 cP to about 60 cP, about 2 cP to about 50 cP, about 2 cP to about 40 cP, about 2 cP to about 30 cP, about 2 cP to about 20 cP, or about 2 cP to about 10 cP. In some embodiments, the otic formulation contains at least one viscosity modulating agent that provides the otic formulation with a viscosity of about 2 cP, about 5 cP, about 10 cP, about 20 cP, about 30 cP, about 40 cP, about 50 cP, about 60 cP, about 70 cP, about 80 cP, about 90 cP, about 100 cP, about 200 cP, about 300 cP, about 400 cP, about 500 cP, about 600 cP, about 700 cP, about 800 cP, about 900 cP, about 1,000 cP, about 5,000 cP, about 10,000 cP, about 20,000 cP, about 50,000 cP, about 100,000 cP, or about 250,000 cP. In some embodiments, the viscosity modulating agent is not a poloxamer. In some embodiments, the viscosity modulating agent is a poloxamer. In some embodiments, the poloxamer is P407. In some embodiments, the viscosity modulating agent is povidone. In some embodiments, the viscosity modulating agent is carbomer. In some embodiments, the viscosity modulating agent is a polymer. In some embodiments, the viscosity modulating agent is silicon dioxide. In some embodiments, the viscosity modulating agent is silicon dioxide, poloxamer, carbomer, povidone, or a combination thereof. In some embodiments, the viscosity modulating agent is a polysaccharide, such as dextran, alginate, or hyaluronic acid. In some embodiments, the viscosity modulating agent is cellulose-based, such as hydroxypropyl cellulose, hydroxypropyl methylcellulose, carboxymethylcellulose, carboxymethylcellulose sodium, methylcellulose, hydroxyethylcellulose, hydroxypropylcellulose, hydroxypropylmethylcellulose phthalate, hydroxypropylmethylcellulose acetate stearate (HPMCAS), and noncrystalline cellulose. In some embodiments, the viscosity modulating agent is polyvinyl alcohol (PVA). In some embodiments, the viscosity modulating agent is polyethylene glycol (PEG) based. In some embodiments, the viscosity modulating agent is silicon dioxide, poloxamer, carbomer, povidone, polysaccharide, cellulose-based, polyvinyl alcohol, polyethylene glycol, or a combination thereof. In some embodiments, the viscosity modulating agent is an oil. In some embodiments, the viscosity modulating agent is beeswax. In some embodiments, the viscosity modulating agent is Vaseline. In some embodiments, the viscosity modulating agent is petroleum jelly. In some embodiments, the viscosity modulating agent is 12-hydroxystearic acid. In some embodiments, the formulation comprises between about 0.01% to about 80% by weight of the viscosity modulating agent. In some

embodiments, the formulation comprises between about 0.01% to about 50% by weight of the viscosity modulating agent. In some embodiments, the formulation comprises between about 0.01% to about 20% by weight of the viscosity modulating agent. In some embodiments, the formulation comprises between about 0.01% to about 15% by weight of the viscosity modulating agent. In some embodiments, the formulation comprises between about 0.01% to about 10% by weight of the viscosity modulating agent. In some embodiments, the formulation comprises between about 0.01% to about 9% by weight of the viscosity modulating agent. In some embodiments, the formulation comprises between about 0.01% to about 8% by weight of the viscosity modulating agent. In some embodiments, the formulation comprises between about 0.01% to about 7% by weight of the viscosity modulating agent. In some embodiments, the formulation comprises between about 0.01% to about 6% by weight of the viscosity modulating agent. In some embodiments, the formulation comprises between about 0.01% to about 5% by weight of the viscosity modulating agent. In some embodiments, the formulation comprises between about 0.01% to about 4% by weight of the viscosity modulating agent. In some embodiments, the formulation comprises between about 0.01% to about 3% by weight of the viscosity modulating agent. In some embodiments, the formulation comprises between about 0.01% to about 2% by weight of the viscosity modulating agent. In some embodiments, the formulation comprises between about 0.01% to about 1% by weight of the viscosity modulating agent. In some embodiments, the formulation comprises between about 0.1% to about 80% by weight of the viscosity modulating agent. In some embodiments, the formulation comprises between about 0.1% to about 50% by weight of the viscosity modulating agent. In some embodiments, the formulation comprises between about 0.1% to about 20% by weight of the viscosity modulating agent. In some embodiments, the formulation comprises between about 0.1% to about 10% by weight of the viscosity modulating agent. In some embodiments, the formulation comprises between about 0.1% to about 5% by weight of the viscosity modulating agent. In some embodiments, the formulation comprises between about 1% to about 80% by weight of the viscosity modulating agent. In some embodiments, the formulation comprises between about 1% to about 50% by weight of the viscosity modulating agent. In some embodiments, the formulation comprises between about 1% to about 20% by weight of the viscosity modulating agent. In some embodiments, the formulation comprises between about 1% to about 10% by weight of the viscosity modulating agent. In some embodiments, the formulation comprises between about 1% to about 5% by weight of the viscosity modulating agent. In some embodiments, the formulation comprises about 0.01%, about 0.05%, about 0.1%, about 0.2%, about 0.3%, about 0.4%, about 0.5%, about 0.6%, about 0.7%, about 0.8%, about 0.9%, about 1.0%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 25%, about 30%, about 35%, about 40%, about 45%, about 50%, about 55%, about 60%, about 65%, about 70%, about 75%, about 80%, about 85%, about 90% by weight of the viscosity modulating agent. In some embodiments, the formulation comprises greater than about 0.01%, greater than about 0.05%, greater than about 0.1%, greater than about 0.2%, greater than about 0.3%, greater than about 0.4%, greater than about 0.5%, greater than about 0.6%, greater than about 0.7%, greater than about 0.8%, greater than about 0.9%, greater than about 1.0%, greater than about 2%, greater than about 3%, greater than about 4%, greater than about 5%, greater than about 6%, greater than about 7%, greater than about 8%, greater than about 9%, greater than about 10%, greater than about 11%, greater than about 12%, greater than about 13%, greater than about 14%, greater than about 15%, greater than about 16%, greater than about 17%, greater than about 18%, greater than about 19%, greater than about 20%, greater than about 25%, greater than about 30%, greater than about 35%, greater than about 40%, greater than about 45%, greater than about 50%, greater than about 55%, greater than about 60%, greater than about 65%, greater than about 70%, greater than about 75%, greater than about 80%, greater than about 85%, greater than about 90% by weight of the viscosity modulating agent. In some embodiments, the formulation comprises less than about 0.01%, less than about 0.05%, less than about 0.1%, less than about 0.2%, less than about 0.3%, less than about 0.4%, less than about 0.5%, less than about 0.6%, less than about 0.7%, less than about 0.8%, less than about 0.9%, less than about 1.0%, less than about 2%, less than about 3%, less than about 4%, less than about 5%, less than about 6%, less than about 7%, less than about 8%, less than about 9%, less than about 10%, less than about 11%, less than about 12%, less than about 13%, less than about 14%, less than about 15%, less than about 16%, less than about 17%, less than about 18%, less than about 19%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, less than about 90% by weight of the viscosity modulating agent.

In some embodiments, the otic formulation is free or substantially free of water. In some embodiments, the otic formulation comprises less than 10% by weight of water. In some embodiments, the otic formulation comprises less than 9% by weight of water. In some embodiments, the otic formulation comprises less than 8% by weight of water. In some embodiments, the otic formulation comprises less than 7% by weight of water. In some embodiments, the otic formulation comprises less than 6% by weight of water. In some embodiments, the otic formulation comprises less than 5% by weight of water. In some embodiments, the otic formulation comprises less than 4% by weight of water. In some embodiments, the otic formulation comprises less than 3% by weight of water. In some embodiments, the otic formulation comprises less than 2% by weight of water. In some embodiments, the otic formulation comprises less than 1% by weight of water. In some embodiments, the otic formulation comprises less than 0.5% by weight of water. In some embodiments, the otic

formulation comprises less than 0.1% by weight of water. In some embodiments, an otic formulation disclosed herein comprises less than about 50 ppm of water. In some embodiments, an otic formulation disclosed herein comprises less than about 25 ppm of water. In some embodiments, an otic formulation disclosed herein comprises less than about 20 ppm of water. In some embodiments, an otic formulation disclosed herein comprises less than about 10 ppm of water. In some embodiments, an otic formulation disclosed herein comprises less than about 5 ppm of water. In some embodiments, an otic formulation disclosed herein comprises less than about 1 ppm of water.

In some embodiments (not claimed), the otic formulation is free or substantially free of poloxamer. In some embodiments (not claimed), the otic formulation is free or substantially free of poloxamer 407.

In some embodiments, the otic formulation is free or substantially free of C1-C6 alcohols or C1-C6 glycols. In some embodiments, the otic formulation is free or substantially free of C1-C6 alcohols. In some embodiments, the otic formulation is free or substantially free of C1-C6 glycols. In some embodiments, the otic formulation comprises less than 10% by weight of C1-C6 alcohols or C1-C6 glycols. In some embodiments, the otic formulation comprises less than 9% by weight of C1-C6 alcohols or C1-C6 glycols. In some embodiments, the otic formulation comprises less than 8% by weight of C1-C6 alcohols or C1-C6 glycols. In some embodiments, the otic formulation comprises less than 7% by weight of C1-C6 alcohols or C1-C6 glycols. In some embodiments, the otic formulation comprises less than 6% by weight of C1-C6 alcohols or C1-C6 glycols. In some embodiments, the otic formulation comprises less than 5% by weight of C1-C6 alcohols or C1-C6 glycols. In some embodiments, the otic formulation comprises less than 4% by weight of C1-C6 alcohols or C1-C6 glycols. In some embodiments, the otic formulation comprises less than 3% by weight of C1-C6 alcohols or C1-C6 glycols. In some embodiments, the otic formulation comprises less than 2% by weight of C1-C6 alcohols or C1-C6 glycols. In some embodiments, the otic formulation comprises less than 1% by weight of C1-C6 alcohols or C1-C6 glycols. In some embodiments, the otic formulation comprises less than 0.5% by weight of C1-C6 alcohols or C1-C6 glycols. In some embodiments, the otic formulation comprises less than 0.1% by weight of C1-C6 alcohols or C1-C6 glycols. In some embodiments, an otic formulation disclosed herein comprises less than about 50 ppm of each of C1-C6 alcohols or C1-C6 glycols. In some embodiments, an otic formulation disclosed herein comprises less than about 25 ppm of each of C1-C6 alcohols or C1-C6 glycols. In some embodiments, an otic formulation disclosed herein comprises less than about 20 ppm of each of C1-C6 alcohols or C1-C6 glycols. In some embodiments, an otic formulation disclosed herein comprises less than about 10 ppm of each of C1-C6 alcohols or C1-C6 glycols. In some embodiments, an otic formulation disclosed herein comprises less than about 5 ppm of each of C1-C6 alcohols or C1-C6 glycols. In some embodiments, an otic formulation disclosed herein comprises less than about 1 ppm of each of C1-C6 alcohols or C1-C6 glycols.

In some embodiments, the otic formulation is free or substantially free of C1-C4 alcohols or C1-C4 glycols. In some embodiments, the otic formulation is free or substantially free of C1-C4 alcohols. In some embodiments, the otic formulation is free or substantially free of C1-C4 glycols. In some embodiments, the otic formulation comprises less than 10% by weight of C1-C4 alcohols or C1-C4 glycols. In some embodiments, the otic formulation comprises less than 9% by weight of C1-C4 alcohols or C1-C4 glycols. In some embodiments, the otic formulation comprises less than 8% by weight of C1-C4 alcohols or C1-C4 glycols. In some embodiments, the otic formulation comprises less than 7% by weight of C1-C4 alcohols or C1-C4 glycols. In some embodiments, the otic formulation comprises less than 6% by weight of C1-C4 alcohols or C1-C4 glycols. In some embodiments, the otic formulation comprises less than 5% by weight of C1-C4 alcohols or C1-C4 glycols. In some embodiments, the otic formulation comprises less than 4% by weight of C1-C4 alcohols or C1-C4 glycols. In some embodiments, the otic formulation comprises less than 3% by weight of C1-C4 alcohols or C1-C4 glycols. In some embodiments, the otic formulation comprises less than 2% by weight of C1-C4 alcohols or C1-C4 glycols. In some embodiments, the otic formulation comprises less than 1% by weight of C1-C4 alcohols or C1-C4 glycols. In some embodiments, the otic formulation comprises less than 0.5% by weight of C1-C4 alcohols or C1-C4 glycols. In some embodiments, the otic formulation comprises less than 0.1% by weight of C1-C4 alcohols or C1-C4 glycols. In some embodiments, an otic formulation disclosed herein comprises less than about 50 ppm of each of C1-C4 alcohols or C1-C4 glycols. In some embodiments, an otic formulation disclosed herein comprises less than about 25 ppm of each of C1-C4 alcohols or C1-C4 glycols. In some embodiments, an otic formulation disclosed herein comprises less than about 20 ppm of each of C1-C4 alcohols or C1-C4 glycols. In some embodiments, an otic formulation disclosed herein comprises less than about 10 ppm of each of C1-C4 alcohols or C1-C4 glycols. In some embodiments, an otic formulation disclosed herein comprises less than about 5 ppm of each of C1-C4 alcohols or C1-C4 glycols. In some embodiments, an otic formulation disclosed herein comprises less than about 1 ppm of each of C1-C4 alcohols or C1-C4 glycols.

By way of non-limiting example, the use of the following commonly used solvents should be limited, reduced or eliminated when formulating agents for administration to the ear: alcohols, propylene glycol, and cyclohexane. Thus, in some embodiments, an otic formulation disclosed herein is free or substantially free of alcohols, propylene glycol, and cyclohexane. In some embodiments, an otic formulation disclosed herein comprises less than about 50 ppm of each of alcohols, propylene glycol, and cyclohexane. In some embodiments, an otic formulation disclosed herein comprises less than about 25 ppm of each of alcohols, propylene glycol, and cyclohexane. In some embodiments, an otic formulation disclosed herein comprises less than about 20 ppm of each of alcohols, propylene glycol, and cyclohexane. In some embodiments, an otic formulation disclosed herein comprises less than about 10 ppm of each of alcohols, propylene

glycol, and cyclohexane. In some embodiments, an otic formulation disclosed herein comprises less than about 5 ppm of each of alcohols, propylene glycol, and cyclohexane. In some embodiments, an otic formulation disclosed herein comprises less than about 1 ppm of each of alcohols, propylene glycol, and cyclohexane.

In some embodiments, therapeutic agent, or pharmaceutically acceptable prodrug or salt thereof, is multiparticulate. In some embodiments, the therapeutic agent, or pharmaceutically acceptable prodrug or salt thereof, is essentially in the form of micronized particles. In some embodiments, the therapeutic agent, or pharmaceutically acceptable prodrug or salt thereof, is essentially dissolved in the otic pharmaceutical formulation .

**Poloxamers**

In some embodiments, the otic formulations described herein further comprise poloxamer. In some embodiments (not claimed), the otic formulations described herein are free or substantially free of poloxamer. An example of a poloxamer includes Poloxamer 407 (PF-127) is a nonionic surfactant composed of polyoxyethylene-polyoxypropylene copolymers. Other commonly used poloxamers also include 188 (F-68 grade), 237 (F-87 grade), 338 (F-108 grade).

In some embodiments, the otic formulations disclosed herein comprise PF-127, 188 (F-68 grade), 237 (F-87 grade), or 338 (F-108 grade). In some embodiments, the otic formulations disclosed herein comprise poloxamer 407.

In some embodiments (not claimed), the otic formulations disclosed herein are free or substantially free of PF-127, 188 (F-68 grade), 237 (F-87 grade), or 338 (F-108 grade). In some embodiments, the otic formulations disclosed here are free or substantially free of poloxamer 407.

In certain embodiments, the thickening agents (i.e., viscosity enhancing agents or viscosity modulating agents) are also utilized in the otic formulations presented herein. In some embodiments, the thickening agent is a cellulose based thickening agent. In some instances, the addition of a thickening agent introduces a diffusional barrier and reduces the rate of release of the therapeutic agent. In some embodiments, the thickening agent or viscosity enhancer or viscosity modulating agent is not a poloxamer. In some embodiments, the thickening agent or viscosity enhancer or viscosity modulating agent is not poloxamer 407. In some embodiments, the thickening agent or viscosity enhancer or viscosity modulating agent is a poloxamer. In some embodiments, the thickening agent or viscosity enhancer or viscosity modulating agent is poloxamer 407.

In some embodiments, the otic formulations disclosed herein also contain preservatives, cosolvents, suspending agents, viscosity enhancing agents, ionic-strength and osmolality adjustors and other excipients in addition to buffering agents. Suitable water soluble preservatives which are employed in the drug delivery vehicle are sodium bisulfite, sodium thiosulfate, ascorbate, benzalkonium chloride, chlorobutanol, thimerosal, parabens, benzyl alcohol, phenylethanol and others. These agents are present, generally, in amounts of about 0.001% to about 5% by weight and, preferably, in the amount of about 0.01 to about 2% by weight.

Suitable water soluble buffering agents are alkali or alkaline earth metal carbonates, phosphates, bicarbonates, citrates, borates, acetates, succinates and the like, such as sodium phosphate, citrate, borate, acetate, bicarbonate, carbonate and tromethamine (TRIS). These agents are present in amounts sufficient to maintain the pH of the system at $7.4\pm0.2$ and preferably, 7.4. As such, the buffering agent is as much as 5% on a weight basis of the total formulation in some instances.

In some embodiments, cosolvents are used to enhance drug solubility; however, some drugs are insoluble.

**Mucoadhesive Excipients**

In some embodiments, mucoadhesive characteristics are also imparted to otic formulations disclosed herein, by incorporation of mucoadhesive carbomers, such as Carbopol 934P, to the formulation (Majithiya et al., AAPS PharmSciTech (2006), 7(3), p. E1; EP0551626).

The term 'mucoadhesion' is commonly used for materials that bind to the mucin layer of a biological membrane. To serve as mucoadhesive polymers, the polymers should possess some general physiochemical features such as predominantly anionic hydrophilicity with numerous hydrogen bond forming groups, suitable surface property for wetting mucus/mucosal tissue surfaces and sufficient flexibility to penetrate the mucus network. In some embodiments, mucoadhesive formulations described herein adhere to the round window and/or the oval window and/or any inner ear structure. In some embodiments, the mucoadhesive agent adheres to the round window membrane. In some embodiments, the mucoadhesive agent is a round window membrane mucoadhesive agent.

Mucoadhesive agents including at least one soluble polyvinylpyrrolidone polymer (PVP); a water-swellable, fibrous, cross-linked carboxy-functional polymer; a crosslinked poly(acrylic acid) (e.g. Carbopol 947P); a carbomer homopolymer; a carbomer copolymer; a hydrophilic polysaccharide gum, maltodextrin, a cross-linked alginate gum gel, a water-dispersible polycarboxylated vinyl polymer, at least two particulate components selected from the group consisting of titanium dioxide, silicon dioxide, and clay, or a mixture thereof. In some embodiments, the mucoadhesive agent is used in combination with a viscosity increasing excipient, or are used alone to increase the interaction of the formulation with a

mucosal layer. In one non-limiting example, the mucoadhesive agent is maltodextrin and/or an alginate gum. Those of ordinary skill in the art will recognize that the mucoadhesive character imparted to the formulation should be at a level that is sufficient to deliver an effective amount of the formulation to, for example, the mucosal membrane of the round window in an amount that coats the mucosal membrane, and thereafter deliver the formulation to the affected areas, including by way of example only, the vestibular and/or cochlear structures of the auris interna. Those of ordinary skill in the art are able to determine the mucoadhesive characteristics of the formulations provided herein, and thus determine appropriate ranges. One method for determining sufficient mucoadhesiveness includes monitoring changes in the interaction of the formulation with a mucosal layer, including measuring changes in residence or retention time of the formulation in the absence and presence of the excipient.

Mucoadhesive agents have been described, for example, in U.S. Patent Nos. 6,638,521, 6,562,363, 6,509,028, 6,348,502, 6,319,513, 6,306,789, 5,814,330, and 4,900,552.

In one non-limiting example, the mucoadhesive agent is maltodextrin. Maltodextrin is a carbohydrate produced by the hydrolysis of starch that are derived from corn, potato, wheat or other plant products. Maltodextrin are used either alone or in combination with other mucoadhesive agents to impart mucoadhesive characteristics on the formulations disclosed herein. In one embodiment, a combination of maltodextrin and a carbopol polymer are used to increase the mucoadhesive characteristics of the formulations disclosed herein.

In another non-limiting example, a mucoadhesive agent is, for example, at least two particulate components selected from titanium dioxide, silicon dioxide, and clay, wherein the formulation is not further diluted with any liquid prior to administration and the level of silicon dioxide, if present, is from about 3% to about 15%, by weight of the formulation. Silicon dioxide, if present, are selected from the group consisting of fumed silicon dioxide, precipitated silicon dioxide, coacervated silicon dioxide, gel silicon dioxide, and mixtures thereof. Clay, if present, are kaolin minerals, serpentine minerals, smectites, illite or a mixture thereof. For example, clay is laponite, bentonite, hectorite, saponite, montmorillonites or a mixture thereof.

In one non-limiting example, the mucoadhesive agent is maltodextrin. Maltodextrin is a carbohydrate produced by the hydrolysis of starch that is optionally derived from corn, potato, wheat, or other plant products. Maltodextrin is optionally used either alone or in combination with other mucoadhesive agents to impart mucoadhesive characteristics on the formulations disclosed herein. In one embodiment, a combination of maltodextrin and a carbopol polymer are used to increase the membrane mucoadhesive characteristics of the formulations or devices disclosed herein.

In another embodiment, the membrane mucoadhesive agent is an alkyl-glycoside and/or a saccharide alkyl ester. As used herein, an "alkyl-glycoside" means a compound comprising any hydrophilic saccharide (e.g. sucrose, maltose, or glucose) linked to a hydrophobic alkyl. In some embodiments, the round window membrane mucoadhesive agent is an alkyl-glycoside wherein the alkyl-glycoside comprises a sugar linked to a hydrophobic alkyl (e.g., an alkyl comprising about 6 to about 25 carbon atoms) by an amide linkage, an amine linkage, a carbamate linkage, an ether linkage, a thioether linkage, an ester linkage, a thioester linkage, a glycosidic linkage, a thioglycosidic linkage, and/or a ureide linkage. In some embodiments, the round window membrane mucoadhesive agent is a hexyl-, heptyl-, octyl-, nonyl-, decyl-, undecyl-, dodecyl-, tridecyl- , tetradecyl-, pentadecyl-, hexadecyl-, heptadecyl-, octadecyl α-D-, or octadecyl β-D-maltoside; hexyl-, heptyl-, octyl-, nonyl-, decyl-, undecyl-, dodecyl-, tridecyl- , tetradecyl-, pentadecyl-, hexadecyl-, heptadecyl-, octadecyl α-D-, or octadecyl β-D-glucoside; hexyl-, heptyl-, octyl-, nonyl-, decyl-, undecyl-, dodecyl-, tridecyl- , tetradecyl-, pentadecyl-, hexadecyl-, heptadecyl-, octadecyl α- D-, or octadecyl β-D-sucroside; hexyl-, heptyl-, octyl-, dodecyl-, tridecyl-, or tetradecyl-β-D-thiomaltoside; dodecyl maltoside; heptyl- or octyl-1-thio-α- or β-D-glucopyranoside; alkyl thiosucroses; alkyl maltotriosides; long chain aliphatic carbonic acid amides of sucrose β-amino-alkyl ethers; derivatives of palatinose or isomaltamine linked by an amide linkage to an alkyl chain and derivatives of isomaltamine linked by urea to an alkyl chain; long chain aliphatic carbonic acid ureides of sucrose β-amino- alkyl ethers and long chain aliphatic carbonic acid amides of sucrose β- amino-alkyl ethers, or any combination thereof. In some embodiments, the mucoadhesive agent is an alkyl-glycoside wherein the alkyl glycoside is maltose, sucrose, glucose, or a combination thereof linked by a glycosidic linkage to an alkyl chain of 9-16 carbon atoms (e.g., nonyl-, decyl-, dodecyl- and tetradecyl sucroside; nonyl-, decyl-, dodecyl- and tetradecyl glucoside; and nonyl-, decyl-, dodecyl- and tetradecyl maltoside). In some embodiments, the mucoadhesive agent is an alkyl-glycoside wherein the alkyl glycoside is dodecylmaltoside, tridecylmaltoside, and tetradecylmaltoside.

In some embodiments, the mucoadhesive agent is an alkyl-glycoside wherein the alkyl-glycoside is a disaccharide with at least one glucose. In some embodiments, the auris-acceptable penetration enhancer is a surfactant comprising α-D-glucopyranosyl-β-glycopyranoside, n-Dodecyl-4-O-α- D-glucopyranosyl-β-glycopyranoside, and/or n-tetradecyl-4-O-α- D-glucopyranosyl-β-glycopyranoside. In some embodiments, the mucoadhesive agent is an alkyl-glycoside wherein the alkyl-glycoside has a critical micelle concentration (CMC) of less than about 1mM in pure water or in aqueous solutions. In some embodiments, the mucoadhesive agent is an alkyl-glycoside wherein an oxygen atom within the alkyl-glycoside is substituted with a sulfur atom. In some embodiments, the mucoadhesive agent is an alkyl-glycoside wherein the alkylglycoside is the β anomer. In some embodiments, the mucoadhesive agent is an alkyl-glycoside wherein the alkylglycoside comprises 90%, 91%, 92%, 93%, 94%, 95%, 96%, 97%, 98%, 99%, 99.1%, 99.5%, or 99.9% of the β

anomer.

**Stabilizers (which are not specifically claimed but may be used in conjunction with the claimed formulations)**

In one embodiment, stabilizers are selected from, for example, fatty acids, fatty alcohols, alcohols, long chain fatty acid esters, long chain ethers, hydrophilic derivatives of fatty acids, polyvinyl pyrrolidones, polyvinyl ethers, polyvinyl alcohols, hydrocarbons, hydrophobic polymers, moisture-absorbing polymers, and combinations thereof. In some embodiments, amide analogues of stabilizers are also used. In a further embodiment, the chosen stabilizer changes the hydrophobicity of the formulation (e.g., oleic acid, waxes), or improves the mixing of various components in the formulation(e.g., ethanol), controls the moisture level in the formula (e.g., PVP or polyvinyl pyrrolidone), controls the mobility of the phase (substances with melting points higher than room temperature such as long chain fatty acids, alcohols, esters, ethers, amides etc. or mixtures thereof; waxes), and/or improves the compatibility of the formula with encapsulating materials (e.g., oleic acid or wax). In another embodiment some of these stabilizers are used as solvents/co-solvents (e.g., ethanol). In a further embodiment, stabilizers are present in sufficient amount to inhibit the degradation of the active pharmaceutical ingredient. Examples of such stabilizing agents, include : (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc; or (n) combinations thereof.

In some embodiments, the stabilizer is silicon dioxide. In some embodiments, the silicon dioxide is stabilizer in suspension formulations. In some embodiments, the silicon dioxide is an anticaking agent (i.e. an agent that prevents the formation of lumps). In some embodiments, the silicon dioxide is an anticaking agent (i.e. an agent that prevents the formation of lumps) that stabilizes suspension formulations. In some embodiments, the stabilizer is an anticaking agent.

In some embodiments, the stabilizer is a carbomer. In some embodiments, the carbomer is a complexing agent for positively charged proteins. In some embodiments, the positively charged protein in the complex has reduced solubility and therefore is released slowly from the formulation.

In some embodiments, the stabilizer is a complexing agent. In some embodiments, stabilizer interacts with the therapeutic agent to form a complex. In some embodiments, the stabilizer is a protein complexing agent. In some embodiments, the protein complexing agent is a polymer with a charge that is opposite to charge of the protein therapeutic agent. In some embodiments, the polymer is carbomer or alginate. In some embodiments, the stabilizer forms a complex with the protein therapeutic agent that reduces the solubility of the protein therapeutic agent. In some embodiments, the stabilizer forms a complex with the protein therapeutic agent that provides for the slow release of the protein therapeutic agent. In some embodiments, the stabilizer forms a complex with the protein therapeutic agent that provides for the sustained release of the protein therapeutic agent.

In some embodiments, the stabilizer is a neutral polymer. Examples of a neutral polymer include povidone, poloxamer, and HMPC. In some embodiments, the neutral polymer forms a polymer matrix that encapsulates the therapeutic agent and provides for the slow release of the therapeutic agent. In some embodiments, the neutral polymer forms a polymer matrix that encapsulates the therapeutic agent and provides for the sustained release of the therapeutic agent.

Additional useful auris-acceptable formulations include one or more anti-aggregation additives to enhance stability of otic formulations by reducing the rate of protein aggregation. The anti-aggregation additive selected depends upon the nature of the conditions to which the therapeutic agents, or otic agents, for example anti-TNF antibodies are exposed. For example, certain formulations undergoing agitation and thermal stress require a different anti-aggregation additive than a formulation undergoing lyophilization and reconstitution. Useful anti-aggregation additives include, by way of example only, urea, guanidinium chloride, simple amino acids such as glycine or arginine, sugars, polyalcohols, polysorbates, polymers such as polyethylene glycol and dextrans, alkyl saccharides, such as alkyl glycoside, and surfactants.

Other useful formulations include one or more antioxidants to enhance chemical stability where required. Suitable antioxidants include, by way of example only, ascorbic acid and sodium metabisulfite. In one embodiment, antioxidants are selected from metal chelating agents, thiol containing compounds and other general stabilizing agents.

Still other useful formulations include one or more surfactants to enhance physical stability or for other purposes. Suitable nonionic surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.

In some embodiments, the pharmaceutical formulations described herein are stable with respect to compound degradation over a period of any of at least about 1 day, at least about 2 days, at least about 3 days, at least about 4 days, at least about 5 days, at least about 6 days, at least about 1 week, at least about 2 weeks, at least about 3 weeks, at least about 4 weeks, at least about 5 weeks, at least about 6 weeks, at least about 7 weeks, at least about 8 weeks, at least about 1 month, at least about 2 months, at least about 3 months, at least about 4 months, at least about 5 months, or at least about 6

months. In other embodiments, the formulations described herein are stable with respect to compound degradation over a period of at least about 1 week. Also described herein are formulations that are stable with respect to compound degradation over a period of at least about 1 month.

In other embodiments, an additional surfactant (co-surfactant) and/or buffering agent is combined with one or more of the pharmaceutically acceptable vehicles previously described herein so that the surfactant and/or buffering agent maintains the product at an optimal pH for stability. Suitable co-surfactants include : a) natural and synthetic lipophilic agents, e.g., phospholipids, cholesterol, and cholesterol fatty acid esters and derivatives thereof; b) nonionic surfactants, which include for example, polyoxyethylene fatty alcohol esters, sorbitan fatty acid esters (Spans), polyoxyethylene sorbitan fatty acid esters (e.g., polyoxyethylene (20) sorbitan monooleate (Tween 80), polyoxyethylene (20) sorbitan monostearate (Tween 60), polyoxyethylene (20) sorbitan monolaurate (Tween 20) and other Tweens, sorbitan esters, glycerol esters, e.g., Myrj and glycerol triacetate (triacetin), polyethylene glycols, cetyl alcohol, cetostearyl alcohol, stearyl alcohol, polysorbate 80, poloxamers, poloxamines, polyoxyethylene castor oil derivatives (e.g., Cremophor® RH40, Cremophor A25, Cremophor A20, Cremophor® EL) and other Cremophors, sulfosuccinates, alkyl sulfates (SLS); PEG glyceryl fatty acid esters such as PEG-8 glyceryl caprylate/caprate (Labrasol), PEG-4 glyceryl caprylate/caprate (Labrafac Hydro WL 1219), PEG-32 glyceryl laurate (Gelucire 444/14), PEG-6 glyceryl mono oleate (Labrafil M 1944 CS), PEG-6 glyceryl linoleate (Labrafil M 2125 CS); propylene glycol mono- and di-fatty acid esters, such as propylene glycol laurate, propylene glycol caprylate/caprate; Brij® 700, ascorbyl-6-palmitate, stearylamine, sodium lauryl sulfate, polyoxethyleneglycerol triiricinoleate, and any combinations or mixtures thereof; c) anionic surfactants include calcium carboxymethylcellulose, sodium carboxymethylcellulose, sodium sulfosuccinate, dioctyl, sodium alginate, alkyl polyoxyethylene sulfates, sodium lauryl sulfate, triethanolamine stearate, potassium laurate, bile salts, and any combinations or mixtures thereof; and d) cationic surfactants such as quaternary ammonium compounds, benzalkonium chloride, cetyltrimethylammonium bromide, and lauryldimethylbenzyl-ammonium chloride.

In a further embodiment, when one or more co-surfactants are utilized in the formulations of the present disclosure, they are combined, e.g., with a pharmaceutically acceptable vehicle and is present in the final formulation, e.g., in an amount ranging from about 0.1% to about 20%, from about 0.5% to about 10%. In one embodiment, the surfactant has an HLB value of 0 to 20. In additional embodiments, the surfactant has an HLB value of 0 to 3, of 4 to 6, of 7 to 9, of 8 to 18, of 13 to 15, of 10 to 18.

**Preservatives (which are not specifically claimed but may be used in conjunction with the claimed formulations)**

In some embodiments, the otic formulations described herein is free of preservatives. In some embodiments, a formulation disclosed herein comprises a preservative. Suitable auris-acceptable preservatives for use in a formulation disclosed herein include benzoic acid, boric acid, p-hydroxybenzoates, benzyl alcohol, lower alkyl alcohols (e.g., ethanol, butanol or the like), quaternary compounds, stabilized chlorine dioxide, mercurials, such as merfen and thimerosal, mixtures of the foregoing and the like. Suitable preservatives for use with a formulation disclosed herein are not ototoxic. In some embodiments, a formulation disclosed herein does not include a preservative that is ototoxic. In some embodiments, a formulation disclosed herein does not include benzalkonium chloride or benzethonium chloride.

In certain embodiments, any otic formulation described herein has an endotoxin level of less than 0.5 EU/kg, less than 0.4 EU/kg or less than 0.3 EU/kg. In certain embodiments, any otic formulation described herein has less than about 60 colony forming units (CFU), has less than about 50 colony forming units, has less than about 40 colony forming units, has less than about 30 colony forming units of microbial agents per gram of formulation . In certain embodiments, any controlled release formulation described herein is substantially free of pyrogens.

In a further embodiment, the preservative is, by way of example only, an antimicrobial agent, within the formulation presented herein. In one embodiment, the formulation includes a preservative such as by way of example only, methyl paraben. In another embodiment, the methyl paraben is at a concentration of about 0.05% to about 1.0%, about 0.1% to about 0.2%. In certain embodiments, the preservative employed in any auris-compatible formulation described herein is an antioxidant (e.g., butyl hydroxytoluene (BHT) or the like, as described herein). In certain embodiments, an antioxidant preservative is nontoxic and/or non-irritating to the inner ear environment.

**Carriers (which are not specifically claimed but may be used in conjunction with the claimed formulations)**

Suitable carriers for use in a formulation described herein include any pharmaceutically acceptable solvent. For example, suitable solvents include polyalkylene glycols such as polyethylene glycol (PEG) and any combinations or mixtures thereof. In other embodiments, the base is a combination of a pharmaceutically acceptable surfactant and solvent.

In some embodiments, other excipients include, sodium stearyl fumarate, diethanolamine cetyl sulfate, isostearate, polyethoxylated castor oil, benzalkonium chloride, nonoxyl 10, octoxynol 9, sodium lauryl sulfate, sorbitan esters

(sorbitan monolaurate, sorbitan monooleate, sorbitan monopalmitate, sorbitan monostearate, sorbitan sesquioleate, sorbitan trioleate, sorbitan tristearate, sorbitan laurate, sorbitan oleate, sorbitan palmitate, sorbitan stearate, sorbitan dioleate, sorbitan sesqui-isostearate, sorbitan sesquistearate, sorbitan tri-isostearate), lecithins, phospholipids, phosphatidyl cholines (c8-c18), phosphatidylethanolamines (c8-c18), phosphatidylglycerols (c8-c18), pharmaceutical acceptable salts thereof and combinations or mixtures thereof.

In further embodiments, the carrier is polyethylene glycol. Polyethylene glycol is available in many different grades having varying molecular weights. For example, polyethylene glycol is available as PEG 200; PEG 300; PEG 400; PEG 540 (blend); PEG 600; PEG 900; PEG 1000; PEG 1450; PEG 1540; PEG 2000; PEG 3000; PEG 3350; PEG 4000; PEG 4600, and PEG 8000. For purposes of the present disclosure, all grades of polyethylene glycol are contemplated for use in preparation of a formulation described herein. In some embodiments the polyethylene glycol used to prepare a formulation described herein is PEG 300.

In other embodiments, the carrier is a polysorbate. Polysorbates are nonionic surfactants of sorbitan esters. Polysorbates useful in the present disclosure include polysorbate 20, polysorbate 40, polysorbate 60, polysorbate 80 (Tween 80) and any combinations or mixtures thereof. In further embodiments, polysorbate 80 is utilized as the pharmaceutically acceptable carrier.

In one embodiment, water-soluble glycerin-based auris-acceptable enhanced viscosity formulations utilized in the preparation of pharmaceutical delivery vehicles comprise at least one active agent containing at least about 0.1% of the water-soluble glycerin compound or more. In some embodiments, the percentage of active agent is varied between about 1% and about 95%, between about 5% and about 80%, between about 10% and about 60% or more of the weight or volume of the total pharmaceutical formulation. In some embodiments, the amount of the compound(s) in each therapeutically useful formulation is prepared in such a way that a suitable dosage will be obtained in any given unit dose of the compound. Factors such as solubility, bioavailability, biological half-life, route of administration, product shelf life, as well as other pharmacological considerations are contemplated herein.

If desired, the auris-acceptable pharmaceutical gels also contain co-solvents, preservatives, cosolvents, ionic strength and osmolality adjustors and other excipients in addition to buffering agents. Suitable auris-acceptable water soluble buffering agents are alkali or alkaline earth metal carbonates, phosphates, bicarbonates, citrates, borates, acetates, succinates and the like, such as sodium phosphate, citrate, borate, acetate, bicarbonate, carbonate, and tromethamine (TRIS). These agents are present in amounts sufficient to maintain the pH of the system at $7.4\pm0.2$ and preferably, 7.4. As such, the buffering agent is as much as 5% on a weight basis of the total formulation.

Cosolvents are used to enhance the active agent solubility, however, some active agents are insoluble. These are often suspended in the polymer vehicle with the aid of suitable suspending or viscosity enhancing agents.

Moreover, some pharmaceutical excipients, diluents or carriers are potentially ototoxic. For example, benzalkonium chloride, a common preservative, is ototoxic and therefore potentially harmful if introduced into the vestibular or cochlear structures. In formulating a controlled-release formulation, it is advised to avoid or combine the appropriate excipients, diluents or carriers to lessen or eliminate potential ototoxic components from the formulation, or to decrease the amount of such excipients, diluents, or carriers. Optionally, a controlled-release formulation includes otoprotective agents, such as antioxidants, alpha lipoic acid, calcium, fosfomycin or iron chelators, to counteract potential ototoxic effects that may arise from the use of specific therapeutic agents or excipients, diluents, or carriers.

In some embodiments, therapeutically acceptable otic formulations are:

| Example Formulation | Example Characteristics |
|---|---|
| Chitosan glycerophosphate (CGP) | • tunable degradation of matrix in vitro<br>• tunable TACE inhibitor release in vitro: e.g., ~50 % of drug released after 24 hrs<br>• biodegradable<br>• compatible with drug delivery to the inner ear<br>• suitable for macromolecules and hydrophobic drugs |
| PEG-PLGA-PEG triblock polymers | • tunable high stability: e.g., maintains mechanical integrity > 1 month in vitro<br>• tunable fast release of hydrophilic drugs: e.g., ~ 50 % of drug released after 24 hrs, and remainder released over ~ 5 days<br>• tunable slow release of hydrophobic drugs: e.g., ~ 80 % released after 8 weeks<br>• biodegradable<br>• subcutaneous injection of solution: e.g., gel forms within seconds and is intact after 1 month |

(continued)

| Example Formulation | Example Characteristics |
|---|---|
| PEO-PPO-PEO triblock copolymers (e.g., | • Tunable sol-gel transition temperature: e.g., decreases with increasing F127 concentration |
| Pluronic or Poloxamers) (e.g., F127) | |
| Chitosan glycerophosphate with drug-loaded liposomes | • CGP formulation tolerates liposomes: e.g., up to 15 μM/ml liposomes.<br>• liposomes tunably reduce drug release time (e.g., up to 2 weeks in vitro).<br>• increase in liposome diameter optionally reduces drug release kinetics (e.g., liposome size between 100 and 300 nm)<br>• release parameters are controlled by changing formulation of liposomes |

The formulations disclosed herein alternatively encompass an otoprotectant agent in addition to the at least one active agent and/or excipients, including such agents as antioxidants, alpha lipoic acid, calcium, fosfomycin or iron chelators, to counteract potential ototoxic effects that may arise from the use of specific therapeutic agents or excipients, diluents or carriers.

In some embodiments, the percentage of active pharmaceutical ingredient is varied between about 0.01% and about 20%, between about 0.01% and about 10%, between about 0.01% and about 5% or more of the weight or volume of the total pharmaceutical formulation.

In some embodiments, the amount of the compound(s) in each therapeutically useful formulation is prepared in such a way that a suitable dosage will be obtained in any given unit dose of the compound. Factors such as solubility, bioavailability, biological half-life, route of administration, product shelf life, as well as other pharmacological considerations are contemplated herein and the preparation of such pharmaceutical formulations is presented herein.

**Suspending Agents (which are not specifically claimed but may be used in conjunction with the claimed formulations)**

In one embodiment is an active pharmaceutical ingredient in a pharmaceutically acceptable formulation wherein the formulation comprises at least one suspending agent. In one embodiment is an active pharmaceutical ingredient in a pharmaceutically acceptable gel formulation wherein the formulation comprises at least one suspending agent.

In one embodiment, at least one therapeutic agent is included in a pharmaceutically acceptable enhanced viscosity formulation wherein the formulation further comprises at least one suspending agent, wherein the suspending agent assists in imparting controlled release characteristics to the formulation . In some embodiments, suspending agents also serve to increase the viscosity of the auris-acceptable therapeutic agent formulations .

Suspending agents include by example only, compounds such as polyvinylpyrrolidone, e.g., polyvinylpyrrolidone K12, polyvinylpyrrolidone K17, polyvinylpyrrolidone K25, or polyvinylpyrrolidone K30, vinyl pyrrolidone/vinyl acetate copolymer (S630), polyethylene glycol, e.g., the polyethylene glycol has a molecular weight of about 300 to about 6000, or about 3350 to about 4000, or about 7000 to about 5400, sodium carboxymethylcellulose, methylcellulose, hydroxypropylmethylcellulose, hydroxymethylcellulose acetate stearate, polysorbate-80, hydroxyethylcellulose, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated sorbitan monolaurate, povidone and the like. In some embodiments, useful aqueous suspensions also contain one or more polymers as suspending agents. Useful polymers include water-soluble polymers such as cellulosic polymers, e.g., hydroxypropyl methylcellulose, and water-insoluble polymers such as cross-linked carboxyl-containing polymers. Useful polymers also include hyaluronic acid.

In one embodiment, the present disclosure provides auris-acceptable gel formulations comprising a therapeutically effective amount of an active agent in a hydroxyethyl cellulose gel. Hydroxyethyl cellulose (HEC) is obtained as a dry powder which is reconstituted in water or an aqueous buffer solution to give the desired viscosity (generally about 200 cps to about 30,000 cps, corresponding to about 0.2% to about 10% HEC). In one embodiment the concentration of HEC is between about 1% and about 15%, about 1% and about 2%, or about 1.5% to about 2%.

In some embodiments, the formulations include excipients, other medicinal or pharmaceutical agents, carriers, adjuvants, such as preserving, stabilizing, wetting or emulsifying agents, solution promoters, and salts. In some embodiments, the excipients, carriers, adjuvants, are useful in forming a pharmaceutically acceptable formulation . In

some embodiments, the formulation comprises a stabilizer. In another embodiment the formulation comprises a solubilizer. In a further embodiment the formulation comprises an antifoaming agent. In yet a further embodiment, the formulation comprises an antioxidant. In yet another embodiment, the formulation comprises a dispersing agent. In one embodiment, the formulation comprises a surfactant. In yet another embodiment, the formulation comprises a wetting agent.

**Viscosity Enhancing Agents**

In one embodiment is a formulation that is free or substantially free of a viscosity enhancing agent. In one embodiment is a formulation comprising at least one active pharmaceutical ingredient and a viscosity agent. Also described herein are controlled release formulations that is free or substantially free of a viscosity enhancing agent. Also described herein are controlled release formulations comprising a therapeutic agent and a viscosity enhancing agent. In some embodiments, suitable viscosity-enhancing agents do not include poloxamers. Suitable viscosity-enhancing agents include by way of example only, thickening agents and suspending agents. In one embodiment, the enhanced viscosity formulation does not include a pharmaceutically acceptable buffer. In other embodiments, the enhanced viscosity formulation includes a pharmaceutically acceptable buffer. Sodium chloride or other tonicity agents are optionally used to adjust tonicity, if necessary.

Described herein are formulations comprising an active pharmaceutical ingredient and a thickening agent. Suitable thickening agents include by way of example only, suspending agents. In one embodiment, the thickened formulation does not include a pharmaceutically acceptable buffer. In another embodiment, the thickened formulation includes a pharmaceutically acceptable buffer.

By way of example only, the auris-acceptable viscosity agents include hydroxypropyl methylcellulose, hydroxyethyl cellulose, polyvinylpyrrolidone (PVP: povidone), carboxymethyl cellulose, polyvinyl alcohol, sodium chondroitin sulfate, sodium hyaluronate. Other viscosity agents that are used in pharmaceutical formulations described herein include acacia (gum arabic), agar, aluminum magnesium silicate, sodium alginate, sodium stearate, bladderwrack, bentonite, carbomer, carrageenan, Carbopol, xanthan, cellulose, microcrystalline cellulose (MCC), ceratonia, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, polyethylene glycol (e.g. PEG 200-4500), gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethylcellulose (HPMC), sodium carboxymethyl-cellulose (CMC), silicon dioxide, Splenda® (dextrose, maltodextrin and sucralose) or combinations thereof. In specific embodiments, the viscosity-enhancing excipient is a combination of methylcellulose (MC) and CMC. In another embodiment, the viscosity-enhancing agent is a combination of carboxymethylated chitosan, or chitin, and alginate. The combination of chitin and alginate with the active agents disclosed herein acts as a controlled release formulation, restricting the diffusion of the active agents from the formulation. Moreover, the combination of carboxymethylated chitosan and alginate is optionally used to assist in increasing the permeability of any active agent described herein through the round window membrane.

In further embodiments, the auris formulation contains a viscosity enhancing agent or viscosity modulating agent sufficient to provide a viscosity of between about 10 and 1,000,000 centipoise, between about 100 and 1,000,000 centipoise, between about 500 and 1,000,000 centipoise, between about 750 and 1,000,000 centipoise; between about 1000 and 40,000 centipoise; between about 2000 and 35,000 centipoise; between about 3000 and 30,000 centipoise; between about 4000 and 25,000 centipoise; between about 5000 and 20,000 centipoise; or between about 6000 and 15,000 centipoise.

In further embodiments, the auris formulation contains a viscosity enhancing agent or viscosity modulating agent sufficient to provide a viscosity of about 2 cP to about 250,000 cP, about 2 cP to about 100,000 cP, about 2 cP to about 50,000 cP, about 2 cP to about 25,000 cP, about 2 cP to about 10,000 cP, about 2 cP to about 5,000 cP, about 2 cP to about 1,000 cP, about 2 cP to about 500 cP, about 2 cP to about 250 cP, about 2 cP to about 100 cP, about 2 cP to about 90 cP, about 2 cP to about 80 cP, about 2 cP to about 70 cP, about 2 cP to about 60 cP, about 2 cP to about 50 cP, about 2 cP to about 40 cP, about 2 cP to about 30 cP, about 2 cP to about 20 cP, or about 2 cP to about 10 cP. In some embodiments, the formulation has a viscosity of about 2 cP, about 5 cP, about 10 cP, about 20 cP, about 30 cP, about 40 cP, about 50 cP, about 60 cP, about 70 cP, about 80 cP, about 90 cP, about 100 cP, about 200 cP, about 300 cP, about 400 cP, about 500 cP, about 600 cP, about 700 cP, about 800 cP, about 900 cP, about 1,000 cP, about 5,000 cP, about 10,000 cP, about 20,000 cP, about 50,000 cP, about 100,000 cP, or about 250,000 cP.

In some embodiments, the viscosity of the otic formulations presented herein are measured by any means described herein. For example, in some embodiments, an LVDV-II+CP Cone Plate Viscometer and a Cone Spindle CPE-40 is used to calculate the viscosity of the formulation described herein. In other embodiments, a Brookfield (spindle

and cup) viscometer is used to calculate the viscosity of the formulation described herein. In some embodiments, the viscosity ranges referred to herein are measured at room temperature. In other embodiments, the viscosity ranges referred to herein are measured at body temperature.

**Auris-Acceptable Penetration Enhancers (which are not specifically claimed but may be used in conjunction with the claimed formulations)**

In another embodiment the formulation further comprises one or more penetration enhancers. Penetration into biological membranes is enhanced by the presence of penetration enhancers. Penetration enhancers are chemical entities that facilitate transport of coadministered substances across biological membranes. Penetration enhancers are grouped according to chemical structure. Surfactants, both ionic and non-ionic, such as sodium lauryl sulfate, sodium laurate, polyoxyethylene-20-cetyl ether, laureth-9, sodium dodecylsulfate, dioctyl sodium sulfosuccinate, polyoxyethylene-9-lauryl ether (PLE), Tween 80, nonylphenoxypolyethylene (NP-POE), polysorbates and the like, function as penetration enhancers. Bile salts (such as sodium glycocholate, sodium deoxycholate, sodium taurocholate, sodium taurodihydrofusidate, sodium glycodihydrofusidate and the like), fatty acids and derivatives (such as oleic acid, caprylic acid, mono- and di-glycerides, lauric acids, acylcholines, caprylic acids, acylcarnitines, sodium caprates and the like), chelating agents (such as EDTA, citric acid, salicylates and the like), sulfoxides (such as dimethyl sulfoxide (DMSO), decylmethyl sulfoxide and the like), and alcohols (such as ethanol, isopropanol, propylene glycol, polyethylene glycol, glycerol, propanediol and the like) also function as penetration enhancers. In addition, the peptide-like penetration enhancers described in U.S. Patent Nos. 7,151,191, 6,221,367 and 5,714,167 are contemplated as an additional embodiment. These penetration enhancers are amino-acid and peptide derivatives and enable drug absorption by passive transcellular diffusion without affecting the integrity of membranes or intercellular tight junctions. In some embodiments, a penetration enhancer is hyaluronic acid.

In some embodiments, the auris-acceptable penetration enhancer is a surfactant. In some embodiments, the auris-acceptable penetration enhancer is a surfactant comprising an alkyl-glycoside and/or a saccharide alkyl ester. As used herein, an "alkyl-glycoside" means a compound comprising any hydrophilic saccharide (e.g. glucose, fructose, sucrose, maltose, or glucose) linked to a hydrophobic alkyl. In some embodiments, the auris-acceptable penetration enhancer is a surfactant comprising an alkyl-glycoside wherein the alkyl-glycoside comprises a sugar linked to a hydrophobic alkyl (e.g., an alkyl comprising about 6 to about 25 carbon atoms) by an amide linkage, an amine linkage, a carbamate linkage, an ether linkage, a thioether linkage, an ester linkage, a thioester linkage, a glycosidic linkage, a thioglycosidic linkage, and/or a ureide linkage. In some embodiments, the auris-acceptable penetration enhancer is a surfactant comprising hexyl-, heptyl-, octyl-, nonyl-, decyl-, undecyl-, dodecyl-, tridecyl- , tetradecyl, pentadecyl-, hexadecyl-, heptadecyl-, and octadecyl α- or β-D-maltoside; hexyl-, heptyl-, octyl-, nonyl-, decyl-, undecyl-, dodecyl-, tridecyl- , tetradecyl, pentadecyl-, hexadecyl-, heptadecyl-, and octadecyl α- or β-D-glucoside; hexyl-, heptyl-, octyl-, nonyl-, decyl-, undecyl-, dodecyl-, tridecyl- , tetradecyl, pentadecyl-, hexadecyl-, heptadecyl-, and octadecyl α- or β-D-sucroside; hexyl-, heptyl-, octyl-, dodecyl-, tridecyl-, and tetradecyl-β-D-thiomaltoside; heptyl- or octyl-1-thio-α- or β-D-glucopyranoside; alkyl thiosucroses; alkyl maltotriosides; long chain aliphatic carbonic acid amides of sucrose β-amino-alkyl ethers; derivatives of palatinose or isomaltamine linked by an amide linkage to an alkyl chain and derivatives of isomaltamine linked by urea to an alkyl chain; long chain aliphatic carbonic acid ureides of sucrose β-amino- alkyl ethers and long chain aliphatic carbonic acid amides of sucrose β- amino-alkyl ethers. In some embodiments, the auris-acceptable penetration enhancer is a surfactant comprising an alkyl-glycoside wherein the alkyl glycoside is maltose, sucrose, glucose, or a combination thereof linked by a glycosidic linkage to an alkyl chain of 9-16 carbon atoms (e.g., nonyl-, decyl-, dodecyl- and tetradecyl sucroside; nonyl-, decyl-, dodecyl- and tetradecyl glucoside; and nonyl-, decyl-, dodecyl- and tetradecyl maltoside). In some embodiments, the auris-acceptable penetration enhancer is a surfactant comprising an alkyl-glycoside wherein the alkyl glycoside is dodecylmaltoside, tridecylmaltoside, and tetradecylmaltoside. In some embodiments, the auris-acceptable penetration enhancer is a surfactant comprising an alkyl-glycoside wherein the alkyl glycoside is tetradecyl- β-D-maltoside. In some embodiments, the auris-acceptable penetration enhancer is a surfactant comprising an alkyl-glycoside wherein the alkyl-glycoside is a disaccharide with at least one glucose. n some embodiments, the auris-acceptable penetration enhancer is a surfactant comprising α-D-glucopyranosyl-β-glycopyranoside, n-Dodecyl-4-O-α- D-glucopyranosyl-β-glycopyranoside, and/or n-tetradecyl-4-O-α- D-glucopyranosyl-β-glycopyranoside. In some embodiments, the auris-acceptable penetration enhancer is a surfactant comprising an alkyl-glycoside wherein the alkyl-glycoside has a critical micelle concentration (CMC) of less than about 1mM in pure water or in aqueous solutions. In some embodiments, the auris-acceptable penetration enhancer is a surfactant comprising an alkyl-glycoside wherein an oxygen atom within the alkyl-glycoside is substituted with a sulfur atom. In some embodiments, the auris-acceptable penetration enhancer is a surfactant comprising an alkyl-glycoside wherein the alkylglycoside is the β anomer. In some embodiments, the auris-acceptable penetration enhancer is a surfactant comprising an alkyl-glycoside wherein the alkylglycoside comprises 90%, 91%, 92%, 93%, 94%, 95%, 96%, 97%, 98%, 99%, 99.1%, 99.5%, or 99.9% of the β anomer.

In certain instances, the penetration enhancing agent is a hyaluronidase. In certain instances, a hyaluronidase is a human or bovine hyaluronidase. In some instances, a hyaluronidase is a human hyaluronidase (e.g., hyaluronidase found in human sperm, PH20 (Halozyme), Hyelenex® (Baxter International, Inc.)). In some instances, a hyaluronidase is a bovine hyaluronidase (e.g., bovine testicular hyaluronidase, Amphadase® (Amphastar Pharmaceuticals), Hydase® (PrimaPharm, Inc). In some instances, a hyaluronidase is an ovine hyaluronidase, Vitrase® (ISTA Pharmaceuticals). In certain instances, a hyaluronidase described herein is a recombinant hyaluronidase. In some instances, a hyaluronidase described herein is a humanized recombinant hyaluronidase. In some instances, a hyaluronidase described herein is a PEGylated hyaluronidase (e.g., PEGPH20 (Halozyme)).

## Foams and Paints

In some embodiments, the auris therapeutic agents disclosed herein are dispensed as an auris-acceptable paint. As used herein, paints (also known as film formers) are solutions comprised of a solvent, a monomer or polymer, an active agent, and optionally one or more pharmaceutically-acceptable excipients. After application to a tissue, the solvent evaporates leaving behind a thin coating comprised of the monomers or polymers, and the active agent. The coating protects active agents and maintains them in an immobilized state at the site of application. This decreases the amount of active agent which are lost and correspondingly increases the amount delivered to the subject. By way of non-limiting example, paints include collodions (e.g. Flexible Collodion, USP), and solutions comprising saccharide siloxane copolymers and a cross-linking agent. Collodions are ethyl ether/ethanol solutions containing pyroxylin (a nitrocellulose). After application, the ethyl ether/ethanol solution evaporates leaving behind a thin film of pyroxylin. In solutions comprising saccharide siloxane copolymers, the saccharide siloxane copolymers form the coating after evaporation of the solvent initiates the cross-linking of the saccharide siloxane copolymers. For additional disclosures regarding paints, see *Remington: The Science and Practice of Pharmacy* which is hereby incorporated in its entirety. The paints contemplated for use herein, are flexible such that they do not interfere with the propagation of pressure waves through the ear. Further, the paints are applied as a liquid (i.e. solution, suspension, or emulsion), a semisolid (i.e. a gel, foam, paste, or jelly) or an aerosol.

In some embodiments, the auris therapeutic agents disclosed herein are dispensed as a controlled-release foam. Examples of suitable foamable carriers for use in the formulations disclosed herein include alginate and derivatives thereof, carboxymethylcellulose and derivatives thereof, collagen, polysaccharides, including, for example, dextran, dextran derivatives, pectin, starch, modified starches such as starches having additional carboxyl and/or carboxamide groups and/or having hydrophilic side-chains, cellulose and derivatives thereof, agar and derivatives thereof, such as agar stabilized with polyacrylamide, polyethylene oxides, glycol methacrylates, gelatin, gums such as xanthum, guar, karaya, gellan, arabic, tragacanth and locust bean gum, or combinations thereof. Also suitable are the salts of the aforementioned carriers, for example, sodium alginate. The formulation optionally further comprises a foaming agent, which promotes the formation of the foam, including a surfactant or external propellant. Examples of suitable foaming agents include cetrimide, lecithin, soaps, silicones and the like. Commercially available surfactants such as Tween® are also suitable.

In some embodiments, other gel formulations are useful depending upon the particular active agent, other pharmaceutical agent, or excipients/additives used. For example, other commercially-available glycerin-based gels, glycerin-derived compounds, conjugated or crosslinked gels, matrices, hydrogels, and polymers, as well as gelatins and their derivatives, alginates, alginate-based gels, and even various native and synthetic hydrogel and hydrogel-derived compounds are all expected to be useful in the otic formulations described herein. In some embodiments, auris-acceptable gels include alginate hydrogels SAF®-Gel (ConvaTec, Princeton, N.J.), Duoderm® Hydroactive Gel (ConvaTec), Nu-gel ®(Johnson & Johnson Medical, Arlington, Tex.), Carrasyn®(V) Acemannan Hydrogel (Carrington Laboratories, Inc., Irving, Tex.) glycerin gels Elta® Hydrogel (Swiss-American Products, Inc., Dallas, Tex.), and K-Y® Sterile (Johnson & Johnson). In further embodiments, biodegradable biocompatible gels also represent compounds present in auris-acceptable formulations disclosed and described herein.

In some formulations developed for administration to a mammal, and for formulations formulated for human administration, the auris-acceptable gel comprises substantially all of the weight of the formulation. In other embodiments, the auris-acceptable gel comprises as much as about 98% or about 99% of the formulation by weight. This is desirable when a substantially non-fluid or substantially viscous formulation is needed. In a further embodiment, when slightly less viscous, or slightly more fluid auris-acceptable pharmaceutical gel formulations are desired, the biocompatible gel portion of the formulation comprises at least about 50% by weight, at least about 60% by weight, at least about 70% by weight, or even at least about 80% or 90% by weight of the compound. All intermediate integers within these ranges are contemplated to fall within the scope of this disclosure, and in some alternative embodiments, even more fluid (and consequently less viscous) auris-acceptable gel formulations are formulated, such as for example, those in which the gel or matrix component of the mixture comprises not more than about 50% by weight, not more than about 40% by weight, not more than about 30% by weight, or even those than comprise not more than about 15% or about 20% by weight of the formulation.

**Auris-Acceptable Actinic Radiation Curable Gel**

In other embodiments, the gel is an actinic radiation curable gel, such that following administration to or near the targeted auris structure, use of actinic radiation (or light, including UV light, visible light, or infrared light) the desired gel properties are formed. By way of example only, fiber optics are used to provide the actinic radiation so as to form the desired gel properties. In some embodiments, the fiber optics and the gel administration device form a single unit. In other embodiments, the fiber optics and the gel administration device are provided separately.

**Auris-Acceptable Solvent Release Gel**

In some embodiments, the gel is a solvent release gel such that the desired gel properties are formed after administration to or near the targeted auris structure, that is, as the solvent in the injected gel formulation diffuses out the gel, a gel having the desired gel properties is formed. For example, a formulation that comprises sucrose acetate isobutyrate, a pharmaceutically acceptable solvent, one or more additives, and the active agent is administered at or near the round window membrane; diffusion of the solvent out of the injected formulation provides a depot having the desired gel properties. For example, use of a water soluble solvent provides a high viscosity depot when the solvent diffuses rapidly out of the injected formulation. On the other hand, use of a hydrophobic solvent (e.g., benzyl benzoate) provides a less viscous depot. One example of an auris-acceptable solvent release gel formulation is the SABER™ Delivery System marketed by DURECT Corporation.

**Auris-Acceptable Spongy Material**

Also contemplated within the scope of the embodiments is the use of a spongy material in the auris interna or auris media. In some embodiments, the spongy material is formed from hyaluronic acid or its derivatives. The spongy material is impregnated with a desired auris therapeutic agent and placed within the auris media so as to provide controlled release of the auris therapeutic agent within the auris media, or in contact with the round window membrane so as to provide controlled release of the auris therapeutic agent into the auris interna. In some embodiments, the spongy material is biodegradable.

**Cyclodextrin formulations (which are not specifically claimed but may be used in conjunction with the claimed formulations)**

In a specific embodiment, the formulation alternatively comprises a cyclodextrin. Cyclodextrins are cyclic oligosaccharides containing 6, 7, or 8 glucopyranose units, referred to as α-cyclodextrin, β-cyclodextrin, or γ-cyclodextrin respectively. Cyclodextrins have been found to be particularly useful in pharmaceutical formulations . Cyclodextrins have a hydrophilic exterior, which enhances water-soluble, and a hydrophobic interior which forms a cavity. In an aqueous environment, hydrophobic portions of other molecules often enter the hydrophobic cavity of cyclodextrin to form inclusion compounds. Additionally, cyclodextrins are also capable of other types of nonbonding interactions with molecules that are not inside the hydrophobic cavity. Cyclodextrins have three free hydroxyl groups for each glucopyranose unit, or 18 hydroxyl groups on α-cyclodextrin, 21 hydroxyl groups on β-cyclodextrin, and 24 hydroxyl groups on γ-cyclodextrin. One or more of these hydroxyl groups are reacted with any of a number of reagents to form a large variety of cyclodextrin derivatives. Some of the more common derivatives of cyclodextrin are hydroxypropyl ethers, sulfonates, and sulfoalkylethers. Shown below is the structure of β-cyclodextrin and the hydroxypropyl-β-cyclodextrin (HPβCD).

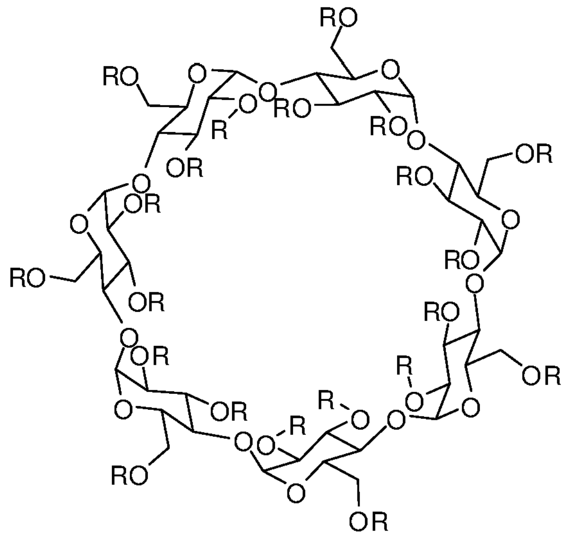

R = H
β-cyclodextrin

R = $CH_2CH(OH)CH_3$
hydroxypropyl β-cyclodextrin

The use of cyclodextrins in pharmaceutical formulations is well known in the art as cyclodextrins and cyclodextrin derivatives are often used to improve the solubility of a drug. Inclusion compounds are involved in many cases of enhanced solubility; however other interactions between cyclodextrins and insoluble compounds improve solubility. Hydroxypropyl-β-cyclodextrin (HPβCD) is commercially available as a pyrogen free product. It is a nonhygroscopic white powder that readily dissolves in water. HPβCD is thermally stable and does not degrade at neutral pH. Thus, cyclodextrins improve the solubility of a therapeutic agent in a formulation. Accordingly, in some embodiments, cyclodextrins are included to increase the solubility of the therapeutic agents, or auris-acceptable otic agents, within the formulations or described herein. In other embodiments, cyclodextrins in addition serve as controlled release excipients within the formulations or described herein.

Preferred cyclodextrin derivatives for use include α-cyclodextrin, β-cyclodextrin, γ-cyclodextrin, hydroxyethyl β-cyclodextrin, hydroxypropyl γ-cyclodextrin, sulfated β-cyclodextrin, sulfated α-cyclodextrin, and sulfobutyl ether β-cyclodextrin.

In some embodiments, the concentration of the cyclodextrin used in the formulations disclosed herein vary according to the physiochemical properties, pharmacokinetic properties, side effect or adverse events, formulation considerations, or other factors associated with the therapeutic agent, or a salt or prodrug thereof. The properties of other excipients in a formulation are also important in some instances. Thus, the concentration or amount of cyclodextrin used in accordance with the formulations disclosed herein vary in some embodiments.

In certain embodiments, the formulation further comprise a suitable viscosity agent, such as hydroxypropyl methylcellulose, hydroxyethyl cellulose, polyvinylpyrrolidone, carboxymethyl cellulose, polyvinyl alcohol, sodium chondroitin sulfate, sodium hyaluronate etc. as a dispersant, if necessary. A nonionic surfactant such as polysorbate 80, polysorbate 20, tyloxapol, Cremophor, HCO 40 etc. is optionally used. In certain embodiments, the preparations optionally contain a suitable buffering system, such as phosphate, citrate, borate, tris, etc., and pH regulators such as sodium hydroxide and hydrochloric acid also are optionally used in the formulations of the disclosures. Sodium chloride or other tonicity agents are also used to adjust tonicity, if necessary.

### Auris-Acceptable Microspheres and Nanospheres (which are not specifically claimed)

Otic agents and/or other pharmaceutical agents disclosed herein are optionally incorporated within controlled release particles, lipid complexes, liposomes, nanoparticles, microspheres, nanocapsules or other agents which enhance or facilitate the localized delivery of the otic agent. In some embodiments, a single formulation is used, in which at least one active pharmaceutical ingredient is present, while in other embodiments, a pharmaceutical formulation that comprises a mixture of two or more distinct formulations is used, in which at least one active pharmaceutical ingredient is present. In certain embodiments, the formulations are cross-linked by one or more agents to alter or improve the properties of the formulation .

Microspheres have been described in the following references: Luzzi, L. A., J. Pharm. Psy. 59:1367 (1970); U.S. Pat. No. 4,530,840; Lewis, D. H., "Controlled Release of Bioactive Agents from Lactides/Glycolide Polymers" in Biodegradable Polymers as Drug Delivery Systems, Chasin, M. and Langer, R., eds., Marcel Decker (1990); U.S. Pat. No. 4,675,189; Beck et al., "Poly(lactic acid) and Poly(lactic acid-co-glycolic acid) Contraceptive Delivery Systems," in Long Acting Steroid Contraception, Mishell, D. R., ed., Raven Press (1983); U.S. Pat. No. 4,758,435; U.S. Pat. No. 3,773,919; U.S. Pat. No. 4,474,572; G. Johns et al. "Broad Applicability of a Continuous Formation Process," Drug Delivery Technology vol. 4 (Jan./Feb. 2004). Examples of protein therapeutics formulated as microspheres include: U.S.

Pat. No. 6,458,387; U.S. Pat. No. 6,268,053; U.S. Pat. No. 6,090,925; U.S. Pat. No. 5,981,719; and U.S. Pat. No. 5,578,709.

Microspheres usually have a spherical shape, although irregularly-shaped microparticles are possible. The microspheres vary in size, ranging from submicron to 1000 micron diameters. Preferably, submicron to 250 micron diameter microspheres, are desirable, allowing administration by injection with a standard gauge needle. The microspheres are thus prepared by any method which produces microspheres in a size range acceptable for use in an injectable formulation . Injections are accomplished with standard gauge needles used for administering liquid formulation .

Suitable examples of polymeric matrix materials include poly(glycolic acid), poly-d,l-lactic acid, poly-l-lactic acid, copolymers of the foregoing, poly(aliphatic carboxylic acids), copolyoxalates, polycaprolactone, polydioxonene, poly(orthocarbonates), poly(acetals), poly(lactic acid-caprolactone), polyorthoesters, poly(glycolic acid-caprolactone), polydioxonene, polyanhydrides, polyphosphazines, and natural polymers including albumin, casein, and some waxes, such as, glycerol mono- and distearate, and the like. Various commercially available poly (lactide-co-glycolide) materials (PLGA) are used in the method disclosed herein. For example, poly (d,l-lactic-co-glycolic acid) is commercially available from Boehringer-Ingelheim as RESOMER RG 503 H. This product has a mole percent composition of 50% lactide and 50% glycolide. These copolymers are available in a wide range of molecular weights and ratios of lactic acid to glycolic acid. A preferred polymer for use is poly(d,l-lactide-co-glycolide). It is preferred that the molar ratio of lactide to glycolide in such a copolymer be in the range of from about 95:5 to about 50:50. In other embodiments, PLGA copolymers with polyethylene glycol (PEG) are suitable polymeric matrices for the formulations disclosed herein. For example, PEG-PLGA-PEG block polymers are biodegradable matrices that provide high mechanical stability of the resulting formulation. Mechanical stabilities of formulations using PEG-PLGA-PEG block polymers have been maintained for more than one month in vitro. In some embodiments, PEG-PLGA-PEG block polymers are used to control the release rate of the active agents with different physical properties. Particularly, in some embodiments, hydrophilic active agents are released more quickly, e.g., approximately 50% of drug release after 24 hours, the remainder released over approximately 5 days, whereas hydrophobic agents are released more slowly, e.g., approximately 80% after 8 weeks.

The molecular weight of the polymeric matrix material is of some importance. The molecular weight should be high enough so that it forms satisfactory polymer coatings, i.e., the polymer should be a good film former. Usually, a satisfactory molecular weight is in the range of 5,000 to 500,000 daltons. The molecular weight of a polymer is also important from the point of view that molecular weight influences the biodegradation rate of the polymer. For a diffusional mechanism of drug release, the polymer should remain intact until all of the drug is released from the microparticles and then degrade. The drug is also released from the microparticles as the polymeric excipient bioerodes. By an appropriate selection of polymeric materials a microsphere formulation are made such that the resulting microspheres exhibit both diffusional release and biodegradation release properties. This is useful in affording multiphasic release patterns.

A variety of methods are known by which compounds are encapsulated in microspheres. In these methods, the active pharmaceutical ingredient is generally dispersed or emulsified, using stirrers, agitators, or other dynamic mixing techniques, in a solvent containing a wall-forming material. Solvent is then removed from the microspheres, and thereafter the microsphere product is obtained.

In one embodiment, controlled release formulations are made through the incorporation of the otic agents and/or other pharmaceutical agents into ethylene-vinyl acetate copolymer matrices. (*See* U.S. Patent No. 6,083,534, incorporated herein for such disclosure). In another embodiment, otic agents are incorporated into poly (lactic-glycolic acid) or poly-L-lactic acid microspheres. In yet another embodiment, the otic agents are encapsulated into alginate microspheres. (*See* U.S. Patent No. 6,036,978, incorporated herein for such disclosure). Biocompatible methacrylate-based polymers to encapsulate the otic agents are optionally used in the formulations disclosed herein. A wide range of methacrylate-based polymer systems are commercially available, such as the EUDRAGIT polymers marketed by Evonik. One useful aspect of methacrylate polymers is that the properties of the formulation are varied by incorporating various co-polymers. For example, poly(acrylic acid-co-methylmethacrylate) microparticles exhibit enhanced mucoadhesion properties as the carboxylic acid groups in the poly(acrylic acid) form hydrogen bonds with mucin (Park et al, Pharm. Res. (1987) 4(6):457-464). Variation of the ratio between acrylic acid and methylmethacrylate monomers serves to modulate the properties of the co-polymer. Methacrylate-based microparticles have also been used in protein therapeutic formulations (Naha et al, Journal of Microencapsulation 04 February, 2008 (online publication)). In one embodiment, the enhanced viscosity auris-acceptable formulations described herein comprise otic agent microspheres wherein the microspheres are formed from a methacrylate polymer or copolymer. In an additional embodiment, the enhanced viscosity formulation described herein comprises otic agent microspheres wherein the microspheres are mucoadhesive. Other controlled release systems, including incorporation or deposit of polymeric materials or matrices onto solid or hollow spheres containing otic agents, are also explicitly contemplated within the embodiments disclosed herein. The types of controlled release systems available without significantly losing activity of the otic agent are determined using the teachings, examples, and principles disclosed herein

An example of a conventional microencapsulation process for pharmaceutical preparations is shown in U.S. Pat. No. 3,737,337. The substances to be encapsulated or embedded are dissolved or dispersed in the organic solution of the

polymer (phase A), using conventional mixers, including (in the preparation of dispersion) vibrators and high-speed stirrers, etc. The dispersion of phase (A), containing the core material in solution or in suspension, is carried out in the aqueous phase (B), again using conventional mixers, such as high-speed mixers, vibration mixers, or even spray nozzles, in which case the particle size of the microspheres will be determined not only by the concentration of phase (A), but also by the emulsate or microsphere size. With conventional techniques for the microencapsulation of active pharmaceutical ingredients, the microspheres form when the solvent containing an active agent and a polymer is emulsified or dispersed in an immiscible solution by stirring, agitating, vibrating, or some other dynamic mixing technique, often for a relatively long period of time.

Conventional methods for the construction of microspheres are also described in U.S. Pat. No. 4,389,330, and U.S. Pat. No. 4,530,840. The desired agent is dissolved or dispersed in an appropriate solvent. To the agent-containing medium is added the polymeric matrix material in an amount relative to the active ingredient which gives a product of the desired loading of active agent. Optionally, all of the ingredients of the microsphere product are blended in the solvent medium together. Suitable solvents for the agent and the polymeric matrix material include organic solvents such as acetone, halogenated hydrocarbons such as chloroform, methylene chloride and the like, aromatic hydrocarbon compounds, halogenated aromatic hydrocarbon compounds, cyclic ethers, alcohols, ethyl acetate ,and the like.

In some embodiments, the controlled-release auris-acceptable microspheres are combined in a controlled-release auris-acceptable increased-viscosity formulation.

A suitable controlled-release auris-acceptable microsphere example for use with the auris-acceptable therapeutic agents disclosed herein includes CHRONIJECT™, a PLGA-based controlled release injectable drug delivery system. Chroniject microspheres are useful for both hydrophobic and hydrophilic auris therapeutic agents, with achieved durations of release ranging from as short as 1 week to as long as 1 year. Release profiles for the microspheres are achieved by modifying polymer and/or process conditions, with initial release or burst of the auris therapeutic agent also available. The manufacturing process is adaptable to aseptic conditions, allowing direct therapeutic use of the manufactured product. Chroniject manufacturing processes are described in U.S. Patent Nos. 5,945,126; 6,270,802 and 6,3361,798.

In some embodiments, the mixture of ingredients in the solvent is emulsified in a continuous-phase processing medium; the continuous-phase medium being such that a dispersion of microdroplets containing the indicated ingredients is formed in the continuous-phase medium. Naturally, the continuous-phase processing medium and the organic solvent must be immiscible, and most commonly is water although nonaqueous media such as xylene and toluene and synthetic oils and natural oils are used. Usually, a surfactant is added to the continuous-phase processing medium to prevent the microparticles from agglomerating and to control the size of the solvent microdroplets in the emulsion. A preferred surfactant-dispersing medium combination is a 1 to 10 wt. % poly vinyl alcohol in water mixture. The dispersion is formed by mechanical agitation of the mixed materials. An emulsion is also formed by adding small drops of the active agent-wall forming material solution to the continuous phase processing medium. The temperature during the formation of the emulsion is not especially critical but in some cases, influences the size and quality of the microspheres and the solubility of the drug in the continuous phase. It is desirable to have as little of the agent in the continuous phase as possible. Moreover, depending on the solvent and continuous-phase processing medium employed, the temperature must not be too low or the solvent and processing medium will solidify or the processing medium will become too viscous for practical purposes, or too high that the processing medium will evaporate, or that the liquid processing medium will not be maintained. Moreover, the temperature of the medium cannot be so high that the stability of the particular agent being incorporated in the microspheres is adversely affected. Accordingly, the dispersion process is conducted at any temperature which maintains stable operating conditions, which preferred temperature being about 30 °C to 60 °C, depending upon the drug and excipient selected.

In some embodiments, the dispersion which is formed is a stable emulsion and from this dispersion the organic solvent immiscible fluid is optionally partially removed in the first step of the solvent removal process. The solvent is easily removed by common techniques such as heating, the application of a reduced pressure or a combination of both. The temperature employed to evaporate solvent from the microdroplets is not critical, but should not be that high that it degrades the agent employed in the preparation of a given microparticle, nor should it be so high as to evaporate solvent at such a rapid rate to cause defects in the wall forming material. Generally, from 5 to 75%, of the solvent is removed in the first solvent removal step.

In some embodiments, after the first stage, the dispersed microparticles in the solvent immiscible fluid medium are isolated from the fluid medium by any convenient means of separation. Thus, for example, the fluid is decanted from the microsphere or the microsphere suspension is filtered. Still other, various combinations of separation techniques are used if desired.

In some embodiments, following the isolation of the microspheres from the continuous-phase processing medium, the remainder of the solvent in the microspheres is removed by extraction. In this step, the microspheres are suspended in the same continuous-phase processing medium used in step one, with or without surfactant, or in another liquid. The extraction medium removes the solvent from the microspheres and yet does not dissolve the

microspheres. During the extraction, the extraction medium with dissolved solvent is optionally removed and replaced with fresh extraction medium. This is best done on a continual basis. Obviously, the rate of extraction medium replenishment of a given process is a variable which is easily determined at the time the process is performed and, therefore, no precise limits for the rate must be predetermined. After the majority of the solvent has been removed from the microspheres, the microspheres are dried by exposure to air or by other conventional drying techniques such as vacuum drying, drying over a desiccant, or the like. This process is very efficient in encapsulating the agent since core loadings of up to 80 wt. %, preferably up to 60 wt. % are obtained.

In some embodiments, controlled release microspheres containing an active pharmaceutical agent are prepared through the use of static mixers. Static or motionless mixers consist of a conduit or tube in which is received a number of static mixing agents. Static mixers provide homogeneous mixing in a relatively short length of conduit, and in a relatively short period of time. With static mixers, the fluid moves through the mixer, rather than some part of the mixer, such as a blade, moving through the fluid.

In some embodiments, a static mixer is used to create an emulsion. When using a static mixer to form an emulsion, several factors determine emulsion particle size, including the density and viscosity of the various solutions or phases to be mixed, volume ratio of the phases, interfacial tension between the phases, static mixer parameters (conduit diameter; length of mixing element; number of mixing elements), and linear velocity through the static mixer. Temperature is a variable because it affects density, viscosity, and interfacial tension. The controlling variables are linear velocity, sheer rate, and pressure drop per unit length of static mixer.

In order to create microspheres containing an active pharmaceutical agent, an organic phase and an aqueous phase are combined in some embodiments. The organic and aqueous phases are largely or substantially immiscible, with the aqueous phase constituting the continuous phase of the emulsion. The organic phase includes an active pharmaceutical agent as well as a wall-forming polymer or polymeric matrix material. In some embodiments, the organic phase is prepared by dissolving an active pharmaceutical agent in an organic or other suitable solvent, or by forming a dispersion or an emulsion containing the active agent. The organic phase and the aqueous phase are pumped so that the two phases flow simultaneously through a static mixer, thereby forming an emulsion which comprises microspheres containing the active pharmaceutical agent encapsulated in the polymeric matrix material. The organic and aqueous phases are pumped through the static mixer into a large volume of quench liquid to extract or remove the organic solvent. Organic solvent are removed from the microspheres while they are washing or being stirred in the quench liquid. After the microspheres are washed in a quench liquid, they are isolated, as through a sieve, and dried.

In some embodiments, the process whereby microspheres are prepared using a static mixer is optionally carried out for a variety of techniques used to encapsulate active agents. In some embodiments, the process is not limited to the solvent extraction technique discussed above and is used with other encapsulation techniques. For example, the process is used with a phase separation encapsulation technique in some instances. To do so, an organic phase is prepared that comprises an active pharmaceutical agent suspended or dispersed in a polymer solution. The non-solvent second phase is free from solvents for the polymer and active agent. A preferred non-solvent second phase is silicone oil. The organic phase and the non-solvent phase are pumped through a static mixer into a non-solvent quench liquid, such as heptane. The semi-solid particles are quenched for complete hardening and washing. The process of microencapsulation also includes spray drying, solvent evaporation, a combination of evaporation and extraction, and melt extrusion.

In another embodiment, the microencapsulation process involves the use of a static mixer with a single solvent. This process is described in detail in U.S. application Ser. No. 08/338,805. An alternative process involves the use of a static mixer with co-solvents. In this process for preparing biodegradable microspheres comprising a biodegradable polymeric binder and an active pharmaceutical agent, a blend of at least two substantially non-toxic solvents, free of halogenated hydrocarbons, is used to dissolve both the agent and the polymer. The solvent blend containing the dissolved agent and polymer is dispersed in an aqueous solution to form droplets. The resulting emulsion is then added to an aqueous extraction medium preferably containing at least one of the solvents of the blend, whereby the rate of extraction of each solvent is controlled, whereupon the biodegradable microspheres containing the pharmaceutically active agent are formed. The process has the advantage that less extraction medium is required because the solubility of one solvent in water is substantially independent of the other and solvent selection is increased, especially with solvents that are particularly difficult to extract.

Nanoparticles are material structures of about 100 nm or less in size. One use of nanoparticles in pharmaceutical formulations is the formation of suspensions as the interaction of the particle surface with solvent is strong enough to overcome differences in density. Nanoparticle suspensions are sterilized as the nanoparticles are small enough to be subjected to sterilizing filtration (U.S. 6,139,870). Nanoparticles comprise at least one hydrophobic, water-insoluble and water-indispersible polymer or copolymer emulsified in a solution or aqueous dispersion of surfactants, phospholipids or fatty acids. The active pharmaceutical ingredient is introduced with the polymer or the copolymer into the nanoparticles.

Lipid nanocapsules act as controlled release structures, as well for penetrating the round window membrane and reaching auris interna targets, is also contemplated herein. *See* Zou et al. J. Biomed. Materials Res., online pub. (April 24, 2008). Lipid nanocapsules are formed by emulsifying 1.028 g capric and caprylic acid triglycerides (LABRAFAC WL 1349;

avg. mw 512), 0.075 g soybean lecithin (LIPOID S75-3; 69% phosphatidylcholine and other phospholipids), 0.846 g surfactant (SOLUTOL HS15), mixture of polyethylene glycol 660 hydroxystearate and free polyethylene glycol 660; 0.089 g NaCl and 2.962 g water. The mixture is stirred at room temperature to obtain an oil emulsion in water. After progressive heating at a rate of 4 °C/min under magnetic stirring, a short interval of transparency should occur close to 70 °C, and the inverted phase (water droplets in oil) obtained at 85 °C. Three cycles of cooling and heating is then applied between 85 °C and 60 °C at the rate of 4 °C/min, and a fast dilution in cold water at a temperature close to 0 °C to produce a suspension of nanocapsules. To encapsulate auris interna active agents, the agent are added just prior to the dilution with cold water.

In some instances, agents are inserted into the lipid nanocapsules by incubation for 90 minutes with an aqueous micellar solution of the auris interna active agent. The suspension is then vortexed every 15 minutes, and then quenched in an ice bath for 1 minute.

Suitable surfactants are, by way of example, cholic acid or taurocholic acid salts. Taurocholic acid, the conjugate formed from cholic acid and taurine, is a fully metabolizable sulfonic acid surfactant. An analog of taurocholic acid, tauroursodeoxycholic acid (TUDCA), is a naturally occurring bile acid and is a conjugate of taurine and ursodeoxycholic acid (UDCA). Other naturally occurring anionic (e.g., galactocerebroside sulfate), neutral (e.g., lactosylceramide) or zwitterionic surfactants (e.g., sphingomyelin, phosphatidyl choline, palmitoyl carnitine) are used to prepare nanoparticles in some instances.

The phospholipids are chosen, by way of example, from natural, synthetic or semisynthetic phospholipids; lecithins (phosphatidylcholine) such as, for example, purified egg or soya lecithins (lecithin E100, lecithin E80 and phospholipons, for example phospholipon 90), phosphatidylethanolamine, phosphatidylserine, phosphatidylinositol, phosphatidylglycerol, dipalmitoylphosphatidylcholine, dipalmitoylglycerophosphatidylcholine, dimyristoylphosphatidylcholine, distearoylphosphatidylcholine and phosphatidic acid or mixtures thereof are used more particularly.

The fatty acids are chosen from, by way of example, from lauric acid, mysristic acid, palmitic acid, stearic acid, isostearic acid, arachidic acid, behenic acid, oleic acid, myristoleic acid, palmitoleic acid, linoleic acid, alpha-linoleic acid, arachidonic acid, eicosapentaenoic acid, erucic acid, docosahexaenoic acid, and the like.

Suitable surfactants are preferably selected from known organic and inorganic pharmaceutical excipients. Such excipients include various polymers, low molecular weight oligomers, natural products, and surfactants. Preferred surface modifiers include nonionic and ionic surfactants. Two or more surface modifiers are used in combination for some embodiments.

Representative examples of surfactants include cetyl pyridinium chloride, gelatin, casein, lecithin (phosphatides), dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters; polyethylene glycols, dodecyl trimethyl ammonium bromide, polyoxyethylenestearates, colloidal silicon dioxide, phosphates, sodium dodecylsulfate, carboxymethylcellulose calcium, hydroxypropyl cellulose (HPC, HPC-SL, and HPC-L), hydroxypropyl methylcellulose (HPMC), carboxymethylcellulose sodium, methylcellulose, hydroxyethylcellulose, hydroxypropylcellulose, hydroxypropylmethylcellulose phthalate, noncrystalline cellulose, magnesium aluminum silicate, triethanolamine, polyvinyl alcohol (PVA), polyvinylpyrrolidone (PVP), 4-(1,1,3,3-tetaamethylbutyl)-phenol polymer with ethylene oxide and formaldehyde (also known as tyloxapol, superione, and triton), poloxamers, poloxamines, a charged phospholipid such as dimyristoyl phosphatidyl glycerol, dioctylsulfosuccinate (DOSS); Tetronic 1508, dialkylesters of sodium sulfosuccinic acid, Duponol P, Tritons X-200, Crodestas F-110, p-isononylphenoxypoly-(glycidol), Crodestas SL-40.RTM. (Croda, Inc.); and SA9OH-CO, which is $C_{18}H_{37}CH_2(CON(CH_3)-CH_2(CHOH)_4(CH_2OH)_2$ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucarmide; n-octyl-β-D-glucopyranoside; octyl β-D-thioglucopyranoside; and the like.

Most of these surfactants are known pharmaceutical excipients and are described in detail in the Handbook of Pharmaceutical Excipients, published jointly by the American Pharmaceutical Association and The Pharmaceutical Society of Great Britain (The Pharmaceutical Press, 1986).

The hydrophobic, water-insoluble and water-indispersible polymer or copolymer are chosen from biocompatible and biodegradable polymers, for example lactic or glycolic acid polymers and copolymers thereof, or polylactic/polyethylene (or polypropylene) oxide copolymers, preferably with molecular weights of between 1000 and 200000, polyhydroxybutyric acid polymers, polylactones of fatty acids containing at least 12 carbon atoms, or polyanhydrides.

In one embodiment, the nanoparticles are suitable for use with hydrophobic active principles. In some embodiments, the active principles are chosen from the major classes of medicaments for use in human or veterinary medicine. In some embodiments, the active principles are chosen from principles for use in the cosmetics or agrifood industry or sports medicine or from diagnostic agents. By way of example, active principles which are of interest in the pharmaceutical industry are chosen, in a non-limiting manner, from antirheumatic, non-steroidal anti-inflammatory (e.g., NSAIDs), analgesic, antitussive and psychotropic agents, steroids, barbiturates, antimicrobial, antiallergenic, antiasthmatic, antispasmodic, antisecretory and cardiovascular agents, cerebral vasodilators, cerebral and hepatic protective agents, therapeutic agents of the gastrointestinal tract, anticancer or antiviral agents, vitamins, contraceptives, vaccines, etc.

The nanoparticles are obtained by the technique of evaporation of solvent, from an aqueous dispersion or solution of phospholipids and of an oleic acid salt into which is added an immiscible organic phase comprising the active principle and the hydrophobic, water-insoluble and water-indispersible polymer or copolymer. The mixture is pre-emulsified and then subjected to homogenization and evaporation of the organic solvent to obtain an aqueous suspension of very small-sized nanoparticles.

A variety of methods are employed to fabricate nanoparticles. These methods include vaporization methods, such as free jet expansion, laser vaporization, spark erosion, electro explosion, and chemical vapor deposition; physical methods involving mechanical attrition (e.g., "pearlmilling" technology, Elan Nanosystems), super critical CO2 and interfacial deposition following solvent displacement. In one embodiment, the solvent displacement method is used. The size of nanoparticles produced by this method is sensitive to the concentration of polymer in the organic solvent; the rate of mixing; and to the surfactant employed in the process. Continuous flow mixers provide the necessary turbulence to ensure small particle size. One type of continuous flow mixing device that is used to prepare nanoparticles has been described (Hansen et al. J Phys Chem 92, 2189-96, 1988). In other embodiments, ultrasonic devices, flow through homogenizers or supercritical CO2 devices are used to prepare nanoparticles.

If suitable nanoparticle homogeneity is not obtained on direct synthesis, then size-exclusion chromatography is used to produce highly uniform drug-containing particles that are freed of other components involved in their fabrication. Size-exclusion chromatography (SEC) techniques, such as gel- filtration chromatography, is used to separate particle-bound drug from free drug or to select a suitable size range of drug-containing nanoparticles. Various SEC media, such as Superdex 200, Superose 6, and Sephacryl 1000 are commercially available and are readily employed by persons of skill in the art for the size-based fractionation of mixture. Additionally, nanoparticles are purified by centrifugation, membrane filtration and by use of other molecular sieving devices, crosslinked gels/materials and membranes.

In some embodiments, liposomes or lipid particles are also employed to encapsulate the otic agent formulations. Phospholipids that are gently dispersed in an aqueous medium form multilayer vesicles with areas of entrapped aqueous media separating the lipid layers. Sonication, or turbulent agitation, of these multilayer vesicles results in the formation of single layer vesicles, commonly referred to as liposomes, with sizes of about 10-1000 nm. These liposomes have many advantages as drug carriers. They are biologically inert, biodegradable, non-toxic and non-antigenic. Liposomes are formed in various sizes and with varying compositions and surface properties. Additionally, they are able to entrap a wide variety of small molecule drugs and release the drug at the site of liposome collapse.

Suitable phospholipids for use in the present formulations are, for example, phosphatidyl cholines, ethanolamines and serines, sphingomyelins, cardiolipins, plasmalogens, phosphatictic acids and cerebrosides, in particular those which are soluble together with piroxicam in non-toxic, pharmaceutically acceptable organic solvents. Preferred phospholipids are, for example, phosphatidyl choline, phosphatidyl ethanolamine, phosphatidyl serine, phosphatidyl inositol, lysophosphatidyl choline, phosphatidyl glycerol and the like, and mixtures thereof especially lecithin, e.g. soya lecithin. The amount of phospholipid used in the present formulation ranges from about 10 to about 30%, preferably from about 15 to about 25% and in particular is about 20%.

Lipophilic additives are employed advantageously to modify selectively the characteristics of the liposomes. Examples of such additives include, for example, stearylamine, phosphatictic acid, tocopherol, cholesterol, cholesterol hemisuccinate and lanolin extracts. The amount of lipophilic additive used ranges from 0.5 to 8%, preferably from 1.5 to 4% and in particular is about 2%. Generally, the ratio of the amount of lipophilic additive to the amount of phospholipid ranges from about 1:8 to about 1:12 and in particular is about 1:10. Said phospholipid, lipophilic additive and the active ingredient piroxicam are employed in conjunction with a non-toxic, pharmaceutically acceptable organic solvent system which dissolves said ingredients. Said solvent system not only must dissolve the active pharmaceutical ingredient completely, but it also has to allow the formulation of stable single bilayered liposomes. The solvent system comprises dimethylisosorbide and tetraglycol (glycofurol, tetrahydrofurfuryl alcohol polyethylene glycol ether) in an amount of about 8 to about 30%. In said solvent system, the ratio of the amount of dimethylisosorbide to the amount of tetraglycol ranges from about 2:1 to about 1:3, in particular from about 1:1 to about 1:2.5 and preferably is about 1:2. The amount of tetraglycol in the final formulation thus varies from 5 to 20%, in particular from 5 to 15% and preferably is approximately 10%. The amount of dimethylisosorbide in the final formulation thus ranges from 3 to 10%, in particular from 3 to 7% and preferably is approximately 5%.

The term "organic component" as used hereinafter refers to mixtures comprising said phospholipid, lipophilic additives and organic solvents.

The active pharmaceutical ingredient is dissolved in the organic component. It is advantageous to use micronized forms of the active ingredient to facilitate its dissolution. The amount of active ingredient in the final formulation ranges from 0.1 to 5.0%. In addition, other ingredients such as anti-oxidants are added to the organic component. Examples include tocopherol, butylated hydroxyanisole, butylated hydroxytoluene, ascorbyl palmitate, ascorbyl oleate, and the like.

In some embodiments, the aqueous component of the present formulation comprises mainly water and optionally contains various additives such as electrolytes, buffer systems, preservatives and the like. Suitable electrolytes include metal salts, in particular alkali metal and earth alkaline metal salts such as, for example, calcium chlorides, sodium chloride, potassium chloride, preferably sodium chloride. In some instances, the concentration of the electrolytes varies over a wide range and depends on the nature and the concentration of each of the ingredients in the final formulation and should be sufficient to stabilize the liposomal membranes. In the present formulation the amount of sodium chloride ranges from 0.05 to 0.2%. Buffer systems comprise mixtures of appropriate amounts of an acid such as phosphoric, succinic, or preferably citric acid, and a base, in particular sodium hydroxide. Said buffer systems should maintain the pH of the formulation within the range of 3 to 9, alternatively within the range or 6 to 8 or between the range of 5 to 7. Preservatives which are employed in the present formulation to prevent degradation by microorganisms comprise benzoic acid, methylparaben and propylparaben in some embodiments.

Liposomal formulations are optionally prepared by (a) heating the phospholipid and the organic solvent system to about 60-80 °C in a vessel, dissolving the active ingredient, then adding any additional formulating agents, and stirring the mixture until complete dissolution is obtained; (b) heating the aqueous solution to 90-95 °C in a second vessel and dissolving the preservatives therein, allowing the mixture to cool and then adding the remainder of the auxiliary formulating agents and the remainder of the water, and stirring the mixture until complete dissolution is obtained; thus preparing the aqueous component; (c) transferring the organic phase directly into the aqueous component, while homogenizing the combination with a high performance mixing apparatus, in particular a high-shear mixer; and (d) adding a thickener to the resulting mixture while further homogenizing. Preferably, the aqueous component is placed in a suitable vessel which is equipped with a homogenizer and homogenization is effected by creating great turbulence during the injection of the organic component. Any mixing means or homogenizer which exerts high shear forces on the mixture is employed. Generally, a mixer capable of speeds from about 1,500 to 20,000 rpm, in particular from about 3,000 to about 6,000 rpm are employed. Suitable thickening agents for use in process step (d) are for example, xanthan gum, hydroxypropyl cellulose, hydroxypropyl methylcellulose or mixtures thereof, cellulose derivatives being preferred. The amount of thickening agent depends on the nature and the concentration of the other ingredients and in general ranges from about 0.5 to 1.5%, and in particular is approximately 1.5%. In order to prevent degradation of the materials used during the preparation of the liposomal formulation, it is advantageous to purge all solutions with an inert gas such as nitrogen or argon, and to conduct all steps under an inert atmosphere. Liposomes prepared by the above described method usually contain most of the active ingredient bound in the lipid bilayer and separation of the liposomes from unencapsulated material is not required.

**Auris-Acceptable Lipid Formulations (which are not specifically claimed)**

In some embodiments, the drug delivery formulation is a lipid-based formulation . In some embodiments, the lipid-based drug delivery formulation is a lipid emulsion (e.g., microemulsions and oil-in-water emulsions), a lipid vesicle (e.g., liposomes, micelles and transfersomes) or a combination thereof. In some embodiments, the lipid-based drug delivery formulation is a lipid vesicle wherein the lipid vesicle is a liposome. In some embodiments, the lipid-based drug delivery formulation is a phospholipid-based formulation . In some embodiments, the lipid-based drug delivery formulation is a phospholipid-based formulation wherein the natural or synthetic phospholipid is phosphatidylethanolamine, phosphatidylserine, phosphatidylinositol, phosphatidylglycerol, phosphatidic acid, lysophospholipids, egg or soybean phospholipid, or a combination thereof. The phospholipid is optionally salted or desalted, hydrogenated or partially hydrogenated, natural, synthetic, or semisynthetic. In some embodiments, the lipid-based drug delivery formulation is a phospholipid-based formulation (e.g., hydrogenated or nonhydrogenated phospholipids, lecithins, phosphatidyl cholines (C8-C18), phosphatidylethanolamines (C8-C18), phosphatidylglycerols (C8-C18)) wherein the phospholipid is phospholipon 90H (1,2-dia-cyl-SN-glycero-3-phosphatidyl choline), egg phospholipids P123, Lipoid E80; Phospholipon 80H®, 80G®, 90H® and 100H®, or combinations thereof.

In some embodiments, the lipid-based drug delivery formulation comprises a water-soluble preservative (i.e., a component that prevents microbes from substantially growing and multiplying). In some embodiments, the lipid-based drug delivery formulation comprises a water-soluble preservative wherein the preservative is a benzethonium salt (e.g., benzethonium chloride), benzoic acid, and/or a benzylkonium salt (e.g., benzylkonium chloride). As used herein, water soluble means that the component has a solubility in water from about 100 μg/mL (0.01%) to about 0.01 mg/mL (0.1%).

In some embodiments, the lipid-based drug delivery formulation comprises a lipid soluble anti-oxidant. In some embodiments, the lipid-based drug delivery formulation comprises vitamin E.

In some embodiments, the lipid-based drug delivery formulation comprises less than about 2% w/w, less than about 1.5%, less than about 1.0%, less than about 0.5%, or less than about 0.25% of a viscosity enhancing agent.

In some embodiments, the lipid-based drug delivery formulation has a viscosity of at least about 10,000 centipoise, at least about 20,000 centipoise, at least about 30,000 centipoise, at least about 40,000 centipoise, at least about 50,000 centipoise, at least about 60,000 centipoise, or at least about 70,000 centipoise, all at 58° C, without the presence of any methyl-cellulose or other viscosity enhancing agents. In some embodiments, the lipid-based drug delivery

formulation comprises oleyl alcohol to enhance the transmembrane penetration.

In some embodiments, the lipid-based drug delivery formulation comprises a penetration enhancer (e.g., a low molecular weight alcohol (e.g., ethanol, oleyl alcohol), alkyl methanol sulphoxides, N-methyl-2-pyrrolidone, fatty amines (e.g., oleylamine), fatty acids (e.g., oleic acid, palmitoleic acid, linoleic acid, myristate acid), gluconic acid (the hexonic acid derived from glucose by oxidation of the aldehyde group at C-1 to a carboxyl group) and its derivatives, such as gluconolactone (especially, glucono-D-lactone, a chelating agent produced by the oxidation of glucose), azone and propylene glycol, singly or in combination). In some embodiments, the lipid-based drug delivery formulation comprises a penetration enhancer wherein the penetration enhancer is propylene glycol, either alone or in up to a 1:1 ratio with another enhancer, such as oleic acid or ethanol. In some embodiments, the lipid-based drug delivery formulation comprises a penetration enhancer wherein the penetration enhancer is gluconolactone (e.g., glucono-D-lactone), either alone or in up to a 1:1 ratio with another enhancer, such as propylene glycol.

In some embodiments, the lipid-based drug delivery formulation comprises about 25% v/v or less of any one or more chemical penetration enhancer(s), most preferably from about 2% to 15% v/v, although the exact formulation will vary depending on the presence and amounts of excipients, preservatives, water, pH modulators, and the like included therein.

In some embodiments, prepared liposomes loaded with the active agents herein are gently mixed with viscosity, mucosal adhesives or absorption penetration enhancers. For example, the active agents loaded into liposomes are mixed with a chitosan-glycerophosphate composition. The liposome size are optionally increased or decreased to modulate the release kinetics of the controlled release particles. In additional aspects, release kinetics is altered by changing the lipid composition of the liposomes as described above.

The formulations described herein are administered in any suitable form. By way of non-limiting examples, the formulations are administered as otic drops, as intratympanic injections, as foams or as otic paints. The formulations are administered via cannula and/or injection, via a drop dispenser, as a spray in the ear canal, or as a paint via a cotton tipped stick.

**Controlled Release Kinetics**

The goal of every drug delivery technique is to deliver the proper amount of drug to the site of action at the right time to obtain a therapeutic benefit. In general, controlled release drug formulations impart control over the release of drug with respect to site of release and time of release within the body. As discussed herein, controlled release refers to any release other than solely immediate release. In some instances, controlled release is delayed release, extended release, sustained release and/or pulsatile release (e.g., a combination of extended release and immediate release) or a combination thereof. Many advantages are offered by controlled release. First, controlled release of a pharmaceutical agent allows less frequent dosing and thus minimizes repeated treatment. Second, controlled release treatment results in more efficient drug utilization and less of the compound remains as a residue. Third, controlled release offers the possibility of localized drug delivery by placement of a delivery device or formulation at the site of disease. Still further, controlled release offers the opportunity to administer and release two or more different drugs, each having a unique release profile, or to release the same drug at different rates or for different durations, by means of a single dosage unit.

In a specific embodiment the formulations described herein provide a therapeutically effective amount of at least one active pharmaceutical ingredient at the site of disease with no systemic exposure. In an additional embodiment the formulations described herein provide a therapeutically effective amount of at least one active pharmaceutical ingredient at the site of disease with no detectable systemic exposure.

In certain embodiments, the formulations comprise an excipient that increases the release rate of the therapeutic agent. In certain embodiments, the formulations comprise an excipient that decreases the release rate of the therapeutic agent.

The formulation is designed to provide drug delivery over a desired period of time, including periods up to several weeks. As such, the patient will not need repeated administration of the drug, or at the least, fewer and less frequent administration of the drug.

Drugs delivered to the auris interna have commonly been administered systemically via oral, intravenous or intramuscular routes. However, systemic administration for pathologies local to the auris interna increases the likelihood of systemic toxicities and side effects and creates a non-productive distribution of drug in which high levels drug are found in the serum and correspondingly lower levels are found at the auris interna.

In one embodiment, the formulations disclosed herein additionally provides an immediate release of the therapeutic agent, or otic agent, from the formulation , or within 1 minute, or within 5 minutes, or within 10 minutes, or within 15 minutes, or within 30 minutes, or within 60 minutes, or within 90 minutes. In other embodiments, a therapeutically effective amount of at least one therapeutic agent, or otic agent, is released from the formulation immediately, or within 1 minute, or within 5 minutes, or within 10 minutes, or within 15 minutes, or within 30 minutes, or within 60 minutes, or within 90 minutes. Additional embodiments of the formulation also include an agent that enhances the viscosity of the formulations included herein.

An immediate or rapid release option includes use of different viscosity-enhancing polymers, multi-component formulations and nanospheres (or sub-micron spheres). In addition, the microspheres are optionally coated with an immediate-release component and a controlled-release component.

In certain embodiments the formulation provides immediate release of at least one active pharmaceutical ingredient. Additional embodiments of the formulation also include a thickener that thickens the formulations included herein. In other embodiments the formulation comprises a liposomal formulation providing immediate release of at least one active pharmaceutical ingredient. In certain other embodiments the formulation comprises a cyclodextrin-containing formulation providing immediate release of at least one active pharmaceutical ingredient. In additional embodiments the formulation comprises a microsphere formulation providing immediate release of at least one active pharmaceutical ingredient. In additional embodiments the formulation comprises a nanoparticle formulation providing immediate release of at least one active pharmaceutical ingredient.

In other or further embodiments, the formulation provides a controlled release formulation of at least one therapeutic agent, or otic agent. In certain embodiments, diffusion of at least one otic agent from the formulation occurs for a time period exceeding 5 minutes, or 15 minutes, or 30 minutes, or 1 hour, or 4 hours, or 6 hours, or 12 hours, or 18 hours, or 1 day, or 2 days, or 3 days, or 4 days, or 5 days, or 6 days, or 7 days, or 10 days, or 12 days, or 14 days, or 18 days, or 21 days, or 25 days, or 30 days, or 45 days, or 2 months, or 3 months, or 4 months, or 5 months, or 6 months, or 9 months, or 1 year. In other embodiments, a therapeutically effective amount of at least one otic agent is released from the formulation for a time period exceeding 5 minutes, or 15 minutes, or 30 minutes, or 1 hour, or 4 hours, or 6 hours, or 12 hours, or 18 hours, or 1 day, or 2 days, or 3 days, or 4 days, or 5 days, or 6 days, or 7 days, or 10 days, or 12 days, or 14 days, or 18 days, or 21 days, or 25 days, or 30 days, or 45 days, or 2 months, or 3 months, or 4 months, or 5 months, or 6 months, or 9 months, or 1 year.

In other embodiments, the formulation provides both an immediate release and an extended release formulation of a therapeutic agent, or an otic agent. In yet other embodiments, the formulation contains a 0.25:1 ratio, or a 0.5:1 ratio, or a 1:1 ratio, or a 1:2 ratio, or a 1:3, or a 1:4 ratio, or a 1:5 ratio, or a 1:7 ratio, or a 1:10 ratio, or a 1: 15 ratio, or a 1:20 ratio of immediate release and extended release formulations . In a further embodiment the formulation provides an immediate release of a first otic agent and an extended release of a second otic agent or other therapeutic agent. In yet other embodiments, the formulation provides an immediate release and extended release formulation of at least one otic agent, and at least one therapeutic agent. In some embodiments, the formulation provides a 0.25:1 ratio, or a 0.5:1 ratio, or a 1:1 ratio, or a 1:2 ratio, or a 1:3, or a 1:4 ratio, or a 1:5 ratio, or a 1:7 ratio, or a 1:10 ratio, or a 1: 15 ratio, or a 1:20 ratio of immediate release and extended release formulations of a first otic agent and second therapeutic agent, respectively.

In a specific embodiment the formulation provides a therapeutically effective amount of at least one otic agent at the site of disease with essentially no systemic exposure. In an additional embodiment the formulation provides a therapeutically effective amount of at least one otic agent at the site of disease with essentially no detectable systemic exposure. In other embodiments, the formulation provides a therapeutically effective amount of at least one otic therapeutic agent at the site of disease with little or no detectable systemic exposure.

In some instances, upon administration (e.g., intratympanic injection) of a conventional otic formulation (e.g., DSP in a buffer), the concentration of a drug in the perilymph of an individual will rise sharply ($C_{max}$ at about 1-2 hours) and then taper off to below $C_{min}$. In some instances, administration of an otic formulation described herein lowers the ratio of $C_{max}$ to $C_{min}$ and provides a larger Area Under the Curve (AUC) with a prolonged PK profile based on the $C_{min}$. In certain instances, controlled release formulations described herein delay the time to $C_{max}$. In certain instances, the controlled steady release of a drug prolongs the time the concentration of the drug will stay above the minimum therapeutic concentration (i.e., $C_{min}$). In some instances, controlled release of an otic agent provided by the formulations described herein allows for release of an otic agent at concentrations greater than $C_{min}$ for a period of at least 1 day, 3 days, 4 days, 5 days, 6 days, 7 days, 10 days, 14 days, 3 weeks, 1 month, or 6 months. In some embodiments, auris formulations described herein prolong the residence time of a drug in the inner ear. In certain instances, once drug exposure (e.g., concentration in the perilymph) of a drug reaches steady state, the concentration of the drug in the perilymph stays at or about the therapeutic dose for an extended period of time (e.g., one day, 2 days, 3 days, 4 days, 5 days, 6 days, 1 week, 2 weeks, 1 month, or 6 months). In some embodiments, otic formulations described herein increase the bioavailability and/or steady state levels of a drug in auris structures (e.g., in inner ear and/or the endolymph and/or the perilymph).

In some instances, upon administration of a controlled release auris formulation described herein (e.g., a formulation comprising a therapeutic agent), drug concentrations relative to the binding constants of one or more otic receptors are relevant in determining a biologically meaningful PK profile or the minimum concentration of an active agent required for a therapeutic effect. In some instances, upon administration of a controlled release auris formulation described herein, drug concentrations relative to the binding constants of two receptors, such as, by way of example only, mineralcorticoid receptor (MR) and glucocorticoid receptor (GR), are relevant in determining the $C_{min}$ or the biologically most meaningful PK profile. In some instances, for example, a drug saturates a first receptor (e.g.GR) first, then saturates a second receptor (e.g., MR), and there is therapeutic benefit even when the first receptor is saturated and the second receptor is not yet saturated. In some instances, the drug concentration for saturation the second receptor is

about the same as the $C_{min}$. In some of such instances, for example, a next dose is administered when drug concentration drops below saturation levels of the second receptor and/or the $C_{min}$

The combination of immediate release, delayed release and/or extended release otic formulations are combined with other pharmaceutical agents, as well as the excipients, diluents, stabilizers, tonicity agents and other components disclosed herein. As such, depending upon the otic agent used, the thickness or viscosity desired, or the mode of delivery chosen, alternative aspects of the embodiments disclosed herein are combined with the immediate release, delayed release and/or extended release embodiments accordingly.

In certain embodiments, the pharmacokinetics of the otic formulations described herein are determined by injecting the formulation on or near the round window membrane of a test animal (including by way of example, a guinea pig, a chinchilla, or a rat). At a determined period of time (e.g., 6 hours, 12 hours, 1 day, 2 days, 3 days, 4 days, 5 days, 6 days, 7 days, and 14 days for testing the pharmacokinetics of a formulation over a 1 or 2 week period), the test animal is euthanized and the inner ear removed and tested for the presence of the otic agent. As needed, the level of otic agent is measured in other organs. In addition, the systemic level of the otic agent is measured by withdrawing a blood sample from the test animal. In order to determine whether the formulation impedes hearing, the hearing of the test animal is optionally tested.

Alternatively, an inner ear is provided (as removed from a test animal) and the migration of the otic agent is measured. As yet another alternative, an in vitro model of a round window membrane is provided, and the migration of the otic agent is measured.

**Retention Time**

In some embodiments, the formulation has a retention time in the ear of about 5 minutes, about 15 minutes, about 30 minutes, about 1 hour, about 4 hours, about 6 hours, about 12 hours, about 18 hours, about 1 day, about 2 days, about 3 days, about 4 days, about 5 days, about 6 days, about 7 days, about 10 days, about 12 days, about 14 days, about 18 days, about 21 days, about 25 days, about 30 days, about 45 days, about 2 months, about 3 months, about 4 months, about 5 months, about 6 months, about 9 months or about 1 year. In some embodiments, the formulation has a retention time in the ear of at least 5 minutes, at least 15 minutes, at least 30 minutes, at least 1 hour, at least 4 hours, at least 6 hours, at least 12 hours, at least 18 hours, at least 1 day, at least 2 days, at least 3 days, at least 4 days, at least 5 days, at least 6 days, at least 7 days, at least 10 days, at least 12 days, at least 14 days, at least18 days, at least 21 days, at least 25 days, at least 30 days, at least 45 days, at least 2 months, at least 3 months, at least 4 months, at least 5 months, at least 6 months, at least 9 months or at least 1 year.

In some embodiments, the ear is the outer ear, middle ear, or inner ear. In some embodiments, the ear is the middle ear. In some embodiments, the ear is the inner ear. In some embodiments, the ear is the outer ear. In some embodiments, the outer ear is the external auditory canal, the outer surface of the tympanic membrane, or a combination thereof.

**Modes of Otic Administration (Methods of administration described herein do not form part of the invention, but it will be understood that the formulations as claimed may be for use with such methods)**

In some examples, the auris formulations described herein are administered into the ear canal, or in the vestibule of the ear. Access to, for example, the vestibular and cochlear apparatus occurs through the auris media including the round window membrane, the oval window/stapes footplate, the annular ligament and through the otic capsule/temporal bone. In some examples, otic administration of the formulations described herein avoids toxicity associated with systemic administration (e.g., hepatotoxicity, cardiotoxicity, gastrointestinal side effects, and renal toxicity) of the active agents. In some examples, localized administration in the ear allows an active agent to reach a target organ (e.g., inner ear) in the absence of systemic accumulation of the active agent. In some examples, local administration to the ear provides a higher therapeutic index for an active agent that otherwise have dose-limiting systemic toxicity.

Provided but not claimed are modes of treatment for otic formulations that ameliorate or lessen otic disorders described herein. Drugs delivered to the inner ear have been administered systemically via oral, intravenous or intramuscular routes. However, systemic administration for pathologies local to the inner ear increases the likelihood of systemic toxicities and adverse side effects and creates a non-productive distribution of drug in which high levels of drug are found in the serum and correspondingly lower levels are found at the inner ear.

Provided but not claimed are methods comprising the administration of said auris formulations on or near the round window membrane via intratympanic injection. In some examples, a formulation disclosed herein is administered on or near the round window or the crista fenestrae cochleae through entry via a post-auricular incision and surgical manipulation into or near the round window or the crista fenestrae cochleae area. Alternatively, a formulation disclosed herein is applied via syringe and needle, wherein the needle is inserted through the tympanic membrane and guided to the area of the round window or crista fenestrae cochleae. In some examples, a formulation disclosed herein is then deposited

on or near the round window or crista fenestrae cochleae for localized treatment. In other examples, a formulation disclosed herein is applied via microcathethers implanted into the patient, and in yet further examplesa formulation disclosed herein is administered via a pump device onto or near the round window membrane. In still further examples, a formulation disclosed herein is applied at or near the round window membrane via a microinjection device. In yet other examples, a formulation disclosed herein is applied in the tympanic cavity. In some examples, a formulation disclosed herein is applied on the tympanic membrane. In still other examples, a formulation disclosed herein is applied onto or in the auditory canal. The formulations described herein, and modes of administration thereof, are also applicable to methods of direct instillation or perfusion of the inner ear compartments. Thus, the formulations described herein are useful in surgical procedures including, by way of non-limiting examples, cochleostomy, labyrinthotomy, mastoidectomy, stapedectomy, endolymphatic sacculotomy or the like.

*Intratympanic Injections* (*reference to methods of treatment do not form part of the invention)*

In some examples, a surgical microscope is used to visualize the tympanic membrane. In some examples, the tympanic membrane is anesthetized by any suitable method (e.g., use of phenol, lidocaine, and xylocaine). In some examples, the anterior-superior and posterior-inferior quadrants of the tympanic membrane are anesthetized.

In some examples, a puncture is made in the tympanic membrane to vent any gases behind the tympanic membrane. In some examples, a puncture is made in the anterior-superior quadrant of the tympanic membrane to vent any gases behind the tympanic membrane. In some examples, the puncture is made with a needle (e.g., a 25 gauge needle). In some examples, the puncture is made with a laser (e.g., a $CO_2$ laser). In one example the delivery system is a syringe and needle apparatus that is capable of piercing the tympanic membrane and directly accessing the round window membrane or crista fenestrae cochleae of the auris interna.

In one example, the needle is a hypodermic needle used for instant delivery of the formulation. The hypodermic needle is a single use needle or a disposable needle. In some example, a syringe is used for delivery of the pharmaceutically acceptable otic agent-containing formulations as disclosed herein wherein the syringe has a press-fit (Luer) or twist-on (Luer-lock) fitting. In one example, the syringe is a hypodermic syringe. In another example, the syringe is made of plastic or glass. In yet another example, the hypodermic syringe is a single use syringe. In a further example, the glass syringe is capable of being sterilized. In yet a further example, the sterilization occurs through an autoclave. In another example, the syringe comprises a cylindrical syringe body wherein the formulation is stored before use. In other examples, the syringe comprises a cylindrical syringe body wherein the pharmaceutically acceptable otic formulations as disclosed herein is stored before use which conveniently allows for mixing with a suitable pharmaceutically acceptable buffer. In other examples, the syringe contains other excipients, stabilizers, suspending agents, diluents, or a combination thereof to stabilize or otherwise stably store the otic agent or other pharmaceutical compounds contained therein.

In some example, the syringe comprises a cylindrical syringe body wherein the body is compartmentalized in that each compartment is able to store at least one component of the auris-acceptable otic formulation. In a further example, the syringe having a compartmentalized body allows for mixing of the components prior to injection into the auris media or auris interna. In other examples, the delivery system comprises multiple syringes, each syringe of the multiple syringes contains at least one component of the formulation such that each component is pre-mixed prior to injection or is mixed subsequent to injection. In a further example, the syringes disclosed herein comprise at least one reservoir wherein the at least one reservoir comprises an otic agent, or a pharmaceutically acceptable buffer, or a viscosity enhancing agent, or a combination thereof. Commercially available injection devices are optionally employed in their simplest form as ready-to-use plastic syringes with a syringe barrel, needle assembly with a needle, plunger with a plunger rod, and holding flange, to perform an intratympanic injection.

In some examples, a needle is used to deliver the formulations described herein. In some examples, a needle punctures the posterior-inferior quadrant of the tympanic membrane. In some examples, the needle is a standard gauge needle. In some examples, the needle is a narrow gauge needle. In some examples, the needle is wider than an 18 gauge needle. In another example, the needle gauge is from about 18 gauge to about 30 gauge. In some examples, the needle gauge is from about 20 gauge to about 30 gauge. In some examples, the needle gauge is from about 25 gauge to about 30 gauge. In some examples, the needle gauge is about 18 gauge, about 19 gauge, about 20 gauge, about 21 gauge, about 22 gauge, about 23 gauge, about 24 gauge, about 25 gauge, about 26 gauge, about 27 gauge, about 28 gauge, about 29 gauge, or about 30 gauge. In a further example, the needle is a 25 gauge needle. Depending upon the thickness or viscosity of a formulation disclosed herein, the gauge level of the syringe or hypodermic needle is varied accordingly. In some examples, the formulations described herein are liquids and are administered via narrow gauge needles or cannulas (e.g., 22 gauge needle, 25 gauge needle, or cannula), minimizing damage to the tympanic membrane upon administration. The formulations described herein are administered with minimal discomfort to a patient.

In some examples, an otoendoscope (e.g., about 1.7 mm in diameter) is used to visualize the round window membrane. In some examples, any obstructions to the round window membrane (e.g., a false round window membrane, a fat plug, fibrous tissue) are removed.

In some examples, a formulation disclosed herein is injected onto the round window membrane. In some examples, 0.1 to 0.5 cc of a formulation disclosed herein is injected onto the round window membrane.

In some examples, the tympanic membrane puncture is left to heal spontaneously. In some examples, a paper patch myringoplasty is performed by a trained physician. In some examples, a tympanoplasty is performed by a trained physician. In some examples, an individual is advised to avoid water. In some examples, a cotton ball soaked in petroleum-jelly is utilized as a barrier to water and other environmental agents.

*Other Delivery Routes* (*reference to methods of treatment do not form part of the invention*)

In some examples, a formulation disclosed herein is administered locally to the outer ear, such as the external auditory canal, the outer surface of the tympanic membrane, or a combination thereof. In some examples, the formulations described herein are not administered through the tympanic membrane.

In some examples, a formulation disclosed herein is administered to the inner ear. In some examples, a formulation disclosed herein is administered to the inner ear via an incision in the stapes footplate. In some examples, a formulation disclosed herein is administered to the cochlea via a cochleostomy. In some examples, a formulation disclosed herein is administered to the vestibular apparatus (e.g., semicircular canals or vestibule).

In some examples, a formulation disclosed herein is applied via syringe and needle. In other examples, a formulation disclosed herein is applied via microcatheters implanted into the patient. In some examples, a formulation disclosed herein is administered via a pump device. In still further examples, a formulation disclosed herein is applied via a microinjection device. In some examples, a formulation disclosed herein is administered via a prosthesis, a cochlear implant, a constant infusion pump, or a wick.

In some examples, the delivery device is an apparatus designed for administration of therapeutic agents to the middle and/or inner ear. By way of example only: GYRUS Medical GmbH offers micro-otoscopes for visualization of and drug delivery to the round window niche; Arenberg has described a medical treatment device to deliver fluids to inner ear structures in U.S. Patent Nos. 5,421,818; 5,474,529; and 5,476,446. U.S. Patent Application No. 08/874,208 describes a surgical method for implanting a fluid transfer conduit to deliver therapeutic agents to the inner ear. U.S. Patent Application Publication 2007/0167918 further describes a combined otic aspirator and medication dispenser for intratympanic fluid sampling and medicament application.

*Dosage*

In some embodiments, auris formulations described herein are controlled release formulations, and are administered at reduced dosing frequency compared to the current standard of care. In certain instances, when an auris formulation is administered via intratympanic injection, a reduced frequency of administration alleviates discomfort caused by multiple intratympanic injections in individuals undergoing treatment for a middle and/or inner ear disease, disorder or condition. In certain instances, a reduced frequency of administration of intratympanic injections reduces the risk of permanent damage (e.g., perforation) to the ear drum. In some embodiments, formulations described herein provide a constant, sustained, extended, delayed or pulsatile rate of release of an active agent into the inner ear environment and thus avoid any variability in drug exposure in treatment of otic disorders.

The formulations containing the compound(s) described herein are administered for prophylactic and/or therapeutic treatments. In therapeutic applications, the formulations are administered to a patient already suffering from a disease, condition or disorder, in an amount sufficient to cure or at least partially arrest the symptoms of the disease, disorder or condition. Amounts effective for this use will depend on the severity and course of the disease, disorder or condition, previous therapy, the patient's health status and response to the drugs, and the judgment of the treating physician.

The amount of a given agent that will correspond to such an amount will vary depending upon factors such as the particular compound, disease condition and its severity, but is nevertheless routinely determined in a manner known in the art according to the particular circumstances surrounding the case, including, e.g., the specific agent being administered, the route of administration, the condition being treated, and the subject or host being treated. In general, however, doses employed for adult human treatment will typically be in the range of 0.02-50 mg per administration, preferably 1-15 mg per administration. In some embodiments, the desired dose is conveniently presented in a single dose or as divided doses administered simultaneously (or over a short period of time) or at appropriate intervals.

*Frequency of administration*

In the case wherein the patient's condition does not improve, upon the doctor's discretion the administration of the compounds is administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the administration of the compounds are given continuously; alternatively, the dose of drug being administered are temporarily reduced or temporarily suspended for a certain length of time (*i.e.,* a "drug holiday"). The length of the drug holiday varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, and 365 days. The dose reduction during a drug holiday are from 10%-100%, including by way of example only 10%, 15%, 20%, 25%, 30%, 35%, 40%, 45%, 50%, 55%, 60%, 65%, 70%, 75%, 80%, 85%, 90%, 95%, and 100%.

Once improvement of the patient's conditions has occurred, a maintenance dose is administered if necessary. Subsequently, the dosage or the frequency of administration, or both, is reduced, as a function of the symptoms, to a level at which the improved disease, disorder or condition is retained. Patients, however, require intermittent treatment on a long-term basis upon any recurrence of symptoms in some embodiments.

In some embodiments, the initial administration is of a particular formulation and the subsequent administration is of a different formulation or active pharmaceutical ingredient.

**Otic Surgery and Implants (any reference to methods of treatment do not form part of the invention)**

In some embodiments, the pharmaceutical formulations described herein are used in combination with (e.g., implantation, short-term use, long-term use, or removal of) implants (e.g., cochlear implants). As used herein, implants include auris-interna or auris-media medical devices, examples of which include cochlear implants, hearing sparing devices, hearing-improvement devices, short electrodes, micro-prostheses or piston-like prostheses; needles; stem cell transplants; drug delivery devices; any cell-based therapeutic; or the like. In some instances, the implants are used in conjunction with a patient experiencing hearing loss. In some instances, the hearing loss is present at birth. In some instances, the hearing loss is associated with conditions such as AIED, bacterial meningitis or the like that lead to osteoneogenesis and/or nerve damage with rapid obliteration of cochlear structures and profound hearing loss.

In some instances, an implant is an immune cell or a stem cell transplant in the ear. In some instances, an implant is a small electronic device that has an external portion placed behind the ear, and a second portion that is surgically placed under the skin that helps provide a sense of sound to a person who is profoundly deaf or severely hard-of-hearing. By way of example, such cochlear medical device implants bypass damaged portions of the ear and directly stimulate the auditory nerve. In some instances cochlear implants are used in single sided deafness. In some instances cochlear implants are used for deafness in both ears.

In some embodiments, administration of a formulation or device described herein in combination with an otic intervention (e.g., an intratympanic injection, a stapedectomy, a medical device implant, or a cell-based transplant) delays or prevents collateral damage to auris structures, e.g., irritation, cell damage, cell death, osteoneogenesis, and/or further neuronal degeneration, caused by the external otic intervention (e.g., installation of an external device and/or cells in the ear). In some embodiments, administration of a formulation or device described herein in combination with an implant allows for a more effective restoration of hearing loss compared to an implant alone.

In some embodiments, administration of formulation or device described herein reduces damage to cochlear structures caused by underlying conditions (e.g., bacterial meningitis, autoimmune ear disease (AIED)) allowing for successful cochlear device implantation. In some embodiments, administration of a formulation or device described herein, in conjunction with otic surgery, medical device implantation, and/or cell transplantation, reduces or prevents cell damage and/or death (e.g., auris sensory hair cell death and/or damage) associated with otic surgery, medical device implantation, and/or cell transplantation.

In some embodiments, administration of a formulation or device described herein in conjunction with a cochlear implant or stem cell transplant has a trophic effect (e.g., promotes healthy growth of cells and/or healing of tissue in the area of an implant or transplant). In some embodiments, a trophic effect is desirable during otic surgery or during intratympanic injection procedures. In some embodiments, a trophic effect is desirable after installation of a medical device or after a cell transplant. In some of such embodiments, the formulations or devices described herein are administered via direct cochlear injection, through a cochleostomy or via deposition on the round window.

In some embodiments, administration of the formulations described herein reduces inflammation and/or infections associated with otic surgery, implantation of a medical device or a cell transplant. In some instances, perfusion of a surgical area with a formulation described herein reduces or eliminates post-surgical and/or post-implantation complications (e.g., inflammation, hair cell damage, neuronal degeneration, osteoneogenesis, or the like). In some instances, perfusion of a surgical area with a formulation described herein reduces post-surgery or post-implantation recuperation time. In some embodiments, a medical device is coated with a formulation described herein prior to implantation in the ear.

In one aspect, the formulations described herein, and modes of administration thereof, may be applicable to methods of direct perfusion of the inner ear compartments. Thus, the formulations described herein are useful in

combination with otic interventions. In some embodiments, an otic intervention is an implantation procedure (e.g., implantation of a hearing device in the cochlea). In some embodiments, an otic intervention is a surgical procedure including, by way of non-limiting examples, cochleostomy, labyrinthotomy, mastoidectomy, stapedectomy, stapedotomy, endolymphatic sacculotomy, tympanostomy, or the like. In some embodiments, the inner ear compartments are perfused with a formulation described herein prior to otic intervention, during otic intervention, or after otic intervention, or a combination thereof.

In some embodiments, when perfusion is carried out in combination with otic intervention, the formulations are immediate release formulations. In some of such embodiments, the immediate release formulations described herein are substantially free of extended release components.

**Kits and Other Articles of Manufacture**

The disclosure also provides kits for preventing, treating or ameliorating the symptoms of a diseases or disorder in a mammal. Such kits generally will comprise one or more of the pharmaceutically acceptable formulations as disclosed herein, and instructions for using the kit. The disclosure also contemplates the use of one or more of the formulations, in the manufacture of medicaments for treating, abating, reducing, or ameliorating the symptoms of a disease, dysfunction, or disorder in a mammal, such as a human that has, is suspected of having, or at risk for developing an auris interna disorder.

In some embodiments, a kit disclosed herein comprises a needle that penetrates a tympanic membrane and/or a round window. In some embodiments, a kit disclosed herein further comprises a hydrogel with a penetration enhancer (e.g., an alkylglycoside and/or a saccharide alkyl ester).

In some embodiments, kits include a carrier, package, or container that is compartmentalized to receive one or more containers such as vials, tubes, and the like, each of the container(s) including one of the separate elements to be used in a method described herein. Suitable containers include, for example, bottles, vials, syringes, and test tubes. In other embodiments, the containers are formed from a variety of materials such as glass or plastic.

The articles of manufacture provided herein contain packaging materials. Packaging materials for use in packaging pharmaceutical products presented herein. See, e.g., U.S. Patent Nos. 5,323,907, 5,052,558 and 5,033,252. Examples of pharmaceutical packaging materials include blister packs, bottles, tubes, inhalers, pumps, bags, vials, containers, syringes, bottles, and any packaging material suitable for a selected formulation and intended mode of administration and treatment. A wide array of formulations of the compounds and formulations provided herein are contemplated as are a variety of treatments for any disease, disorder, or condition that would benefit by extended release administration of a therapeutic agent to the auris interna.

In some embodiments, a kit will typically include one or more additional containers, each with one or more of various materials (such as reagents, optionally in concentrated form, and/or devices) desirable from a commercial and user standpoint for use of a formulation described herein. Non-limiting examples of such materials include buffers, diluents, filters, needles, syringes; carrier, package, container, vial, and/or tube labels listing contents and/or instructions for use, and package inserts with instructions for use. A set of instructions will also typically be included.

In a further embodiment, a label is on or associated with the container. In yet a further embodiment, a label is on a container when letters, numbers or other characters forming the label are attached, molded or etched into the container itself; a label is associated with a container when it is present within a receptacle or carrier that also holds the container, e.g., as a package insert. In other embodiments a label is used to indicate that the contents are to be used for a specific therapeutic application. In yet another embodiment, a label also indicates directions for use of the contents, such as in the methods described herein.

In certain embodiments, the pharmaceutical formulations are presented in a pack or dispenser device which contains one or more unit dosage forms containing a compound provided herein. In another embodiment, the pack for example contains metal or plastic foil, such as a blister pack. In a further embodiment, the pack or dispenser device is accompanied by instructions for administration. In yet a further embodiment, the pack or dispenser is also accompanied with a notice associated with the container in form prescribed by a governmental agency regulating the manufacture, use, or sale of pharmaceuticals, which notice is reflective of approval by the agency of the form of the drug for human or veterinary administration. In another embodiment, such notice, for example, is the labeling approved by the U.S. Food and Drug Administration for prescription drugs, or the approved product insert. In yet another embodiment, formulations containing a compound provided herein formulated in a compatible pharmaceutical carrier are also prepared, placed in an appropriate container, and labeled for treatment of an indicated condition. The invention is defined in the appended claims. Any of the following examples that do not fall within the scope of the claims are provided for comparative purposes only.

## EXAMPLES

### Example A1 - Effects of BDNF on Noise-Induced Hearing Loss In Vivo

In some forms of hearing loss, a reduction in auditory brainstem response (ABR) wave I amplitude and loss of synaptic ribbon synapses of the cochlear inner hair cells are common features. BDNF is known to provide trophic support to spiral ganglion neurons in the cochlea and to promote their survival and synaptic integrity. Rodent models of noise exposure can be used to test the in vivo efficacy of BDNF against hearing loss, specifically wave I amplitude reduction and synaptopathy.

*BDNF in Poloxamer 407*

Poloxamer 407 gel at 16% was prepared using the cold method. In brief, a 16% w/w stock solution of poloxamer 407 was prepared by slowly adding it to a cold buffer solution (10 mM PBS, pH 7.4). Sterilization was achieved by filtration. Human recombinant BDNF was suspended with an appropriate amount of poloxamer 407 solution to reach a range of concentration of 0.05 to 5.0 mg/ml BDNF.

*Noise-induced hearing loss/ synaptopathy*

Male rats (Charles River) approximately 10-11 months of age served as subjects (N = 6 per group). Prior to any procedures, animals were anesthetized using a combination of xylazine (10 mg/kg) and ketamine (90 mg/kg) for up to one hour via the intraperitoneal route. If needed, an intraoperative booster was administered intraperitoneally representing one-tenth of the original dose.

*Noise exposure* - Anesthetized animals (4 at a time) were placed in an enclosure and exposed to noise. The characteristics of the acoustic trauma were as follows: a single period of 1h, sound pressure level of 105 dB SPL with a narrow band pass of 8-16 kHz. Noise was delivered using a Grason Stadler white noise generator, filtered through a Krohn Hite 3750 unit, amplified using a Crown D75 unit, and conveyed via a JBL speaker (model 2446H). Following treatment, the animals were returned to the vivarium. Noise exposure was calibrated over a range of 80-115 dB SPL across a wide range of frequencies using the Acoustical Interface Precision Microphone System and the Tucker Davis Technologies system.

*Intratympanic injection* - Each animal was positioned so that the head was tilted at an angle to favor injection towards the round window niche. Briefly, under visualization with an operating microscope, 20 μL of the formulation was injected using a 25G (Gauge) 1½ inch needle through the tympanic membrane into the superior posterior quadrant. Formulations were delivered using a perfusion pump at the rate of 2 μL/sec. Contact with the round window membrane was maintained for 30 minutes by placing the animal in a recumbent position. During the procedure and until recovery, animals were placed on a temperature controlled (40 °C) heating pad until consciousness was regained at which time they were returned to the vivarium.

*Auditory Brainstem Response (ABR) assessment (wave* 1) - During the procedure, additional anesthetic (xylazine and ketamine) was administered if needed to maintain the depth of anesthesia sufficient to insure immobilization and relaxation. ABRs were recorded in an electrically and acoustically shielded chamber, one ear at a time. Needle electrodes were placed at the vertex (active) and immediately below the pinna of the test ear (reference) and contralateral ear (ground). Tucker Davis Technologies (TDT) System III hardware and SigGen/BioSig software (TDT) were used to present the stimulus and record the ABR responses. Tones were delivered through a Tucker-Davis open-field ES1 driver placed 5 cm above the animal's ear. Acoustic calibration was performed with TDT software (SigCal) and thresholds were expressed as dB SPL in conditions identical to that of threshold recordings in animals. Stimulus presentation (15 ms tone bursts, with 1 ms rise/fall times) were presented 10 per second. Up to 512 responses were averaged for each stimulus level. Responses were collected for stimulus levels in 5 dB decrement steps at 4 frequencies: 4 kHz, 10 kHz, 20 kHz and 40 kHz. Thresholds were interpolated between the lowest stimulus level where a response was observed, and 5 dB lower, where no response was observed. The threshold was then reported as the mean value between these two stimuli conditions. Wave 1 was quantified by averaging the wave 1 amplitude from intensities ranging between 60-80 dB SPL.

*Tissue collection* - At 28 days post-IT injection, animals were sacrificed as follows. Animals were anesthetized with a combination of xylazine (10 mg/kg) and ketamine (90 mg/kg). Anesthetized animals were then given a lethal dose of anesthesia via cardiac puncture. Once dead, animals were immediately decapitated, the temporal bones were isolated, and the middle ear bullae were opened to expose the cochleae. The cochleae were then perfused directly with fixative as follows. A small gauge needle was used to puncture the round window membrane and the bone within the oval window of the cochlea. A small hole was drilled into the apical turn of the cochlea, and 1-2 mls of cold PFA were slowly perfused through the apical hole. Perfused cochleae were then immersed in 4% PFA and post-fixed for 2-6 hrs. Cochleae were then rinsed in PBS and stored in fresh PBS at 4°C.

*Cochlear Decalcification/Dissection* - Fixed cochleae were decalcified in a large volume of 10% EDTA in PBS at

room temperature with mild shaking for 72 hours. After decalcification, samples were rinsed in PBS, then carefully dissected to remove the residual bone from the tissue so that just the cochlear duct attached to the modiolar core remained. Dissected tissue was then permeabilized in 0.5% PBS-Triton (PBS-T) at room temperature for a minimum of 3 hours.

*Immunohistochemistry* - Fixed and dissected cochleae were incubated in PBS-T with 10% normal goat serum with the following primary antibodies overnight at 4°C with nutation: CtBP2 (1:250; mouse monoclonal IgG1, BD Biosciences), GluR2 (1:2000; mouse monoclonal IgG2a, Millipore), MyosinVIIa (1:1000; rabbit polyclonal, Proteus Biosciences). Cochleae were then rinsed in PBS-T and incubated in the appropriate fluorescent secondary antibodies (1:1000; anti-mouse IgG1-546, anti-mouse IgG2a-488, anti-rabbit IgG-633) in PBS-T with 10% goat serum for 2 hrs at room temperature. Cochleae were then rinsed in PBS, stained with DAPI to label nuclei then finely dissected in PBS and flat-mounted on glass slides with anti-fade mounting medium (Southern Biotech).

*Synaptic Analysis* - Flat-mounted cochlear pieces from each ear were imaged at low magnification (10x) and the total length of the imaged tissue was measured and the frequency domains (4, 10, 20 and 40 kHz) were mapped onto the images. High-magnification confocal Z-stack images were then acquired using a Zeiss LSM880 laser scanning confocal microscope at each specific frequency at a minimum of 0.35 μm steps such that the inner hair cells were fully imaged from their basal pole to the cuticular plate. Images included at least 10 contiguous inner hair cells per frequency domain. The number of aligned pre/post-synaptic puncta was then counted for each inner hair cell per region and the average number of puncta per inner hair cell per frequency domain was calculated.

*Study design*

Animals were exposed to noise on Day 0. On Day 1 (24 hrs later) animals received a single unilateral intratympanic administration of vehicle (Poloxamer 407) or the BDNF formulation (0.005% BDNF in Poloxamer 407). ABR threshold and ABR wave I amplitude were measured weekly in live animals for up to 4 weeks. Synaptic counts were performed on processed tissue obtained from animals sacrificed on Day 28. Naïve animals were not exposed to noise, nor did they receive an intratympanic injection.

**FIG. 2** shows the visualization of synapses on hair cells. **FIG. 3A** shows that treatment with sustained release BDNF formulations leads to improved hearing function (wave I amplitude) in noise exposed adult rats compared to vehicle treatment. **FIG. 3B** shows that treatment with sustained release BDNF formulations leads to an increased number of ribbon synapses per inner hair cell in noise exposed adult rats compared to vehicle treatment.

**Example A2 - Sustained Release of BDNF in Poloxamer and MCT Formulations**

*BDNF in Poloxamer 407*

Poloxamer 407 solution at 32% was prepared by slowly adding it to a cold buffer solution (50 mM tris buffer with saline, pH 7.4). Sterilization was achieved by filtration. Human recombinant BDNF was either diluted or concentrated with the same tris buffer solution to a concentration twice the target concentration. The solutions were sterilized by filtration. Combining the above two solutions at 1:1 ratio yields a solution or a suspension containing 16% P407 and a concentration range of 0.005% to 0.5% BDNF.

*BDNF/PVP (K25) in MCT SiO2*

BDNF/PVP (2% or 10%) in MCT/SiO2 (0.5%) formulations were prepared as follows. PVP was dissolved in water for injection at 2 or 10% concentration. The BDNF frozen solution was thawed at 5°C overnight and was then diluted with water for injection to a concentration of 0.15% BDNF. Combining the above two solutions at a ratio of 1:1 (w/w) yields a solution with 0.075% BDNF and 1% or 5% PVP. The solution was sterile filtered via a 0.22 um filter, filled into vials and then lyophilized. The dry powder was then dispersed into sterile filtered MCT by bead-blasting. Finally, the heat sterilized SiO2 was added. The final suspension contains 0.15% BDNF, 2 or 10% PVP and 0.5% SiO2 in MCT.

*BDNF/Carbopol (971P) in MCT/SiO2*

BDNF/Carbopol (0.15%) in MCT/SiO2 (0.5%) formulation was prepared as follows. Carbopol was dissolved in water for injection at 0.04% concentration. The BDNF frozen solution was thawed at 5°C overnight and was then diluted with water for injection to a concentration of 0.04% BDNF. Combining the above two solutions at a ratio of 1:1 (w/w) yields a solution with 0.02% BDNF and 0.02% Carbopol. The solution was sterile filtered via a 0.22 um filter, filled into vials and then lyophilized. The dry powder was then dispersed into sterile filtered MCT by bead-blasting. Finally, the heat sterilized SiO2 was added. The final suspension contains 0.15% BDNF, 0.15% Carbopol and 0.5% SiO2 in MCT. *BDNF/Poloxamer (P407) in MCT/SiO2*

BDNF/P407 (10%) in MCT/SiO2 (0.5%) formulation was prepared as follows. P407 was dissolved in water for injection at10% concentration. The BDNF frozen solution was thawed at 5C overnight and was then diluted with water for injection to a concentration of 0.15% BDNF. Combining the above two solutions at a ratio of 1:1 (w/w) yields a solution with 0.075% BDNF and 5% P407. The solution was sterile filtered via a 0.22 um filter, filled into vials and then lyophilized. The dry powder was then dispersed into sterile filtered MCT by bead-blasting. Finally, the heat sterilized SiO2 was added. The final suspension contains 0.15% BDNF, 10% P407 and 0.5% SiO2 in MCT.

*Pharmacokinetics*

Female rats (Charles River) weighing 200-300 g of approximately 12-16 weeks of age served as subjects (N = 4 per group). Prior to any procedures, animals were anesthetized using a combination of xylazine (10 mg/kg) and ketamine (90 mg/kg) for up to an hour via the intraperitoneal route. If needed, an intraoperative booster was administered intraperitoneally representing a one-tenth of the original dose.

*Intratympanic injection* - Each animal was positioned so that the head was tilted at an angle to favor injection towards the round window niche. Briefly, under visualization with an operating microscope, 20 μL of the formulation was injected using a 25G (Gauge) 11/2 needle through the tympanic membrane into the superior posterior quadrant. Formulations were delivered using a perfusion pump at the rate of 2 μL/sec. Contact with the round window membrane was maintained for 30 minutes by placing the animal in a recumbent position. During the procedure and until recovery, animals were placed on a temperature controlled (40 °C) heating pad until consciousness was regained at which time they were returned to the vivarium.

*Perilymph sampling procedure* - The skin behind the ear of anesthetized rats was shaved and disinfected with povidone-iodine. An incision was then made behind the ear, and muscles were carefully retracted from over the bulla. A hole was drilled through the bulla using a dental burr so that the middle ear was exposed and accessed. The cochlea and the round window membrane were visualized under a stereo surgical microscope. The basal turn of bulla was cleaned by using small cotton ball. A unique microhole was hand drilled through the bony shell of the cochlea (cochlear capsule) adjacent to the round window. Perilymph (about 2 μL) was then collected using a microcapillary inserted into the cochlear scala tympani. Perilymph samples were added to a vial containing 18 μL of acetonitrile/water (50/50, v/v), stored at -80 °C until analysis.

*Analytical method*

Concentrations of BDNF in perilymph and samples were determined using commercially available ELISA kits. The limits of detection of human BDNF were 4 pg/mL.

**FIG. 4** shows the perilymph concentrations of BDNF following a single IT injection in rats in poloxamer 407 formulation. BDNF was administered at the indicated doses. Perilymph BDNF is in arbitrary units.

**FIG.5A, 5B,** and **5C** show perilymph concentrations of BDNF following a single IT injection in rats in MCT formulations. BDNF was administered at a dose of 0.15% (1.5 mg/ml) in the MCT formulation containing different polymers: PVP (polyvinylpyrrolidone) at 2 or 10% **(FIG. 5A);** 0.15% carbomer **(FIG. 5B)**; or P407 (poloxamer 407) at 10% **(FIG.5C).** Perilymph BDNF is in arbitrary units.

**Example B1 - Preparation of a Thermoreversible Gel Formulation**

**Table A. Thermoreversible Gel Growth Factor Otic Formulation**

| Ingredient | Concentration in 1000mL aqueous solution |
|---|---|
| Growth Factor | 0.001-10 (wt%) |
| Polyoxyethylene-polypropylene triblock copolymer (e.g. Poloxamer 407) | 14 - 21 (wt%) |
| pH adjusting agent (e.g. HCl) | q.s. for pH= 5.5-8.0 |
| Sterile water | q.s. to 1000mL |

An exemplary batch of gel formulation containing, for example, 1.5% of a growth factor described herein is prepared by dissolving Poloxamer 407 (BASF Corp.) in 50 mM Tris buffer and 77 mM NaCl solution with a pH between 5.5-8.0. The appropriate amount of the growth factor is added and the formulation is mixed until a homogenous suspension

is produced. The mixture is maintained below room temperature until use.

**Example B2 - In Vitro Comparison of Gelation Temperature**

The effect of Poloxamer 188 and any one of the growth factors described herein on the gelation temperature and viscosity of Poloxamer 407 formulations is evaluated with the purpose of manipulating the gelation temperature. In some embodiments, the growth factor is BDNF.

A 25% Poloxamer 407 stock solution in PBS buffer and Poloxamer 188NF from BASF are used. An appropriate amount of the growth factor is added to the solutions described in Table B to provide a 2% formulation of the growth factor.

A PBS buffer (pH 7.3) is prepared by dissolving 805.5 mg of sodium chloride (Fisher Scientific), 606 mg of sodium phosphate dibasic anhydrous (Fisher Scientific), 247 mg of sodium phosphate monobasic anhydrous (Fisher Scientific), then QS to 200g with sterile filtered DI water.

**Table B. Preparation of Samples Containing Poloxamer 407/Poloxamer 188**

| Sample | 25%P407 Stock Solution (g) | Poloxamer 188 (mg) | PBS Buffer (g) |
|---|---|---|---|
| 16%P407/10%P188 | 3.207 | 501 | 1.3036 |
| 17%P407/10%P188 | 3.4089 | 500 | 1.1056 |
| 18%P407/10%P188 | 3.6156 | 502 | 0.9072 |
| 19%P407/10%P188 | 3.8183 | 500 | 0.7050 |
| 20%P407/10%P188 | 4.008 | 501 | 0.5032 |
| 20%P407/5%P188 | 4.01 | 256 | 0.770 |

Gelation temperature of the above formulations are measured using procedures described herein.

An equation is fitted to the data obtained and is utilized to estimate the gelation temperature of F127/F68 mixtures (for 17-20% F127 and 0-10% F68).

$$T_{gel} = -1.8\ (\%F127) + 1.3\ (\%F68) + 53$$

An equation is fitted to the data obtained and can be utilized to estimate the Mean Dissolution Time (hr) based on the gelation temperature of F127/F68 mixtures (for 17-25% F127 and 0-10% F68), using results obtained in examples above:

$$MDT = -0.2\ (T_{gel}) + 8\ .$$

**Example B3 - Preparation of Medium Chain Triglyceride Formulations**

Formulations 1, 2, and 3 are prepared with the appropriate amounts of a growth factor and medium chain triglycerides (CRODAMOL, GTCC-LQ-(MV), PhEur) as shown in the below table (Table C).

The formulations are prepared by adding the target weight percentage of any one of the growth factors described herein to the appropriate amount of medium chain triglyceride for a total volume of about 100 mL. The formulations are mixed until complete dissolution. The formulations are then sterilized by passing the formulations through 0.22 $\mu$m sterilizing grade filters under aseptic conditions. The sterilized solutions are then filled into vials or pre-filled syringes, which were then used to test the formulations.

**Table C**

| Component | Formulation 1 | Formulation 2 | Formulation 3 |
|---|---|---|---|
| Growth Factor | 0.01 wt % | 0.15 wt% | 0.5 wt% |
| CRODAMOL, GTCC-LQ-(MV), PhEur | QS to 100 mL | QS to 100 mL | QS to 100 mL |

**Example B4 - Additional Preparation of Medium Chain Triglyceride Formulations - Growth Factor**

Formulations 4, 5, and 6 are prepared with the appropriate amounts of the growth factor and medium chain triglyceride (CRODAMOL, GTCC-LQ-(MV), PhEur) as shown in the below table (Table D).

The formulations are prepared by adding the target weight percentage of the growth factor to the appropriate amount of medium chain triglyceride. The formulations are mixed until complete dissolution. The formulations are then sterilized by passing the formulations through 0.22 μm sterilizing grade filters under aseptic conditions. The sterilized solutions are then filled into vials or pre-filled syringes, which are then used to test the formulations.

**Table D**

| Component | Formulation 4 | Formulation 5 | Formulation 6 |
|---|---|---|---|
| Growth Factor | 1 wt% | 5 wt% | 10 wt% |
| CRODAMOL, GTCC-LQ-(MV), PhEur | QS to 100 mL | QS to 100 mL | QS to 100 mL |

**Example B5 -Preparation of Medium Chain Triglyceride Formulations**

Medium chain triglyceride formulations are prepared with the appropriate amount of growth factor, medium chain triglyceride, and viscosity modulating agents as shown in the below tables (Tables E-H). In some embodiments, the formulations further comprise cholesterol as shown in the below tables (Tables I-L). Also in some instances, the medium chain triglyercide as shown in below tables is replaced with a mixture of mixture of long-chain triglyceride and medium-chain triglycerides (0.1:99.9 to 99.9:0.1),

*Growth Factor Solution in MCT*

The formulation is prepared by dissolving the appropriate amount of a growth factorand one or more than one of the viscosity modulating agents, such as PVP, carbomer, and P407, in water for injection and sterile filtering the solution. The sterilized solution is lyophilized to form the dry cake. The appropriate amount of the dry cake is aseptically added to the appropriate amount of sterile filtered medium chain triglyceride. The formulation is mixed until a uniform suspension is achieved. If needed, the suspension is homogenized to reduce the particle size to below 10 microns (D50). Then the appropriate amount of sterilized silicon dioxide is added to the suspension, if needed. The final formulation is mixed until a uniform suspension is achieved and then is filled into vials.

*Growth Factor Suspension in MCT and SiO2*

The formulation is prepared by adding the target weight percentage of a growth factor that has been micronized and gamma irradiated to the appropriate amount of medium chain triglyceride that has been sterilized via filtration. The formulation is mixed until a uniform suspension is formed. The appropriate amount of SiO2 is then added and is mixed until uniform. The resulting uniform suspension is then filled into vials.

*Growth Factor Nano-Suspension in MCT and SiO2*

The formulation is prepared by adding the target weight percentage of a growth factor that has been micronized and gamma irradiated to the appropriate amount of medium chain triglyceride that has been sterilized via filtration. The formulation is mixed until a uniform suspension is formed. Ball milling equipment is then used to reduce the particle size to below 0.2 μm. The appropriate amount of SiO2 is then added and is mixed until uniform. The resulting uniform suspension is then filled into vials.

**Table E**

| Component | Amount (wt%) |
|---|---|
| Growth Factor | 0.0001%-10% |
| Medium Chain Triglyceride | QS to 100 mL |

**Table F**

| Component | Amount (wt%) |
|---|---|
| Growth Factor | 0.0001%-10% |
| Medium Chain Triglyceride | QS to 100 mL |
| SiO2 | 0.01%-10% |

**Table G**

| Component | Amount (wt%) |
|---|---|
| Growth Factor | 0.0001%-10% |
| Medium Chain Triglyceride | QS to 100 mL |
| PVP, P407, or carbomer | 0.01-20% |

**Table H**

| Component | Amount (wt%) |
|---|---|
| Growth Factor | 0.0001%-10% |
| Medium Chain Triglyceride | QS to 100 mL |
| SiO2 | 0.01%-10% |
| PVP, P407, or carbomer | 0.01%-20% |

**Table I**

| Component | Amount (wt%) |
|---|---|
| Growth Factor | 0.0001%-10% |
| Cholesterol | 0.01%-20% |
| Medium Chain Triglyceride | QS to 100 mL |

**Table J**

| Component | Amount (wt%) |
|---|---|
| Growth Factor | 0.0001%-10% |
| Medium Chain Triglyceride | QS to 100 mL |
| SiO2 | 0.01%-10% |
| Cholesterol | 0.01%-20% |

**Table K**

| Component | Amount (wt%) |
|---|---|
| Growth Factor | 0.0001%-10% |
| Medium Chain Triglyceride | QS to 100 mL |
| PVP, P407, or carbomer | 0.01-20% |
| Cholesterol | 0.01%-20% |

**Table L**

| Component | Amount (wt%) |
|---|---|
| Growth Factor | 0.0001%-10% |

(continued)

| Component | Amount (wt%) |
|---|---|
| Medium Chain Triglyceride | QS to 100 mL |
| SiO2 | 0.01%-10% |
| PVP, P407, or carbomer | 0.01%-20% |
| Cholesterol | 0.01%-20% |

**Example C-Survival of Spiral Ganglion Neurons**

Spiral ganglion neurons (SGNs) were harvested from P1-P3 rates, dissociated, and cultured in 96-well plates. The SGNs were treated with neurotrophins and/or antibodies at various concentrations. After about 24 hours, media was exchanged to serum-free media and the neurotrophins and/or antibodies were replenished. Cells were cultured for an additional 48-72 hours before being fixed using 4% paraformaldehyde (PFA) and stained using 4',6-diamidino-2-phenylindole (DAPI). **FIG. 6A** schematically depicts this experimental scheme, while **FIG. 6B** shows neuronal cell bodies and neurites stained with neurofilament (green) and nuclei stained with DAPI. Surviving neurons were identified as cells with intact soma and at least one neurite.

**FIG. 6C** shows SGN survival normalized to 1 nanomolar (nM) of NT-3 for treatment with neurotrophins and antibodies at various concentrations including 1 nM NT-3, 10 nM BDNF, 10 nM M1, 10 nM M2, 10 nM M3, 10 nM M4, 10 nM M5, 10 nM M6, 10 nM M7, 10 nM mIgG1, 10 nM hIgG4, 1 μM LM22b10, and 0.01% DMSO. As shown in the figure, the number of surviving SGNs was increased when cultured with neurotrophins or M-antibodies, compared to when treated with mouse IgG1 and human IgG4 iso-type controls. Survival of SGNs was not improved with LM22b10 compared to a vehicle-only control, 0.01% DMSO.

**FIG. 6D** shows SGN survival normalized to 1 nM of NT-3 for treatment with neurotrophins and antibodies at various concentrations. As shown in the figure, normalized SGN survival was comparatively lower for M1, M2, M6, and M7 at all concentrations.

**Example D-TrkB/TrkC Agonists Promote SGN Complexity (Dissociated SGNs)**

SGNs were immunostained, imaged, and batch analyzed with PerkinElmer Harmony 4.6 software. Changes in the complexities of SGNs upon treatment with various agents were examined. **FIG. 7A** shows SGNs immunostained for neurofilament and DAPI and imaged at 20x. All of the images in a single well were stitched together and analyzed. SGN somas are shown in the upper right panel and SGN neurites are shown in the lower left panel. As shown in the lower right panel, each neurite tree is assigned to a single cell for analysis. Depending on the stitching, occasionally neurites will not meet the criteria due to gaps that exceed a user-defined threshold. **FIG. 7B** shows roots (indicated with asterices), extremeties (indicated with white dots), nodes (indicated with arrows), segments (indicated with discrete sections of neurite between nodes or extremities), and total neurite length measured for each neuron. **FIG. 7C** shows the percentage of SGNs having various numbers of roots upon treatment with 10 nM BDNF, 1 nM NT-3, or 1 nM M3 compared to the IgG-treated control (*p<0.05, **p<0.005, and p<0.001). **FIG. 7D** shows total neurite length (left panel) as well as total number of extremities, segments, and nodes per cell upon treatment with BDNF, NT-3, or M3. These differences were not statistically significant.

**Example E-Neurite Outgrowth (SGN Explants)**

Treatment with various agents may promote neurite extension in SGN explants. SGNs were harvested from P7-8 rats, cut into ~1 millimeter (mm) sections and cultured in 96-well plates coated with human fibronectin. After about 24 hours, media was exchanged to serum-free media, and neurotrophins and/or antibodies were added at various concentrations. Tissues were cultured for an additional 48-72 hours and then fixed with 4% PFA and immunostained. **FIG. 8A** schematically depicts this experimental scheme, while **FIG. 8B** shows stained neurofilaments for samples treated with no neurotrophin/antibody (upper left image), 10 nM BDNF (upper right image), 10 nM NT-3 (middle left image), 100 nM NT-3 (middle right image), 10 nM M3 (lower left image), and 100 nM M3 (lower right image).

**FIGs. 8C** and **8D** show neurites per tissue at various concentrations of NT-3 **(FIG. 8C)** and BDNF **(FIG. 8D). FIG. 8E** shows neurites per tissue at various concentrations of hIgG4 (left) and M3 (right). In **FIGs. 8C-8E,** * indicates p<0.05, ** indicates p<0.005, and *** indicates p<0.0005 vs untreated.

As shown in **FIG. 8F,** explants were imaged and analyzed individually using Harmony analysis software to examine neurite length, nodes (arrows), segments (arrowheads), and the number of extremities (asterices). **FIG. 8G**

shows the effect of BDNF, NT-3, and M3 on neurite complexity of SGN explants compared to IgG-treated or untreated controls.

**Example F-Synaptopathy Model (Cochlear Explants)**

Cochlear synaptopathy is a primary pathology of various otic insults, including aggeg-related hearing loss. It is characterized by the loss of afferent synapses, the synaptic connections between inner hair cells and type I spiral ganglion neurons. Cochlear synaptopathy in humans has been implicated in "hidden hearing loss," i.e., speech-in-noise hearing difficulties.

Neurotrophins such as BDNF and NT-3 and their mimetics establish afferent synapses during development, and disruption of BDNF, NT-3, or their receptors (TrkB and TrkC) affects afferent synapses and hearing function.

Neurotrophin and/or antibody treatment may be combined with excitotoxin treatment. An excitotoxin is a chemical such as an amino acid that overstimulates neuron receptors, causing impulses to be fired at such a rate as to exhaust the neuron receptors.

*Restoration of SGN fibers.* P2-P3 rat cochleae were established and then exposed to excitotoxin (NK: 0.5 mM NMDA + 0.5 mM kainate) for about 2 hours. Neurotrophins and/or antibodies (e.g., Trk agonists) were added in various concentrations after excitotoxin was removed. Explants were cultured for an addittional 18 or 72 hours before being fixed with 4% PFA and immunostained. **FIG. 9A** schematically depicts this experimental scheme. As shown in **FIG. 9B,** type I SGNs were estimated by counting the number of fibers approaching inner hair cells (IHCs; triangles) and subtracting the number of fibers passing through to the outer hair cells (OHCs; circles). **FIG. 9C** shows SGN fibers/IHC normalized to CTL for various agents for no excitotoxin and 72 hour culture (left panel) and excitotoxin treatment and 18 hour culture (right panel). Trk agonists increased the number of fibers in explants.

**FIG. 9D** shows images of cochlear explants taken under various conditions. The upper left image corresponds to a control that is not treated with excitotoxin. The middle left image corresponds to a sample 72 hours post-excitotoxin treatment. The upper, middle, and lower right images correspond to samples 72-hours post excitotoxin and BDNF, NT-3, or M3 treatment, respectively. The lower left image shows post-synaptic density protein 95 (PSD-95) and C-terminal-binding protein 2 (CtBP2).

*Restoration of synapses.* Cochleae were exposed to excitotoxin and Trk agonists, as above, and then cultured for 72 hours. Synapses were stained for PSD-95, imaged, and counted. Trk agonists increased the number of synaptic puncta and restored them to control levels after excitotoxic insult.

**FIG. 9E** shows an image of cochlear explants with PSD-95, CtBP2, neurofilament (lines), and Myo7a. **FIG. 9F** shows puncta (PSD95)/IHC normalized to CTL for various Trk agonists for no excitotoxin and 72 hour culture (left panel) and excitotoxin and 72 hour culture (right panel) (*$p<0.05$ (CTL vs. NK)).

**Example G-Inner ear pharmacokinetics of BDNF in P407**

Animals: Male Sprague-Dawley rats (5-6 months of age) were used for efficacy studies and females (2-3 months) were used for pharmacokinetic studies.

<u>Formulations:</u> Human recombinant BDNF was suspended in the thermoreversible polymer Poloxamer 407 (16%) to reach a range of BDNF concentrations.

<u>Intratympanic (IT) injection:</u> Formulations were administered under anesthesia as a single IT injection (20 $\mu$l) through the tympanic membrane into the middle ear, targeting the round window membrane (RWM).

<u>Inner ear pharmacokinetics:</u> Animals (n=4 per time point) received a single unilateral intratympanic injection of the different treatment doses. BDNF levels in the perilymph were determined at the indicated times by ELISA.

<u>Noise-induced cochlear synaptopathy:</u> Rats (n= 44 per treatment group) received a single acoustic trauma (105 dB SPL, 8-16 kHz, 1h) under anesthesia. 24 hours later (Day 1) animals received a single bilateral IT injection of the treatment regimens. Auditory function (ABR and ABR wave 1 amplitude) was monitored at day 1 and then every 7 days for 28 days. At termination (Day 28), cochleae were collected and assessed for evidence of cochlear synaptopathy (IHC synaptic puncta counts).

<u>Immunohistochemistry:</u> Dissected cochleae were immunostained with antibodies against presynaptic (CtBP2) and postsynaptic (GluR2) proteins and MyosinVIIa to label hair cells. Cochleae were then frequency mapped and high-magnification images were obtained with a Zeiss LSM-880 confocal microscope and imaged at an interval of 0.35 $\mu$m.

**FIG. 10** schematically illustrates non-invasive intratympanic (IT) delivery of an agent such as BDNF to the inner ear.

*BDNF levels in the cochlear perilymph.* Adult rats received a unilateral intratympanic injection of 20 $\mu$l of BDNF/P407 formulation on Day 0. Perilymph was collected from the base of the cochlea via cochleostomy at days 1, 3, 7, and 14 and BDNF levels were quantified by ELISA. **FIG. 11** shows BDNF in perilymph (pg/ml) at various times post IT injection for various concentrations of BDNF in P407.

*Intratympanic BDNF improves Auditory Brainstem Response (ABR) thresholds and wave I amplitudes in adult rats.* The Auditory Brainstem Response (ABR) is an objective test that measures hearing sensitivity and can identify abnormalities in the peripheral auditory system and the auditory pathway up through the brainstem. Adult male rats were bilaterally exposed to noise followed by an intratympanic injection of 0.05 mg/ml BDNF in P407 24 hours after noise exposure. ABR recordings were obtained at days 1, 7, 14, and 28. BDNF-treated animals showed a more rapid recovery of initial threshold shifts and overall reduced levels of hearing loss. **FIG. 12A** shows ABR threshold shift (dB SPL) at various frequencies for P407 vehicle alone and 0.05 mg/ml BDNF in P407.

The ABR Wave I amplitude is an indicator of the strength of the distal cochlear nerve signal, i.e., the response of the spiral ganglion neurons/synapses. **FIG. 12B** shows ABR wave components (left panel) and noise-induced wave I deficits (right panel). Wave I amplitude shift at 10 kHz in the same ear at baseline prior to noise exposure (black line) and 28 days post-noise exposure. The inset dashed lines highlight the significant reduction in wave I after noise exposure. As shown in **FIG. 12C,** ABR wave I amplitudes were measured in adult male rats after BDNF or vehicle treatment following noise exposure or in naive undamaged ears. Significant improvements in wave I amplitudes were detected in BDNF-treated animals across many frequencies.

*Intratympanic BDNF repairs cochlear synaptopathy in adult rats.* Functional inner hair cell synapses are defined by colocalization of pre-synaptic and post-synaptic proteins associated with the inner hair cells and SGNs, respectively. **FIG. 13A** shows inner hair cell innervation (upper panel) and hair cell ribbon synapses (lower panel). **FIG. 13B** shows high-magnification views of inner hair cells from the 40 kHz region from adult rats after BDNF or vehicle treatment following noise damage, or in a naive undamaged cochlea. Digital X-compressed cross-sections are shown at right.

As shown in **FIG. 13C,** increased synaptic puncta (defined by co-expression of CtBp2 and GluR2) per inner hair cell were observed for each frequency region throughout the cochlea following BDNF treatment. As shown in **FIG. 13D,** the total length of the dissected cochlea was measured and then frequencies along the tonotopic axis were mapped for assessment.

## Example H-Study Design for Growth Factor Formulations

A randomized, double-blind, placebo-controlled study is performed in subjects with hearing impairment characteriezd by speench-in-noise deficits and mild-to-moderate age-related hearing loss. A single ascending is used for 4 dose level cohorts of 8 subjects each. Of the 8 subjects of each dose level cohort, 6 were assigned a therapeutic agent and 2 were assigned a placebo. The study is performed to assess the safety, pharmacokinetics, and exploratory efficacy of intratympanic therapeutic composition. Study outcomes include safety (e.g., otoscopy, tympanometry, vital signs, clinical safety labs, adverse events, suicidality), plasma pharmacokinetics, synaptopathy assessments (e.g., middle ear muscle reflex, ABR Wave 1/5 ratio, envelope following response), speech-in-noise tests, and hearing handicap questionnaire. **FIG. 14** schematically depicts the scheme for this study. The therapeutic composition will be configured to otic administration and will include a growth factor and an auris acceptable vehicle such as a thermoreversible gel.

## Example I-Doses for Growth Factor Formulations

Good laboratory practice toxicology doses for otic formulations comprising a growth factor range from a low dose of about 0.05% (about 0.5 mg/ml), to a medium dose of about 0.15% (about 1.5 mg/ml), to a higher dose about 0.5% (about 5 mg/ml) of the growth factor. In some cases, the growth factor is BDNF. The remainder of the formulation includes auris acceptable vehicle such as a thermoreversible gel and optional other components, as described herein. In some cases, preparing such doses requires a 50 mg/ml growth factor (e.g., BDNF) concentrate. Such a concentrate may have limited stability over time.

Clinical doses range from about 0.01% to about 0.25% (e.g., about 0.1 mg/ml to about 2.5 mg/ml) of growth factor (e.g., BDNF). In some cases, preparing such clinical doses (e.g., for use in a Phase 1 or Phase 2 clinincal trial) may require a 10X growth factor concentrate of 1 mg/ml to 25 mg/ml.

Solubility and stability of growth factor formulations are evaluated for concentrates (e.g., BDNF concentrates) between 1 mg/ml to 15 mg/ml to determine the highest usable concentrations. Solubility of growth factor (e.g., BDNF) is assessed in three buffer systems, phosphate buffer (PB), phosphate buffered saline (PBS), and Tris, to determine concentration limits of active vial concentrates.

The injection volume for an otic formulation comprising a growth factor is about 0.2 ml.

## Example J-Sustained Release of BDNF in Poloxamer Formulations (Feline PK)

*BDNF in Poloxamer 407.* Poloxamer 407 solution at 16% was prepared by slowly adding it to a cold buffer solution (50 mM tris buffer with saline, pH 7.4). Sterilization was achieved by filtration. Human recombinant BDNF was formulated as a concentrate with the same tris buffer solution to a concentration 10 X the target concentration. The solutions were

sterilized by filtration. Combining the above two solutions at 1:10 ratio yields a solution or a suspension containing 16% P407 and a concentration of 0.05% and 0.5% BDNF.

*Pharmacokinetics.* Adult male cats (Liberty Research) weighing 2-4 kg served as subjects (N = 3 per group). Prior to any procedures, animals were anesthetized using a combination of xylazine (0.11 mg/kg) and ketamine (20 mg/kg) for up to an hour via the intramuscular route. If needed, an intraoperative booster was administered intramuscularly representing a one-tenth of the original dose.

*Intratympanic injection.* Each animal was positioned so that the head was tilted at an angle to favor injection towards the round window niche. Briefly, under visualization with an operating microscope, 150 μL of the formulation was injected using a 25G (Gauge) 11/2 needle through the tympanic membrane into the superior posterior quadrant. Formulations were delivered using a perfusion pump at the rate of 2 μL/sec. Contact with the round window membrane was maintained for 30 minutes by placing the animal in a recumbent position. During the procedure and until recovery, animals were placed on a temperature controlled (40 °C) heating pad until consciousness was regained at which time they were returned to the vivarium.

*Perilymph sampling procedure.* The skin extending from the ventral neck to the sternal notch was shaved and disinfected with povidone-iodine. An incision was then made medially and parallel with the digastric muscle in the ventral aspect of the neck, which is directly over the hollow bulla (the tympanic part of the temporal bone). The skin, subcutaneous tissue, platysma, and mylohyoid muscle were incised. The bulla was then exposed by blunt dissection between the digastric, styloglossus, and hyoglossus muscles. A 2-cm-diameter access site in the bulla was made using a rotary burr tool mounted on a dental drill. The mucosa internal to the bulla was freed to allow visualization of the round window in the posterolateral wall of the bulla. The location of round window was verified by identification of the promontory and the attachment of the stapes to the oval window. A1 mm diameter hole was drilled with a diamond burr under a surgical microscope in the basal turn, and a microcapillary tube was used to collect perilymph (20 μl).

*Analysis.* Concentrations of BDNF in perilymph and samples were determined using commercially available ELISA kits. The limits of detection of human BDNF were 4 pg/mL. **FIG. 15** shows perilymph concentrations of BDNF following a single intratympanic injection in cats of BDNF poloxamer 407 formulation. BDNF was administerred at the indicated doses. Perilymph BDNF is in arbitrary units.

## Claims

**1.** An otic formulation comprising a therapeutically effective amount of a growth factor and an auris-acceptable vehicle, wherein the otic formulation is formulated to provide sustained release of the growth factor into the inner ear, wherein the auris-acceptable vehicle is a thermoreversible gel comprising poloxamer 407, wherein the otic formulation comprises from 0.0001% to 1% by weight of the growth factor, wherein the growth factor is brain-derived neurotrophic factor (BDNF), ciliary neurotrophic factor (CNTF), glial cell-line derived neurotrophic factor (GDNF), neurotrophin-3, neurotrophin-4, or any combination thereof, and wherein:

(i) the thermoreversible gel has a gelation viscosity from 15,000 cP to 3,000,000 cP;
(ii) the otic formulation has an osmolarity from 100 mOsm/L to 1000 mOsm/L; or
(iii) the otic formulation has a gelation temperature from 19°C to 42°C.

**2.** The otic formulation of claim 1, wherein growth factor is neurotrophin-3.

**3.** The otic formulation of claim 1, wherein the growth factor is brain-derived neurotrophic factor (BDNF).

**4.** The otic formulation of claim 3, wherein the otic formulation has a pH from 7.0 to 8.0.

**5.** The otic formulation of any of claims 1 to 4, wherein the otic formulation comprises from 14 wt% to 18 wt% poloxamer 407.

**6.** The otic formulation of claim 5, wherein the otic formulation comprises from 15 wt% to 17 wt% poloxamer 407.

**7.** The otic formulation of any of claims 1 to 6, wherein the otic formulation further comprises at least one viscosity modulating agent.

**8.** The otic formulation of claim 1, wherein the otic formulation comprises between 0.05% to 0.5% by weight of the growth factor.

9. The otic formulation of any of claims 1 to 8, wherein the otic formulation provides sustained release of the growth factor over a period of at least 7 days.

10. The otic formulation of any one of claims 1 to 8, wherein the otic formulation is formulated to provide sustained release of the growth factor over a period of at least 5 days.

11. The otic formulation of any of claims 1 to 10, wherein the growth factor is essentially dissolved in the otic formulation.

12. The otic formulation of any of claims 1 to 11, for use in the treatment of hearing loss.

13. The otic formulation for the use as claimed in claim 12, wherein the ribbon synapses are repaired.

**Patentansprüche**

1. Otische Formulierung, umfassend eine therapeutisch wirksame Menge eines Wachstumsfaktors und ein für die Auris annehmbares Vehikel, wobei die otische Formulierung so formuliert ist, dass sie eine verzögerte Freisetzung des Wachstumsfaktors in das Innenohr bereitstellt, wobei das für die Auris annehmbare Vehikel ein thermoreversibles Gel ist, das Poloxamer 407 umfasst, wobei die otische Formulierung von 0.0001 Gew.-% bis 1 Gew.-% des Wachstumsfaktors umfasst, wobei der Wachstumsfaktor ein vom Gehirn stammender neurotropher Faktor (BDNF), ein ziliärer neurotropher Faktor (CNTF), ein von Gliazelllinie- abgeleiteter neurotropher Faktor (GDNF), Neurotrophin-3, Neurotrophin-4 oder eine beliebige Kombination davon ist, und wobei:

   (i) das thermoreversible Gel eine Gelierviskosität von 15.000 cP bis 3.000.000 cP aufweist;
   (ii) die otische Formulierung eine Osmolarität von 100 mOsm/L bis 1000 mOsm/L aufweist; oder
   (iii) die otische Formulierung eine Geliertemperatur von 19 °C bis 42 °C aufweist.

2. Otische Formulierung nach Anspruch 1, wobei der Wachstumsfaktor Neurotrophin-3 ist.

3. Otische Formulierung nach Anspruch 1, wobei der Wachstumsfaktor ein vom Gehirn stammender neurotropher Faktor (BDNF) ist.

4. Otische Formulierung nach Anspruch 3, wobei die otische Formulierung einen pH-Wert von 7,0 bis 8,0 aufweist.

5. Otische Formulierung nach einem der Ansprüche 1 bis 4, wobei die otische Formulierung von 14 Gew.-% bis 18 Gew.-% Poloxamer 407 umfasst.

6. Otische Formulierung nach Anspruch 5, wobei die otische Formulierung von 15 Gew.-% bis 17 Gew.-% Poloxamer 407 umfasst.

7. Otische Formulierung nach einem der Ansprüche 1 bis 6, wobei die otische Formulierung ferner mindestens ein viskositätsmodulierendes Mittel umfasst.

8. Otische Formulierung nach Anspruch 1, wobei die otische Formulierung zwischen 0,05 Gew.-% und 0,5 Gew.-% des Wachstumsfaktors umfasst.

9. Otische Formulierung nach einem der Ansprüche 1 bis 8, wobei die otische Formulierung eine verzögerte Freisetzung des Wachstumsfaktors über einen Zeitraum von mindestens 7 Tagen bereitstellt.

10. Otische Formulierung nach einem der Ansprüche 1 bis 8, wobei die otische Formulierung so formuliert ist, dass sie eine verzögerte Freisetzung des Wachstumsfaktors über einen Zeitraum von mindestens 5 Tagen bereitstellt.

11. Otische Formulierung nach einem der Ansprüche 1 bis 10, wobei der Wachstumsfaktor im Wesentlichen in der otischen Formulierung gelöst wird.

12. Otische Formulierung nach einem der Ansprüche 1 bis 11 zur Verwendung bei der Behandlung von Hörverlust.

13. Otische Formulierung für die Verwendung nach Anspruch 12, wobei die Bandsynapsen repariert werden.

## Revendications

1. Formulation otique comprenant une quantité thérapeutiquement efficace d'un facteur de croissance et un vecteur acceptable pour l'oreille, dans laquelle la formulation otique est formulée pour fournir une libération prolongée du facteur de croissance dans l'oreille interne, dans laquelle le vecteur acceptable pour l'oreille est un gel thermoréversible comprenant du poloxamère 407, dans laquelle la formulation otique comprend de 0,0001 % à 1 % en poids du facteur de croissance, dans laquelle le facteur de croissance est le facteur neurotrophique dérivé du cerveau (BDNF), le facteur neurotrophique ciliaire (CNTF), le facteur neurotrophique dérivé d'une lignée de cellules gliales (GDNF), la neurotrophine-3, la neurotrophine-4, ou toute combinaison de ceux-ci, et dans laquelle :

   (i) le gel thermoréversible possède une viscosité de gélification de 15 000 cP à 3 000 000 cP ;
   (ii) la formulation otique possède une osmolarité de 100 mOsm/L à 1000 mOsm/L ; ou
   (iii) la formulation otique possède une température de gélification de 19 °C à 42 °C.

2. Formulation otique selon la revendication 1, dans laquelle le facteur de croissance est la neurotrophine-3.

3. Formulation otique selon la revendication 1, dans laquelle le facteur de croissance est le facteur neurotrophique dérivé du cerveau (BDNF).

4. Formulation otique selon la revendication 3, dans laquelle la formulation otique possède un pH de 7,0 à 8,0.

5. Formulation otique selon l'une quelconque des revendications 1 à 4, dans laquelle la formulation otique comprend de 14 % en poids à 18 % en poids de poloxamère 407.

6. Formulation otique selon la revendication 5, dans laquelle la formulation otique comprend de 15 % en poids à 17 % en poids de poloxamère 407.

7. Formulation otique selon l'une quelconque des revendications 1 à 6, dans laquelle la formulation otique comprend en outre au moins un agent de modulation de la viscosité.

8. Formulation otique selon la revendication 1, dans laquelle la formulation otique comprend entre 0,05 % et 0,5 % en poids du facteur de croissance.

9. Formulation otique selon l'une quelconque des revendications 1 à 8, dans laquelle la formulation otique fournit une libération prolongée du facteur de croissance sur une période d'au moins 7 jours.

10. Formulation otique selon l'une quelconque des revendications 1 à 8, dans laquelle la formulation otique est formulée pour fournir une libération prolongée du facteur de croissance sur une période d'au moins 5 jours.

11. Formulation otique selon l'une quelconque des revendications 1 à 10, dans laquelle le facteur de croissance est essentiellement dissous dans la formulation otique.

12. Formulation otique selon l'une quelconque des revendications 1 à 11, pour une utilisation dans le traitement de la perte auditive.

13. Formulation otique pour l'utilisation selon la revendication 12, dans laquelle les synapses à ruban sont réparées.

FIG. 1

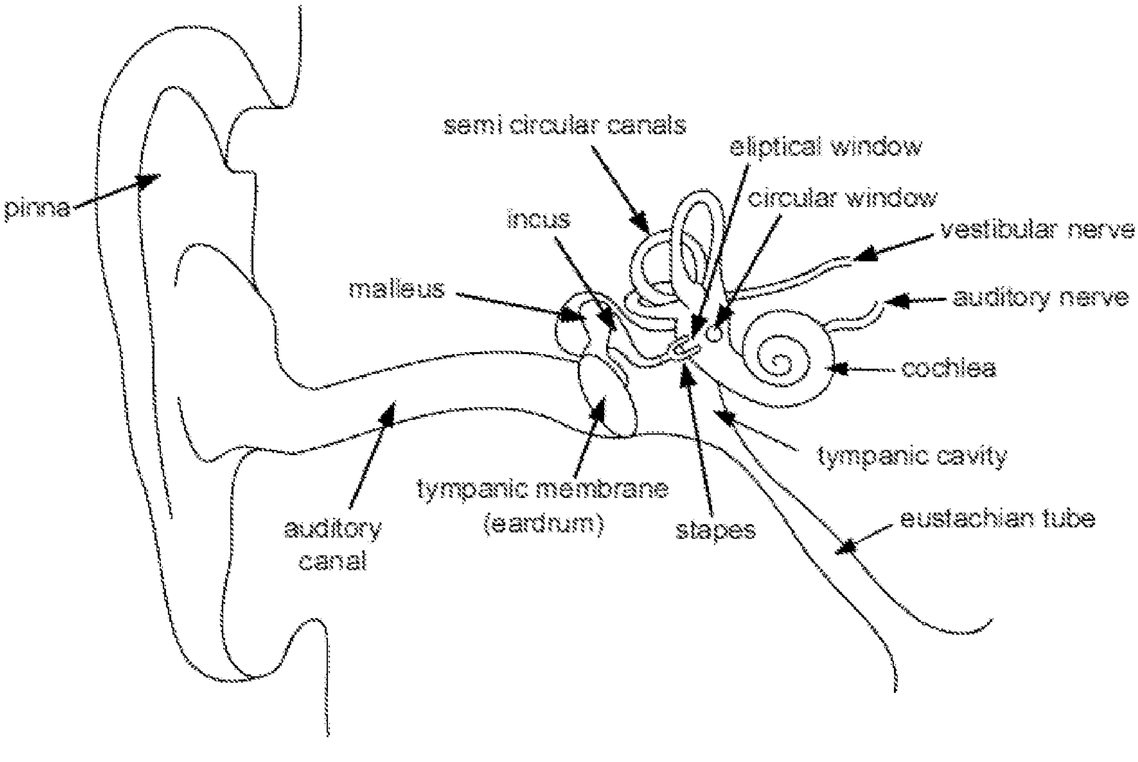
semi circular canals
eliptical window
circular window
vestibular nerve
pinna
incus
malleus
auditory nerve
cochlea
tympanic cavity
tympanic membrane
(eardrum)
auditory
canal
stapes
eustachian tube

FIG. 2

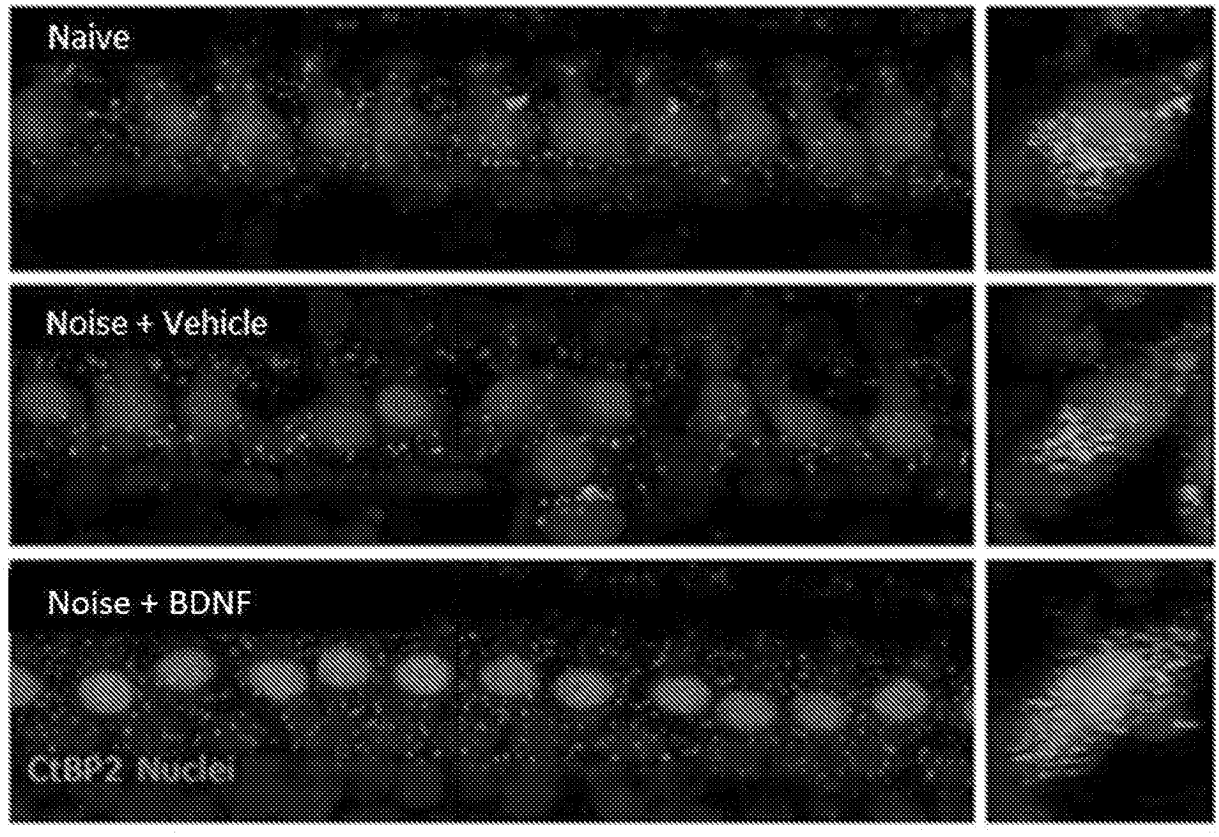

FIG. 3A

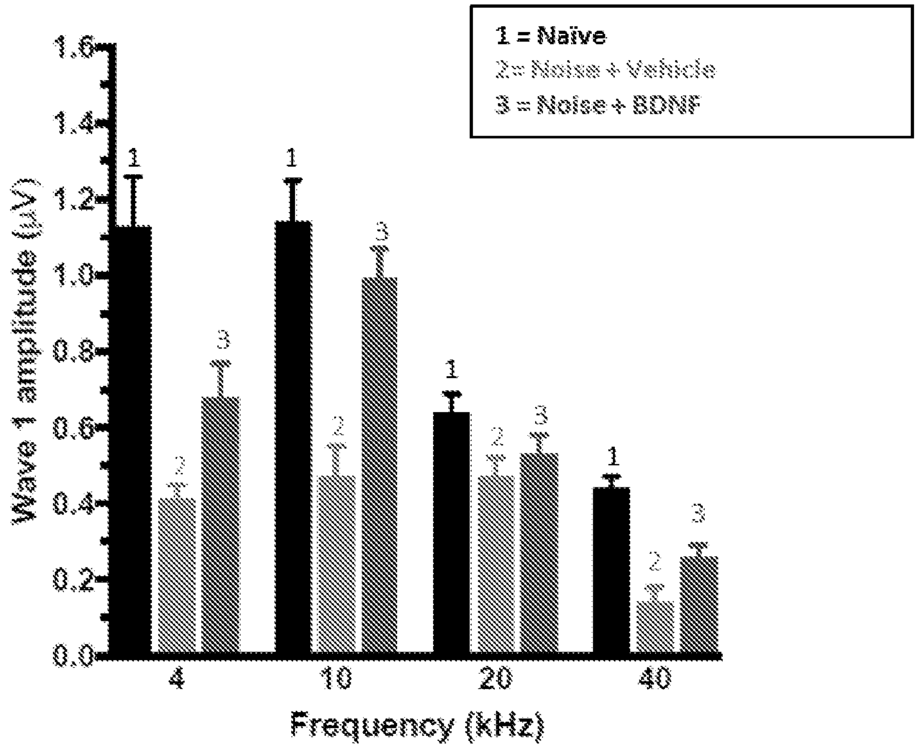
1 = Naïve
2= Noise + Vehicle
3 = Noise + BDNF
Wave 1 amplitude (μV)
1.6
1.4
1.2
1.0
0.8
0.6
0.4
0.2
0.0
4
10
20
40
Frequency (kHz)

FIG. 3B

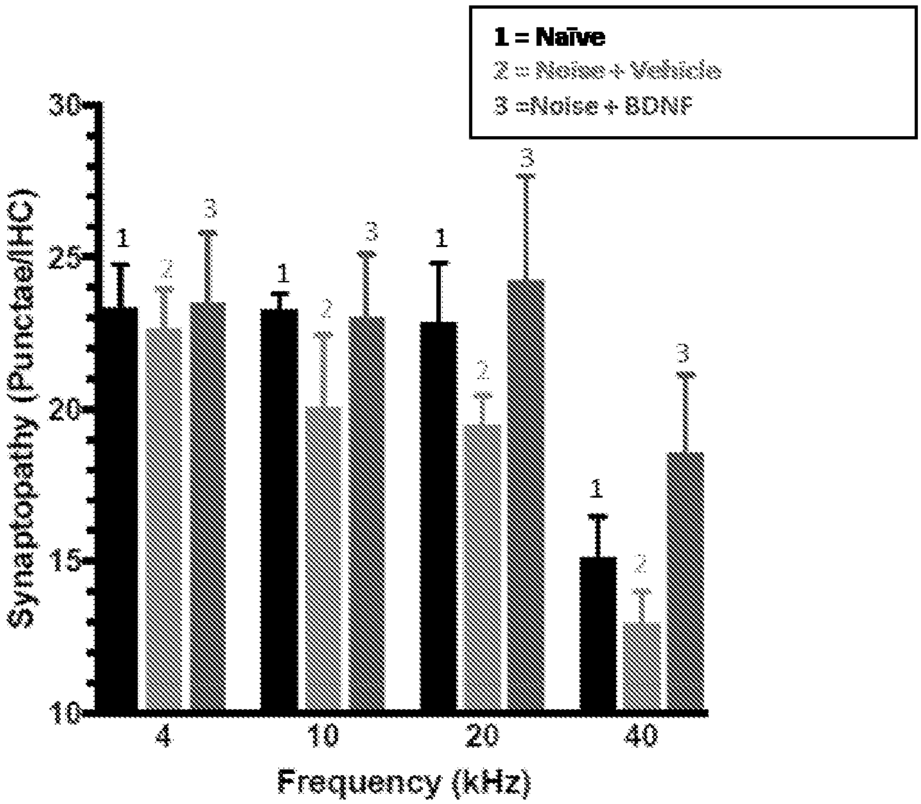
1 = Naïve
2 = Noise + Vehicle
3 =Noise + BDNF
Synaptopathy (Punctae/IHC)
30
25
20
15
10
4
10
20
40
Frequency (kHz)

FIG. 4

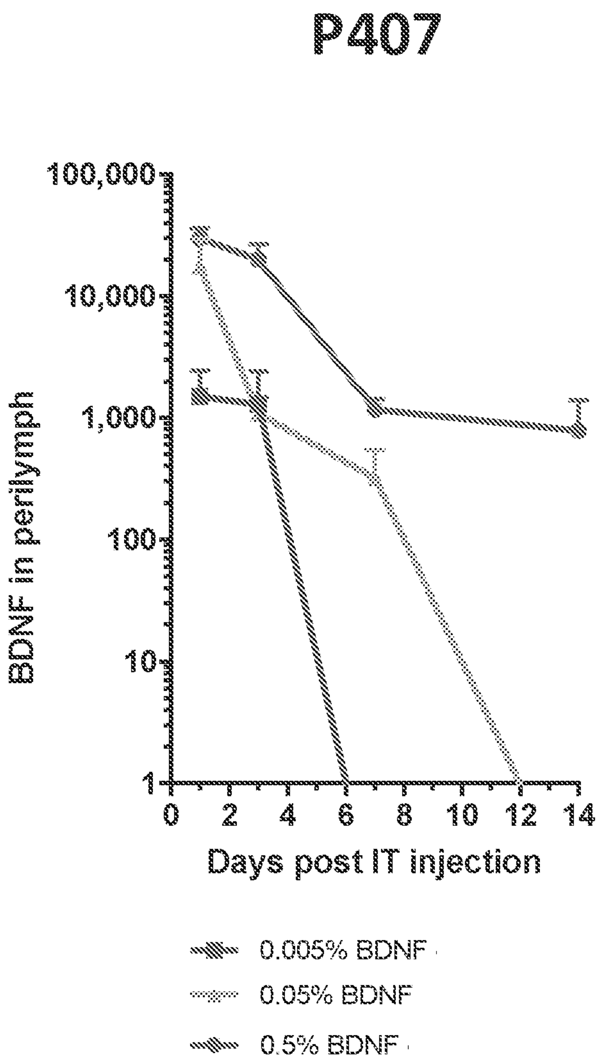
P407
100,000
10,000
1,000
100
10
1
0
2
4
6
8
10
12
14
BDNF in perilymph
Days post IT injection
0.005% BDNF
0.05% BDNF
0.5% BDNF

FIG. 5A

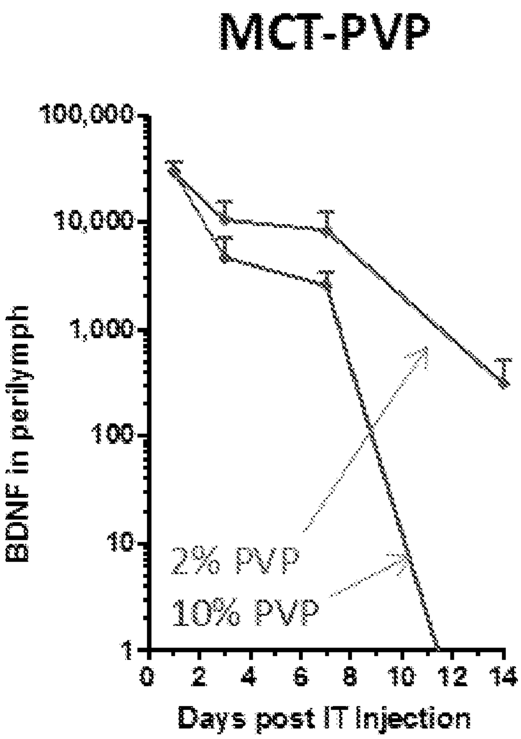
MCT-PVP
100,000
10,000
1,000
100
10
1
BDNF in perilymph
0 2 4 6 8 10 12 14
Days post IT injection
2% PVP
10% PVP

FIG. 5B

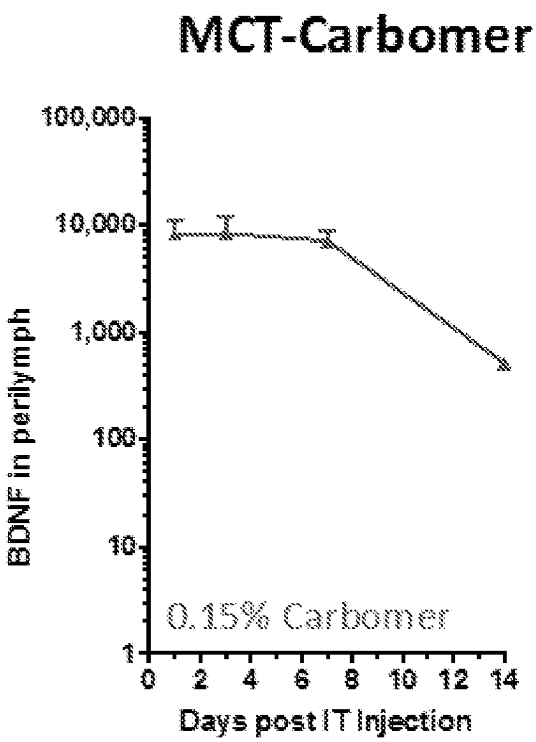
MCT-Carbomer
100,000
10,000
1,000
100
10
1
BDNF in perilymph
0 2 4 6 8 10 12 14
Days post IT injection
0.15% Carbomer

FIG. 5C

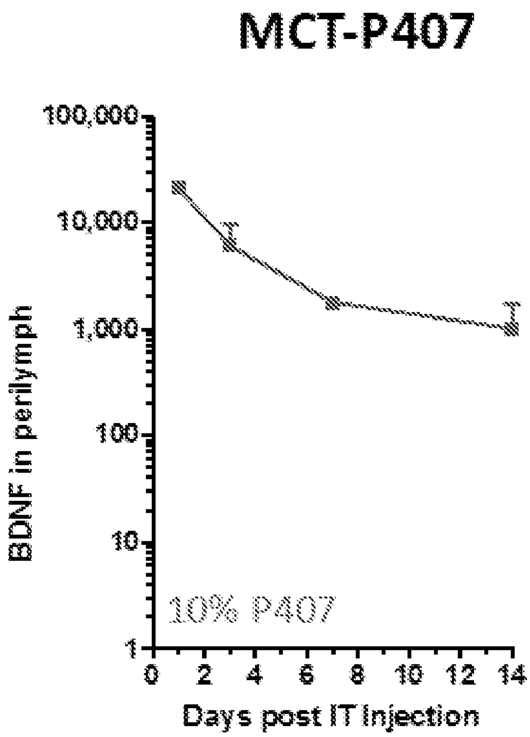
MCT-P407
100,000
10,000
1,000
100
10
1
BDNF in perilymph
10% P407
0
2
4
6
8
10
12
14
Days post IT Injection

FIG. 6A

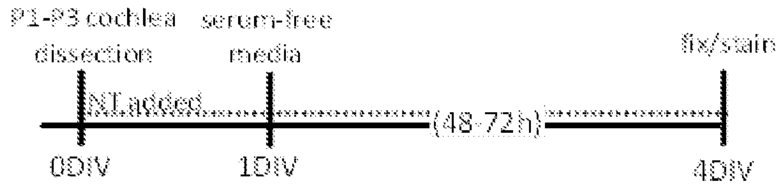


FIG. 6B

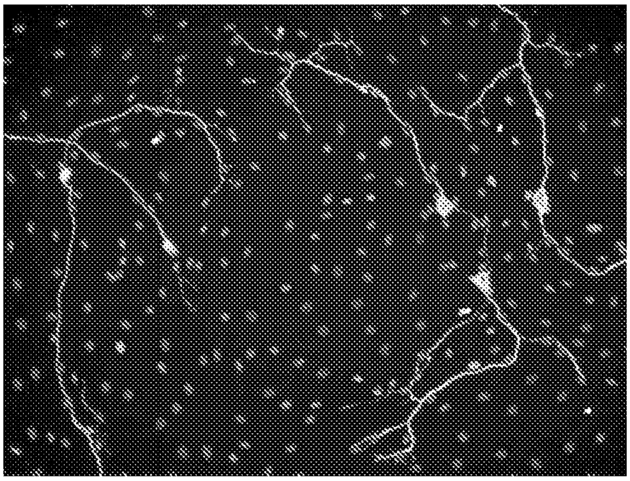

FIG. 6C

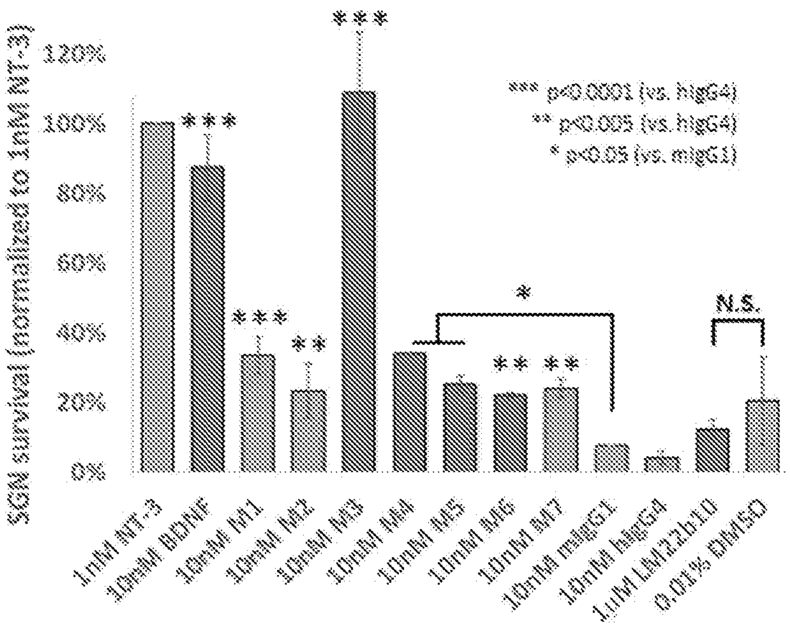

FIG. 6D

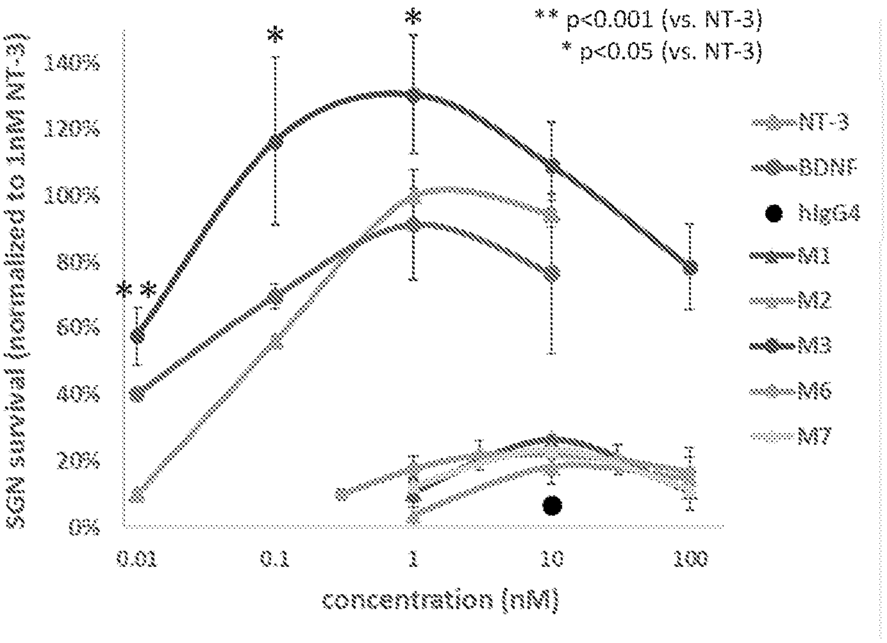
** p<0.001 (vs. NT-3)
* p<0.05 (vs. NT-3)
SGN survival (normalized to 1nM NT-3)
140%
120%
100%
80%
60%
40%
20%
0%
0.01
0.1
1
10
100
concentration (nM)
NT-3
BDNF
hIgG4
M1
M2
M3
M6
M7

FIG. 7A

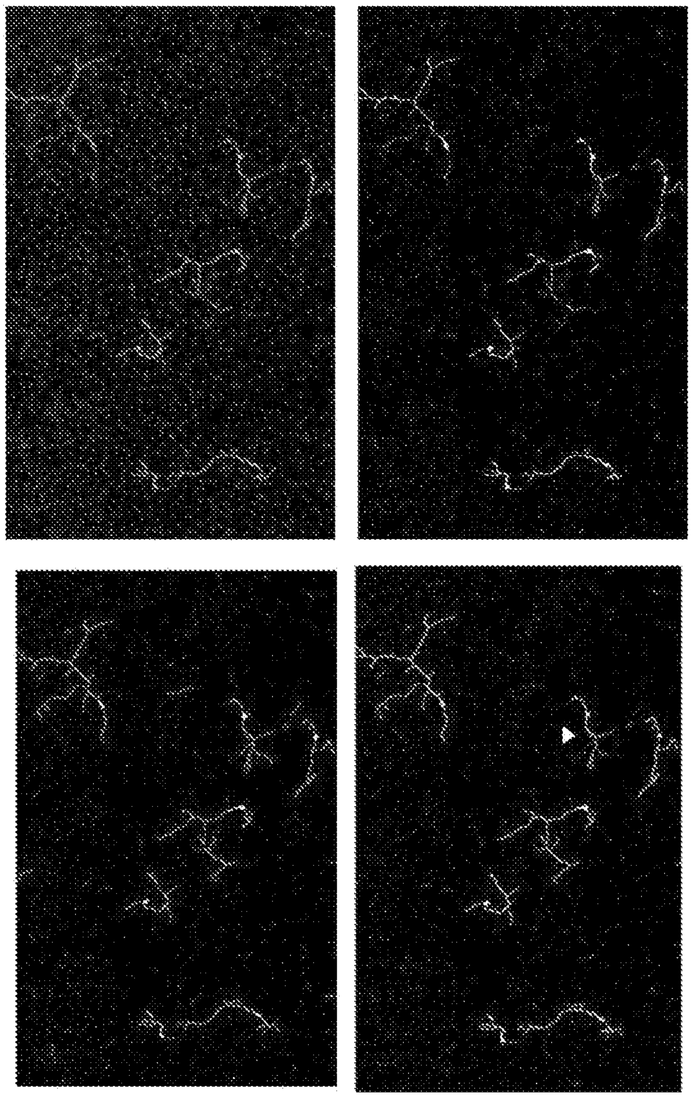

FIG. 7B

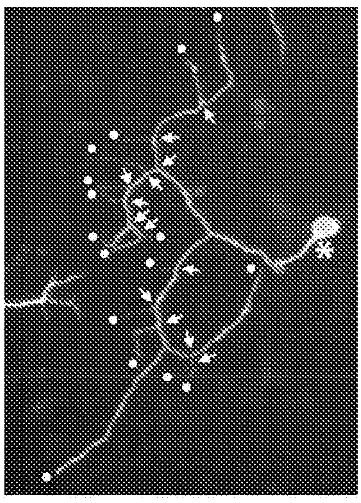

FIG. 7C

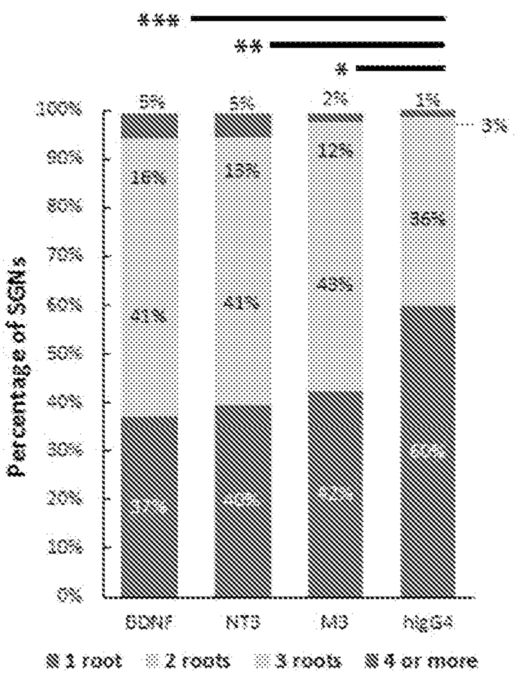
***
**
*
Percentage of SGNs
100%
90%
80%
70%
60%
50%
40%
30%
20%
10%
0%
BDNF
NT3
M3
hIgG4
1 root
2 roots
3 roots
4 or more

FIG. 7D

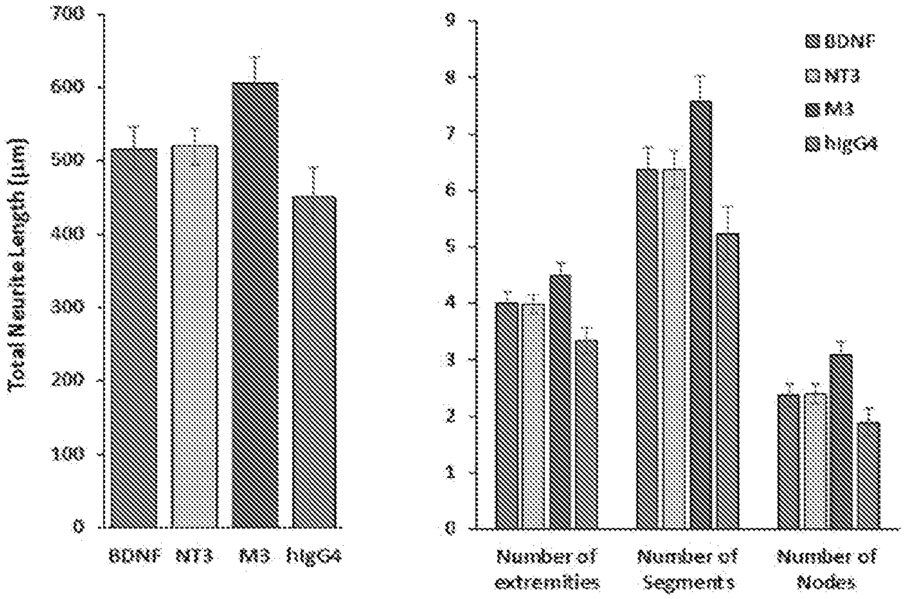
Total Neurite Length (μm)
BDNF
NT3
M3
hIgG4
Number of extremities
Number of Segments
Number of Nodes

FIG. 8A

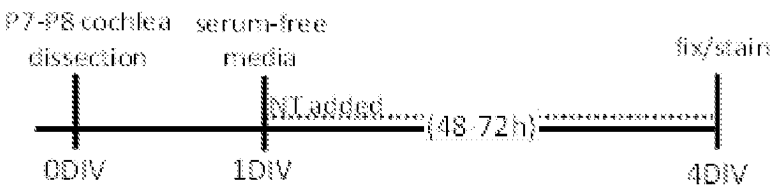
P7-P8 cochlea
dissection
serum-free
media
fix/stain
NT added
(48-72h)
0DIV
1DIV
4DIV

FIG. 8B

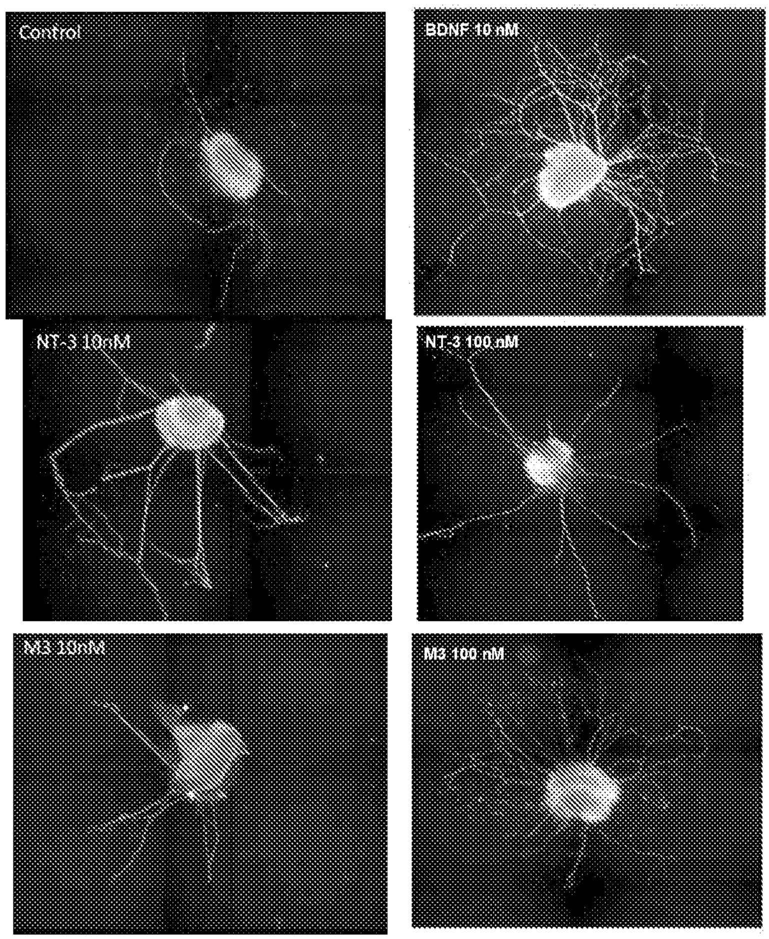
Control
BDNF 10 nM
NT-3 10nM
NT-3 100 nM
M3 10nM
M3 100 nM

FIG. 8C

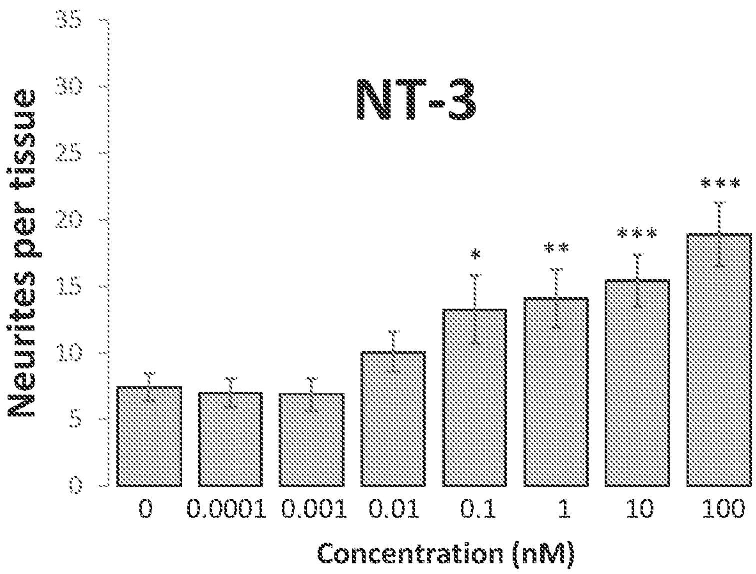
NT-3
Neurites per tissue
35
30
25
20
15
10
5
0
*
**
***
***
0
0.0001
0.001
0.01
0.1
1
10
100
Concentration (nM)

FIG. 8D

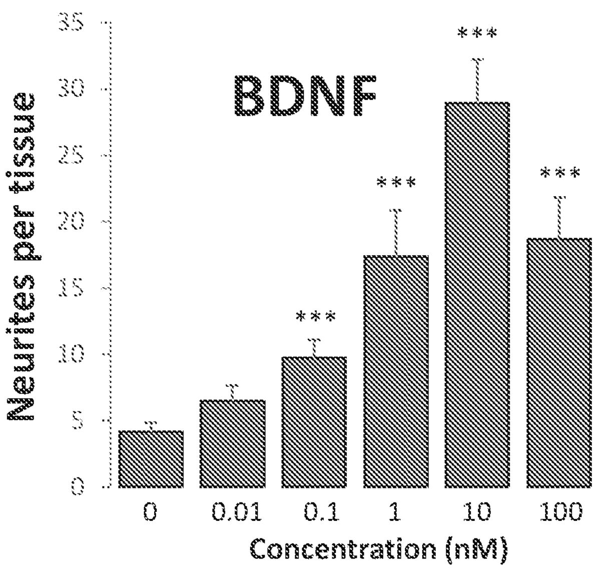
BDNF
Neurites per tissue
35
30
25
20
15
10
5
0
***
***
***
***
0
0.01
0.1
1
10
100
Concentration (nM)

FIG. 8E

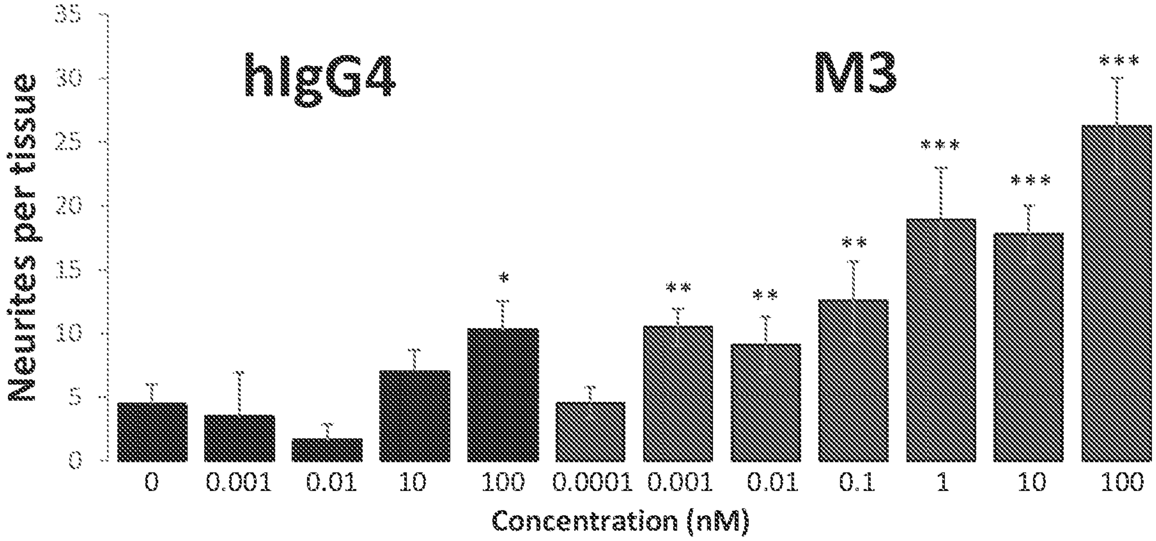
hIgG4
M3
Neurites per tissue
35
30
25
20
15
10
5
0
*
**
**
**
***
***
***
0
0.001
0.01
10
100
0.0001
0.001
0.01
0.1
1
10
100
Concentration (nM)

FIG. 8F

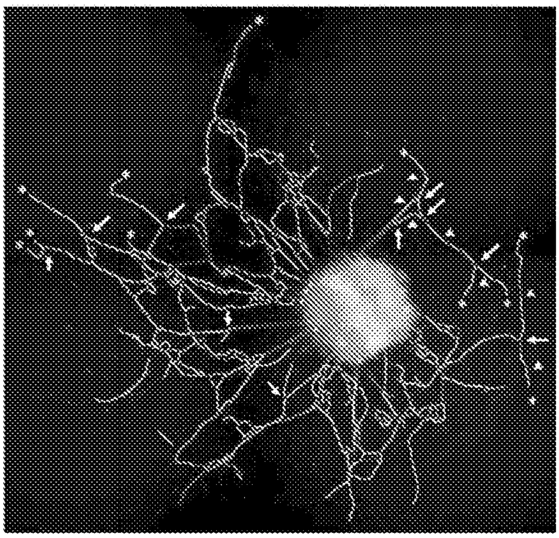

FIG. 8G

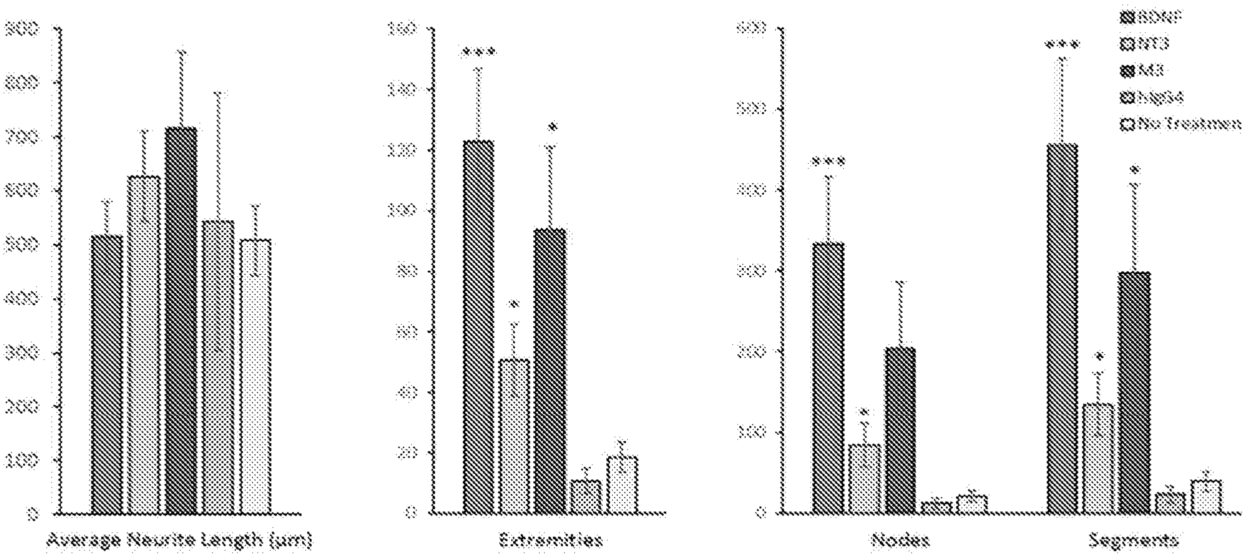
BDNF
NT3
M3
hIgG4
No Treatmen
***
*
Average Neurite Length (um)
Extremities
Nodes
Segments
0
100
200
300
400
500
600
700
800
900
20
40
60
80
100
120
140
160

FIG. 9A

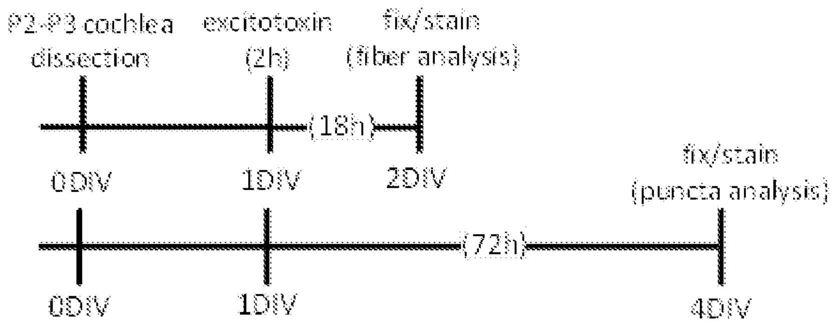
P2-P3 cochlea dissection
excitotoxin (2h)
fix/stain (fiber analysis)
(18h)
0DIV
1DIV
2DIV
fix/stain (puncta analysis)
(72h)
0DIV
1DIV
4DIV

FIG. 9B

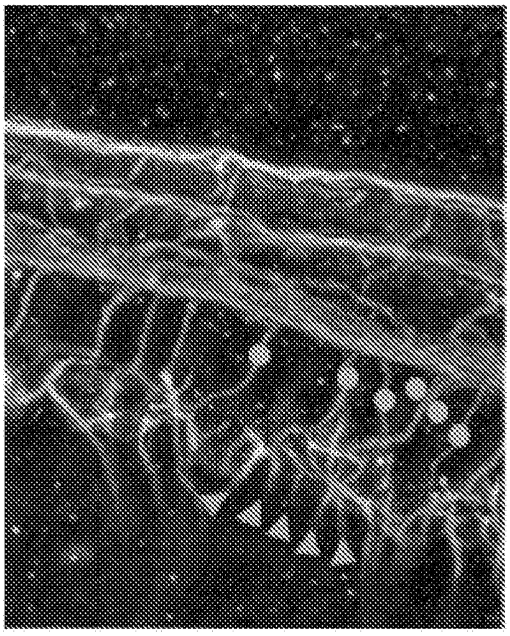

FIG. 9C

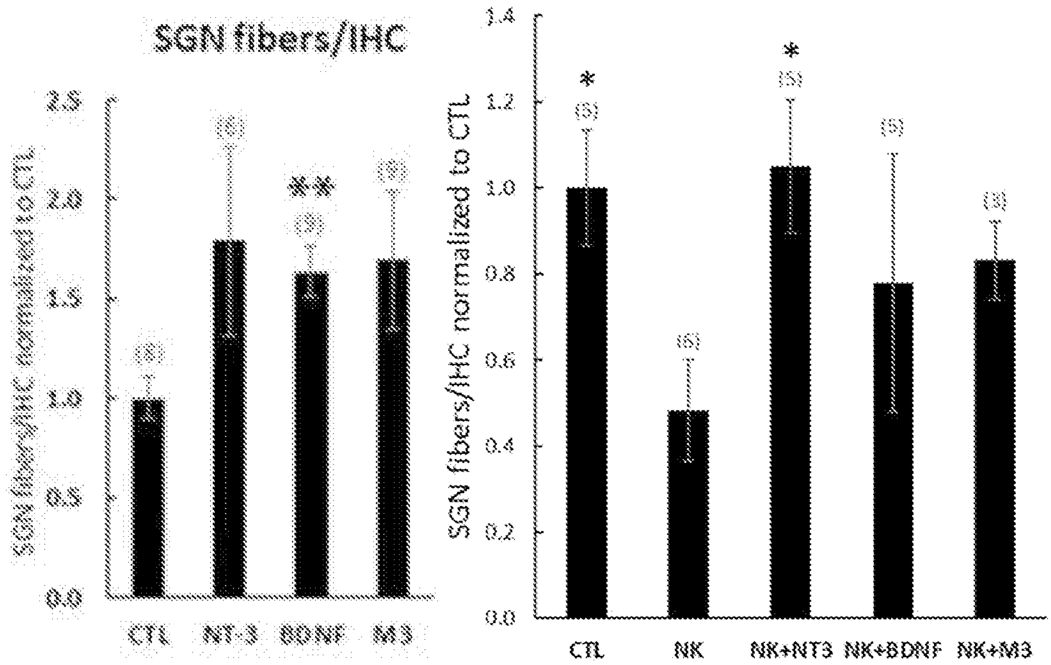
SGN fibers/IHC
SGN fibers/IHC normalized to CTL
2.5
2.0
1.5
1.0
0.5
0.0
(8)
(6)
**
(9)
(8)
CTL
NT-3
BDNF
M3
SGN fibers/IHC normalized to CTL
1.4
1.2
1.0
0.8
0.6
0.4
0.2
0.0
*
(5)
(6)
*
(5)
(5)
(3)
CTL
NK
NK+NT3
NK+BDNF
NK+M3

FIG. 9D

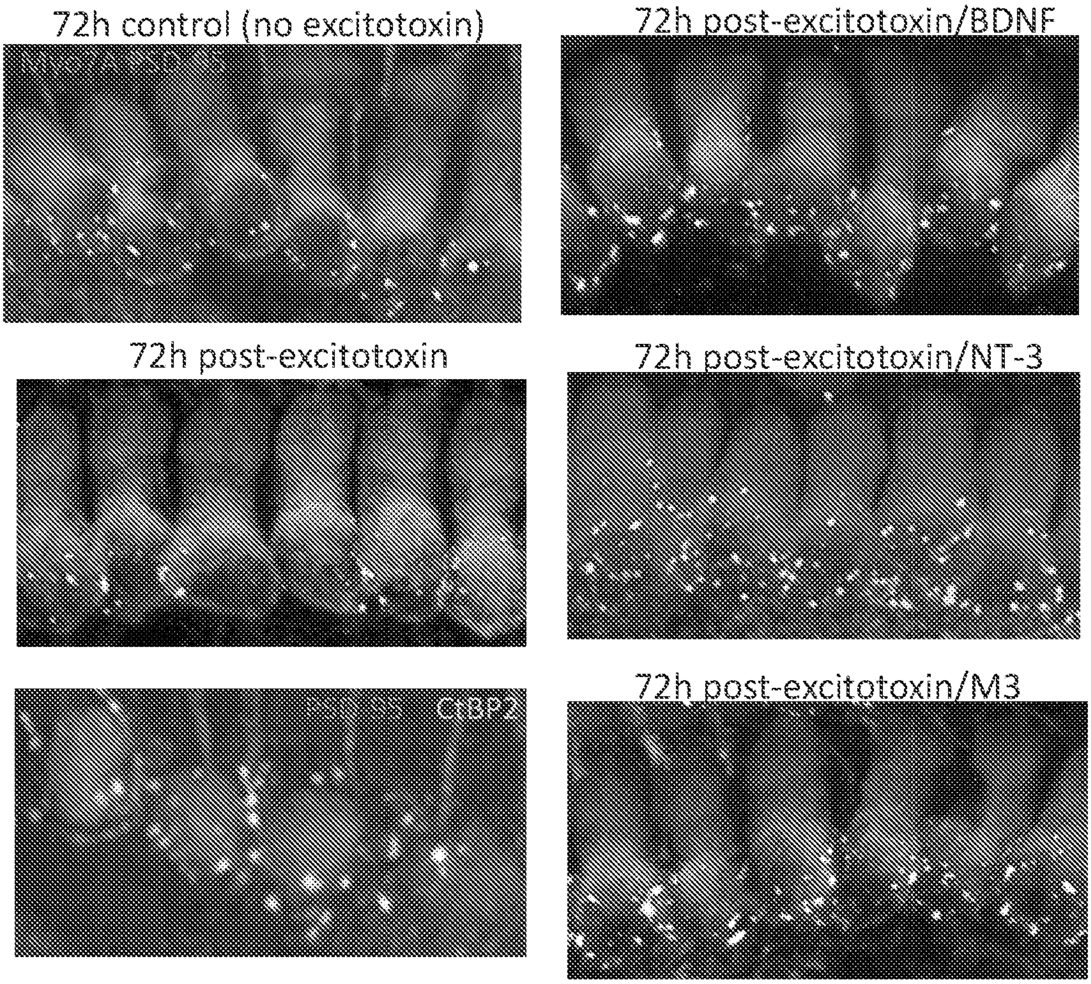
72h control (no excitotoxin)
72h post-excitotoxin/BDNF
72h post-excitotoxin
72h post-excitotoxin/NT-3
72h post-excitotoxin/M3

FIG. 9E

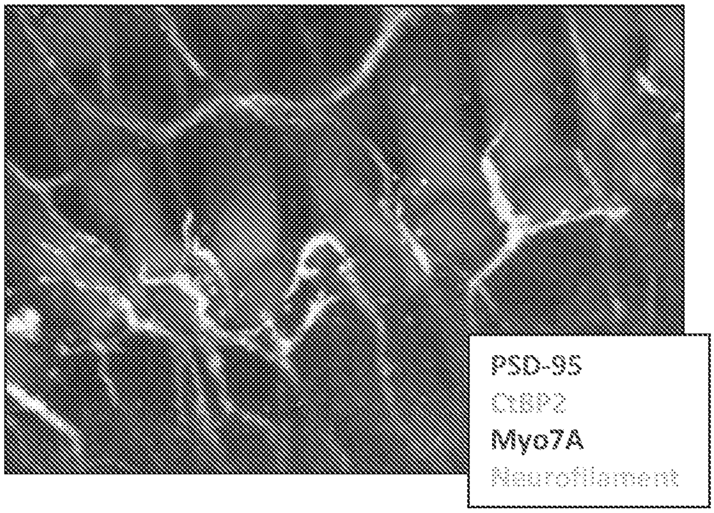
PSD-95
CtBP2
Myo7A
Neurofilament

FIG. 9F

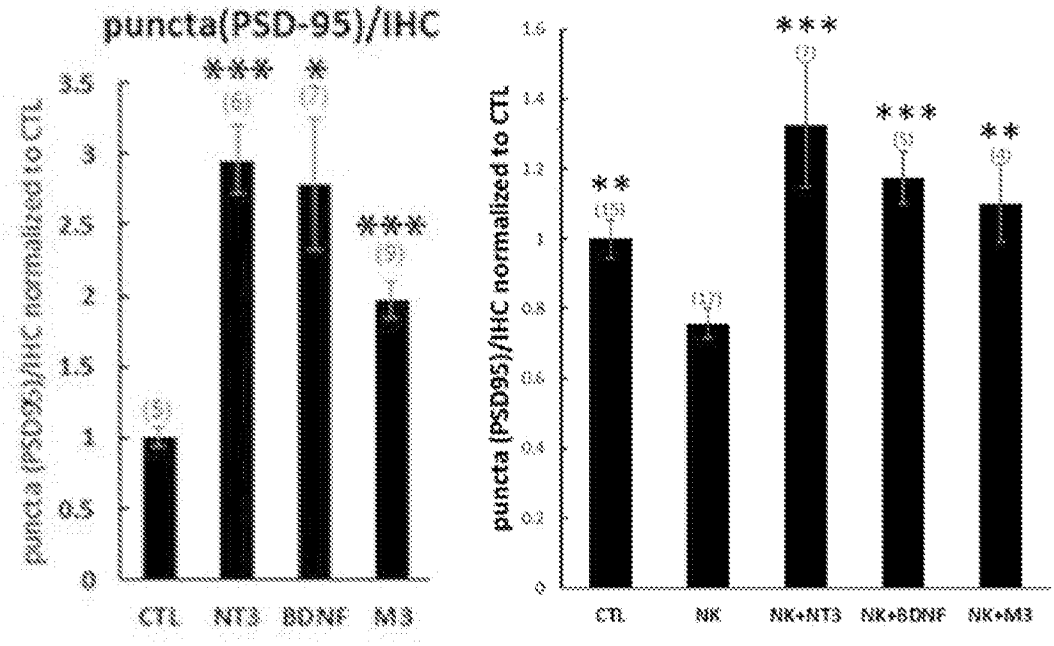
puncta(PSD-95)/IHC
puncta (PSD95)/IHC normalized to CTL
CTL
NT3
BDNF
M3
CTL
NK
NK+NT3
NK+BDNF
NK+M3

FIG. 10

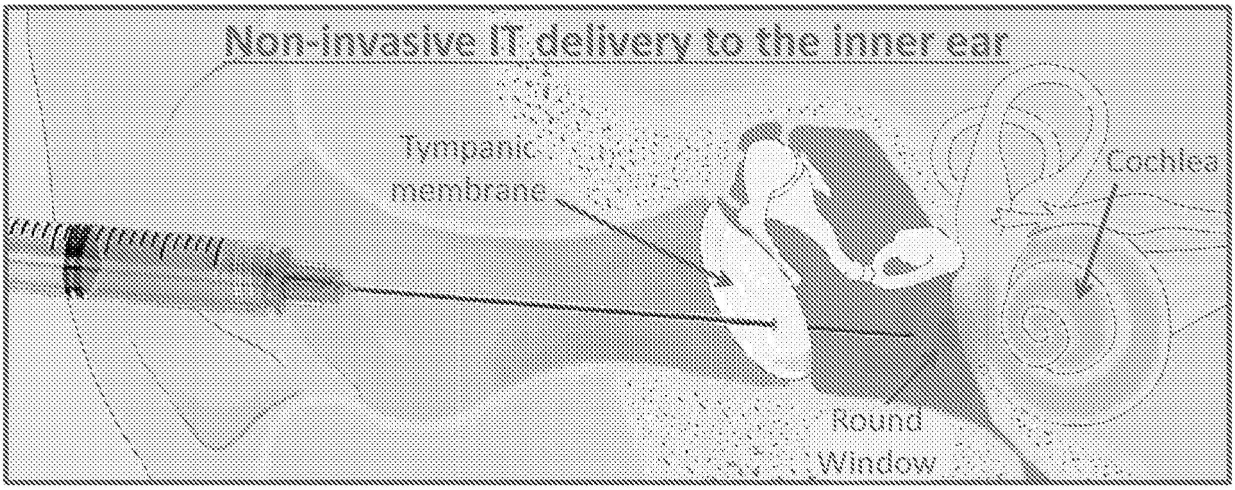
Non-invasive IT delivery to the inner ear
Tympanic membrane
Cochlea
Round Window

FIG. 11

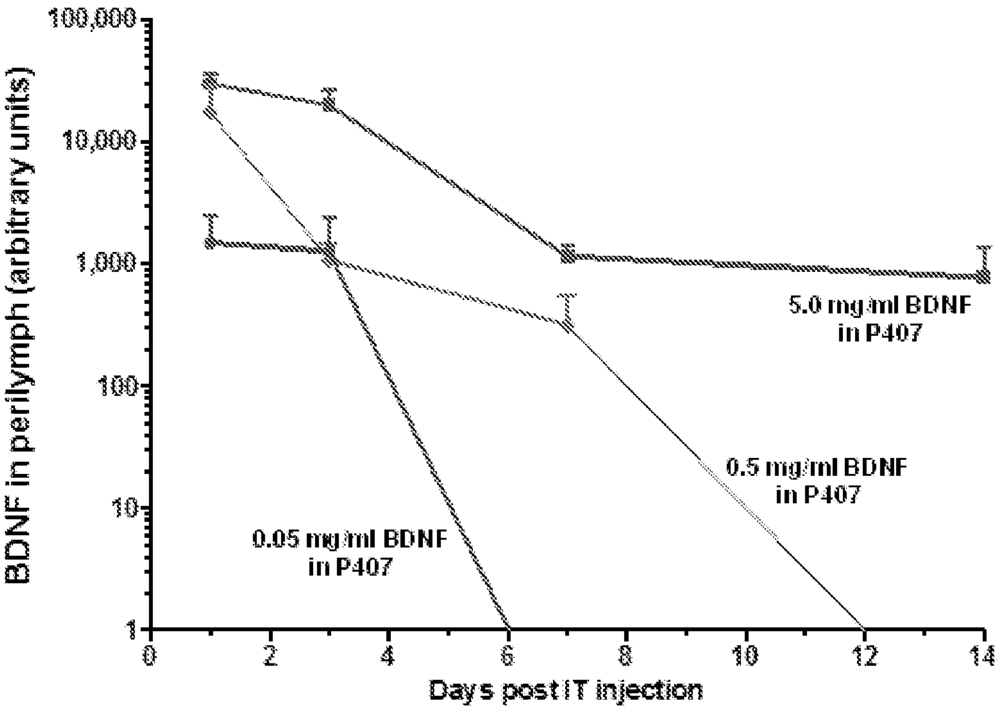
BDNF in perilymph (arbitrary units)
100,000
10,000
1,000
100
10
1
0
2
4
6
8
10
12
14
Days post IT injection
5.0 mg/ml BDNF in P407
0.5 mg/ml BDNF in P407
0.05 mg/ml BDNF in P407

FIG. 12A

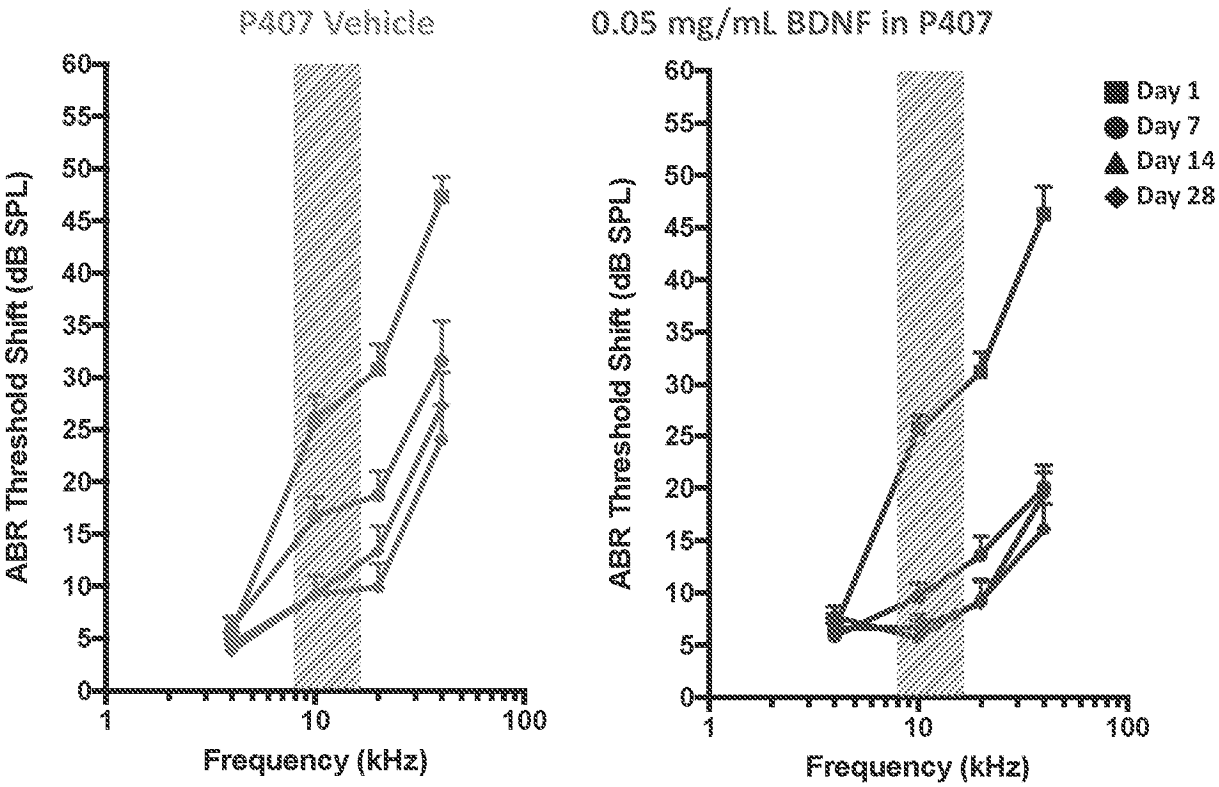


FIG. 12B

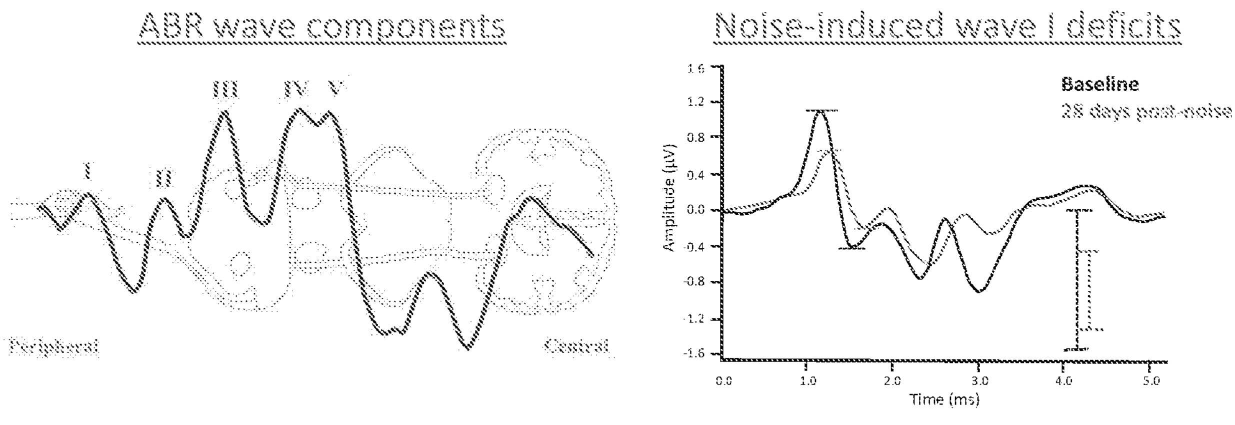

FIG. 12C

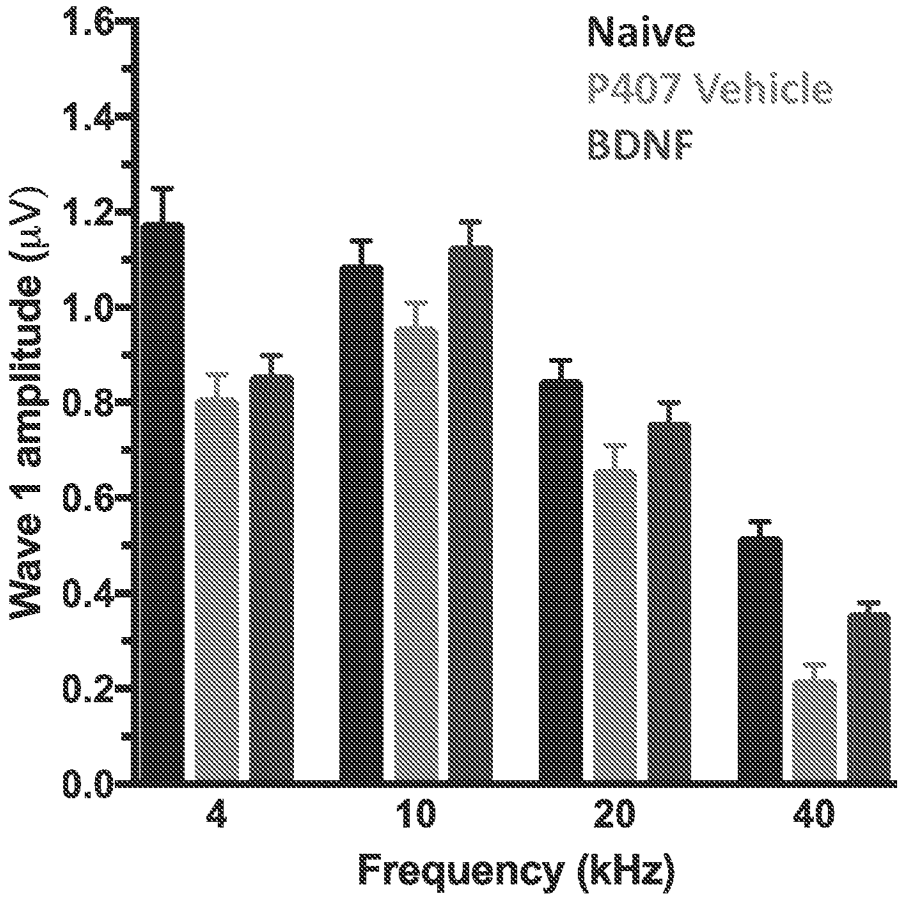
Naive
P407 Vehicle
BDNF
Wave 1 amplitude (μV)
1.6
1.4
1.2
1.0
0.8
0.6
0.4
0.2
0.0
4
10
20
40
Frequency (kHz)

FIG. 13A

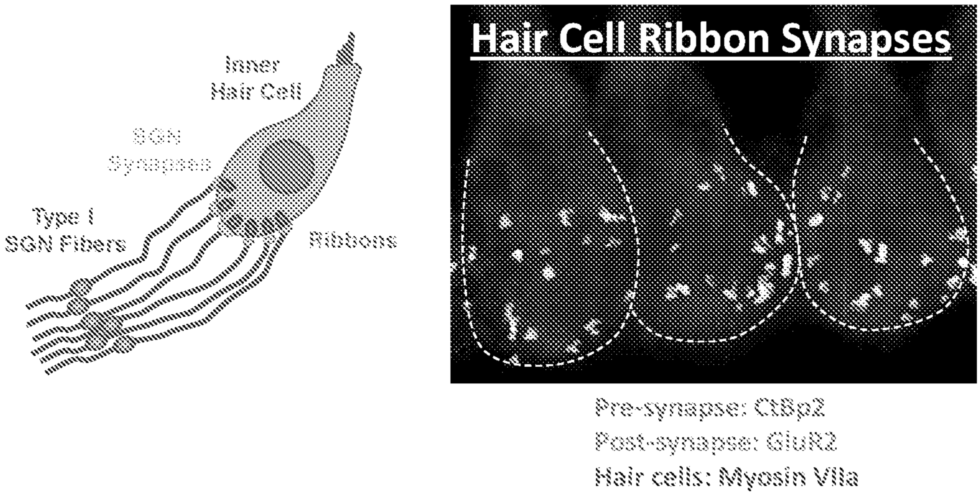


FIG. 13B

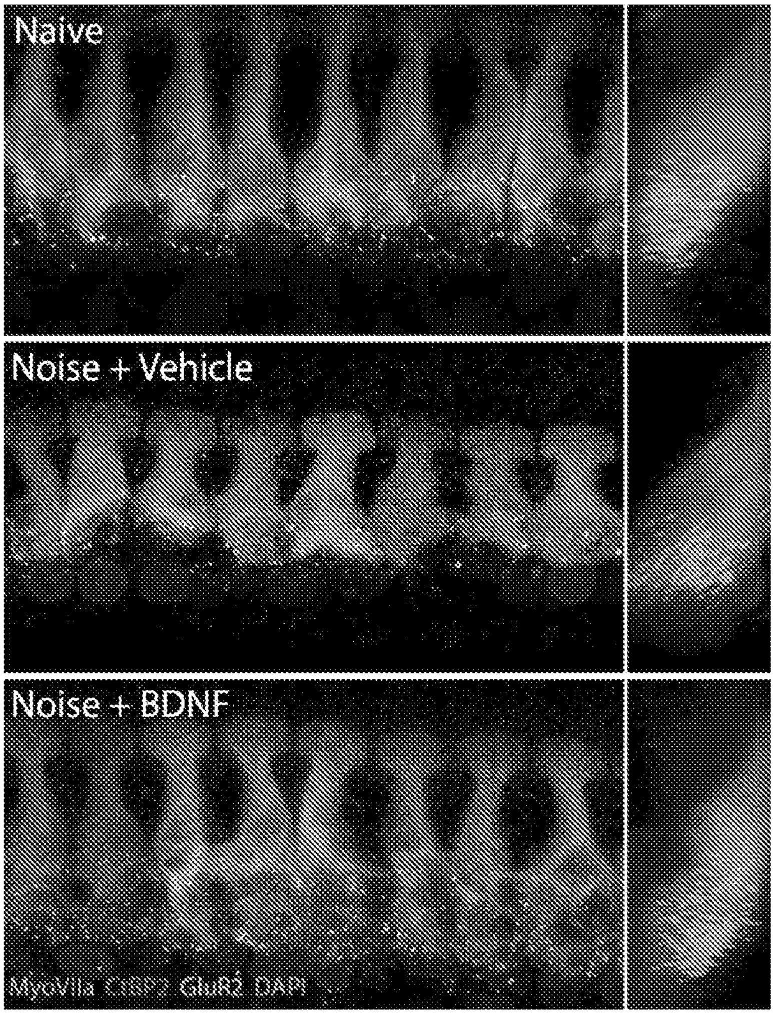

FIG. 13C

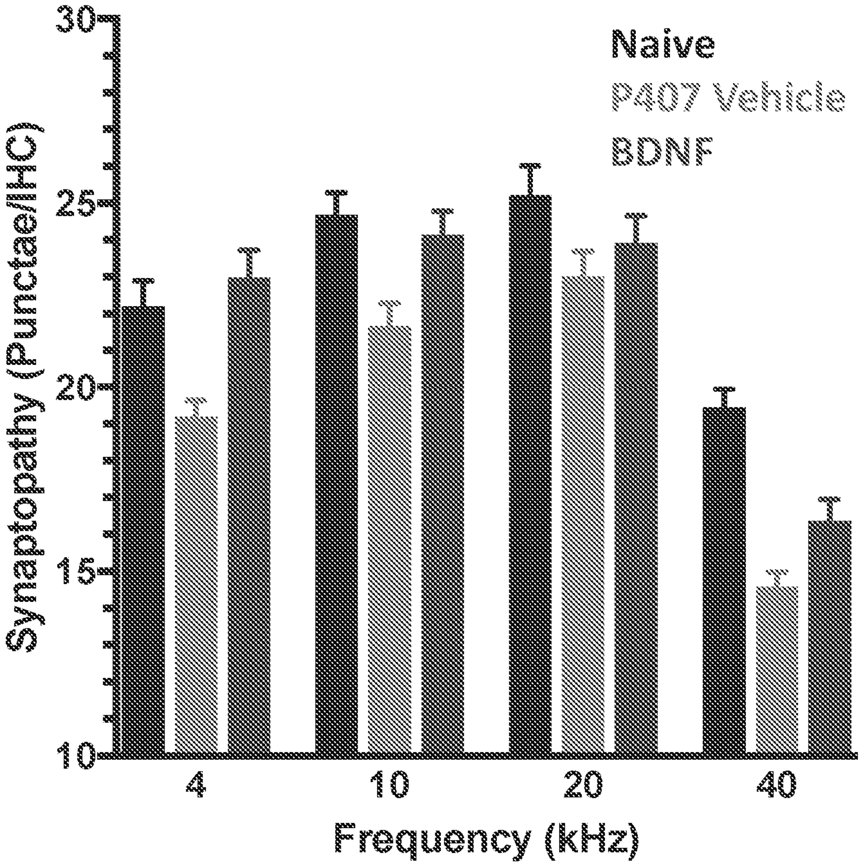


FIG. 13D

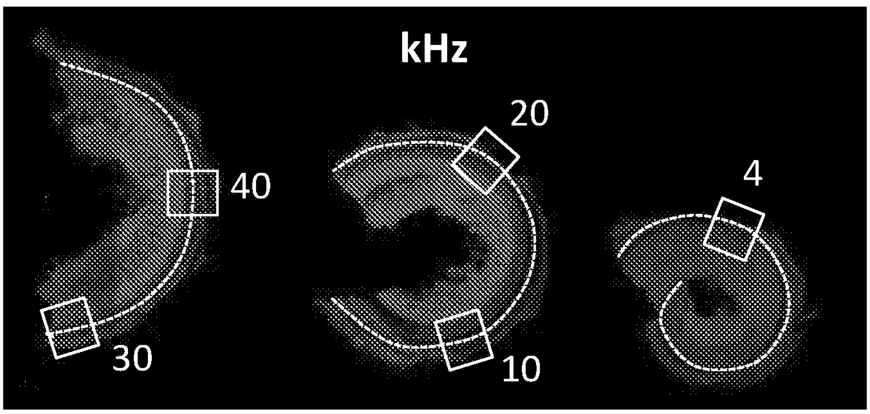

FIG. 14

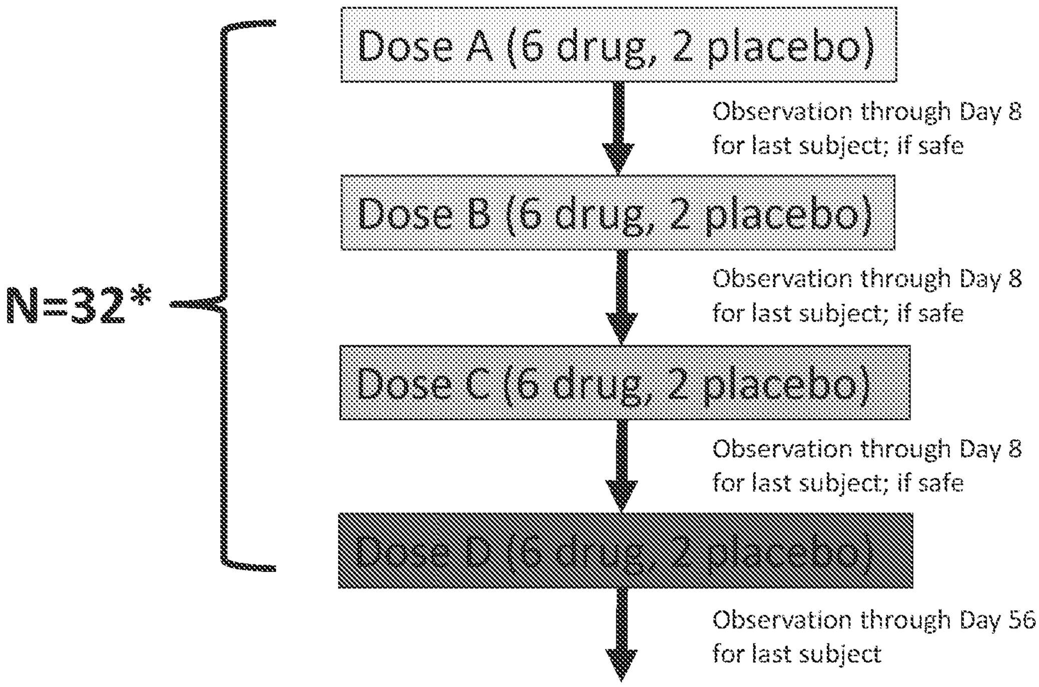


*Approximately 32 subjects will be enrolled into this single dose study of drug. The number of subjects may be higher if some subjects are replaced and/or if some cohort sizes are expanded to obtain further experience with some dose levels.

FIG. 15

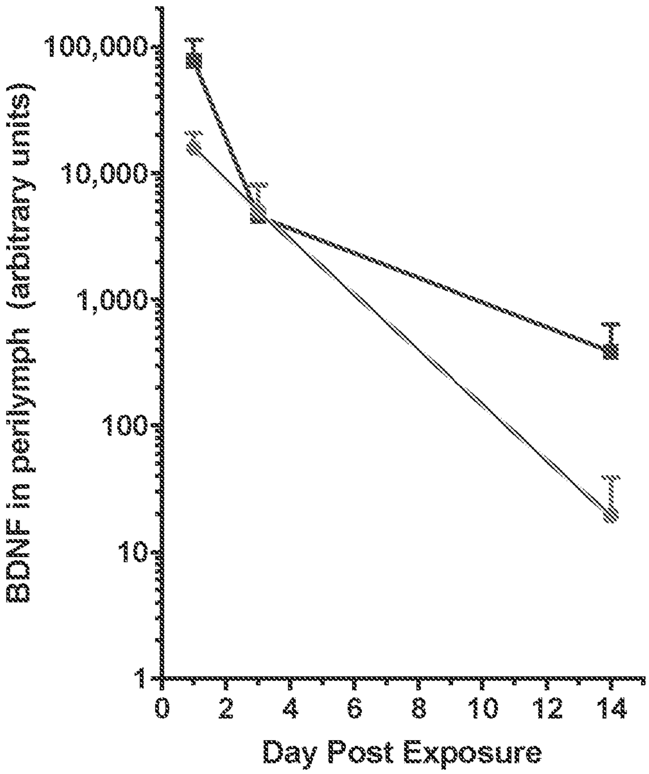
100,000
10,000
1,000
100
10
1
BDNF in perilymph (arbitrary units)
0
2
4
6
8
10
12
14
Day Post Exposure

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2016243028 A1
- JP 2005220070 A
- WO 2015031393 A1
- WO 2018140792 A
- US 6139870 A
- US 6004573 A
- US 6117949 A
- US 6201072 B
- US 6287588 B
- US 906041 A
- US 09559799 B
- US 10919603 B
- US 5324519 A
- US 4938763 A
- US 5702716 A
- US 5744153 A
- US 5990194 A
- EP 0551626 A
- US 6638521 B
- US 6562363 B
- US 6509028 B
- US 6348502 B
- US 6319513 B
- US 6306789 B
- US 5814330 A
- US 4900552 A
- US 7151191 B
- US 6221367 B
- US 5714167 A
- US 4530840 A
- US 4675189 A
- US 4758435 A
- US 3773919 A
- US 4474572 A
- US 6458387 B
- US 6268053 B
- US 6090925 A
- US 5981719 A
- US 5578709 A
- US 6083534 A
- US 6036978 A
- US 3737337 A
- US 4389330 A
- US 5945126 A
- US 6270802 B
- US 63361798 B
- US 338805 A
- US 5421818 A
- US 5474529 A
- US 5476446 A
- US 874208 A
- US 20070167918
- US 5323907 A
- US 5052558 A
- US 5033252 A

### Non-patent literature cited in the description

- **WAN et al.** Neurotrophin-3 regulates ribbon synapse density in the cochlea and induces synapse regeneration after acoustic trauma. *eLife*, 20 October 2014, vol. 3, 03564
- **ZUCCOTTI et al.** Lack of Brain-Derived Neurotrophic Factor Hampers Inner Hair Cell Synapse Physiology, But Protects against Noise-Induced Hearing Loss. *The Journal of Neuroscience*, 2012, vol. 32 (25), 8545-8553
- **BAFTI, S**. *Regeneration And Protection Of Synapses After Noise-Induced Cochlear Synaptopathy*, 2019
- **WISE K.** ; **GILLESPIE L.** Drug Delivery to the Inner Ear. *J Neural Eng.*, 2012, vol. 9 (6), 065002
- **SANGHI et al.** *Eur. Heart J.*, 2005, vol. 26, 538-543
- **PALM et al.** *Nephrol. Dial Transplant*, 1999, vol. 14, 2559-2562
- **HARRIS** ; **KEITHLEY**. Autoimmune inner ear disease. *Otorhinolaryngology Head and Neck Surgery*, 2002, vol. 91, 18-32
- **GLODDEK**. *Acta Otolaryngol.*, 1989, vol. 108, 68-75
- **SATOH, J.** *Assoc. Res. Otolaryngol.*, 2003, vol. 4, 139-147
- **CARFRAE et al.** *Laryngoscope*, March 2008, vol. 118, 501-505
- The Science and Practice of Pharmacy. Lippincott, Williams & Wilkins
- Microbiological Evaluation of Filters for Sterilizing Liquids. Health Industry Manufacturers Association, 1981, vol. 4

- **RICHARD et al.** *International Journal of Pharmaceutics*, 2006, vol. 312 (1-2), 144-50
- **TAKTAK et al.** *J. Pharm. Pharmacol.*, 1990, vol. 43, 578-82
- **VIEGAS**. *Int. J. Pharm.*, 1998, vol. 160, 157-162
- **JEONG et al.** *Nature*, 1997, vol. 388, 860-2
- **JEONG et al.** *J. Control. Release*, 2000, vol. 63, 155-63
- **JEONG et al.** *Adv. Drug Delivery Rev.*, 2002, vol. 54, 37-51
- **MAJITHIYA et al.** *AAPS PharmSciTech*, 2006, vol. 7 (3), 1
- **LUZZI, L. A.** *J. Pharm. Psy.*, 1970, vol. 59, 1367
- Controlled Release of Bioactive Agents from Lactides/Glycolide Polymers. **LEWIS, D. H.** Biodegradable Polymers as Drug Delivery Systems. Marcel Decker, 1990
- Poly(lactic acid) and Poly(lactic acid-co-glycolic acid) Contraceptive Delivery Systems. **BECK et al.** Long Acting Steroid Contraception. Raven Press, 1983
- **G. JOHNS et al.** Broad Applicability of a Continuous Formation Process. *Drug Delivery Technology*, January 2004, vol. 4
- **PARK et al.** *Pharm. Res.*, 1987, vol. 4 (6), 457-464
- **NAHA et al.** *Journal of Microencapsulation*, 04 February 2008
- **ZOU et al.** *J. Biomed. Materials Res.*, 24 April 2008
- Handbook of Pharmaceutical Excipients. The Pharmaceutical Press, 1986
- **HANSEN et al.** *J Phys Chem*, 1988, vol. 92, 2189-96